# EUROPEAN PATENT SPECIFICATION

(11) **EP 2 079 690 B1**
(45) Date of publication and mention of the grant of the patent: **15.09.2010**
(21) Application number: 07826577.4
(22) Date of filing: 28.09.2007
(51) Int. Cl.: C07D 209/52, C07D 405/14, C07D 409/12, C07D 413/14, C07D 417/12, C07D 417/14, C07D 471/04, C07D 487/04, C07D 495/04, C07D 513/04, A61K 31/403, A61P 25/00

(54) **3-AZA-BICYCLO[3.1.0]HEXANE DERIVATIVES**
3-AZA-BICYCLO[3.1.0]HEXANDERIVATE
DERIVES DU 3-AZA-BICYCLO[3.1.0]HEXANE

(30) Priority: 29.09.2006 WO PCT/IB2006/053570
(43) Date of publication of application: 22.07.2009
(73) Proprietor: Actelion Pharmaceuticals Ltd., 4123 Allschwil (CH)
(72) Inventor: AISSAOUI, Hamed, 68840 Pulversheim (FR); BOSS, Christoph, 4123 Allschwil (CH); GUDE, Markus, 4123 Allschwil (CH); KOBERSTEIN, Ralf, 79539 Lörrach (DE); LEHMANN, David, 4058 Basel (CH); SIFFERLEN, Thierry, 68116 Guewenheim (FR); TRACHSEL, Daniel, 4416 Bubendorf (CH)
(74) Representative: Schager, Frank
(86) International application number: PCT/IB2007/053947
(87) International publication number: WO 2008/038251

(56) References cited:
- WO-A-01/96302
- WO-A-02/44172
- WO-A-03/041711
- WO-A-03/051368
- WO-A-2004/026866
- WO-A-2004/041791
- WO-A-2004/041807

## Description

The present invention relates to novel 3-aza-bicyclo[3.1.0]hexane derivatives of formula (I) and their use as pharmaceuticals. The invention also concerns related aspects including processes for the preparation of the compounds, pharmaceutical compositions containing one or more compounds of formula (1), and especially their use as orexin receptor antagonists.
Orexins (orexin A or OX-A and orexin B or OX-B) are novel neuropeptides found in 1998 by two research groups, orexin A is a 33 amino acid peptide and orexin B is a 28 amino acid peptide (Sakurai T. et al., Cell, 1998, 92, 573-585). Orexins are produced in discrete neurons of the lateral hypothalamus and bind to G-protein-coupled receptors (OX₁ and OX₂ receptors). The orexin-1 receptor (OX₁) is selective for OX-A, and the orexin-2 receptor (OX₂) is capable to bind OX-A as well as OX-B. Orexins are found to stimulate food consumption in rats suggesting a physiological role for these peptides as mediators in the central feedback mechanism that regulates feeding behaviour (Sakurai T. et al., Cell, 1998, 92, 573-585). On the other hand, it was also observed that orexins regulate states of sleep and wakefulness opening potentially novel therapeutic approaches to narcolepsy as well as insomnia and other sleep disorders (Chemelli R.M. et al., Cell, 1999, 98, 437-451).
Orexin receptors are found in the mammalian brain and may have numerous implications in pathologies as known from the literature.
The present invention provides 3-aza-bicyclo[3.1.0]hexane derivatives, which are non-peptide antagonists of human orexin receptors. These compounds are in particular of potential use in the treatment of e.g. eating disorders, drinking disorders, sleep disorders, or cognitive dysfunctions in psychiatric and neurologic disorders.
Up to now, several low molecular weight compounds are known having a potential to antagonise either specifically OX₁ or OX₂, or both receptors at the same time. Piperidine derivatives useful as orexin receptor antagonists are disclosed in WO2001/96302.
The present invention describes for the first time 3-aza-bicyclo[3.1.0]hexane derivatives as orexin receptor antagonists.
i) A first aspect of the invention consists of a compound of the formula (I) 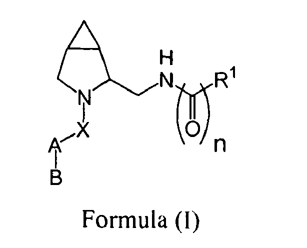 wherein
   X represents C(O) or SO₂;
   A represents aryl or heterocyclyl, wherein the aryl or heterocyclyl is unsubstituted or independently mono- or di-substituted, wherein the substituents are independently selected from the group consisting of (C₁₋₄)alkyl, (C₃₋₆)cycloalkyl, (C₂₋₆)alkynyl, hydroxy-(C₁₋₄)alkyl, hydroxy-(C₂₋₆)alkynyl, trimethylsilyl-ethynyl, (C₃₋₆)cycloalkyl-ethynyl, (C₁₋₄)alkoxy, trifluoromethyl, trifluoromethoxy, NR²K³, N(R²)C(O)R³, C(O)NR²R³, and halogen;
   B represents a hydrogen atom or an aryl- or heterocyclyl-group, wherein the aryl or heterocyclyl is unsubstituted or independently mono-, di-, or trisubstituted, wherein the substituents are independently selected from the group consisting of (C₁₋₄)alkyl, (C₃₋₆)cycloalkyl, (C₁₋₄)alkoxy, methoxy-(C₁₋₄)alkoxy, cyano, trifluoromethyl, trifluoromethoxy, NR²R³, N(R²)C(O)R³, C(O)NR²R³, and halogen; or B represents a 2,3-dihydro-benzo[1,4]dioxinyl group;
   or A and B together represent a tricyclic group;
   n represents 0 or 1;
   R¹ represents aryl or heterocyclyl, wherein the aryl or heterocyclyl is unsubstituted or independently mono-, di-, or trisubstituted wherein the substituents are independently selected from the group consisting of (C₁₋₄)alkyl, (C₂₋₆)alkynyl, (C₃₋₆)cycloalkyl, (C₁₋₄)alkoxy, (C₁₋₄)alkylthio, halogen, hydroxy, cyano, trifluoromethyl, trifluoromethoxy, NR²R³, N(R²)C(O)R³, COOR², and C(O)NR²R³; or R¹ represents a heterocyclyl-ethenyl-, a heterocyclyl-(C₁₋₄)alkyl or an aryloxy-(C₁₋₄)alkyl-group, which groups are unsubstituted or independently mono- or di-substituted at the aryl- or heterocyclyl-part wherein the substituents are independently selected from the group consisting of (C₁₋₄)alkyl, (C₁₋₄)alkoxy, halogen, trifluoromethyl, trifluoromethoxy, and NR²R³; or R¹ represents a 2,3-dihydro-benzofuranyl-, a benzo[1,3]dioxolyl-, a 2,3-dihydro-benzo[1,4]dioxinyl-, a 4-oxo-4*H*-chromenyl-, a 2*H*-chromenyl, a chromanyl-, a 4*H*-benzo[1,3]dioxinyl-, a 2,3-dihydro-thieno [3,4-b][1,4]dioxinyl-, a morpholin-4-yl-phenyl-, a piperazin-1-yl-phenyl-, a 3,4-dihydro-2*H*-benzo[1,4]oxazinyl-, a 3-oxo-3,4-dihydro-2*H*-benzo[1,4]oxazinyl- or a 2,3,6,7-tetrahydro-benzo[1,2-b;4,5-b']difuranyl-group, wherein said groups are unsubstituted or independently mono- or di-substituted wherein the substituents are independently selected from the group consisting of (C₁₋₄)alkyl, (C₁₋₄)alkoxy, and halogen;
   R² represents hydrogen or (C₁₋₄)alkyl; and
   R³ represents hydrogen or (C₁₋₄)alkyl.

The compounds of formula (I) may contain one or more stereogenic or asymmetric centers, such as one or more asymmetric carbon atoms. Substituents at a double bond or a ring may be present in cis- (= Z-) or trans (= E-) form unless indicated otherwise. The compounds of formula (I) may thus be present as mixtures of stereoisomers or preferably as pure stereoisomers. Mixtures of stereoisomers may be separated in a manner known to a person skilled in the art.

Any reference to a compound of formula (I) is to be understood as referring also to salts (especially pharmaceutically acceptable salts) of a compound of formula (I), respectively, as appropriate and expedient.

The term "pharmaceutically acceptable salts" refers to non-toxic, inorganic or organic acid and/or base addition salts. Reference can be made to "Salt selection for basic drugs", Int. J. Pharm. (1986), 33, 201-217.

The term "halogen" means fluorine, chlorine, bromine, or iodine, preferably fluorine or chlorine.
The term "(C₁₋₄)alkyl", alone or in combination, means a straight-chain or branched-chain alkyl group with 1 to 4 carbon atoms. Examples of (C₁₋₄)alkyl groups are methyl, ethyl, propyl, isopropyl, n-butyl, isobutyl, sec-butyl or tert.-butyl. Preferred are methyl and ethyl.
The term "(C₃₋₆)cycloalkyl", alone or in combination, means a cycloalkyl group with 3 to 6 carbon atoms. Examples of (C₃₋₆)cycloalkyl groups are cyclopropyl, cyclobutyl, cyclopentyl or cyclohexyl. Preferred is cyclopropyl.
The term "(C₁₋₄)alkoxy", alone or in combination, means a group of the formula (C₁₋₄)alkyl-O- in which the term "(C₁₋₄)alkyl" has the previously given significance, such as methoxy, ethoxy, n-propoxy, isopropoxy, n-butoxy, isobutoxy, sec.-butoxy or tert.-butoxy. Preferred are methoxy and ethoxy.
The term "aryl", alone or in combination, means a phenyl or a naphthyl group. Preferred is a phenyl group. The aryl group may be unsubstituted or independently mono-, di-, or trisubstituted wherein the substituents are independently selected from the group consisting of (C₁₋₄)alkyl, (C₃₋₆)cycloalkyl, (C₂₋₆)alkynyl, hydroxy-(C₁₋₄)alkyl, hydroxy-(C₂₋₆)alkynyl, trimethylsilyl-ethynyl, (C₃₋₆)cycloalkyl-ethynyl, (C₁₋₄)alkoxy, trifluoromethyl, trifluoromethoxy, methoxy-(C₁₋₄)alkoxy, cyano, (C₁₋₄)alkylthio, hydroxy, COOR², NR²R³, N(R²)C(O)R³, C(O)NR²R³, and halogen.
In case "A" represents "aryl" the term preferably means the above-mentioned group which is unsubstituted or independently mono- or di-substituted, wherein the substituents are independently selected from the group consisting of (C₁₋₄)alkyl, (C₃₋₆)cycloalkyl, (C₂₋₆)alkynyl, hydroxy-(C₁₋₄)alkyl, hydroxy-(C₂₋₆)alkynyl, trimethylsilyl-ethynyl, (C₃₋₆)cycloalkyl-ethynyl, (C₁₋₄)alkoxy, trifluoromethyl, trifluoromethoxy, NR²R³, N(R²)C(O)R³, C(O)NR²R³, and halogen. Especially, the substituents are independently selected from the group consisting of (C₁₋₄)alkyl, (C₃₋₆)cycloalkyl, (C₁₋₄)alkoxy, trifluoromethyl, trifluoromethoxy, NR²R³, N(R²)C(O)R³, C(O)NR²R³, and halogen. Especially preferred examples wherein "A" represents "aryl" are unsubstituted or mono-substituted phenyl, wherein the substituent is (C₁₋₄)alkyl. In another embodiment, especially preferred examples wherein "A" represents "aryl" are mono-, or di-substituted phenyl, wherein one substituent is selected from the group consisting of (C₂₋₆)alkynyl, and (C₃₋₆)cycloalkyl-ethynyl; and the other substituent (if present) is selected from the group consisting of (C₁₋₄)alkyl, (C₁₋₄)alkoxy, and trifluoromethoxy (especially (C₁₋₄)alkyl). In addition to the above-mentioned substituents, the substituent "A" is also substituted by the substituent "B", whereby, in case B represents aryl or heterocyclyl, B is preferably attached in ortho position to the point of attachment of the group X. Examples wherein "A" represents "aryl" are phenyl, naphthyl, 2-methylphenyl, 3-methylphenyl, 4-methylphenyl, 3-trifluoromethyl-phenyl, 2-trifluoromethoxyphenyl, 2-(cyclopropyl-ethynyl)-4-methylphenyl, 2-(ethyl-ethynyl)-4-methylphenyl, and 2-(isobutyl-ethynyl)-4-methylphenyl.
In case "B" represents "aryl" the term preferably means the above-mentioned group which is unsubstituted or independently mono-, di-, or trisubstituted, wherein the substituents are independently selected from the group consisting of (C₁₋₄)alkyl, (C₃₋₆)cycloalkyl, (C₁₋₄)alkoxy, methoxy-(C₁₋₄)alkoxy, cyano, trifluoromethyl, trifluoromethoxy, NR²R³, N(R²)C(O)R³, C(O)NR²R³, and halogen. Especially, the substituents are independently selected from the group consisting of (C₁₋₄)alkyl, (C₃₋₆)cycloalkyl, (C₁₋₄)alkoxy, trifluoromethyl, trifluoromethoxy, NR²R³, N(R²)C(O)R³, C(O)NR²R³, and halogen. Preferred examples wherein "B" represents "aryl" are unsubstituted or independently mono-, di-, or trisubstituted phenyl (preferred mono-substituted phenyl), wherein the substituents are independently selected from the group consisting of (C₁₋₄)alkyl, (C₁₋₄)alkoxy, trifluoromethyl, and halogen. In addition to the above-mentioned substituents, the substituent "B" is attached to the substituent "A". Examples wherein "B" represents "aryl" are phenyl, 2-methylphenyl, 3-methylphenyl, 4-methylphenyl, 3,4-dimethylphenyl, 2,3-dimethylphenyl, 4-ethylphenyl, 3-isopropylphenyl, 2-methoxyphenyl, 3-methoxyphenyl, 4-methoxyphenyl, 2-fluorophenyl, 3-fluorophenyl, 4-fluorophenyl, 2-chlorophenyl, 3-chlorophenyl, 4-chlorophenyl, 2-trifluoromethylphenyl, 3-trifluoromethylphenyl, 4-trifluoromethylphenyl, 3-trifluoromethoxyphenyl, 4-fluoro-3-methylphenyl, 3-cyanophenyl, and 3-(2-methoxyethoxy)-phenyl.
In case "A" and "B" both represents "aryl" the combination "A-B" preferably means a biphenyl group which is unsubstituted or independently mono- or di-substituted for "A" and unsubstituted or mono-, di- or trisubstituted for "B", wherein the substituents are independently selected from the group consisting of (C₁₋₄)alkyl, (C₃₋₆)cycloalkyl, (C₁₋₄)alkoxy, trifluoromethyl, trifluoromethoxy, NR²R³, N(R²)C(O)R³, C(O)NR²R³, and halogen. Preferred examples wherein "A" and "B" both represents "aryl" are biphenyl groups which are unsubstituted or independently mono- or di-substituted for "A" and unsubstituted or mono-, di- or trisubstituted for "B", wherein the substituents are independently selected from the group consisting of (C₁₋₄)alkyl, (C₁₋₄)alkoxy, trifluoromethyl, and halogen. Especially preferred examples wherein "A" and "B" both represents "aryl" are biphenyl groups which are unsubstituted or mono-substituted with methyl for "A" and unsubstituted or mono-, di- or trisubstituted for "B", wherein the substituents are independently selected from the group consisting of (C₁₋₄)alkyl, (C₁₋₄)alkoxy, trifluoromethyl, and halogen.
Examples are: 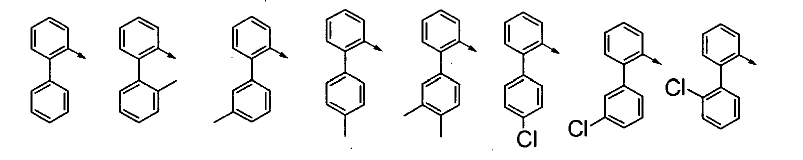 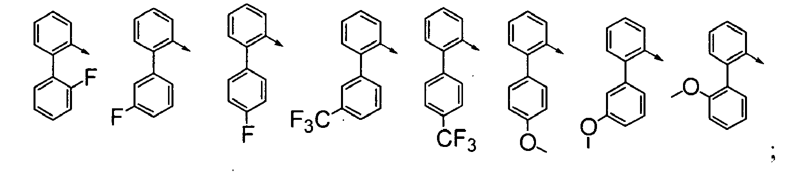 whereby in the above examples the phenyl ring representing "A" may also be further mono-substituted with methyl.

In case R¹ represents "aryl" the term preferably means the above-mentioned groups which are unsubstituted or independently mono-, di-, or trisubstituted wherein the substituents are independently selected from the group consisting of (C₁₋₄)alkyl, (C₂₋₆)alkynyl, (C₃₋₆)cycloalkyl, (C₁₋₄)alkoxy, (C₁₋₄)alkylthio, halogen, hydroxy, cyano, trifluoromethyl, trifluoromethoxy, NR²R³, N(R²)C(O)R³, COOR², and C(O)NR²R³. Especially, the substituents are independently selected from the group consisting of (C₁₋₄)alkyl, (C₃₋₆)cycloalkyl, (C₁₋₄)alkoxy, halogen, hydroxy, cyano, trifluoromethyl, trifluoromethoxy, NR²R³, N(R²)C(O)R³, and C(O)NR²R³. Preferred examples wherein "R¹" represents "aryl" are unsubstituted or independently mono-, di-, or trisubstituted phenyl (preferred mono-substituted phenyl), wherein the substituents are independently selected from the group consisting of (C₁₋₄)alkyl, (C₁₋₄)alkoxy, halogen, hydroxy, cyano, trifluoromethyl, trifluoromethoxy, and COOR². Examples wherein R¹ represents "aryl" are phenyl, naphthyl, 2-chloro-4,5-difluorophenyl, 3-bromo-6-chlorophenyl, 2-chloro-5-trifluoromethylphenyl, 2-chlorophenyl, 4-chlorophenyl, 2,4-dichlorophenyl, 3,4-dichlorophenyl, 2-chloro-4-fluorophenyl, 2-chloro-3-fluorophenyl, 2-chloro-3-methylphenyl, 3-chloro-2-methylphenyl, 4-fluorophenyl, 2-fluoro-5-methylphenyl, 3-fluoro-2-methylphenyl, 3-fluoro-6-methoxyphenyl, 3-fluoro-4-methoxyphenyl, 2-bromo-5-methylphenyl, 2-bromo-3-methylphenyl, 2,5-dimethylphenyl, 3,4-dimethylphenyl, 3,5-dimethylphenyl, 2-methyl-4-methoxyphenyl, 3-methyl-4-methoxyphenyl, 4-ethylphenyl, 4-tert.-butylphenyl, 2-hydroxyphenyl, 3-hydroxyphenyl, 3-methoxyphenyl, 4-methoxyphenyl, 2,3-dimethoxyphenyl, 2,4-dimethoxyphenyl, 3,4-dimethoxyphenyl, 2,5-dimethoxyphenyl, 3,5-dimethoxyphenyl, 3,5-dichloro-4-hydroxyphenyl, 3-iodophenyl, 3-bromophenyl, 3-cyanophenyl, 4-cyanophenyl, 3-ethynylphenyl, 4-methyl-3-trifluoromethylphenyl, 4-methoxy-3-trifluoromethylphenyl, 3-trifluoromethylphenyl, 4-trifluoromethylphenyl, 3-methoxycarbonylphenyl, and 4-dimethylaminophenyl.

The term "aryloxy-(C₁₋₄)alkyl" means a (C₁₋₄)alkyl group as previously defined in which one hydrogen atom has been replaced by a group of the formula aryl-O-wherein "aryl" has the meaning as defined previously and is unsubstituted or independently mono- or di-substituted wherein the substituents are independently selected from the group consisting of (C₁₋₄)alkyl, (C₁₋₄)alkoxy, halogen, trifluoromethyl, trifluoromethoxy, and NR²R³. Examples wherein R¹ represents "aryloxy-(C₁₋₄)alkyl" are naphthalen-2-yloxy-methyl, 2-methoxy-phenoxy-methyl and 3-methoxy-phenoxy-methyl.

The term "heterocyclyl", alone or in combination, means a 5- to 10-membered monocyclic or bicyclic aromatic ring containing for example 1, 2 or 3 heteroatoms selected from oxygen, nitrogen and sulfur which may be the same or different. Examples of such heterocyclyl groups are furanyl, oxazolyl, isoxazolyl, oxadiazolyl, thienyl, thiazolyl, isothiazolyl, thiadiazolyl, pyrrolyl, imidazolyl, pyrazolyl, triazolyl, pyridyl, pyrimidyl, pyridazinyl, pyrazinyl, indolyl, isoindolyl, benzofuranyl, isobenzofuranyl, benzothiophenyl, indazolyl, benzimidazolyl, benzoxazolyl, benzisoxazolyl, benzothiazolyl, benzotriazolyl, benzoxadiazolyl, benzothiadiazolyl, quinolinyl, isoquinolinyl, naphthyridinyl, cinnolinyl, quinazolinyl, quinoxalinyl, phthalazinyl, pyrazolo[1,5-a]pyridyl, pyrazolo[1,5-a]pyrimidyl, imidazo[1,2-a]pyridyl or imidazo[2,1-b]thiazolyl. The above-mentioned heterocyclyl groups may also be independently mono-, di-, or trisubstituted wherein the substitucnts are independently selected from the group consisting of (C₁₋₄)alkyl, (C₃₋₆)cycloalkyl, (C₂₋₆)alkynyl, hydroxy-(C₁₋₄)alkyl, hydroxy-(C₂₋₆)alkynyl, trimethylsilyl-ethynyl, (C₃₋₆)cycloalkyl-ethynyl, (C₁₋₄)alkoxy, trifluoromethyl, trifluoromethoxy, methoxy-(C₁₋₄)alkoxy, cyano, (C₁₋₄)alkylthio, hydroxy, COOR², NR²R³, N(R²)C(O)R³, C(O)NR²R³, and halogen.
In case "A" represents "heterocyclyl" the term preferably means the above-mentioned groups which is unsubstituted or independently mono- or di-substituted (preferred unsubstituted or mono-substituted) wherein the substituents are independently selected from the group consisting of (C₁₋₄)alkyl, (C₃₋₆)cycloalkyl, (C₂₋₆)alkynyl, hydroxy-(C₁₋₄)alkyl, hydroxy-(C₂₋₆)alkynyl, trimethylsilyl-ethynyl, (C₃₋₆)cycloalkyl-ethynyl, (C₁₋₄)alkoxy, trifluoromethyl, trifluoromethoxy, NR²R³, N(R²)C(O)R³, C(O)NR²R³, and halogen. Especially, the substituents are independently selected from the group consisting of (C₁₋₄)alkyl, (C₃₋₆)cycloalkyl, (C₁₋₄)alkoxy, trifluoromethyl, trifluoromethoxy, NR²R³, N(R²)C(O)R³, C(O)NR²R³, and halogen. In another embodiment, the substituents are independently selected from the group consisting of (C₁₋₄)alkyl, hydroxy-(C₁₋₄)alkyl, hydroxy-(C₂₋₆)alkynyl, trimethylsilyl-ethynyl, (C₁₋₄)alkoxy, NR²R³, and halogen. In a further preferred embodiment, in case "A" represents "heterocyclyl" the term means a 5- to 6-membered monocyclic heterocyclyl as defined above which is unsubstituted or independently mono- or di-substituted (preferred unsubstituted or mono-substituted) wherein the substituents are independently selected from the group consisting of (C₁₋₄)alkyl, hydroxy-(C₁₋₄)alkyl, hydroxy-(C₂₋₆)alkynyl, trimethylsilyl-ethynyl, (C₁₋₄)alkoxy, NR²R³, and halogen. Preferred examples wherein "A" represents "heterocyclyl" are unsubstituted or mono-substituted heterocyclyl as mentioned above (preferred thiazolyl, especially preferred thiazol-4-yl) wherein the substituent is selected from (C₁₋₄)alkyl, (C₁₋₄)alkoxy, halogen (especially bromo), and NR²R³ (especially the substituent is selected from (C₁₋₄)alkyl and NR²R³). In another embodiment the substituent is selected from hydroxy-(C₁₋₄)alkyl, and hydroxy-(C₂₋₆)alkynyl. In addition to the above-mentioned substituents, the substituent "A" is also substituted by the substituent "B", whereby, in case B represents aryl or heterocyclyl, B is preferably attached in ortho position to the point of attachment of the group X.
Particular examples wherein "A" represents "heterocyclyl" and one of the substituents is represented by "B" are: 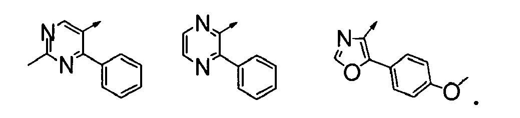 Further particular examples are: 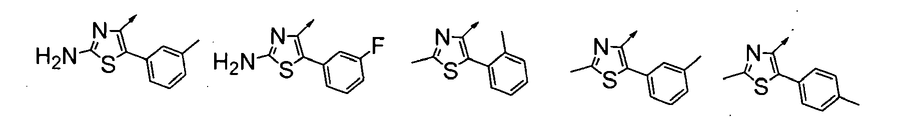 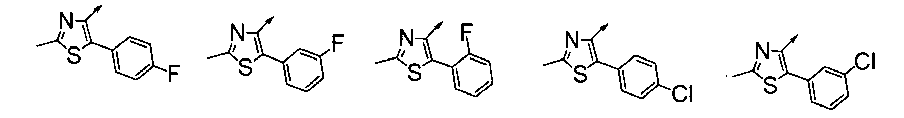 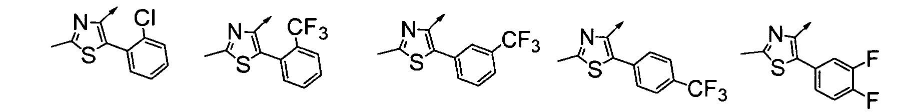 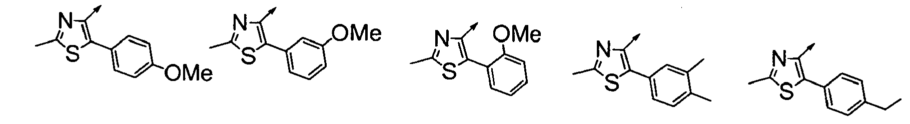 Further particular examples are: 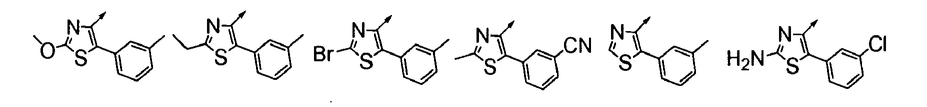 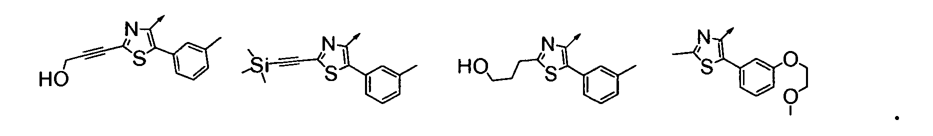 In case "B" represents "heterocyclyl" the term preferably means the above-mentioned groups which is unsubstituted or independently mono-, di-, or trisubstituted (preferred mono- or di-substituted) wherein the substituents are independently selected from the group consisting of (C₁₋₄)alkyl, (C₃₋₆)cycloalkyl, (C₁₋₄)alkoxy, methoxy-(C₁₋₄)alkoxy, cyano, trifluoromethyl, trifluoromethoxy, NR²R³, N(R²)C(O)R³, C(O)NR²R³, and halogen. Especially, the substituents are independently selected from the group consisting of (C₁₋₄)alkyl, (C₃₋₆)cycloalkyl, (C₁₋₄)alkoxy, trifluoromethyl, trifluoromethoxy, NR²R³, N(R²)C(O)R³, C(O)NR²R³, and halogen. In addition to the above-mentioned substituents, the substituent "B" is attached to the substituent "A". Examples wherein "B" represents "heterocyclyl" are pyrazolyl (especially 2-methylpyrazole-5-yl or pyrazole-5-yl), pyridyl (especially 3-pyridyl), thienyl (especially 4-methyl-thien-2-yl) and thiazolyl (especially 2-aminothiazol-4-yl).

In case R¹ represents "heterocyclyl" the term preferably means the above-mentioned groups which is unsubstituted or independently mono-, di-, or trisubstituted (preferred unsubstituted or mono-substituted) wherein the substituents are independently selected from the group consisting of (C₁₋₄)alkyl, (C₂₋₆)alkynyl, (C₃₋₆)cycloalkyl, (C₁₋₄)alkoxy, (C₁₋₄)alkylthio, halogen, hydroxy, cyano, trifluoromethyl, trifluoromethoxy, NR²R³, N(R²)C(O)R³, COOR², and C(O)NR²R³. Especially, the substituents are independently selected from the group consisting of (C₁₋₄)alkyl, (C₃₋₆)cycloalkyl, (C₁₋₄)alkoxy, halogen, hydroxy, cyano, trifluoromethyl, trifluoromethoxy, NR²R³, N(R²)C(O)R³, and C(O)NR²R³. In a further preferred embodiment, in case R¹ represents "heterocyclyl" the term means the above-mentioned groups which are unsubstituted or independently mono-, di-, or trisubstituted (preferred unsubstituted or mono-substituted) wherein the substituents are independently selected from the group consisting of (C₁₋₄)alkyl, (C₁₋₄)alkoxy, and halogen. In another embodiment, the substituents are independently selected from the group consisting of (C₁₋₄)alkyl, trifluoromethyl, and halogen. In a further preferred embodiment, in case R¹ represents "heterocyclyl" the term means the above-mentioned groups which are unsubstituted or independently mono-, di-, or trisubstituted (preferred unsubstituted or mono-substituted) wherein the substituent is methyl. Preferred examples wherein "R¹" represents "heterocyclyl" are unsubstituted or independently mono-, di-, or trisubstituted (preferred unsubstituted or mono-substituted) heterocyclyl; wherein the heterocyclyl is selected from the group consisting of furanyl, oxazolyl, isoxazolyl, thiazolyl, pyrrolyl, pyrazolyl, pyridyl, pyrimidyl, indolyl, benzofuranyl, benzothiophenyl, indazolyl, benzimidazolyl, benzisoxazolyl, benzothiazolyl, benzoxadiazolyl, benzothiadiazolyl, quinolinyl, isoquinolinyl, naphthyridinyl, quinoxalinyl, pyrazolo[1,5-a]pyridyl, pyrazolo [1,5-a]pyrimidyl, imidazo[1,2-a]pyridyl and imidazo[2,1-b]thiazolyl (especially imidazo[2,1-b]thiazolyl); wherein the substituents are independently selected from (C₁₋₄)alkyl, trifluoromethyl, and halogen. Examples wherein R¹ represents "heterocyclyl" are: 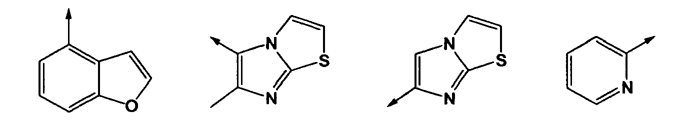 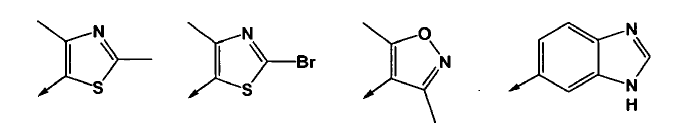 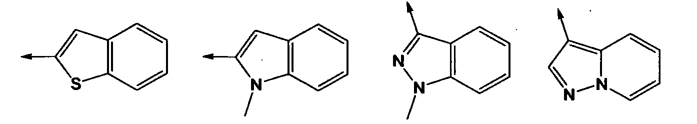 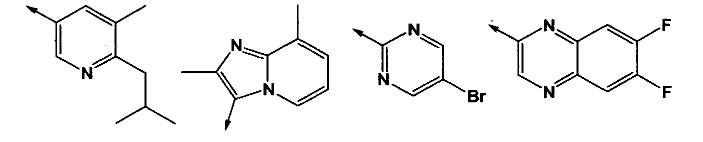 In another embodiment, preferred examples wherein R¹ represents "heterocyclyl" are imidazo[2,1-b]thiazolyl and imidazo[1,2-a]pyridyl (especially imidazo [2,1-b]thiazolyl).

The term "heterocyclyl-ethenyl" means an ethenyl group in which one hydrogen atom has been replaced by a heterocyclyl group as previously defined. The heterocyclyl group may be unsubstituted or independently mono- or di-substituted wherein the substituents are independently selected from the group consisting of (C₁₋₄)alkyl, (C₁₋₄)alkoxy, halogen, trifluoromethyl, trifluoromethoxy, and NR²R³. An example is 2-furanyl-ethenyl.
The term "heterocyclyl-(C₁₋₄)alkyl" means a (C₁₋₄)alkyl group as previously defined in which one hydrogen atom has been replaced by a heterocyclyl group as previously defined. The heterocyclyl group may be unsubstituted or independently mono- or di-substituted wherein the substituents are independently selected from the group consisting of (C₁₋₄)alkyl, (C₁₋₄)alkoxy, halogen, trifluoromethyl, trifluoromethoxy, and NR²R³. An example is 2,5-dimethyl-thiazol-4-ylmethyl.

The term "tricyclic group" means a fluorenyl, a carbazolyl, a dibenzofuranyl, or a dibenzothiophenyl group which groups are unsubstituted or independently mono- or di-substituted wherein the substituents are independently selected from the group consisting of (C₁₋₄)alkyl, (C₃₋₆)cycloalkyl, (C₁₋₄)alkoxy, halogen, cyano, trifluoromethyl, trifluoromethoxy, NR²R³, N(R²)C(O)R³, and C(O)NR²R³. An example is a fluorenyl group.
2,3-Dihydro-benzofuranyl-groups as used for the substituent R¹ are preferably unsubstituted, or di-substituted in position 2 with methyl.
Benzo[1,3]dioxolyl-groups as used for the substituent R¹ are preferably unsubstituted, or di-substituted in position 2 with fluoro.
4*H*-Benzo[1,3]dioxinyl-groups as used for the substituent R¹ are preferably unsubstituted, or mono-substituted in position 6 with fluoro.
3,4-Dihydro-2*H-*benzo[1,4]oxazinyl-, and 3-oxo-3,4-dihydro-2*H*-benzo[1,4]oxazinyl-groups as used for the substituent R¹ are preferably unsubstituted, or mono-substituted on the nitrogen atom with methyl.
2,3-Dihydro-benzo[1,4]dioxinyl-, 4-oxo-4*H*-chromenyl-, 2*H*-chromenyl, chromanyl-, 2,3-dihydro-thieno[3,4-b][1,4]dioxinyl-, morpholin-4-yl-phenyl-, piperazin-1-yl-phenyl-, and 2,3,6,7-tetrahydro-benzo[1,2-b;4,5-b']difuranyl-groups as used for the substituent R¹ are preferably unsubstituted.
The term "NR²R³" means for example NH₂ and N(CH₃)₂ (especially NH₂).
The term "N(R²)C(O)R³" means for example N(CH₃)C(O)CH₃.
The term "C(O)NR²R³" means for example C(O)N(CH₃)₂.
The term "COOR²" means for example COOCH₃.
The term "(C₂₋₆)alkynyl", alone or in combination, means a straight-chain or branched-chain alkynyl group, preferably a straight-chain or branched-chain alkyn-1-yl group, with 2 to 6 carbon atoms. Examples are ethynyl, ethyl-ethynyl, or isobutyl-ethynyl.
The term "hydroxy-(C₁₋₄)alkyl"means a (C₁₋₄)alkyl group as defined before which is substitued with hydroxy. An example is 3-hydroxy-n-propyl.
The term "hydroxy-(C₂₋₆)alkynyl" means a (C₂₋₆)alkynyl group as defined before which is substitued with hydroxy. An example is hydroxymethyl-ethynyl.
The term "(C₃₋₆)cycloalkyl-ethynyl" means an ethynyl group which is substituted with a (C₃₋₆)cycloalkyl group as defined before. An example is cyclopropyl-ethynyl.
The term "methoxy-(C₁₋₄)alkoxy" means for example 2-methoxy-ethoxy.
The term "(C₁₋₄)alkylthio" means a group of the formula (C₁₋₄)alkyl-S-in which the term "(C₁₋₄)alkyl" has the previously given significance, such as methyl-thio.
ii) A further embodiment of the invention relates to compounds according to embodiment i), wherein the stereogenic centers are in a relative *cis*-configuration 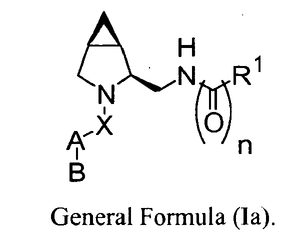
iii) A further embodiment of the invention relates to compounds according to embodiments i) or ii), wherein at least one, preferably all of the following characteristics are present:
   X represents C(O) or SO₂;
   A represents aryl or heterocyclyl, wherein the aryl or heterocyclyl is unsubstituted or independently mono- or di-substituted, wherein the substituents are independently selected from the group consisting of (C₁₋₄)alkyl, (C₃₋₆)cycloalkyl, (C₁₋₄)alkoxy, trifluoromethyl, trifluoromethoxy, NR²R³, N(R²)C(O)R³, C(O)NR²R³, and halogen; B represents a hydrogen atom or an aryl- or heterocyclyl-group, wherein the aryl or heterocyclyl is unsubstituted or independently mono-, di-, or trisubstituted, wherein the substituents are independently selected from the group consisting of (C₁₋₄)alkyl, (C₃₋₆)cycloalkyl, (C₁₋₄)alkoxy, trifluoromethyl, trifluoromethoxy, NR²R³, N(R²)C(O)R³, C(O)NR²R³, and halogen;
   or A and B together represent a tricyclic group;
   n represents 0 or 1; and
   R¹ represents aryl or heterocyclyl, wherein the aryl or heterocyclyl is unsubstituted or independently mono-, di-, or trisubstituted wherein the substituents arc independently selected from the group consisting of (C₁₋₄)alkyl, (C₃₋₆)cycloalkyl, (C₁₋₄)alkoxy, halogen, hydroxy, cyano, trifluoromethyl, trifluoromethoxy, NR²R³, N(R²)C(O)R³, and C(O)NR²R³; or R¹ represents a heterocyclyl-ethenyl-, a heterocyclyl-(C₁₋₄)alkyl or an aryloxy-(C₁₋₄)alkyl-group, which groups are unsubstituted or independently mono- or di-substituted at the aryl- or heterocyclyl-part wherein the substituents are independently selected from the group consisting of (C₁₋₄)alkyl, (C₁₋₄)alkoxy, halogen, trifluoromethyl, trifluoromethoxy, and NR²R³; or R¹ represents a 2,3-dihydro-benzofuranyl-, a benzo[1,3]dioxolyl-, a 2,3-dihydro-benzo[1,4]dioxinyl- or a 4-oxo-4*H*-chromenyl group, wherein said groups are unsubstituted or mono-substituted at the aromatic ring with substituents independently selected from the group consisting of (C₁₋₄)alkyl, (C₁₋₄)alkoxy, and halogen;
   R² represents hydrogen or (C₁₋₄)alkyl;
   R³ represents hydrogen or (C₁₋₄)alkyl.
iv) A further embodiment of the invention relates to compounds according to any one of embodiments i) to iii), wherein at least one, preferably all of the following characteristics are present:
   A represents heterocyclyl, wherein the heterocyclyl is unsubstituted or mono-substituted, wherein the substituent is selected from the group consisting of (C₁₋₄)alkyl, and NR²R³;
   B represents aryl, wherein the aryl is unsubstituted or independently mono-, di- or trisubstituted, wherein the substituents are independently selected from the group consisting of (C₁₋₄)alkyl, (C₁₋₄)alkoxy, trifluoromethyl, and halogen; and
      R¹ represents aryl or heterocyclyl, wherein the aryl or heterocyclyl is unsubstituted or independently mono-, di-, or trisubstituted wherein the substituents are independently selected from the group consisting of (C₁₋₄)alkyl, (C₁₋₄)alkoxy, and halogen; or R¹ represents a 2,3-dihydro-benzofuranyl-, a benzo[1,3]dioxolyl- or a 2,3-dihydro-benzo[1,4]dioxinyl-group.
v) A further embodiment of the invention relates to compounds according to any one of embodiments i) to iv), wherein at least one, preferably all of the following characteristics are present:
   A represents an oxazolyl, a thiazolyl, a pyrimidyl or a pyrazinyl group, wherein said groups are unsubstituted or mono-substituted, wherein the substituent is selected from the group consisting of (C₁₋₄)alkyl, and NR²R³;
   B represents phenyl, wherein the phenyl is unsubstituted or independently mono- or di-substituted, wherein the substituents are independently selected from the group consisting of (C₁₋₄)alkyl, (C₁₋₄)alkoxy, trifluoromethyl, and halogen; and
      R¹ represents a phenyl, a naphthyl, a benzofuranyl, a imidazo[2,1-b]thiazolyl, a imidazo[1,2-a]pyridyl, a pyrazolo[1,5-a]pyridyl, a thiazolyl, a isoxazolyl, a pyrazolyl, an indolyl, an indazolyl, a benzimidazolyl or a benzothiophenyl group, wherein said groups are unsubstituted or independently mono- or di-substituted wherein the substituents are independently selected from the group consisting of (C₁₋₄)alkyl, (C₁₋₄)alkoxy, and halogen; or R¹ represents a 2,3-dihydro-benzofuranyl-, a benzo[1,3]dioxolyl- or a 2,3-dihydro-benzo[1,4]dioxinyl-group.
vi) A further embodiment of the invention relates to compounds according to any one of embodiments i) to v), wherein X represents C(O).
vii) A further embodiment of the invention relates to compounds according to any one of embodiments i) to vi), wherein n represents 1.
vii) A further embodiment of the invention relates to compounds according to any one of embodiments i), ii), vi), or vii), wherein
   A represents aryl or heterocyclyl, wherein the aryl or heterocyclyl is unsubstituted or mono-substituted, wherein the substituent is selected from the group consisting of (C₁₋₄)alkyl, (C₃₋₆)cycloalkyl, (C₂₋₆)alkynyl, hydroxy-(C₁₋₄)alkyl, hydroxy-(C₂₋₆)alkynyl, (C₃₋₆)cycloalkyl-ethynyl, (C₁₋₄)alkoxy, NR²R³, and halogen.
ix) A further embodiment of the invention relates to compounds according to any one of embodiments i), ii), or vi) to viii), wherein
   B represents aryl or heterocyclyl, wherein the aryl or heterocyclyl is unsubstituted or independently mono-, di-, or trisubstituted, wherein the substituents are independently selected from the group consisting of (C₁₋₄)alkyl, (C₁₋₄)alkoxy, cyano, trifluoromethyl, NR²R³, and halogen.
x) A further embodiment of the invention relates to compounds according to any one of embodiments i), ii), or vi) to ix), wherein
   R¹ represents aryl or heterocyclyl, wherein the aryl or heterocyclyl is unsubstituted or independently mono-, di-, or trisubstituted wherein the substituents are independently selected from the group consisting of (C₁₋₄)alkyl, (C₁₋₄)alkoxy, halogen, hydroxy, cyano, trifluoromethyl, and COOR²; or R¹ represents a 2,3-dihydro-benzofuranyl-, a benzo[1,3]dioxolyl-, a 2,3-dihydro-benzo[1,4]dioxinyl-, a 2*H*-chromenyl, a chromanyl-, a 4*H*-benzo[1,3]dioxinyl-, a 2,3-dihydro-thieno[3,4-b][1,4]dioxinyl-, a 3,4-dihydro-2*H*-benzo[1,4]oxazinyl- or a 2,3,6,7-tetrahydro-benzo[1,2-b;4,5-b'] difuranyl-group, wherein said groups are unsubstituted or independently mono- or di-substituted wherein the substituents are independently selected from the group consisting of (C₁₋₄)alkyl, (C₁₋₄)alkoxy, and halogen.
xi) A further embodiment of the invention relates to compounds according to any one of embodiments i), ii), or vi) to x), wherein
   A represents aryl, wherein the aryl is unsubstituted or mono-substituted, wherein the substituent is selected from the group consisting of (C₁₋₄)alkyl, (C₃₋₆)cycloalkyl, (C₃₋₆)cycloalkyl-ethynyl, and halogen.
xii) A further embodiment of the invention relates to compounds according to any one of embodiments i), ii), or vi) to x), wherein
   A represents heterocyclyl, wherein the heterocyclyl is unsubstituted or mono-substituted, wherein the substituent is selected from the group consisting of (C₁₋₄)alkyl, (C₃₋₆)cycloalkyl, hydroxy-(C₁₋₄)alkyl, hydroxy-(C₂₋₆)alkynyl, (C₁₋₄)alkoxy, NR²R³, and halogen.
xiii) A further embodiment of the invention relates to compounds according to any one of embodiments i), ii), or vi) to xii), wherein
   B represents phenyl, wherein the phenyl is unsubstituted or independently mono-, di-, or trisubstituted, wherein the substituents are independently selected from the group consisting of(C₁₋₄)alkyl, (C₁₋₄)alkoxy, trifluoromethyl, and halogen.
xiv) A further embodiment of the invention relates to compounds according to any one of embodiments i) to iii), or vi) to xiii), wherein
   R¹ represents heterocyclyl, wherein the heterocyclyl is unsubstituted or independently mono-, di-, or trisubstituted wherein the substituents are independently selected from the group consisting of(C₁₋₄)alkyl, (C₁₋₄)alkoxy, halogen, and trifluoromethyl.
xv) A further embodiment of the invention relates to compounds according to any one of embodiments i) to iii), or vi) to xiii), wherein
   R¹ represents aryl, wherein the aryl is unsubstituted or independently mono-, di-, or trisubstituted wherein the substituents are independently selected from the group consisting of(C₁₋₄)alkyl, (C₁₋₄)alkoxy, halogen, hydroxy, cyano, and trifluoromethyl.
xvi) A further embodiment of the invention relates to compounds according to any one of embodiments i), ii), or vi) to xiii), wherein
   R¹ represents a 2,3-dihydro-benzofuranyl-, a 2,3-dihydro-benzo[1,4]dioxinyl-, a chromanyl-, a 2,3-dihydro-thieno[3,4-b][1,4]dioxinyl- or a 3,4-dihydro-2*H-*benzo[1,4]oxazinyl-group, wherein said groups are unsubstituted or mono-substituted wherein the substituent is selected from the group consisting of (C₁₋₄)alkyl, and halogen.
xvii) A further embodiment of the invention relates to compounds according to any one of embodiments i) to iii), or vi) to xiv), wherein, in case R¹ represents heterocyclyl, said heterocyclyl is an imidazo[2,1-b]thiazolyl or an imidazo [1,2-a]pyridyl group (especially imidazo[2,1-b]thiazolyl), wherein said groups are unsubstituted or mono-substituted, wherein the substituent is selected from the group consisting of (C₁₋₄)alkyl, halogen, and trifluoromethyl.
xviii) A further embodiment of the invention relates to compounds according to any one of embodiments i), ii), vi) to x), or xii) to xvii), wherein, in case A represents heterocyclyl, said heterocyclyl is a thiazole group, which is unsubstituted or mono-substituted, wherein the substituent is selected from the group consisting of (C₁₋₄)alkyl, hydroxy-(C₁₋₄)alkyl, hydroxy-(C₂₋₆)alkynyl, (C₁₋₄)alkoxy, NR²R³, and halogen.
xix) A further embodiment of the invention relates to compounds according to any one of embodiments i), ii), vi), vii), x), or xiv) to xvii), wherein A represents mono-, or di-substituted phenyl, wherein one substituent is selected from the group consisting of (C₂₋₆)alkynyl, and (C₃₋₆)cycloalkyl-ethynyl; and the other substituent (if present) is selected from the group consisting of (C₁₋₄)alkyl, (C₁₋₄)alkoxy, and trifluoromethoxy (especially (C₁₋₄)alkyl); and
   B represents hydrogen.
xx) A further embodiment of the invention comprises compounds of the formula (Ib), wherein the stereogenic centers are in a (1R,2S,5S)-configuration 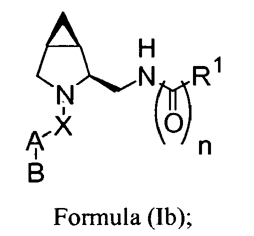 whereby any preference indicated for the compounds of formula (I) or (Ia) (whether for the compounds themselves as indicated in embodiments iii) to xix), salts thereof, compositions containing the compounds or salts thereof, uses of the compounds or salts thereof, etc.) apply mutatis mutandis to compounds of formula (Ib).
xxi) Examples of compounds according to embodiment i) are selected from the group consisting of:
   4-fluoro-N- {(1R*,2S*,5S*)-3-[5-(3-fluoro-phenyl)-2-methyl-thiazole-4-carbonyl]-3-aza-bicyclo[3.1.0]hex-2-ylmethyl}-benzamide;
   benzofuran-4-carboxylic acid [(1R*,2S*,5S*)-3-(biphenyl-2-carbonyl)-3-aza-bicyclo[3.1.0]hex-2-ylmethyl]-amide;
   benzofuran-4-carboxylic acid [(1R*,2S*,5S*)-3-(2-methyl-5-m-tolyl-thiazole-4-carbonyl)-3-aza-bicyclo[3.1.0]hex-2-ylmethyl]-amide;
   benzofuran-4-carboxylic acid {(1R*,2S*,5S*)-3-[5-(4-fluoro-phenyl)-2-methylthiazole-4-carbonyl]-3-aza-bicyclo[3.1.0]hex-2-ylmethyl}-amide;
   benzofuran-4-carboxylic acid {(1R*,2S*,5S*)-3-[5-(3-fluoro-phenyl)-2-methylthiazole-4-carbonyl]-3-aza-bicyclo[3.1.0]hex-2-ylmethyl}-amide;
   benzofuran-4-carboxylic acid [(1R*,2S*,5S*)-3-(2-methyl-5-p-tolyl-thiazole-4-carbonyl)-3-aza-bicyclo[3.1.0]hex-2-ylmethyl]-amide;
   benzofuran-4-carboxylic acid {(1R*,2S*,5S*)-3-[5-(4-ethyl-phenyl)-2-methylthiazole-4-carbonyl]-3-aza-bicyclo[3.1.0]hex-2-ylmethyl}-amide;
   benzofuran-4-carboxylic acid {(1R*,2S*,5S*)-3-[5-(2-fluoro-phenyl)-2-methylthiazole-4-carbonyl]-3-aza-bicyclo[3.1.0]hex-2-ylmethyl }-amide;
   benzofuran-4-carboxylic acid {(1R*,2S*,5S*)-3-[5-(2-chloro-phenyl)-2-methylthiazole-4-carbonyl]-3-aza-bicyclo[3.1.0]hex-2-ylmethyl}-amide;
   benzofuran-4-carboxylic acid {(1R*,2S*,5S*)-3-[5-(3-chloro-phenyl)-2-methylthiazole-4-carbonyl]-3-aza-bicyclo[3.1.0]hex-2-ylmethyl}-amide;
   benzofuran-4-carboxylic acid {(1R*,2S*,5S*)-3-[5-(2-methoxy-phenyl)-2-methylthiazole-4-carbonyl]-3-aza-bicyclo[3.1.0]hex-2-ylmethyl -amide;
   benzofuran-4-carboxylic acid {(1R*,2S*,5S*)-3-[5-(3-methoxy-phenyl)-2-methylthiazole-4-carbonyl]-3-aza-bicyclo[3.1.0]hex-2-ylmethyl}-amide;
   benzofuran-4-carboxylic acid {(1R*,2S*,5S*)-3-[2-methyl-5-(3-trifluoromethylphenyl)-thiazole-4-carbonyl]-3-aza-bicycclo[3.1.0]hex-2-ylmethyl}-amide;
   benzofuran-4-carboxylic acid {(1R*,2S*,5S*)-3-[2-methyl-5-(2-trifluoromethylphenyl)-thiazole-4-carbonyl]-3-aza-bicyclo[3.1.0]hex-2-ylmethyl }-amide;
   benzofuran-4-carboxylic acid [(1R*,2S*,5S*)-3-(2-methyl-5-o-tolyl-thiazole-4-carbonyl)-3-aza-bicyclo[3.1.0]hex-2-ylmethyl]-amide;
   benzofuran-4-carboxylic acid {(1R*,2S*;,5S*)-3-[5-(3,4-dimethyl-phenyl)-2-methylthiazole-4-carbonyl]-3-aza-bicyclo[3.1.0]hex-2-ylmethyl}-amide;
   benzofuran-4-carboxylic acid [(1R*,2S*,5S*)-3-(2-amino-5-m-tolyl-thiazole-4-carbonyl)-3-aza-bicyclo[3.1.0]hex-2-ylmethyl]-amide;
   benzofuran-4-carboxylic acid {(1R*,2S*,5S*)-3-[2-amino-5-(3-fluoro-phenyl)-thiazole-4-carbonyl]-3-aza-bicyclo[3.1.0]hex-2-ylmethyl}-amide;
   benzofuran-4-carboxylic acid [(1R*,2S*,5S*)-3-(2-methyl-4-phenyl-pyrimidine-5-carbonyl)-3-aza-bicyclo[3.1.0]hex-2-ylmethyl]-amide;
   benzofuran-4-carboxylic acid {(1R*,2S*,5S*)-3-[2-(2-amino-thiazol-4-yl)-benzoyl]-3-aza-bicyclo[3.1.0]hex-2-ylmethyl}-amide;
   benzofuran-4-carboxylic acid [(1R*,2S*,5S*)-3-(9H-fluorene-4-carbonyl)-3-aza-bicyclo[3.1.0]hex-2-ylmethyl]-amide;
   benzofuran-4-carboxylic acid [(1R*,2S*,5S*)-3-(3-phenyl-pyrazine-2-carbonyl)-3-aza-bicyclo[3.1.0]hex-2-ylmethyl]-amide;
   benzofuran-4-carboxylic acid {(1R*,2S*,5S*)-3-[5-(4-methoxy-phenyl)-oxazole-4-carbonyl]-3-aza-bicyclo[3.1.0]hex-2-ylmethyl}-amide;
   benzofuran-4-carboxylic acid [(1R*,2S*,5S*)-3-(2'-fluoro-biphenyl-2-carbonyl)-3-aza-bicyclo[3.1.0]hex-2-ylmethyl]-amide;
   benzofuran-4-carboxylic acid [(1R*,2S*,5S*)-3-(4'-fluoro-biphenyl-2-carbonyl)-3-aza-bicyclo[3.1.0]hex-2-ylmethyl]-amide;
   benzofuran-4-carboxylic acid [(1R*,2S*,5S*)-3-(2'-chloro-biphenyl-2-carbonyl)-3-aza-bicyclo[3.1.0]hex-2-ylmethyl]-amide;
   benzofuran-4-carboxylic acid [(1R*,2S*,5S*)-3-(3'-chloro-biphenyl-2-carbonyl)-3-aza-bicyclo[3.1.0]hex-2-ylmethyl]-amide;
   benzofuran-4-carboxylic acid [(1R*,2S*,5S*)-3-(4'-chloro-biphenyl-2-carbonyl)-3-aza-bicyclo[3.1.0]hex-2-ylmethyl]-amide;
   benzofuran-4-carboxylic acid [(1R*,2S*,5S*)-3-(4'-methyl-biphenyl-2-carbonyl)-3-aza-bicyclo[3.1.0]hex-2-ylmethyl]-amide;
   benzofuran-4-carboxylic acid [(1R*,2S*,5S*)-3-(3'-methyl-biphenyl-2-carbonyl)-3-aza-bicyclo[3.1.0]hex-2-ylmethyl]-amide;
   benzofuran-4-carboxylic acid [(1R*,2S*,5S*)-3-(3'-methoxy-biphenyl-2-carbonyl)-3-aza-bicyclo[3.1.0]hex-2-ylmethyl]-amide;
   benzofuran-4-carboxylic acid [(1R*,2S*,5S*)-3-(4'-methoxy-biphenyl-2-carbonyl)-3-aza-bicyclo[3.1.0]hex-2-ylmethyl]-amide;
   benzofuran-4-carboxylic acid [(1R*,2S*,5S*)-3-(3'-trifluoromethyl-biphenyl-2-carbonyl)-3-aza-bicyclo[3.1.0]hex-2-ylmethyl]-amide;
   benzofuran-4-carboxylic acid [(1R*,2S*,5S*)-3-(2-pyridin-3-yl-benzoyl)-3-aza-bicyclo[3.1.0]hex-2-ylmethyl]-amide;
   6-methyl-imidazo[2,1-b]thiazole-5-carboxylic acid [(1R*,2S*,5S*)-3-(2-methyl-5-p-tolyl-thiazole-4-carbonyl)-3-aza-bicyclo[3.1.0]hex-2-ylmethyl]-amide;
   6-methyl-imidazo[2,1-b]thiazole-5-carboxylic acid [(1R*,2S*,5S*)-3-(2-methyl-5-m-tolyl-thiazole-4-carbonyl)-3-aza-bicyclo[3.1.0]hex-2-ylmethyl]-amide;
   6-methyl-imidazo[2, 1-b]thiazole-5-carboxylic acid {(1R*,2S*,5S*)-3-[5-(3-fluorophenyl)-2-methyl-thiazole-4-carbonyl]-3-aza-bicyclo[3.1.0]hex-2-ylmethyl }-amide;
   6-methyl-imidazo[2,1-b]thiazole-5-carboxylic acid {(1R*,2S*,5S*)-3-[5-(4-ethylphenyl)-2-methyl-thiazole-4-carbonyl]-3-aza-bicyclo[3.1.0]hex-2-ylmethyl}-amide;
   6-methyl-imidazo[2,1-b]thiazole-5-carboxylic acid {(1R*,2S*,5S*)-3-[5-(4-fLuorophenyl)-2-methyl-thiazole-4-carbonyl]-3-aza-bicyclo[3.1.0]hex-2-ylmethyl}-amide;
   6-methyl-imidazo[2,1-b]thiazole-5-carboxylicacid [(1R*,2S*,5S*)-3-(biphenyl-2-carbonyl)-3-aza-bicyclo[3.1.0]hex-2-ylmethyl]-amide;
   {(1R*,2S*,5S*)-2-[(6,7-difluoro-quinoxalin-2-ylamino)-methyl]-3-aza-bicyclo[3.1.0]hex-3-yl}-(2-methyl-5-m-tolyl-thiazol-4-yl)-methanone;
   {(1R*,2S*,5S*)-2-[(6,7-difluoro-quinoxalin-2-ylamino)-methyl]-3-aza-bicyclo[3.1.0]hex-3-yl}-[5-(4-fluoro-phenyl)-2-methyl-thiazol-4-yl]-methanone;
   {(1R*,2S*,5S*)-2-[(5-bromo-pyrimidin-2-ylamino)-methyl]-3-aza-bicyclo[3.1.0]hex-3-yl}-(2-methyl-5-m-tolyl-thiazol-4-yl)-methanone;
   naphthalene-2-carboxylic acid [(1R*,2S*,5S*)-3-(2-methyl-5-m-tolyl-thiazole-4-carbonyl)-3-aza-bicyclo[3.1.0]hex-2-ylmethyl]-amide;
   naphthalene-1-carboxylic acid [(1R*,2S*,5S*)-3-(2-methyl-5-m-tolyl-thiazole-4-carbonyl)-3-aza-bicyclo[3.1.0]hex-2-ylmethyl]-amide;
   2,3-dihydro-benzofuran-7-carboxylic acid [(1R*,2S*,5S*)-3-(2-methyl-5-m-tolyl-thiazole-4-carbonyl)-3-aza-bicyclo[3.1.0]hex-2-ylmethyl]-amide;
   1H-indole-5-carboxylic acid [(1R*,2S*,5S*)-3-(2-methyl-5-m-tolyl-thiazole-4-carbonyl)-3-aza-bicyclo[3.1.0]hex-2-ylmethyl]-amide;
   2-hydroxy-N-[(1R*,2S*,5S*)-3-(2-methyl-5-m-tolyl-thiazole-4-carbonyl)-3-aza-bicyclo[3.1.0]hex-2-ylmethyl]-benzamide;
   2-bromo-4-methyl-thiazole-5-carboxylic acid [(1R*,2S*,5S*)-3-(2-methyl-5-m-tolyl-thiazole-4-carbonyl)-3-aza-bicyclo[3.1.0]hex-2-ylmethyl]-amide;
   furan-3-carboxylic acid [(1R*,2S*,5S*)-3-(2-methyl-5-m-tolyl-thiazole-4-carbonyl)-3-aza-bicyclo[3.1.0]hex-2-ylmethyl]-amide;
   3,5-dimethyl-isoxazole-4-carboxylic acid [(1R*,2S*,5S*)-3-(2-methyl-5-m-tolyl-thiazole-4-carbonyl)-3-aza-bicyclo[3.1.0]hex-2-ylmethyl]-amide;
   3,5-dimethoxy-N-[(1R*,2S*,5S*)-3-(2-methyl-5-m-tolyl-thiazole-4-carbonyl)-3-aza-bicyclo[3.1.0]hex-2-ylmethyl]-benzamide;
   benzo[1,3]dioxole-5-carboxylic acid [(1R*,2S*,5S*)-3-(2-methyl-5-m-tolyl-thiazole-4-carbonyl)-3-aza-bicyclo[3.1.0]hex-2-ylmethyl]-amide;
   2,4-dimethoxy-N-[(1R*,2S*,5S*)-3-(2-methyl-5-m-tolyl-thiazole-4-carbonyl)-3-aza-bicyclo[3.1.0]hex-2-ylmethyl]-benzamide;
   2,4-dimethyl-thiazole-5-carboxylic acid [(1R*,2S*,5S*)-3-(2-methyl-5-m-tolyl-thiazole-4-carbonyl)-3-aza-bicyclo[3.1.0]hex-2-ylmethyl]-amide;
   1-methyl-1H-indole-2-carboxylic acid [(1R*,2S*,5S*)-3-(2-methyl-5-m-tolyl-thiazole-4-carbonyl)-3-aza-bicyclo[3.1.0]hex-2-ylmethyl]-amide;
   3H-benzoimidazole-5-carboxylic acid [(1R*,2S*,5S*)-3-(2-methyl-5-m-tolyl-thiazole-4-carbonyl)-3-aza-bicyclo[3.1.0]hex-2-ylmethyl]-amide;
   benzo[2,1,3]oxadiazole-5-carboxylic acid [(1R*,2S*,5S*)-3-(2-methyl-5-m-tolyl-thiazole-4-carbonyl)-3-aza-bicyclo[3.1.0]hex-2-ylmethyl]-amide;
   2,3-dihydro-benzo[1,4]dioxine-5-carboxylic acid [(1R*,2S*,5S*)-3-(2-methyl-5-m-tolyl-thiazole-4-carbonyl)-3-aza-bicyclo[3.1.0]hex-2-ylmethyl]-amide;
   5-chloro-1,3-dimethyl-1H-pyrazole-4-carboxylic acid [(1R*,2S*,5S*)-3-(2-methyl-5-m-tolyl-thiazole-4-carbonyl)-3-aza-bicyclo[3.1.0]hex-2-ylmethyl]-amide;
   benzo[b]thiophene-2-carboxylic acid [(1R*,2S*,5S*)-3-(2-methyl-5-m-tolyl-thiazole-4-carbonyl)-3-aza-bicyclo[3.1.0]hex-2-ylmethyl]-amide;
   5-*tert*-butyl-2-methyl-2H-pyrazole-3-carboxylic acid [(1R*,2S*,5S*)-3-(2-methyl-5-m-tolyl-thiazole-4-carbonyl)-3-aza-bicyclo[3.1.0]hex-2-ylmethyl]-amide;
   1-methyl-1H-indazole-3-carboxylic acid [(1R*,2S*,5S*)-3-(2-methyl-5-m-tolyl-thiazole-4-carbonyl)-3-aza-bicyclo[3.1.0]hex-2-ylmethyl]-amide;
   1-methyl-1H-pyrrole-2-carboxylic acid [(1R*,2S*,5S*)-3-(2-methyl-5-m-tolyl-thiazole-4-carbonyl)-3-aza-bicyclo[3.1.0]hex-2-ylmethyl]-amide;
   2,8-dimethyl-imidazo[1,2-a]pyridine-3-carboxylic acid [(1R*,2S*,5S*)-3-(2-methyl-5-m-tolyl-thiazole-4-carbonyl)-3-aza-bicyclo[3.1.0]hex-2-ylmethyl]-amide;
   6-isobutyl-5-methyl-N-[(1R*,2S*,5S*)-3-(2-methyl-5-m-tolyl-thiazole-4-carbonyl)-3-aza-bicyclo[3.1.0]hex-2-ylmethyl]-nicotinamide;
   pyrazolo[1,5-a]pyridine-3-carboxylic acid [(1R*,2S*,5S*)-3-(2-methyl-5-m-tolyl-thiazole-4-carbonyl)-3-aza-bicyclo[3.1.0]hex-2-ylmethyl]-amide;
   benzo[d]isoxazole-3-carboxylic acid [(1R*,2S*,5S*)-3-(2-methyl-5-m-tolyl-thiazole-4-carbonyl)-3-aza-bicyclo[3.1.0]hex-2-ylmethyl]-amide;
   6-fluoro-4H-benzo[1,3]dioxine-8-carboxylic acid [(1R*,2S*,5S*)-3-(2-methyl-5-m-tolyl-thiazole-4-carbonyl)-3-aza-bicyclo[3.1.0]hex-2-ylmethyl]-amide;
   2,3-dihydro-benzofuran-4-carboxylic acid [(1R*,2S*,5S*)-3-(2-methyl-5-m-tolyl-thiazole-4-carbonyl)-3-aza-bicyclo[3.1.0]hex-2-ylmethyl]-amide;
   isoquinoline-1-carboxylic acid [(1R*,2S*,5S*)-3-(2-methyl-5-m-tolyl-thiazole-4-carbonyl)-3-aza-bicyclo[3.1.0]hex-2-ylmethyl]-amide;
   quinoline-8-carboxylic acid [(1R*,2S*,5S*)-3-(2-methyl-5-m-tolyl-thiazole-4-carbonyl)-3-aza-bicyclo[3.1.0]hex-2-ylmethyl]-amide;
   quinoline-2-carboxylic acid [(1R*,2S*,5S*)-3-(2-methyl-5-m-tolyl-thiazole-4-carbonyl)-3-aza-bicyclo[3.1.0]hex-2-ylmethyl]-amide;
   imidazo[2,1-b]thiazole-5-carboxylic acid [(1R*,2S*,5S*)-3-(2-methyl-5-m-tolyl-thiazole-4-carbonyl)-3-aza-bicyclo[3.1.0]hex-2-ylmethyl]-amide;
   3-methyl-imidazo[2,1-b]thiazole-2-carboxylic acid [(1R*,2S*,5S*)-3-(2-methyl-5-m-tolyl-thiazole-4-carbonyl)-3-aza-bicyclo[3.1.0]hex-2-ylmethyl]-amide;
   1-methyl-1H-indole-3-carboxylic acid [(1R*,2S*,5S*)-3-(2-methyl-5-m-tolyl-thiazole-4-carbonyl)-3-aza-bicyclo[3.1.0]hex-2-ylmethyl]-amide;
   1H-indole-3-carboxylic acid [(1R*,2S*,5S*)-3-(2-methyl-5-m-tolyl-thiazole-4-carbonyl)-3-aza-bicyclo[3.1.0]hex-2-ylmethyl]-amide;
   1H-indazole-3-carboxylic acid [(1R*,2S*,5S*)-3-(2-methyl-5-m-tolyl-thiazole-4-carbonyl)-3-aza-bicyclo[3.1.0]hex-2-ylmethyl]-amide;
   imidazo[ 1,2-a]pyridine-3-carboxylic acid [(1R*,2S*,5S*)-3-(2-methyl-5-m-tolyl-thiazole-4-carbonyl)-3-aza-bicyclo[3.1.0]hex-2-ylmethyl]-amide;
   2,3-dihydro-thieno[3,4-b][1,4]dioxine-5-carboxylic acid [(1R*,2S*,5S*)-3-(2-methyl-5-m-tolyl-thiazole-4-carbonyl)-3-aza-bicyclo[3.1.0]hex-2-ylmethyl]-amide;
   3-bromo-N-[(1R*,2S*,5S*)-3-(2-methyl-5-m-tolyl-thiazole-4-carbonyl)-3-aza-bicyclo[3.1.0]hex-2-ylmethyl]-benzamide;
   N-[(1R*,2S*,5S*)-3-(2-methyl-5-m-tolyl-thiazole-4-carbonyl)-3-aza-bicyclo[3.1.0]hex-2-ylmethyl]-3-trifluoromethyl-benzamide;
   3-methoxy-N-[(1R*,2S*,5S*)-3-(2-methyl-5-m-tolyl-thiazole-4-carbonyl)-3-aza-bicyclo[3.1.0]hex-2-ylmethyl]-benzamide;
   3-fluoro-4-methoxy-N-[(1R*,2S*,5S*)-3-(2-methyl-5-m-tolyl-thiazole-4-carbonyl)-3-aza-bicyclo[3.1.0]hex-2-ylmethyl]-benzamide;
   3,4-dichloro-N-[(1R*,2S*,5S*)-3-(2-methyl-5-m-tolyl-thiazole-4-carbonyl)-3-aza-bicyclo[3.1.0]hex-2-ylmethyl]-benzamide;
   2-chloro-4,5-difluoro-N-[(1R*,2S*,5S*)-3-(2-methyl-5-m-tolyl-thiazole-4-carbonyl)-3-aza-bicyclo[3.1.0]hex-2-ylmethyl]-benzamide;
   2-fluoro-5-methyl-N-[(1R*,2S*,5S*)-3-(2-methyl-5-m-tolyl-thiazole-4-carbonyl)-3-aza-bicyclo[3.1.0]hex-2-ylmethyl]-benzamide;
   3-fluoro-2-methyl-N-[(1R*,2S*,5S*)-3-(2-methyl-5-m-tolyl-thiazole-4-carbonyl)-3-aza-bicyclo[3.1.0]hex-2-ylmethyl]-benzamide;
   5-fluoro-2-methoxy-N-[(1R*,2S*,5S*)-3-(2-methyl-5-m-tolyl-thiazole-4-carbonyl)-3-aza-bicyclo[3.1.0]hex-2-ylmethyl]-benzamide;
   2-chloro-3-fluoro-N-[( 1R*,2S*,5S*)-3-(2-methyl-5-m-tolyl-thiazole-4-carbonyl)-3-aza-bicyclo[3.1.0]hex-2-ylmethyl]-benzamide;
   2,5-dimethyl-N-[(1R*,2S*,5S*)-3-(2-methyl-5-m-tolyl-thiazole-4-carbonyl)-3-aza-bicyclo[3.1.0]hex-2-ylmethyl]-benzamide;
   3,4-dimethyl-N-[(1R*,2S*-,5S*)-3-(2-methyl-5-m-tolyl-thiazole-4-carbonyl)-3-aza bicyclo[3.1.0]hex-2-ylmethyl]-benzamide;
   2,5-dimethoxy-N-[(1R*,2S*,5S*)-3-(2-methyl-5-m-tolyl-thiazole-4-carbonyl)-3-aza-bicyclo[3.1.0]hex-2-ylmethyl]-benzamide;
   N-[(1R*,2S*,5S*)-3-(2-methyl-5-m-tolyl-thiazole-4-carbonyl)-3-aza-bicyclo[3.1.0]hex-2-ylmethyl]-isophthalamic acid methyl ester;
   2-chloro-4-fluoro-N-[(1R*,2S*,5S*)-3-(2-methyl-5-m-tolyl-thiazole-4-carbonyl)-3-aza-bicyclo[3.1.0]hex-2-ylmethyl]-benzamide;
   2-chloro-3-methyl-N-[(1R*,2S*,5S*)-3-(2-methyl-5-m-tolyl-thiazole-4-carbonyl)-3-aza-bicyclo[3.1.0]hex-2-ylmethyl]-benzamide;
   3,5-dichloro-4-hydroxy-N-[(1R*,2S*,5S*)-3-(2-methyl-5-m-tolyl-thiazole-4-carbonyl)-3-aza-bicyclo[3.1.0]hex-2-ylmethyl]-benzamide;
   2,4-dichloro-N-[(1R*,2S*,5S*)-3-(2-methyl-5-m-tolyl-thiazole-4-carbonyl)-3-aza-bicyclo[3.1.0]hex-2-ylmethyl]-benzamide;
   4-methyl-N-[(1R*,2S*,5S*)-3-(2-methyl-5-m-tolyl-thiazole-4-carbonyl)-3-aza-bicyclo[3.1.0]hex-2-ylmethyl]-3-trifluoromethyl-benzamide;
   4-methoxy-2-methyl-N-[(1R*,2S*,5S*)-3-(2-methyl-5-m-tolyl-thiazole-4-carbonyl)-3-aza-bicyclo[3.1.0]hex-2-ylmethyl]-benzamide;
   4-ethyl-N-[(1R*;2S*,5S*)-3-(2-methyl-5-m-tolyl-thiazole-4-carbonyl)-3-aza-bicyclo[3.1.0]hex-2-ylmethyl]-benzamide;
   4-methoxy-3-methyl-N-[(1R*,2S*,5S*)-3-(2-methyl-5-m-tolyl-thiazole-4-carbonyl)-3-aza-bicyclo[3.1.0]hex-2-ylmethyl]-benzamide;
   3,5-dimethyl-N-[(1R*,2S*,5S*)-3-(2-methyl-5-m-tolyl-thiazole-4-carbonyl)-3-aza-bicyclo[3.1.0]hex-2-ylmethyl]-benzamide;
   5-bromo-2-chloro-N-[(1R*,2S*,5S*)-3-(2-methyl-5-m-tolyl-thiazole-4-carbonyl)-3-aza-bicyclo[3.1.0]hex-2-ylmethyl]-benzamide;
   3-cyano-N-[(1R*,2S*,5S*)-3-(2-methyl-5-m-tolyl-thiazole-4-carbonyl)-3-aza-bicyclo[3.1.0]hex-2-ylmethyl]-benzamide;
   4-cyano-N-[(1R*,2S*,5S*)-3-(2-methyl-5-m-tolyl-thiazole-4-carbonyl)-3-aza-bicyclo[3.1.0]hex-2-ylmethyl]-benzamide;
   4-chloro-N-[(1R*,2S*,5S*)-3-(2-methyl-5-m-tolyl-thiazole-4-carbonyl)-3-aza-bicyclo[3.1.0]hex-2-ylmethyl]-benzamide;
   3-iodo-N-[(1R*,2S*,5S*)-3-(2-methyl-5-m-tolyl-thiazole-4-carbonyl)-3-aza-bicyclo[3.1.0]hex-2-ylmethyl]-benzamide;
   2-bromo-3-methyl-N-[(1R*,2S*,5S*)-3-(2-methyl-5-m-tolyl-thiazole-4-carbonyl)-3-aza-bicyclo[3.1.0]hex-2-ylmethyl]-benzamide;
   5-methyl-imidazo[2,1-b]thiazole-6-carboxylic acid [(1R*,2S*,5S*)-3-(2-methyl-5-m-tolyl-thiazole-4-carbonyl)-3-aza-bicyclo[3.1.0]hex-2-ylmethyl]-amide;
   3,5-dimethyl-imidazo[2,1-b]thiazole-6-carboxylic acid [(1R*,2S*,5S*)-3-(2-methyl-5-m-tolyl-thiazole-4-carbonyl)-3-aza-bicyclo[3.1.0]hex-2-ylmethyl]-amide;
   2,6-dimethyl-imidazo[2,1-b]thiazole-5-carboxylic acid [(1R*,2S*,5S*)-3-(2-methyl-5-m-tolyl-thiazole-4-carbonyl)-3-aza-bicyclo[3.1.0]hex-2-ylmethyl]-amide;
   2,3,5-trimethyl-imidazo[2,1-b]thiazole-6-carboxylic acid [(1R*,2S*,5S*)-3-(2-methyl-5-m-tolyl-thiazole-4-carbonyl)-3-aza-bicyclo[3.1.0]hex-2-ylmethyl]-amide;
   2-methyl-imidazo[2,1-b]thiazole-5-carboxylic acid [(1R*,2S*,5S*)-3-(2-methyl-5-m-tolyl-thiazole-4-carbonyl)-3-aza-bicyclo[3.1.0]hex-2-ylmethyl]-amide;
   6-trifluoromethyl-imidazo[2,1-b]thiazole-5-carboxylic acid [(1R*,2S*,5S*)-3-(2-methyl-5-m-tolyl-thiazole-4-carbonyl)-3-aza-bicyclo[3.1.0]hex-2-ylmethyl]-amide;
   3,6-dimethyl-imidazo[2,1-b]thiazole-5-carboxylic acid [(1R*,2S*,5S*)-3-(2-methyl-5-m-tolyl-thiazole-4-carbonyl)-3-aza-bicyclo[3.1.0]hex-2-ylmethyl]-amide;
   3-methyl-imidazo[2,1-b]thiazole-5-carboxylic acid [(1R*,2S*,5S*)-3-(2-methyl-5-m-tolyl-thiazole-4-carbonyl)-3-aza-bicyclo[3.1.0]hex-2-ylmethyl]-amide;
   6-chloro-imidazo[2,1-b]thiazole-5-carboxylic acid [(1R*,2S*,5S*)-3-(2-methyl-5-m-tolyl-thiazole-4-carbonyl)-3-aza-bicyclo[3.1.0]hex-2-ylmethyl]-amide;
   2H-chromene-5-carboxylic acid [(1R*,2S*,5S*)-3-(2-methyl-5-m-tolyl-thiazole-4-carbonyl)-3-aza-bicyclo[3.1.0]hex-2-ylmethyl]-amide;
   4-methyl-3,4-dihydro-2H-benzo[1,4]oxazine-8-carboxylic acid [(1R*,2S*,5S*)-3-(2-methyl-5-m-tolyl-thiazole-4-carbonyl)-3-aza-bicyclo[3.1.0]hex-2-ylmethyl]-amide;
   chroman-8-carboxylic acid [(1R*,2S*,5S*)-3-(2-methyl-5-m-tolyl-thiazole-4-carbonyl)-3-aza-bicyclo[3.1.0]hex-2-ylmethyl]-amide;
   3-oxo-3,4-dihydro-2H-benzo[1,4]oxazine-5-carboxylic acid [(1R*,2S*,5S*)-3-(2-methyl-5-m-tolyl-thiazole-4-carbonyl)-3-aza-bicyclo[3.1.0]hex-2-ylmethyl]-amide;
   3,4-dihydro-2H-benzo[1,4]oxazine-8-carboxylic acid [(1R*,2S*,5S*)-3-(2-methyl-5-m-tolyl-thiazole-4-carbonyl)-3-aza-bicyclo[3.1.0]hex-2-ylmethyl]-amide;
   3,4-dihydro-2H-benzo[1,4]oxazine-5-carboxylic acid [(1R*,2S*,5S*)-3-(2-methyl-5-m-tolyl-thiazole-4-carbonyl)-3-aza-bicyclo[3.1.0]hex-2-ylmethyl]-amide;
   2-methyl-benzofuran-4-carboxylic acid [(1R*,2S*,5S*)-3-(2-methyl-5-m-tolyl-thiazole-4-carbonyl)-3-aza-bicyclo[3.1.0]hex-2-ylmethyl]-amide;
   benzo[d]isoxazole-3-carboxylic acid {(1R*,2S*,5S*)-3-[5-(3-chloro-phenyl)-2-methyl-thiazole-4-carbonyl]-3-aza-bicyclo[3.1.0]hex-2-ylmethyl}-amide;
   2,3-dihydro-benzo[1,4]dioxine-5-carboxylic acid {(1R*,2S*,5S*)-3-[5-(3-chlorophenyl)-2-methyl-thiazole-4-carbonyl]-3-aza-bicyclo[3.1.0]hex-2-ylmethyl}-amide;
   6-fluoro-4H-benzo[1,3]dioxine-8-carboxylic acid {(1R*,2S*,5S*)-3-[5-(3-chlorophenyl)-2-methyl-thiazole-4-carbonyl]-3-aza-bicyclo[3.1.0]hex-2-ylmethyl}-amide;
   2,3-dihydro-benzofuran-4-carboxylic acid {(1R*,2S*,5S*)-3-[5-(3-chloro-phenyl)-2-methyl-thiazole-4-carbonyl]-3-aza-bicyclo[3.1.0]hex-2-ylmethyl}-amide;
   isoquinoline-1-carboxylic acid {(1R*,2S*,5S*)-3-[5-(3-chloro-phenyl)-2-methylthiazole-4-carbonyl]-3-aza-bicyclo[3.1.0]hex-2-ylmethyl }-amide;
   quinoline-8-carboxylic acid {(1R*,2S*,5S*)-3-[5-(3-chloro-phenyl)-2-methylthiazole-4-carbonyl]-3-aza-bicyclo[3.1.0]hex-2-ylmethyl}-amide;
   quinoline-2-carboxylic acid {(1R*,2S*,5S*)-3-[5-(3-chloro-phenyl)-2-methylthiazole-4-carbonyl]-3-aza-bicyclo[3.1.0]hex-2-ylmethyl }-amide;
   imidazo[2,1-b]thiazole-5-carboxylic acid {(1R*,2S*,5S*)-3-[5-(3-chloro-phenyl)-2-methyl-thiazole-4-carbonyl]-3-aza-bicyclo[3.1.0]hex-2-ylmethyl -amide;
   3-methyl-imidazo[2,1-b]thiazole-2-carboxylic acid {(1R*,2S*,5S*)-3-[5-(3-chlorophenyl)-2-methyl-thiazole-4-carbonyl]-3-aza-bicyclo[3.1.0]hex-2-ylmethyl}-amide;
   1-methyl-1H-indole-3-carboxylic acid {(1R*,2S*,5S*)-3-[5-(3-chloro-phenyl)-2-methyl-thiazole-4-carbonyl]-3-aza-bicyclo[3.1.0]hex-2-ylmethyl}-amide;
   1,2-dimethyl-1H-indole-3-carboxylic acid {(1R*,2S*,5S*)-3-[5-(3-chloro-phenyl)-2-methyl-thiazole-4-carbonyl]-3-aza-bicyclo[3.1.0]hex-2-ylmethyl -amide;
   1H-indole-3-carboxylic acid {(1R*,2S*,5S*)-3-[5-(3-chloro-phenyl)-2-methylthiazole-4-carbonyl]-3-aza-bicyclo[3.1.0]hex-2-ylmethyl}-amide;
   1H-indazole-3-carboxylic acid {(1R*,2S*,5S*)-3-[5-(3-chloro-phenyl)-2-methylthiazole-4-carbonyl]-3-aza-bicyclo[3.1.0]hex-2-ylmethyl}-amide;
   imidazo[1,2-a]pyridine-3-carboxylic acid {(1R*,2S*,5S*)-3-[5-(3-chloro-phenyl)-2-methyl-thiazole-4-carbonyl]-3-aza-bicyclo[3.1.0]hex-2-ylmethyl -amide;
   2,5-dimethyl-2H-pyrazole-3-carboxylic acid {(1R*,2S*,5S*)-3-[5-(3-chloro-phenyl)-2-methyl-thiazole-4-carbonyl]-3-aza-bicyclo[3.1.0]hex-2-ylmethyl}-amide;
   2-ethyl-5-methyl-2H-pyrazole-3-carboxylic acid {(1R*,2S*,5S*)-3-[5-(3-chlorophenyl)-2-methyl-thiazole-4-carbonyl]-3-aza-bicyclo[3.1.0]hex-2-ylmethyl}-amide;
   2,3-dihydro-thieno[3,4-b][1,4]dioxine-5-carboxylic acid {(1R*,2S*,5S*)-3-[5-(3-chloro-phenyl)-2-methyl-thiazole-4-carbonyl]-3-aza-bicyclo[3.1.0]hex-2-ylmethyl}-amide;
   3-bromo-N-{(1R*,2S*,5S*)-3-[5-(3-chloro-phenyl)-2-methyl-thiazole-4-carbonyl]-3-aza-bicyclo[3.1.0]hex-2-ylmethyl} -benzamide;
   N-{(1R*,2S*,5S*)-3-[5-(3-chloro-phenyl)-2-methyl-thiazole-4-carbonyl]-3-aza-bicyclo[3.1.0]hex-2-ylmethyl}-3-trifluoromethyl-benzamide;
   N-{(1R*,2S*,5S*)-3-[5-(3-chloro-phenyl)-2-methyl-thiazole-4-carbonyl]-3-aza-bicyclo[3.1.0]hex-2-ylmethyl}-3-methoxy-benzamide;
   3-Methyl-imidazo[2,1-b]thiazole-5-carboxylic acid {(1R*,2S*,5S*)-3-[5-(3-fluorophenyl)-2-methyl-thiazole-4-carbonyl]-3-aza-bicyclo[3.1.0]hex-2-ylmethyl}-amide;
   2-methyl-imidazo[2,1-b]thiazole-5-carboxylic acid {(1R*,2S*,5S*)-3-[5-(3-fluorophenyl)-2-methyl-thiazole-4-carbonyl]-3-aza-bicyclo[3.1.0]hex-2-ylmethyl}-amide;
   imidazo[2,1-b]thiazolc-5-carboxylic acid {(1R*,2S*,5S*)-3-[5-(3-fluoro-phenyl)-2-methyl-thiazole-4-carbonyl]-3-aza-bicyclo[3.1.0]hex-2-ylmethyl}-amide;
   1-methyl-1H-indole-3-carboxylic acid {(1R*,2S*,5S*)-3-[5-(3-fluoro-phenyl)-2-methyl-thiazole-4-carbonyl]-3-aza-bicyclo[3.1.0]hex-2-ylmethyl} -amide;
   3-methyl-imidazo[2,1-b]thiazole-2-carboxylic acid {(1R*,2S*,5S*)-3-[5-(3-fluorophenyl)-2-methyl-thiazole-4-carbonyl]-3-aza-bicyclo[3.1.0]hex-2-ylmethyl}-amide;
   1-ethyl-3-methyl-1H-pyrazole-4-carboxylic acid {(1R*,2S*,5S*)-3-[5-(3-fluorophenyl)-2-methyl-thiazole-4-carbonyl]-3-aza-bicyclo[3.1.0]hex-2-ylmethyl}-amide;
   5-tert-butyl-2-methyl-2H-pyrazole-3-carboxylic acid {(1R*,2S*,5S*)-3-[5-(3-fluorophenyl)-2-methyl-thiazole-4-carbonyl]-3-aza-bicyclo[3.1.0]hex-2-ylmethyl}-amide;
   isoquinoline-1-carboxylic acid {(1R*,2S*,5S*)-3-[5-(3-fluoro-phenyl)-2-methylthiazole-4-carbonyl]-3-aza-bicyclo[3.1.0]hex-2-ylmethyl}-amide;
   1H-indazole-3-carboxylic acid {(1R*,2S*,5S*)-3-[5-(3-fluoro-phenyl)-2-methylthiazole-4-carbonyl]-3-aza-bicyclo[3.1.0]hex-2-ylmethyl}-amide;
   4-methoxy-quinoline-2-carboxylic acid {(1R*,2S*,5S*)-3-[5-(3-fluoro-phenyl)-2-methyl-thiazole-4-carbonyl]-3-aza-bicyclo[3.1.0]hex-2-ylmethyl}-amide;
   quinoline-2-carboxylic acid {(1R*,2S*,5S*)-3-[5-(3-fluoro-phenyl)-2-methylthiazole-4-carbonyl]-3-aza-bicyclo[3.1.0]hex-2-ylmethyl}-amide;
   1H-indole-3-carboxylic acid {(1R*,2S*,5S*)-3-[5-(3-fluoro-phenyl)-2-methylthiazole-4-carbonyl]-3-aza-bicyclo[3.1.0]hex-2-ylmethyl}-amide;
   6-fluoro-4H-benzo[1,3]dioxine-8-carboxylic acid {(1R*,2S*,5S*)-3-[5-(3-fluorophenyl)-2-methyl-thiazole-4-carbonyl]-3-aza-bicyclo[3.1.0]hex-2-ylmethyl} -amide;
   benzo[d]isoxazole-3-carboxylic acid {(1R*,2S*,5S*)-3-[5-(3-fluoro-phenyl)-2-methyl-thiazole-4-carbonyl]-3-aza-bicyclo[3.1.0]hex-2-ylmethyl}-amide;
   benzo[1,3]dioxole-4-carboxylic acid {(1R*,2S*,5S*)-3-[5-(3-fluoro-phenyl)-2-methyl-thiazole-4-carbonyl]-3-aza-bicyclo[3.1.0]hex-2-ylmethyl}-amide;
   2-ethyl-5-methyl-2H-pyrazole-3-carboxylic acid {(1R*,2S*,5S*)-3-[5-(3-fluorophenyl)-2-methyl-thiazole-4-carbonyl]-3-aza-bicyclo[3.1.0]hex-2-ylmethyl}-amide;
   imidazo[1,2-a]pyridine-3-carboxylic acid {(1R*,2S*,5S*)-3-[5-(3-fluoro-phenyl)-2-methyl-thiazole-4-carbonyl]-3-aza-bicyclo[3.1.0]hex-2-ylmethyl}-amide;
   1-methyl-5-trifluoromethyl-1H-pyrazole-4-carboxylic acid {(1R*,2S*,5S*)-3-[5-(3-fluoro-phenyl)-2-methyl-thiazole-4-carbonyl]-3-aza-bicyclo[3.1.0]hex-2-ylmethyl}-amide;
   1,5-dimethyl-1H-pyrazole-3-carboxylic acid {(1R*,2S*,5S*)-3-[5-(3-fluoro-phenyl)-2-methyl-thiazole-4-carbonyl]-3-aza-bicyclo[3.1.0]hex-2-ylmethyl}-amide;
   2,5-dimethyl-2H-pyrazole-3-carboxylic acid {(1R*,2S*,5S*)-3-[5-(3-fluoro-phenyl)-2-methyl-thiazole-4-carbonyl]-3-aza-bicyclo[3.1.0]hex-2-ylmethyl}-amide;
   2,5-dimethyl-oxazole-4-carboxylic acid {(1R*,2S*,5S*)-3-[5-(3-fluoro-phenyl)-2-methyl-thiazole-4-carbonyl]-3-aza-bicyclo[3.1.0]hex-2-ylmethyl}-amide;
   2,3-dihydro-benzo[1,4]dioxine-5-carboxylic acid {(1R*,2S*,5S*)-3-[5-(3-fluorophenyl)-2-methyl-thiazole-4-carbonyl]-3-aza-bicyclo[3.1.0]hex-2-ylmethy}-amide;
   2,3-dihydro-benzofuran-4-carboxylic acid {(1R*,2S*,5S*)-3-[5-(3-fluoro-phenyl)-2-methyl-thiazole-4-carbonyl]-3-aza-bicyclo[3.1.0]hex-2-ylmethyl}-amide;
   1,3-dimethyl-1H-pyrazole-4-carboxylic acid {(1R*,2S*,5S*)-3-[5-(3-fluoro-phenyl)-2-methyl-thiazole-4-carbonyl]-3-aza-bicyclo[3.1.0]hex-2-ylmethyl}-amide;
   7-fluoro-1H-indole-2-carboxylic acid {(1R*,2S*,5S*)-3-[5-(3-fluoro-phenyl)-2-methyl-thiazole-4-carbonyl]-3-aza-bicyclo[3.1.0]hex-2-ylmethyl}-amide;
   2-trifluoromethyl-1H-benzoimidazole-5-carboxylic acid {(1R*,2S*,5S*)-3-[5-(3-fluoro-phenyl)-2-methyl-thiazole-4-carbonyl]-3-aza-bicyclo[3.1.0]hex-2-ylmethyl}-amide;
   3-bromo-N-{(1R*,2S*,5S*)-3-[5-(3-fluoro-phenyl)-2-methyl-thiazole-4-carbonyl]-3-aza-bicyclo[3.1.0]hex-2-ylmethyl}-benzamide;
   N-{(1R*,2S*,5S*)-3-[5-(3-fluoro-phenyl)-2-methyl-thiazole-4-carbonyl]-3-aza-bicyclo[3.1.0]hex-2-ylmethyl}-3-trifluoromethyl-benzamide;
   benzo[d]isoxazole-3-carboxylic acid [(1R*,2S*,5S*)-3-(2-amino-5-m-tolyl-thiazole-4-carbonyl)-3-aza-bicyclo[3.1.0]hex-2-ylmethyl]-amide;
   2,3-dihydro-benzo[1,4]dioxine-5-carboxylic acid [(1R*,2S*,5S*)-3-(2-amino-5-m-tolyl-thiazole-4-carbonyl)-3-aza-bicyclo[3.1.0]hex-2-ylmethyl]-amide;
   6-fluoro-4H-benzo[1,3]dioxine-8-carboxylic acid [(1R*,2S*,5S*)-3-(2-amino-5-m-tolyl-thiazole-4-carbonyl)-3-aza-bicyclo[3.1.0]hex-2-ylmethyl]-amide;
   2,3-dihydro-benzofuran-4-carboxylic acid [(1R*,2S*,5S*)-3-(2-amino-5-m-tolyl-thiazole-4-carbonyl)-3-aza-bicyclo[3.1.0]hex-2-ylmethyl]-amide;
   2,2-dimethyl-2,3-dihydro-benzofuran-7-carboxylic acid [(1R*,2S*,5S*)-3-(2-amino-5-m-tolyl-thiazole-4-carbonyl)-3-aza-bicyclo[3.1.0]hex-2-ylmethyl]-amide;
   isoquinoline-1-carboxylic acid [(1R*,2S*,5S*)-3-(2-amino-5-m-tolyl-thiazole-4-carbonyl)-3-aza-bicyclo[3.1.0]hex-2-ylmethyl]-amide;
   quinoline-8-carboxylic acid [(1R*,2S*,5S*)-3-(2-amino-5-m-tolyl-thiazole-4-carbonyl)-3-aza-bicyclo[3.1.0]hex-2-ylmethyl]-amide;
   imidazo[2,1-b]thiazole-5-carboxylic acid [(1R*,2S*,5S*)-3-(2-amino-5-m-tolyl-thiazole-4-carbonyl)-3-aza-bicyclo[3.1.0]hex-2-ylmethyl]-amide;
   3-methyl-imidazo[2,1-b]thiazole-2-carboxylic acid [(1R*,2S*,5S*)-3-(2-amino-5-m-tolyl-thiazole-4-carbonyl)-3-aza-bicyclo[3.1.0]hex-2-ylmethyl]-amide;
   1-methyl-1H-indole-3-carboxylic acid [(1R*,2S*,5S*)-3-(2-amino-5-m-tolyl-thiazole-4-carbonyl)-3-aza-bicyclo[3.1.0]hex-2-ylmethyl]-amide;
   1H-indole-3-carboxylic acid [(1R*,2S*,5S*)-3-(2-amino-5-m-tolyl-thiazole-4-carbonyl)-3-aza-bicyclo[3.1.0]hex-2-ylmethyl]-amide;
   1H-indazole-3-carboxylic acid [(1R*,2S*,5S*)-3-(2-amino-5-m-tolyl-thiazole-4-carbonyl)-3-aza-bicyclo[3.1.0]hex-2-ylmethyl]-amide;
   imidazo[1,2-a]pyridine-3-carboxylic acid [(1R*,2S*,5S*)-3-(2-amino-5-m-tolyl-thiazole-4-carbonyl)-3-aza-bicyclo[3.1.0]hex-2-ylmethyl]-amide;
   2-ethyl-5-methyl-2H-pyrazole-3-carboxylic acid [(1R*,2S*,5S*)-3-(2-amino-5-m-tolyl-thiazole-4-carbonyl)-3-aza-bicyclo[3.1.0]hex-2-ylmethyl]-amide;
   1-ethyl-3-methyl-1H-pyrazole-4-carboxylic acid [(1R*,2S*,5S*)-3-(2-amino-5-m-tolyl-thiazole-4-carbonyl)-3-aza-bicyclo[3.1.0]hex-2-ylmethyl]-amide;
   2,3-dihydro-thieno[3,4-b][1,4]dioxine-5-carboxylic acid [(1R*,2S*,5S*)-3-(2-amino-5-m-tolyl-thiazole-4-carbonyl)-3-aza-bicyclo[3.1.0]hex-2-ylmethyl]-amide;
   N-[(1R*,2S*,5S*)-3-(2-amino-5-m-tolyl-thiazole-4-carbonyl)-3-aza-bicyclo[3.1.0]hex-2-ylmethyl]-3-bromo-benzamide;
   N-[(1R*,2S*,5S*)-3-(2-amino-5-m-tolyl-thiazole-4-carbonyl)-3-aza-bicyclo[3.1.0]hex-2-ylmethyl]-3-methoxy-benzamide;
   benzo[d]isoxazole-3-carboxylic acid {(1R*,2S*,5S*)-3-[2-amino-5-(3-chlorophenyl)-thiazole-4-carbonyl]-3-aza-bicyclo[3.1.0]hex-2-ylmethyl}-amide;
   2,3-dihydro-benzo[1,4]dioxine-5-carboxylic acid {(1R*,2S*,5S*)-3-[2-amino-5-(3-chloro-phenyl)-thiazole-4-carbonyl]-3-aza-bicyclo[3.1.0]hex-2-ylmethyl}-amide;
   6-fluoro-4H-benzo[1,3]dioxine-8-carboxylic acid {(1R*,2S*,5S*)-3-[2-amino-5-(3-chloro-phenyl)-thiazole-4-carbonyl]-3-aza-bicyclo[3.1.0]hex-2-ylmethyl}-amide;
   2,3-dihydro-benzofuran-4-carboxylic acid {(1R*,2S*,5S*)-3-[2-amino-5-(3-chlorophenyl)-thiazole-4-carbonyl]-3-aza-bicyclo[3.1.0]hex-2-ylmethyl}-amide;
   isoquinoline-1-carboxylic acid {(1R*,2S*,5S*)-3-[2-amino-5-(3-chloro-phenyl)-thiazole-4-carbonyl]-3-aza-bicyclo[3.1.0]hex-2-ylmethyl -amide;
   quinoline-8-carboxylic acid {(1R*,2S*,5S*)-3-[2-amino-5-(3-chloro-phenyl)-thiazole-4-carbonyl]-3-aza-bicyclo[3.1.0]hex-2-ylmethyl}-amide;
   imidazo[2,1-b]thiazole-5-carboxylic acid {(1R*,2S*,5S*)-3-[2-amino-5-(3-chlorophenyl)-thiazole-4-carbonyl]-3-aza-bicyclo[3.1.0]hex-2-ylmethyl}-amide;
   1-methyl-1H-indole-3-carboxylic acid {(1R*,2S*,5S*)-3-[2-amino-5-(3-chlorophenyl)-thiazole-4-carbonyl]-3-aza-bicyclo[3.1.0]hex-2-ylmethyl}-amide;
   1H-indole-3-carboxylic acid {(1R*,2S*,5S*)-3-[2-amino-5-(3-chloro-phenyl)-thiazole-4-carbonyl]-3-aza-bicyclo[3.1.0]hex-2-ylmethyl}-amide;
   1H-indazole-3-carboxylic acid {(1R*,2S*,5S*)-3-[2-amino-5-(3-chloro-phenyl)-thiazole-4-carbonyl]-3-aza-bicyclo[3.1.0]hex-2-ylmethyl}-amide;
   2,5-dimethyl-2H-pyrazole-3-carboxylic acid {(1R*,2S*,5S*)-3-[2-amino-5-(3-chlorophenyl)-thiazole-4-carbonyl]-3-aza-bicyclo[3.1.0]hex-2-ylmethyl}-amide;
   2-ethyl-5-methyl-2H-pyrazole-3-carboxylic acid {(1R*,2S*,5S*)-3-[2-amino-5-(3-chloro-phenyl)-thiazole-4-carbonyl]-3-aza-bicyclo[3.1.0]hex-2-ylmethyl}-amide;
   2,3-dihydro-thieno[3,4-b][1,4]dioxine-5-carboxylic acid {(1R*,2S*,5S*)-3-[2-amino-5-(3-chloro-phenyl)-thiazole-4-carbonyl]-3-aza-bicyclo[3.1.0]hex-2-ylmethyl}-amide;
   N-{(1R*,2S*,5S*)-3-[2-amino-5-(3-chloro-phenyl)-thiazole-4-carbonyl]-3-aza-bicyclo[3.1.0]hex-2-ylmethyl}-3-bromo-benzamide;
   N-{(1R*,2S*,5S*)-3-[2-amino-5-(3-chloro-phenyl)-thiazole-4-carbonyl]-3-aza-bicyclo[3.1.0]hex-2-ylmethyl}-3-methoxy-benzamide;
   6-methyl-imidazo[2,1-b]thiazole-5-carboxylic acid [(1R*,2S*,5S*)-3-(3'-fluoro-5-methyl-biphenyl-2-carbonyl)-3-aza-bicyclo[3.1.0]hex-2-ylmethyl]-amide;
   imidazo[1,2-a]pyridine-3-carboxylic acid [(1R*,2S*,5S*)-3-(3'-fluoro-5-methylbiphenyl-2-carbonyl)-3-aza-bicyclo[3.1.0]hex-2-ylmethyl]-amide;
   2,3-dihydro-benzofuran-4-carboxylic acid [(1R*,2S*,5S*)-3-(3'-fluoro-5-methylbiphenyl-2-carbonyl)-3-aza-bicyclo[3.1.0]hex-2-ylmethyl]-amide;
   2,3-dihydro-benzofuran-7-carboxylic acid [(1R*,2S*,5S*)-3-(3'-fluoro-5-methylbiphenyl-2-carbonyl)-3-aza-bicyclo[3.1.0]hex-2-ylmethyl]-amide;
   6-methyl-imidazo[2,1-b]thiazole-5-carboxylic acid {(1R*,2S*,5S*)-3-[5-(3-cyanophenyl)-2-methyl-thiazole-4-carbonyl]-3-aza-bicyclo[3.1.0]hex-2-ylmethyl}-amide;
   benzofuran-4-carboxylic acid {(1R*,2S*,5S*)-3-[5-(3-cyano-phenyl)-2-methylthiazole-4-carbonyl]-3-aza-bicyclo[3.1.0]hex-2-ylmethyl}-amide;
   2,3-dihydro-benzo[1,4]dioxine-5-carboxylic acid {(1R*,2S*,5S*)-3-[5-(3-cyanophenyl)-2-methyl-thiazole-4-carbonyl]-3-aza-bicyclo[3.1.0]hex-2-ylmethyl}-amide;
   2,3-dihydro-benzo[1,4]dioxine-5-carboxylic acid [(1R*,2S*,5S*)-3-(5-methylbiphenyl-2-carbonyl)-3-aza-bicyclo[3.1.0]hex-2-ylmethyl]-amide;
   2,3-dihydro-benzo[1,4]dioxine-5-carboxylic acid [(1R*,2S*,5S*)-3-(2'-fluoro-5-methyl-biphenyl-2-carbonyl)-3-aza-bicyclo[3.1.0]hex-2-ylmethyl]-amide;
   2,3-dihydro-benzo[1,4]dioxine-5-carboxylic acid [(1R*,2S*,5S*)-3-(5,3'-dimethylbiphenyl-2-carbonyl)-3-aza-bicyclo[3.1.0]hex-2-ylmethyl]-amide;
   2,3-dihydro-benzo[1,4]dioxine-5-carboxylic acid [(1R*,2S*,5S*)-3-(3'-chloro-5-methyl-biphenyl-2-carbonyl)-3-aza-bicyclo[3.1.0]hex-2-ylmethyl]-amide;
   2,3-dihydro-benzo[1,4]dioxine-5-carboxylic acid [(1R*,2S*,5S*)-3-(3'-methoxy-5-methyl-biphenyl-2-carbonyl)-3-aza-bicyclo[3.1.0]hex-2-ylmethyl]-amide;
   2,3-dihydro-benzo[1,4]dioxine-5-carboxylic acid [(1R*,2S*,5S*)-3-(5-methyl-3'-trifluoromethyl-biphenyl-2-carbonyl)-3-aza-bicyclo[3.1.0]hex-2-ylmethyl]-amide;
   2,3-dihydro-benzo[1,4]dioxine-5-carboxylic acid [(1R*,2S*,5S*)-3-(3'-fluoro-5-methyl-biphenyl-2-carbonyl)-3-aza-bicyclo[3.1.0]hex-2-ylmethyl]-amide;
   2,3-dihydro-benzo[1,4]dioxine-5-carboxylic acid [(1R*,2S*,5S*)-3-(5,4'-dimethylbiphenyl-2-carbonyl)-3-aza-bicyclo[3.1.0]hex-2-ylmethyl]-amide;
   2,3-dihydro-benzo[1,4]dioxine-5-carboxylic acid [(1R*,2S*,5S*)-3-(4'-fluoro-5,3'-dimethyl-biphenyl-2-carbonyl)-3-aza-bicyclo[3.1.0]hex-2-ylmethyl]-amide;
   2,3-dihydro-benzo[1,4]dioxine-5-carboxylic acid {(1R*,2S*,5S*)-3-[4-methyl-2-(4-methyl-thiophen-2-yl)-benzoyl]-3-aza-bicyclo[3.1.0]hex-2-ylmethyl}-amide;
   2,3-dihydro-benzo[1,4]dioxine-5-carboxylic acid [(1R*,2S*,5S*)-3-(4'-fluoro-4,3'-dimethyl-biphenyl-2-carbonyl)-3-aza-bicyclo[3.1.0]hex-2-ylmethyl]-amide;
   2,3-dihydro-benzo[1,4]dioxine-5-carboxylic acid [(1R*,2S*,5S*)-3-(6-methylbiphenyl-2-carbonyl)-3-aza-bicyclo[3.1.0]hex-2-ylmethyl]-amide;
   2,3-dihydro-benzo[1,4]dioxine-5-carboxylic acid [(1R*,2S*,5S*)-3-(3'-fluoro-6-methyl-biphenyl-2-carbonyl)-3-aza-bicyclo[3.1.0]hex-2-ylmethyl]-amide;
   2,3-dihydro-benzo[1,4]dioxine-5-carboxylic acid [(1R*,2S*,5S*)-3-(2-cyclopropylethynyl-4-methyl-benzoyl)-3-aza-bicyclo[3.1.0]hex-2-ylmethyl]-amide;
   benzofuran-4-carboxylic acid [(1R,2S,5S)-3-(2-methyl-5-m-tolyl-thiazole-4-carbonyl)-3-aza-bicyclo[3.1.0]hex-2-ylmethyl]-amide;
   benzofuran-4-carboxylic acid {(1R,2S,5S)-3-[5-(3-fluoro-phenyl)-2-methyl-thiazole-4-carbonyl]-3-aza-bicyclo[3.1.0]hex-2-ylmethyl}-amide;
   2,3-dihydro-benzo[1,4]dioxine-5-carboxylic acid [(1R,2S,5S)-3-(2-methyl-5-m-tolyl-thiazole-4-carbonyl)-3-aza-bicyclo[3.1.0]hex-2-ylmethyl]-amide;
   6-methyl-imidazo[2,1-b]thiazole-5-carboxylic acid [(1R*,2S*,5S*)-3-(2-bromo-5-m-tolyl-thiazole-4-carbonyl)-3-aza-bicyclo[3.1.0]hex-2-ylmethyl]-amide;
   6-methyl-imidazo[2,1-b]thiazole-5-carboxylic acid [(1R*,2S*,5S*)-3-(5-m-tolyl-2-trimethylsilanylethynyl-thiazole-4-carbonyl)-3-aza-bicyclo[3.1.0]hex-2-ylmethyl]-amide;
   6-methyl-imidazo[2,1-b]thiazole-5-carboxylic acid {(1R*,2S*,5S*)-3-[2-(3-hydroxy-prop-1-ynyl)-5-m-tolyl-thiazole-4-carbonyl]-3-aza-bicyclo[3.1.0]hex-2-ylmethyl}-amide;
   6-methyl-imidazo[2,1-b]thiazole-5-carboxylic acid [(1R*,2S*,5S*)-3-(2-ethyl-5-m-tolyl-thiazole-4-carbonyl)-3-aza-bicyclo[3.1.0]hex-2-ylmethyl]-amide;
   6-methyl-imidazo[2,1-b]thiazole-5-carboxylic acid {(1R*,2S*,5S*)-3-[2-(3-hydroxypropyl)-5-m-tolyl-thiazole-4-carbonyl]-3-aza-bicyclo[3.1.0]hex-2-ylmethyl}-amide;
   6-methyl-imidazo[2,1-b]thiazole-5-carboxylic acid [(1R*,2S*,5S*)-3-(5-m-tolyl-thiazole-4-carbonyl)-3-aza-bicyclo[3.1.0]hex-2-ylmethyl]-amide; and
   6-methyl-imidazo[2,1-b]thiazole-5-carboxylic acid [(1R*,2S*,5S*)-3-(2-methoxy-5-m-tolyl-thiazole-4-carbonyl)-3-aza-bicyclo[3.1.0]hex-2-ylmethyl]-amide;
      wherein, in case the above compounds have the relative configuration (1R*,2S*,5S*), the respective enantiomers having either the absolute configuration (1R,2S,5S) or (1S,2R,5R), especially the enantiomers having the absolute configuration (1R,2S,5S), are also encompassed.
   Where the plural form is used for compounds, salts, pharmaceutical compositions, diseases or the like, this is intended to mean also a single compound, salt, disease or the like.

The compounds of formula (I) and their pharmaceutically acceptable salts can be used as medicaments, e.g. in the form of pharmaceutical compositions for enteral or parental administration.
A further aspect of the invention is a pharmaceutical composition containing at least one compound according to formula (I), or a pharmaceutically acceptable salt thereof, and a pharmaceutically acceptable carrier material.
The production of the pharmaceutical compositions can be effected in a manner which will be familiar to any person skilled in the art (see for example Mark Gibson, Editor, Pharmaceutical Preformulation and Formulation, IHS Health Group, Englewood, CO, USA, 2001; Remington, The Science and Practice of Pharmacy, 20th Edition, Philadelphia College of Pharmacy and Science) by bringing the described compounds of formula (I) and their pharmaceutically acceptable salts, optionally in combination with other therapeutically valuable substances, into a galenical administration form together with suitable, non-toxic, inert, therapeutically compatible solid or liquid carrier materials and, if desired, usual pharmaceutical adjuvants.

The compounds according to formula (I) may be used for the preparation of a medicament, and are suitable, for the prevention or treatment of diseases selected from the group consisting of dysthymic disorders including major depression and cyclothymia, affective neurosis, manic depression, delirium, psychotic disorders, schizophrenia, delusional paranoia, adjustement disorders and all clusters of personality disorders; anxiety disorders including generalized anxiety, obsessive compulsive disorder, posttraumatic stress disorder, panic attacks, all types of phobic anxiety and avoidance; stress-related syndromes; psychoactive substance use, abuse, seeking and reinstatement; all types of psychological or physical addictions, dissociative disorders including multiple personality syndromes and psychogenic amnesias; sexual dysfunction; psychosexual dysfunction and addiction; tolerance to narcotics or withdrawal from narcotics; hypothalamic-adrenal dysfunctions; disturbed biological and circadian rhythms; all types of sleep disorders; sleep disturbances associated with diseases such as neurological disorders including neuropathic pain and restless leg syndrome; sleep apnea; narcolepsy; insomnias related to psychiatric disorders; all types of idiopathic insomnias and parasomnias; sleep-wake schedule disorders including jet-lag; all dementias and cognitive dysfunctions in the healthy population and in psychiatric and neurologic disorders; mental dysfunctions of aging; severe mental retardation; dyskinesias and muscular diseases; neurodegenerative disorders including Huntington's, Creutzfeld-Jacob's, Alzheimer's diseases and Tourette syndrome; Amyotrophic lateral sclerosis; Parkinson's disease; Cushing's syndrome; traumatic lesions; demyelinating diseases; spinal and cranial nerve diseases; epilepsy; seizure disorders; absence seizures, complex partial and generalized seizures; Lennox-Gastaut syndrome; migraine and headache; pain disorders; anesthesia and analgesia; enhanced or exaggerated sensitivity to pain such as hyperalgesia, causalgia, and allodynia; acute pain; burn pain; atypical facial pain; neuropathic pain; back pain; complex regional pain syndrome **I** and **II**; arthritic pain; sports injury pain; pain related to infection e.g. by HIV; post-chemotherapy pain; post-stroke pain; post-operative pain; neuralgia; conditions associated with visceral pain such as irritable bowel syndrome; eating disorders; diabetes; toxic and dysmetabolic disorders including cerebral anoxia, diabetic neuropathies and alcoholism; appetite, taste, eating, or drinking disorders; somatoform disorders including hypochondriasis; vomiting/nausea; inflammatory bowel disease; gastric dyskinesia; gastric ulcers; Kallman's syndrome (anosmia); impaired glucose tolerance; intestinal motility dyskinesias; hypothalamic diseases; hypophysis diseases; hyperthermia syndromes, pyrexia, febrile seizures; idiopathic growth deficiency; dwarfism; gigantism; acromegaly; basophil adenoma; prolactinoma; hyperprolactinemia; brain tumors, adenomas; benign prostatic hypertrophy, prostate cancer; all types of testicular dysfunctions, fertility control; hypothalamic hypogonadism, functional or psychogenic amenorrhea; urinary bladder incontinence asthma; allergies; all types of dermatitis, acne and cysts, sebaceous gland dysfunctions; cardiovascular disorders; heart and lung diseases, acute and congestive heart failure; hypotension; hypertension; urinary retention; osteoporosis; angina pectoris; myocardial infarction; ischemic or haemorrhagic stroke; all types of cerebrovascular disorders including subarachnoid haemorrhage, ischemic and hemorrhagic stroke and vascular dementia; chronic renal failure and other renal diseases; and other diseases related to general orexin system dysfunctions. Compounds of formula (I) are particularly suitable for use in the treatment of diseases or disorders selected from the group consisting of all types of sleep disorders, of stress-related syndromes, of psychoactive substance use and abuse, of cognitive dysfunctions in the healthy population and in psychiatric and neurologic disorders, of eating or drinking disorders. Eating disorders may be defined as comprising metabolic dysfunction; dysregulated appetite control; compulsive obesities; emeto-bulimia or anorexia nervosa. Pathologically modified food intake may result from disturbed appetite (attraction or aversion for food); altered energy balance (intake vs. expenditure); disturbed perception of food quality (high fat or carbohydrates, high palatability); disturbed food availability (unrestricted diet or deprivation) or disrupted water balance. Drinking disorders include polydipsias in psychiatric disorders and all other types of excessive fluid intake. Sleep disorders include all types of insomnias, narcolepsy and other disorders of excessive sleepiness, sleep-related dystonias; restless leg syndrome; sleep apneas; jet-lag syndrome; shift-work syndrome, delayed or advanced sleep phase syndrome or insomnias related to psychiatric disorders. Insomnias are defined as comprising sleep disorders associated with aging; intermittent treatment of chronic insomnia; situational transient insomnia (new environment, noise) or short-term insomnia due to stress; grief; pain or illness. Insomnia also include stress-related syndromes including post-traumatic stress disorders as well as other types and subtypes of anxiety disorders such as generalized anxiety, obsessive compulsive disorder, panic attacks and all types of phobic anxiety and avoidance; psychoactive substance use, abuse, seeking and reinstatement are defined as all types of psychological or physical addictions and their related tolerance and dependence components. Cognitive dysfunctions include deficits in all types of attention, learning and memory functions occurring transiently or chronically in the normal, healthy, young, adult or aging population, and also occurring transiently or chronically in psychiatric, neurologic, cardiovascular and immune disorders.
In a further embodiment of the invention, compounds of formula (I) are particularly suitable for use in the treatment of diseases or disorders selected from the group consisting of dysthymic, mood, psychotic and anxiety disorders; diabetes and appetite, taste, eating, or drinking disorders; hypothalamic diseases; disturbed biological and circadian rhythms; all types of sleep disorders; sleep disturbances associated with diseases such as neurological disorders including neuropathic pain and restless leg syndrome; insomnias related to psychiatric disorders; sleep apnea; narcolepsy; idiopathic insomnias; parasomnias; stress-related syndromes; benign prostatic hypertrophy; all types of psychoactive substance use and abuse; all dementias and cognitive dysfunctions in the healthy population and in psychiatric and neurologic disorders; and other diseases related to general orexin system dysfunctions.
Another aspect of the present invention is a method for the treatment or prophylaxis of diseases, which are related to the orexin receptors such as eating disorders or sleep disorders comprising the administration to a patient a therapeutically effective amount of a compound of formula (I).

A further aspect of the invention is a process for the preparation of compounds of formula (I). Compounds according to formula (I) of the present invention can be prepared according to the general sequence of reactions outlined in the schemes below wherein A, B, X, n, and R¹ are as defined in the description of formula (I). The compounds obtained may also be converted into pharmaceutically acceptable salts thereof in a manner known *per se.*
In general, all chemical transformations can be performed according to well-known standard methodologies as described in the literature or as described in the procedures below.

### Preparation of compounds of formula (I):

### Abbrevations:

### The following abbreviations are used throughout the specification and the examples:

- aq.: aqueous
- Boc: *tert*-Butoxycarbonyl
- BSA: Bovine serum albumine
- CC: Column chromatography on silica gel
- CHO: Chinese hamster ovary
- conc: Concentrated
- *cy*-: Cyclo-
- d: Day(s)
- DBU: 1,8-Diazabicyclo-[5.4.0]-undec-7-ene
- DCM: Dichloromethane
- DIBAL: Diisobutylaluminium hydride
- DIPEA: Diisopropylethylamine
- DME: 1,2-Dimethoxyethane
- DMF: *N,N*-Dimethylformamide
- ent: Enantiomer
- eq: Equivalent(s)
- ES: Electron spray
- Ether: Diethylether
- EtOAc: Ethyl acetate
- EtOH: Ethanol
- FCS: Foatal calf serum
- FLIPR: Fluorescent imaging plate reader
- h: Hour(s)
- HBSS: Hank's balanced salt solution
- HEPES: 4-(2-hydroxyethyl)-piperazine-1-ethanesulfonic acid
- HPLC: High performance liquid chromatography
- LC: Liquid chromatography
- M: Molar(ity)
- MeCN: Acetonitrile
- MeOH: Methanol
- min: Minute(s)
- MS: Mass spectroscopy
- NEt₃: Triethylamine
- PPh₃: Triphenylphosphine
- RT: Room temperature
- sat: Saturated
- t_{R}: Retention time
- TBAF: Tetrabutylammonium fluoride
- TBME: *tert*-Butyl methyl ether
- TBTU: O-Benzotriazol-1-yl-*N,N,N',N*'-tetramethyluronium tetrafluoroborate
- THF: Tetrahydrofuran

### General preparation methods:

### Preparation of the compounds of formula (I):

The compounds of formula (**I**) can be prepared for example according to the method outlined in *Scheme 1* hereafter.
The preparation of the 3-aza-bicyclo[3.1.0]hexane derivatives started by the protection of the nitrogen atom of the known amino-acid (**1**) [V.V. Tverezovsky et al. Tetrahedron 1997, 53(43), 14773-14792 and cited literature] with Boc₂O. The ester (3) could be obtained e.g. by reaction of acid (**2**) with methyl iodide in the presence of a base like cesium carbonate in a solvent like DMF. After reduction with DIBAL at low temperatures the respective alcohol (**4**) was oxidized to the corresponding aldehyde (**5**) with e.g. Dess-Martin periodinane. After reductive amination of (5) with benzylamine in the presence of a reducing agent like sodium triacetoxyborohydride the benzyl group was removed by hydrogenolysis to yield the primary amine (7). The acylation of (**7**) with a carboxylic acid R¹COOH in the presence of a coupling reagent like TBTU resulted in the formation of amides (**8**) which after removal of the Boc-group were transferred under amide coupling conditions (e.g. B-A-COOH, TBTU or B-A-COCl) or by reaction with sulfonyl chlorides (B-A-SO₂Cl) to compounds (**10**), which are compounds of formula (I), wherein n is 1. Alternatively, amine (**7**) was transferred to intermediates (**9**) by heating a mixture of (**7**) and a heterocyclyl- or aryl-halide (preferably the chloride or bromide) in a solvent like ethanol in the presence of NEt₃ under microwave conditions, or in presence of a palladium catalyst under standard Buchwald or Hartwig amination conditions. After removal of the Boc-group, reaction under amide coupling conditions (e.g. B-A-COOH, TBTU or B-A-COCl) or reaction with sulfonyl chlorides (B-A-SO₂Cl) leads to compounds (**11**), which are compounds of formula (**I**), wherein n is 0. By determination of the crystal structure of one of the derivatives (**9**) it was possible to demonstrate that the reductive amination lead to compounds with a relative *cis*-configuration (see experimental part).

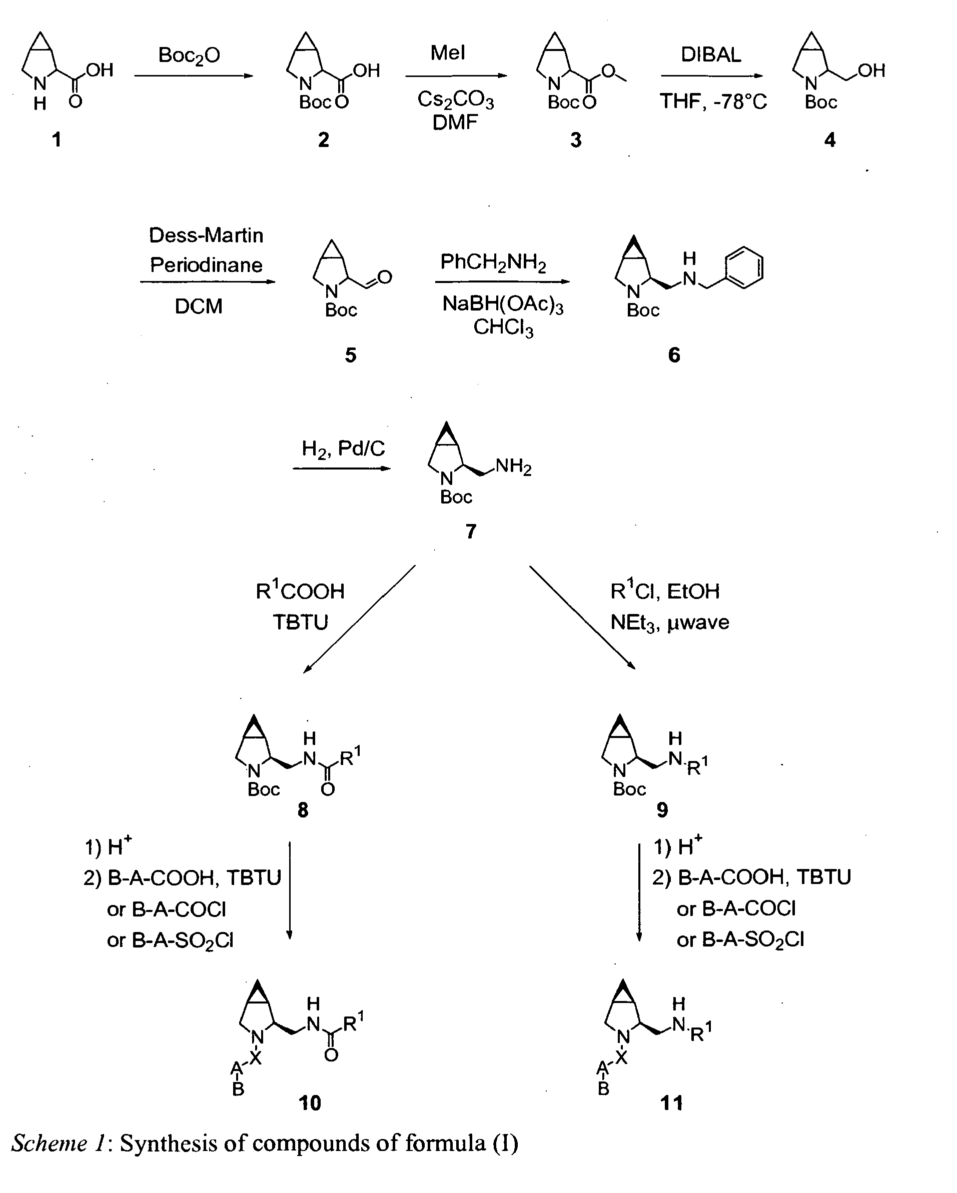

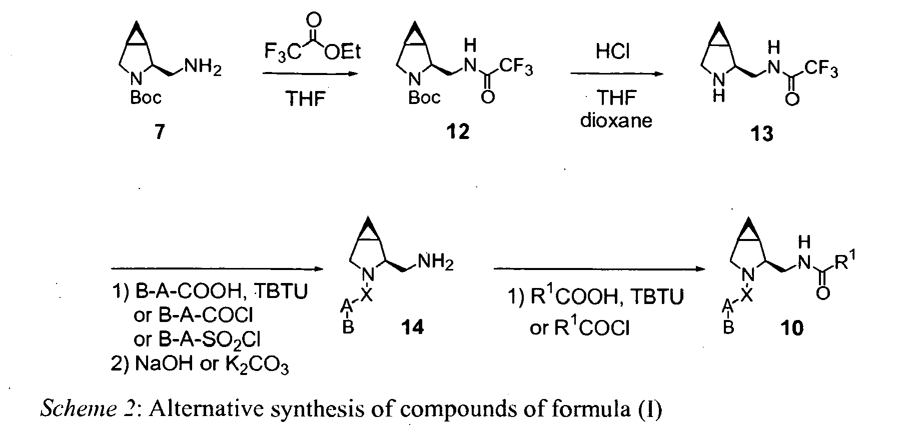

Another approach to compounds of formula (I) started with the protection of amine (**7**) with ethyl trifluoroacetate to give amides (**12**), which were Boc-deprotected with an acid like HCl in a solvent or a mixture of solvents like dioxane or THF. The obtained amine (**13**) was coupled with a carboxylic acid B-A-COOH in the presence of a coupling reagent like TBTU or with an acid chloride B-A-COCl to an amide or with a sulfonyl chloride to a sulfonamide. After deprotection with for instance aq. NaOH or aq. K₂CO₃, amines (**14**) were obtained which were coupled with a carboxylic acid R¹COOH in the presence of a coupling reagent like TBTU or with an acid chloride R¹COCl to compounds (**10**), which are compounds of formula (**I**), wherein n is 1.

Compounds (**15**), which are compounds of formula (I), wherein wherein A represents phenyl substituted with R and B represents an aryl or a heterocyclyl group, could be synthesized according to one of the pathways illustrated in *scheme 3.*

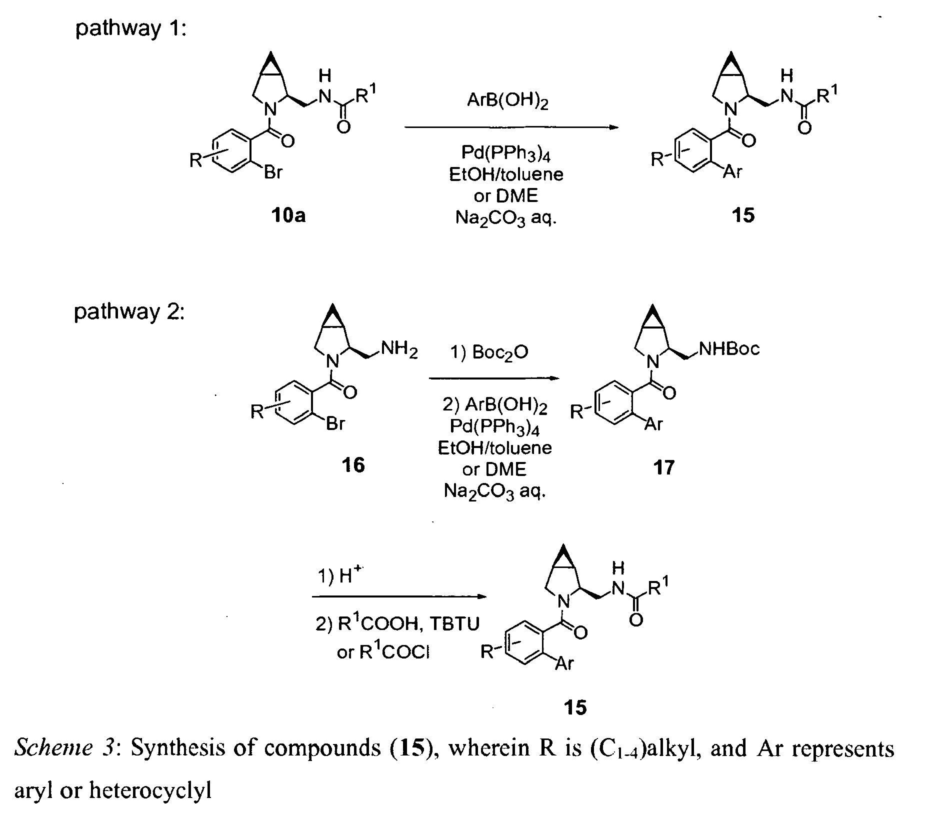

Starting from 2-bromo-benzamide derivatives (**10a**), synthesized according to *scheme 1,* compounds (**15**) were obtained in a Suzuki-type coupling with aryl- or heterocyclylboronic acids in the presence of Pd(PPh₃)₄ as catalyst. Alternatively amines (**16**), obtained according to the procedure illustrated in *scheme* 2, were protected by reaction with di-*tert*-butyl dicarbonate in the presence of a base like NEt₃ and coupled with aryl- or heterocyclylboronic acids in the presence of a palladium catalyst like Pd(PPh₃)₄ to give compounds (**17**). Removal of the Boc group under acidic conditions led to the respective amines which were coupled with a carboxylic acid R¹COOH in the presence of a coupling reagent like TBTU to compounds (**15**).

Compounds (**18**), which are compounds of formula (**I**), wherein A represents mono-, or di-substituted phenyl, wherein one substituent is selected from (C₂₋₆)alkynyl, and (C₃₋₆)cycloalkyl-ethynyl; and the other substituent (if present) is (C₁₋₄)alkyl, and B represents hydrogen, were synthesized according to *scheme 4.*

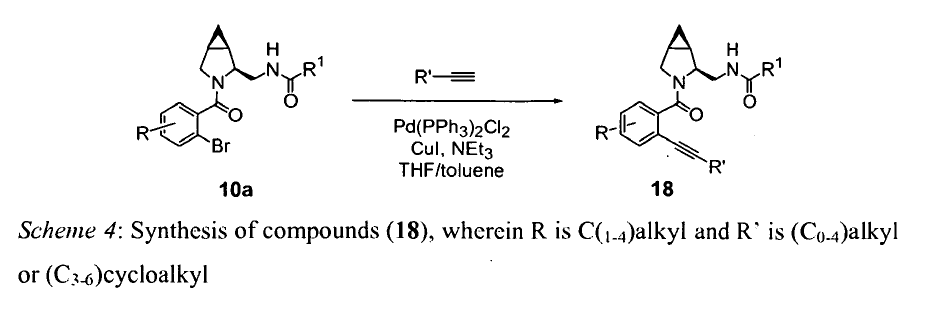

Compounds (**18**) were prepared by palladium catalyzed coupling of aryl bromide derivatives (**10a**) with alkynes in the presence of Pd(PPh₃)₂Cl₂ and copper(**I**) iodide. In case the alkyne is ethyne, the synthesis is preferably conducted using trimethylsilyl-ethyne in analogy to the method described below.

Thiazole-4-carboxylic acid derivatives of formula B-A-COOH were for instance synthesised according to *schema 5.*

By reaction of methyl dichloroacetate (**19**; commercially available) with an aldehyde in the presence of a base like potassium *tert*.-butoxide the 3-chloro-2-oxo-propionic ester derivatives (20**)** were obtained which were transformed in a reaction with thioamides [R = (C₁₋₄)alkyl] to 2-alkyl-substituted thiazole derivatives (**21**) or in a reaction with thioureas (R = NR²R³) to 2-amino-substituted thiazole derivatives (**21**). Saponification of the ester function with an aq. solution of e.g. NaOH in a solvent like MeOH resulted in the formation of the desired carboxylic acids (**22**, R = (C₁₋₄)alkyl or NR²R³). 2-Bromo-thiazole derivatives (**23**) were for instance obtained by reaction of the respective 2-amino-thiazole derivative (**21**, R = NH₂) with isoamylnitrite in the presence of copper(II)bromide. The ester derivatives (**23**) were either saponified to the respective carboxylic acids (**24**) as described above or transferred to 2-methoxy substituted analogues (**25**) by reaction with sodium methoxide and subsequent saponification with NaOH. In addition compounds (**27**) which are unsubsituted in 2-position were synthesized by hydrogenation of (**23**) in the presence of palladium on charcoal and subsequent saponification of the intermediate ester (**26**).

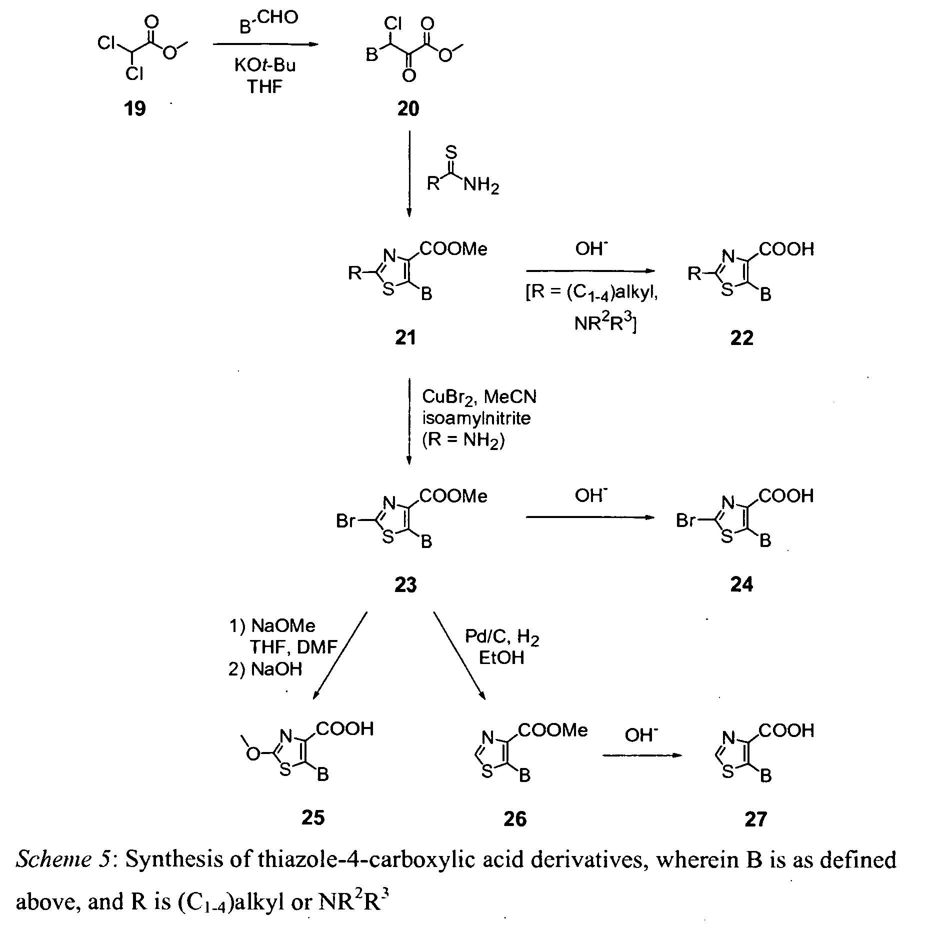

Aldehydes B-CHO are commercially available or may be synthesized by procedures known from the literature like for instance reduction of the respective carboxylic acid or their different derivatives with a reducing agent, by reduction of the respective nitrile or by oxidation of benzylic alcohols and their heterocyclic analogues with oxidating agents (e.g.: J. March, Advanced Organic Chemistry, 4th edition, John Wiley & Sons, p. 447-449, 919-920 and 1167-1171).

Compounds (**29**), which are compounds of formula (I) bearing a 2-substituted thiazole moiety, were for instance synthesized according to *scheme 6.*

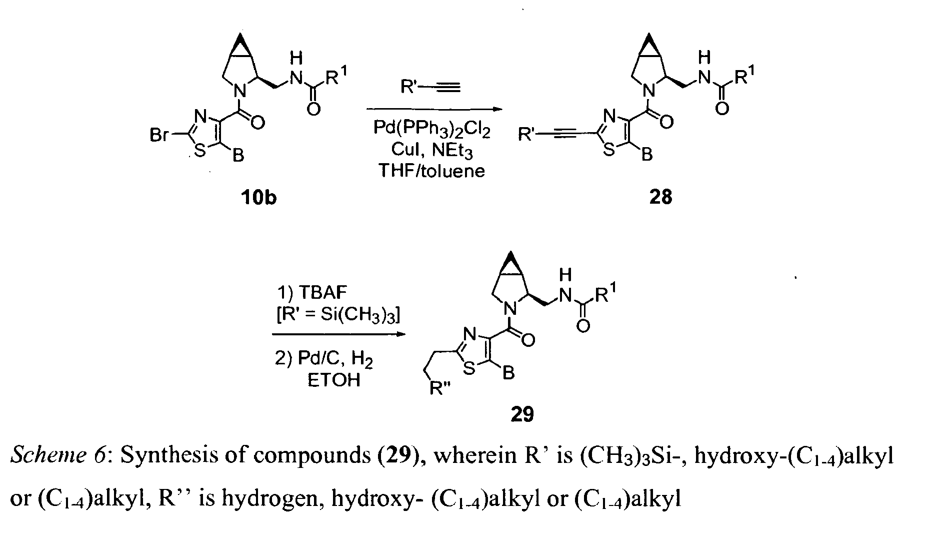

By coupling of 2-bromo-thiazole derivatives (**10b**), obtained according to *scheme 1,* with alkyne derivatives in the presence of Pd(PPh₃)₂Cl₂ and copper(I) iodide compounds (**28**) of formula (**I**) could be obtained. Compounds (**28**) could be reduced, eventually after desilylation with TBAF (R' = SiMe₃), by hydrogenation in the presence of palladium on charcoal to respective compounds (**29**).

Carboxylic acids of formula R¹-COOH are commercially available or well known in the art (Lit. e.g. WO2001/96302; T. Eicher, S. Hauptmann "The chemistry of Heterocycles: Structure, Reactions, Syntheses, and Applications", 2nd Edition 2003, Wiley, ISBN 978-3-527-30720-3).
Derivatives of formula R¹-COOH wherein R¹ is benzo[1,4]oxazine were for instance synthesised according to *scheme 7.*

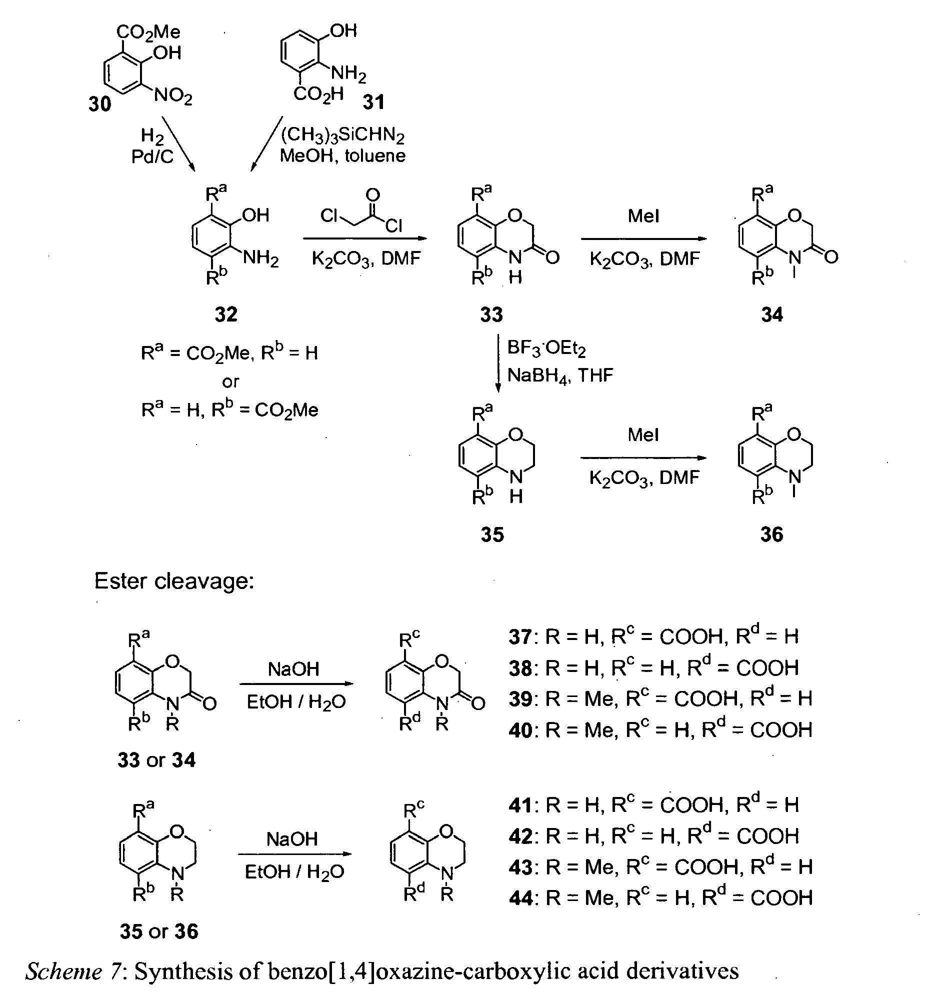

By hydrogenation of 3-nitrosalicylate (commercially available) in MeOH 3-amino-2-hydroxy-benzoic acid methyl ester (**32**, R^{a} = COOMe, R^{b} = H) was obtained. The regioisomer (**32**, R^{a} = H, R^{b} = COOMe) was synthesized by esterification of commercially available 3-hydroxyanthranilic acid with (trimethylsilyl)diazomethane. Cyclization of one or the other amino-hydroxy-benzoic acid (**32**) with chloroacetyl chloride in the presence of a base like K₂CO₃ lead to 3-oxo-3,4-dihydro-2H-benzo[1,4]oxazine derivatives (**33**) which were reduced to 3,4-dihydro-2H-benzo[1,4]oxazine derivatives (**35**) with NaBH₄ in the presence of boron trifluoride diethyl etherate. Compounds (**33**) as well as (**35**) may be alkylated at the nitrogen atom with methyl iodide in the presence of a base like K₂CO₃ in a solvent like DMF to give the respective analogues (**34**) or (**36**). By saponification of the respective ester derivatives (**33, 34, 35** or **36**) with NaOH in a solvent mixture like water/ethanol the desired acids (**37, 38, 39, 40, 41, 42, 43** or **44**) could be obtained.

Derivatives of formula R¹-COOH wherein R¹ is chroman were for instance synthesised according to *scheme 8.*

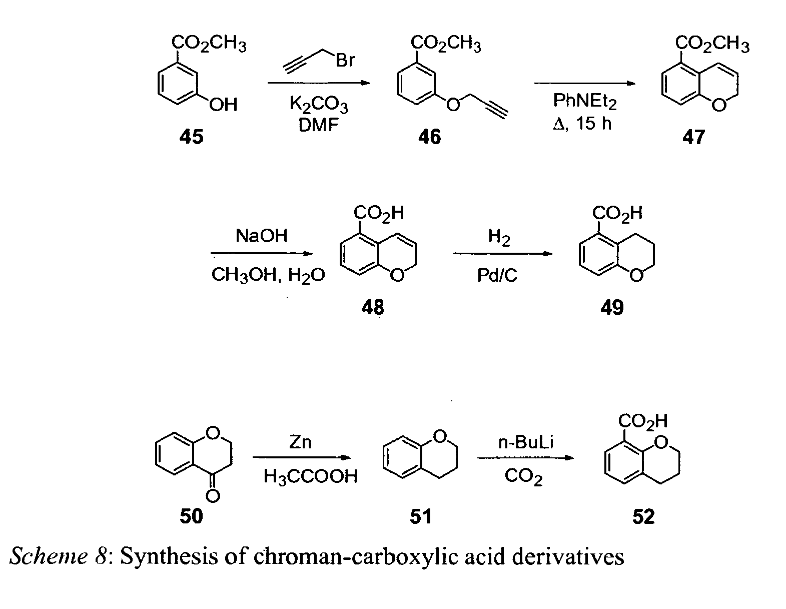

The synthesis of chroman-5-carboxylic acid derivatives started with the alkylation of 3-hydroxy-benzoic acid methyl ester (**45**; commercially available) with propargyl bromide in the presence of K₂CO₃ to give phenylether (**46**) which was cyclised to the chromen derivative (**47**) by heating to reflux in N,N-diethylaniline. The carboxylic ester was saponified by treatment of (**47**) mith NaOH in MeOH and water and the obtained chromen derivative (**48**) was hydrogenated to give the desired acid (**49**). The corresponding chroman-8-carboxylic acid derivatives were synthesized by reduction of 4-chromanone (**50**; commercially available) with zinc in acetic acid and subsequent *ortho*-metalation of the intermediate chroman derivative (**51**) with n-BuLi and trapping with carbon dioxide to give the desired acid (**52**).

Derivatives of formula R¹-COOH wherein R¹ is imidazo[2,1-b]thiazole were for instance synthesised according to one of the different pathways shown in *scheme 9.*

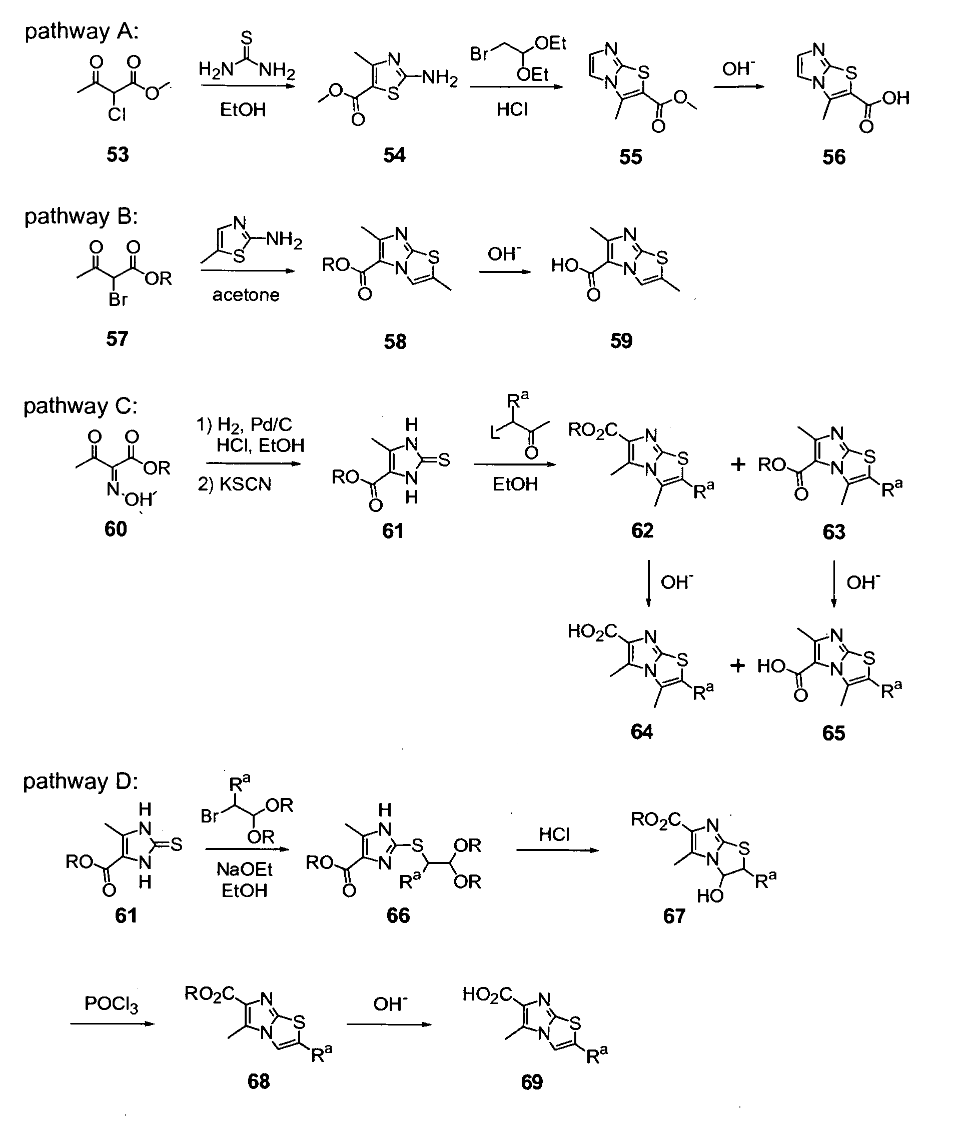

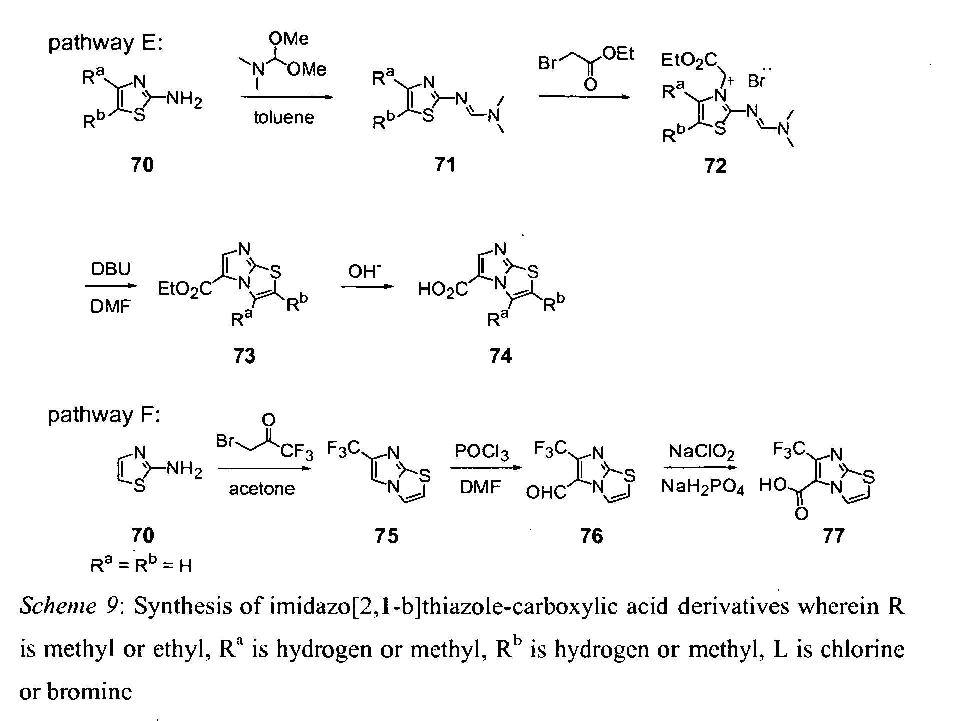

Following pathway A imidazo[2,1-b]thiazole-carboxylic acid derivatives were synthesized starting from 2-chloro-3-oxo-butyric acid methyl ester (**53**; commercially available) by reaction with thiourea in a solvent like ethanol at elevated temperatures. The obtained amino-thiazole (**54**) was converted to the imidazo[2,1-b]thiazole derivative (**55**) by alkylation and subsequent cyclization with bromoacetaldehyde diethyl acetal in the presence of an acid like concentrated hydrochloric acid. By saponification of (**55**) with for instance NaOH in solvents like THF and MeOH the desired acids (**56**) were obtained.
An alternative approach (pathway **B**) started with the reaction of 2-bromo-3-oxo-butyric acid ester (**57**; commercially available) with 2-amino-5-methyl-thiazole in a solvent like acetone to give the imidazo[2,1-b]thiazole derivative (**58**) which was transformed to the desired acid (**59**) by saponification with for instance NaOH in solvents like THF and MeOH.
By hydrogenation of 2-hydroxyimino-3-oxo-butyric acid ester (**60**; commercially available) in the presence of palladium on charcoal under acidic conditions (e.g. HCl in EtOH) and subsequent reaction with potassium thiocyanate the imidazole derivative (**61**) was obtained which was transferred to a mixture of the two possible isomers (**62**) and (**63**) by reaction with the respective α-halogenated propanone or butanone derivative (pathway C). After separation of the isomers (**62**) and (**63**) by chromatography the desired imidazo[2,1-b]thiazole-carboxylic acid derivatives (**64**) and (**65**) were obtained by saponification with for instance NaOH in solvents like THF and MeOH.
Alternatively (pathway D) the imidazole derivative (**61**) may be transferred to the acetal (**66**) by alkylation with a bromoacetaldehyde dialkyl acetal derivative in the presence of a base like sodium ethoxide. Cyclization under acidic conditions (e.g. aq. hydrochloric acid) and dehydration of the intermediate (**67**) with for instance phosphorus oxychloride led to ester (**68**) which was transformed to the desired acid (**69**) by saponification with for instance NaOH in solvents like THF and MeOH.
In still an alternative procedure (pathway **E**) the respective amino-thiazole (**70**; commercially available) was converted to the formamidine derivative (**71**) by heating (**70**; commercially available) with *N,N*-dimethylformamide dimethylacetale in a solvent like toluene. After alkylation with ethyl bromoacetate the respective thiazolium bromide (**72**) was cyclised with DBU to yield the ester (**73**) which was saponified to the desired acid (**74**) with for instance NaOH in solvents like THF and MeOH.
Finally pathway F started with the alkylation of 2-amino-thiazole with 3-bromo-1,1,1-trifluoroacetone to yield the trifluoromethyl-substituted imidazo[2,1-b]thiazole derivative (**75**) which was formylated to the aldehyde (**76**) by reaction with phosphorus oxychloride in a solvent like DMF. By oxidation of aldehyde (**75**) with sodium chlorite the desired imidazo[2,1-b]thiazole-carboxylic acid (**77**) was obtained. In analogy, the commercially available chlorinated aldehyde (**76**, being substituted with Cl instead of CF₃) was oxidized to the corresponding acid.

Whenever the compounds of formula (I) are obtained in the form of mixtures of enantiomers, the enantiomers can be separated using methods known to one skilled in the art: e.g. by formation and separation of diastereomeric salts or by HPLC over a chiral stationary phase such as a Regis Whelk-O1(R,R) (10 µm) column, a Daicel ChiralCel OD-H (5-10 µm) column, or a Daicel ChiralPak IA (10 µm) or AD-H (5 µm) column. Typical conditions of chiral HPLC are an isocratic mixture of eluent A (EtOH, in presence or absence of an amine such as NEt₃, diethylamine) and eluent B (hexane), at a flow rate of 0.8 to 150 mL/min.

### Experimental Section

### I-Chemistry

The following examples illustrate the preparation of pharmacologically active compounds of the invention but do not at all limit the scope thereof.
All temperatures are stated in °C.
Compounds are characterized by:
¹H-NMR: 300 MHz Varian Oxford or 400 MHz Bruker Avance; chemical shifts are given in ppm relative to the solvent used; multiplicities: s = singlet, d = doublet, t = triplet, m = multiplet, b = broad, coupling constants are given in Hz;
LC-MS: Agilent 1100 series with DAD and MS detection (MS: Finnigan single
   quadrupole);
   columns (4.6x50 mm, 5 µm): Zorbax SB-AQ, Zorbax Extend C 18 or Waters
   XBridge C18;
   conditions (if not otherwise stated the acidic gradient is used):
   basic: eluent A: MeCN, eluent B: conc. NH₃ in water (1.0 mL/L), 5% to 95% CH₃CN, flow rate 4.5 mL/min;
   acidic: eluent A: MeCN, eluent B: TFA in water (0.4 mL/L), 5% to 95% CH₃CN, flow rate 4.5 mL/min;
   t_{R} is given in min;
   Compounds are purified by column chromatography on silica gel (CC) or by preparative HPLC using RP-C₁₈ based columns with MeCN/water gradients and formic acid or ammonia additives.

### A. Preparation of precursors and intermediates:

### A.1 Synthesis of thiazole-4-carboxylic acid derivatives

### A.1.1 Synthesis of 3-chloro-2-oxo-propionic ester derivatives (general procedure)

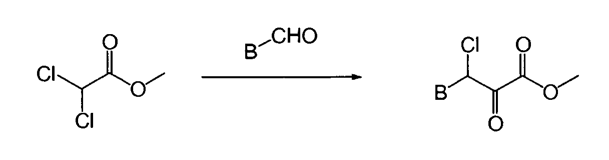

A solution of the respective aldehyde (338 mmol, 1.0 eq) and methyl dichloroacetate (338 mmol, 1.0 eq) in THF (100 mL) is added dropwise to a cold (-60°C) suspension of KOtBu (335 mmol, 1.0 eq) in THF (420 mL). After 4 h the mixture is allowed to reach RT, stirred over night and concentrated in vacuo. DCM and ice-cold water are added, the layers are separated and the aq. layer is extracted twice with DCM. The combined organic layers are washed with ice-cold water and brine, dried over MgSO₄ and concentrated in vacuo to give the desired 3-chloro-2-oxo-propionic ester derivative which is used without further purification.

### 3-chloro-2-oxo-3-m-tolyl-propionic acid methyl ester

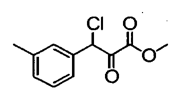

prepared by reaction of 3-methyl-benzaldehyde with methyl dichloroacetate.

### 3-chloro-2-oxo-3-p-tolyl-propionic acid methyl ester

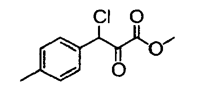

prepared by reaction of 4-methyl-benzaldehyde with methyl dichloroacetate.

### 3-chloro-3-(4-ethyl-phenyl)-2-oxo-propionic acid methyl ester

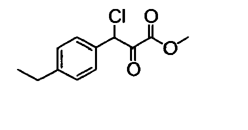

prepared by reaction of 4-ethyl-benzaldehyde with methyl dichloroacetate.

### 3-chloro-3-(3-fluoro-phenyl)-2-oxo-propionic acid methyl ester

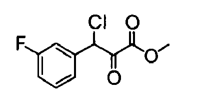

prepared by reaction of 3-fluoro-benzaldehyde with methyl dichloroacetate.

### 3-chloro-3-(4-fluoro-phenyl)-2-oxo-propionic acid methyl ester

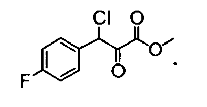

prepared by reaction of 4-fluoro-benzaldehyde with methyl dichloroacetate.

### 3-chloro-3-(4-tritluoromethyl-phenyl)-2-oxo-propionic acid methyl ester

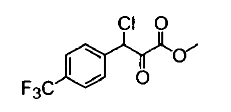

prepared by reaction of 4-trifluoromethyl-benzaldehyde with methyl dichloro-acetate.

### 3-chloro-3-(2-fluoro-phenyl)-2-oxo-propionic acid methyl ester

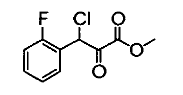

prepared by reaction of 2-fluoro-benzaldehyde with methyl dichloro-acetate.

### 3-chloro-3-(2-chloro-phenyl)-2-oxo-propionic acid methyl ester

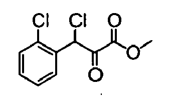

prepared by reaction of 2-chloro-benzaldehyde with methyl dichloro-acetate.

### 3-chloro-3-(3-chloro-phenyl)-2-oxo-propionic acid methyl ester

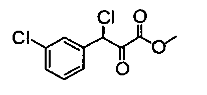

prepared by reaction of 3-chloro-benzaldehyde with methyl dichloro-acetate.

### 3-chloro-2-oxo-3-o-tolyl-propionic acid methyl ester

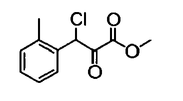

prepared by reaction of 2-methyl-benzaldehyde with methyl dichloro-acetate.

### 3-chloro-3-(2-methoxy-phenyl)-2-oxo-propionic acid methyl ester

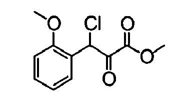

prepared by reaction of 2-methoxy-benzaldehyde with methyl dichloro-acetate.

### 3-chloro-3-(3-methoxy-phenyl)-2-oxo-propionic acid methyl ester

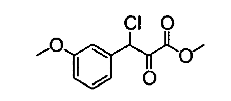

prepared by reaction of 3-methoxy-benzaldehyde with methyl dichloro-acetate.

### 3-chloro-2-oxo-3-(2-trifluoromethyl-phenyl)-propionic acid methyl ester

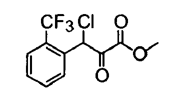

prepared by reaction of 2-trifluoromethyl-benzaldehyde with methyl dichloro-acetate.

### 3-chloro-2-oxo-3-(3-trifluoromethyl-phenyl)-propionic acid methyl ester

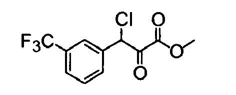

prepared by reaction of 3-trifluoromethyl-benzaldehyde with methyl dichloro-acetate.

### 3-chloro-3-(3,4-dimethyl-phenyl)-2-oxo-propionic acid methyl ester

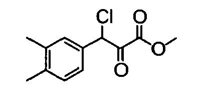

prepared by reaction of 3,4-dimethyl-benzaldehyde with methyl dichloro-acetate.

### 3-chloro-3-(3-cyano-phenyl)-2-oxo-propionic acid methyl ester

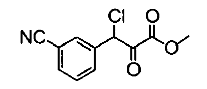

prepared by reaction of 3-cyano-benzaldehyde with methyl dichloro-acetate.

### 3-(3-benzyloxy-phenyl)-3-chloro-2-oxo-propionic acid methyl ester

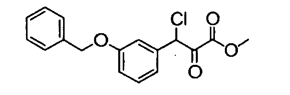

prepared by reaction of 3-benzyloxy-benzaldehyde with methyl dichloro-acetate.

### A.1.2 Synthesis of thiazole-4-carboxylic acid methyl ester derivatives

### (general procedure)

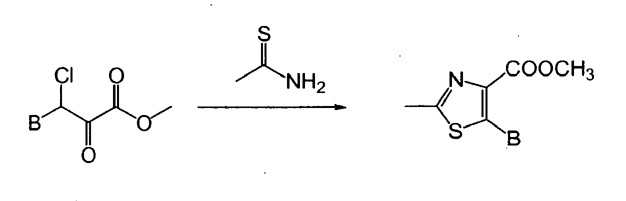

A solution of thioacetamide (132 mmol, 1.0 eq) in MeCN (250 mL) is added to a mixture of the respective 3-chloro-2-oxo-propionic ester derivative (132 mmol, 1.0 eq) and molecular sieves (4Å, 12 g) in MeCN (60 mL). After stirring for 5 h the mixture is cooled in an ice-bath and the obtained precipitate is filtered off. The residue is washed with cold MeCN, dried, dissolved in MeOH (280 mL) and stirred at 50°C for 6 h. The solvents are removed in vacuo to give the desired thiazole derivatives as a white solid.

### 2-methyl-5-m-tolyl-thiazole-4-carboxylic acid methyl ester

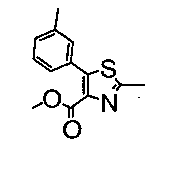

prepared by reaction of 3-chloro-2-oxo-3-m-tolyl-propionic acid methyl ester with thioacetamide. LC-MS: t_{R} = 0.94 min; [M+H]⁺ = 248.0.

### 2-methyl-5-p-tolyl-thiazole-4-carboxylic acid methyl ester

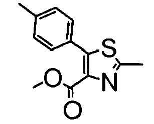

prepared by reaction of 3-chloro-2-oxo-3-p-tolyl-propionic acid methyl ester with thioacetamide. LC-MS: t_{R} = 0.92 min; [M+H]⁺ = 248.2.

### 5-(4-ethyl-phenyl)-2-methyl-thiazole-4-carboxylic acid methyl ester

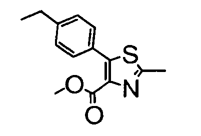

prepared by reaction of 3-chloro-3-(4-ethyl-phenyl)-2-oxo-propionic acid methyl ester with thioacetamide. LC-MS: t_{R} = 0.98 min; [M+H]⁺ = 262.1.

### 5-(3-fluoro-phenyl)-2-methyl-thiazole-4-carboxylic acid methyl ester

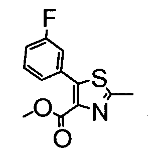

prepared by reaction of 3-chloro-3-(3-fluoro-phenyl)-2-oxo-propionic acid methyl ester with thioacetamide. LC-MS: t_{R} = 0.91 min; [M+H]⁺ = 252.1.

### 5-(4-fluoro-phenyl)-2-methyl-thiazole-4-carboxylic acid methyl ester

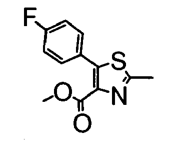

prepared by reaction of 3-chloro-3-(4-fluoro-phenyl)-2-oxo-propionic acid methyl ester with thioacetamide. ¹H-NMR (CDCl₃): δ = 2.75 (s, 3H); 3.84 (s, 3H); 7.10 (m, 2H); 7.47 (m, 2H).

### 2-methyl-5-(4-trifluoromethyl-phenyl)-thiazole-4-carboxylic acid methyl ester

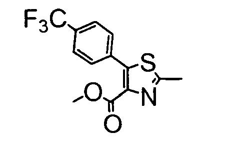

prepared by reaction of 3-chloro-3-(4-trifluoromethyl-phenyl)-2-oxo-propionic acid methyl ester with thioacetamide. LC-MS: t_{R} = 0.98 min; [M+H]⁺ = 302.0.

### 2-methyl-5-(3-trifluoromethyl-phenyl)-thiazole-4-carboxylic acid methyl ester

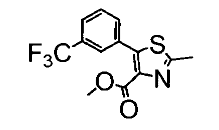

prepared by reaction of 3-chloro-3-(3-trifluoromethyl-phenyl)-2-oxo-propionic acid methyl ester with thioacetamide. LC-MS: t_{R} = 0.98 min; [M+H]⁺ = 302.2.

### 2-methyl-5-(2-trifluoromethyl-phenyl)-thiazole-4-carboxylic acid methyl ester

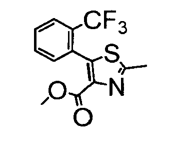

prepared by reaction of 3-chloro-3-(2-trifluoromethyl-phenyl)-2-oxo-propionic acid methyl ester with thioacetamide. LC-MS: t_{R} = 0.94 min; [M+H]⁺ = 302.3.

### 5-(2-fluoro-phenyl)-2-methyl-thiazole-4-carboxylic acid methyl ester

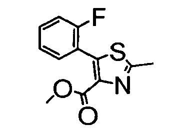

prepared by reaction of 3-chloro-3-(2-fluoro-phenyl)-2-oxo-propionic acid methyl ester with thioacetamide. LC-MS: t_{R} = 0.89 min; [M+H]⁺ = 252.0.

### 5-(2-chloro-phenyl)-2-methyl-thiazole-4-carboxylic acid methyl ester

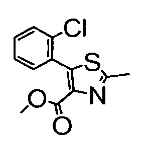

prepared by reaction of 3-chloro-3-(2-chloro-phenyl)-2-oxo-propionic acid methyl ester with thioacetamide. LC-MS: t_{R} = 0.92 min; [M+H]⁺ = 268.0.

### 5-(3-chloro-phenyl)-2-methyl-thiazole-4-carboxylic acid methyl ester

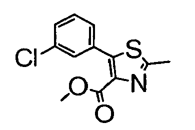

prepared by reaction of 3-chloro-3-(3-chloro-phenyl)-2-oxo-propionic acid methyl ester with thioacetamide. LC-MS: t_{R} = 0.95 min; [M+H]⁺ = 268.0.

### 2-methyl-5-o-tolyl-thiazole-4-carboxylic acid methyl ester

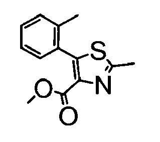

prepared by reaction of 3-chloro-2-oxo-3-o-tolyl-propionic acid methyl ester with thioacetamide. LC-MS: t_{R} = 0.92 min; [M+H]⁺ = 248.1.

### 5-(2-methoxy-phenyl)-2-methyl-thiazole-4-carboxylic acid methyl ester

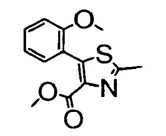

prepared by reaction of 3-chloro-3-(2-methoxy-phenyl)-2-oxo-propionic acid methyl ester with thioacetamide. LC-MS: t_{R} = 0.88 min; [M+H]⁺ = 264.1.

### 5-(3-methoxy-phenyl)-2-methyl-thiazole-4-carboxylic acid methyl ester

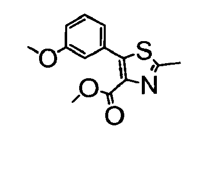

prepared by reaction of 3-chloro-3-(3-methoxy-phenyl)-2-oxo-propionic acid methyl ester with thioacetamide. LC-MS: t_{R} = 0.90 min; [M+H]⁺ = 263.9.

### 5-(3,4-dimethyl-phenyl)-2-methyl-thiazole-4-carboxylic acid methyl ester

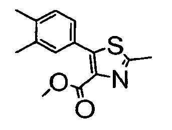

prepared by reaction of 3-chloro-3-(3,4-dimethyl-phenyl)-2-oxo-propionic acid methyl ester with thioacetamide. LC-MS: t_{R} = 0.96 min; [M+H]⁺ = 262.3.

### 5-(3-cyano-phenyl)-2-methyl-thiazole-4-carboxylic acid methyl ester

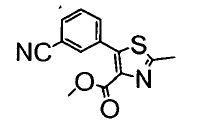

prepared by reaction of 3-chloro-3-(3-cyano-phenyl)-2-oxo-propionic acid methyl ester with thioacetamide. LC-MS: t_{R} = 0.86 min; [M+H]⁺ = 259.3.

### 5-(3-benzyloxy-phenyl)-2-methyl-thiazole-4-carboxylic acid methyl ester

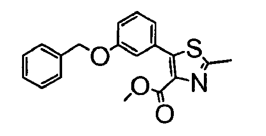

prepared by reaction of 3-(3-benzyloxy-phenyl)-3-chloro-2-oxo-propionic acid methyl ester with thioacetamide. LC-MS: t_{R} = 1.07 min; [M+H]⁺ = 340.2.

### A.1.3 Synthesis of 5-[3-(2-methoxy-ethoxy)-phenyl]-2-methyl-thiazole-4-carboxylic acid methyl ester

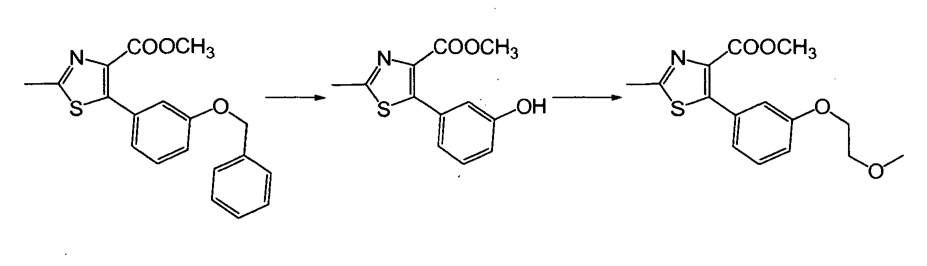

### Synthesis of 5-(3-hydroxy-phenyl)-2-methyl-thiazole-4-carboxylic acid methyl ester

Boron trifluoride diethyl etherate (40.6 mL) is added to a suspension of 5-(3-benzyloxy-phenyl)-2-methyl-thiazole-4-carboxylic acid methyl ester (26.8 mmol) in ethanethiol (50 mL). The mixture is treated with ultrasound for 15 min, stirred for 48 h at RT, poured into a NaOH solution (0.50 M, 500 mL) and extracted twice with EtOAc (250 mL each). The combined organic layers are extracted three times with a solution of NaOH in water (1.0 M, 3x250 mL). The combined aq. layers are made acidic (pH 3) by addition of hydrochloric acid (25%) and the obtained precipitate is filtered off and dried in vacuo to give the desired product as a white solid. LC-MS: t_{R} = 0.82 min; [M+H]⁺ = 250.4.

### Synthesis of 5-[3-(2-methoxy-ethoxy)-phenyl]-2-methyl-thiazole-4-carboxylic acid methyl ester

Under a nitrogen atmosphere azodicarboxylic acid dipiperidide (5.01 mmol) is added to a mixture of 5-(3-hydroxy-phenyl)-2-methyl-thiazole-4-carboxylic acid methyl ester (4.01 mmol) and 2-methoxyethanol (4.41 mmol) in toluene (25 mL). Tributylphosphine (6.02 mmol) is added dropwise at RT and the suspension is heated for 2 h to 100°C. Heptane (25 mL) is added and the suspension is filtered. The residue is washed with heptane (25 mL) and purified by CC (gradient: heptane to heptane/EtOAc 4/1) to give the desired ether. LC-MS: t_{R} = 0.93 min; [M+H]⁺ = 308.3.

### A.1.4 Synthesis of 2-amino-thiazole-4-carboxylic acid methyl ester derivatives (general procedure)

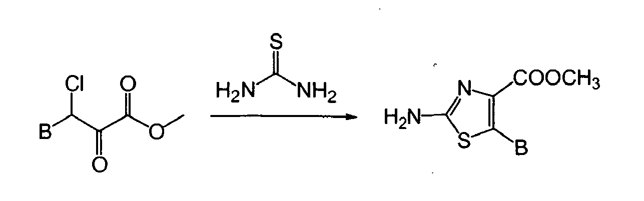

A solution of the respective 3-chloro-2-oxo-propionic ester derivative (22.1 mmol, 1.0 eq) in acetone (25 mL) is added to a suspension of thiourea (22.1 mmol, 1.0 eq) in acetone (45 mL). The mixture is heated to 57°C (bath temperature), stirred for 24h and concentrated to half of the volume. The obtained suspension is filtered and the residue is washed with acetone. After drying the desired amino-thiazole derivative is obtained as a solid.

### 2-amino-5-m-tolyl-thiazole-4-carboxylic acid methyl ester

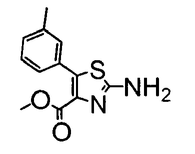

prepared by reaction of 3-chloro-2-oxo-3-m-tolyl-propionic acid methyl ester with thiourea. LC-MS: t_{R} = 0.78 min; [M+H]⁺ = 249.0.

### 2-amino-5-(3-fluoro-phenyl)-thiazole-4-carboxylic acid methyl ester

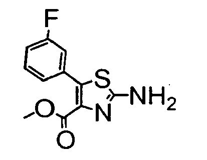

prepared by reaction of 3-chloro-3-(3-fluoro-phenyl)-2-oxo-propionic acid methyl ester with thiourea. LC-MS: t_{R} = 0.78 min; [M+H]⁺ = 252.9.

### 2-Amino-5-(3-chloro-phenyl)-thiazole-4-carboxylic acid methyl ester

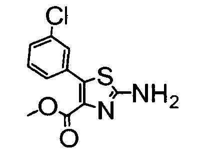

prepared by reaction of 3-chloro-3-(3-chloro-phenyl)-2-oxo-propionic acid methyl ester with thiourea. LC-MS: t_{R} = 0.82 min; [M+H]⁺ = 269.2.

### A.1.5 Synthesis of 2-bromo-5-m-tolyl-thiazole-4-carboxylic acid methyl ester

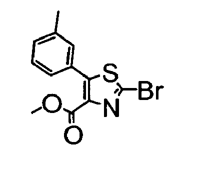

At 15°C under an atmosphere of nitrogen 2-amino-5-m-tolyl-thiazole-4-carboxylic acid methyl ester (7.10 mmol) is added portionwise to a mixture of CuBr₂ (7.10 mmol) and isoamyl nitrite (10.6 mmol) in MeCN (30 mL). The mixture is stirred for 20 min at 15°C, for 30 min at 40°C and for 90 min at 65°C. The solvents are removed in vacuo and the residue is purified by CC (gradient: DCM to DCM/MeOH 98/2) to give the desired product. LC-MS: t_{R} = 1.01 min; [M+H]⁺ = 311.8.

### A.1.6 Synthesis of 5-m-tolyl-thiazole-4-carboxylic acid methyl ester

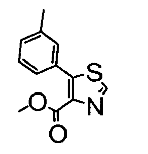

A solution of 2-bromo-5-m-tolyl-thiazole-4-carboxylic acid methyl ester (0.64 mmol) in ethanol (5.0 mL) is treated with Pd/C (100 mg, 10%) and stirred under a hydrogen atmosphere (1 bar) for 18 h. After filtration through celite and removal of the solvents the desired product is obtained which is used without further purification. LC-MS: t_{R} = 0.90 min; [M+H]⁺ = 233.9.

### A.1.7 Synthesis of thiazole-4-carboxylic acid derivatives (general procedure)

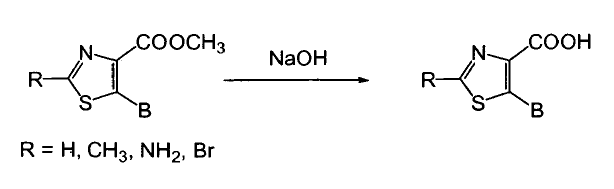

A solution of the respective thiazole-4-carboxylic acid ester (96.2 mmol) in a mixture of THF (150 mL) and MeOH (50 mL) is treated with an aq. NaOH solution (1.0 M, 192 mL). After stirring for 3 h a white suspension is formed and the organic volatiles are removed in vacuo. The remaining mixture is diluted with water (100 mL), cooled in an ice-bath and made acidic (pH = 3-4) by addition of aq. HCl solution (1.0 M). The suspension is filtered and the residue is washed with cold water. After drying the desired acid is obtained as a white solid.

### 2-methyl-5-m-tolyl-thiazole-4-carboxylic acid

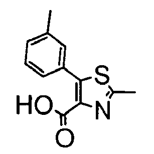

prepared by saponification of 2-methyl-5-m-tolyl-thiazole-4-carboxylic acid methyl ester. LC-MS: t_{R} = 0.83 min; [M+H]⁺ = 234.0.

### 2-methyl-5-p-tolyl-thiazole-4-carboxylic acid

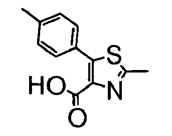

prepared by saponification of 2-methyl-5-p-tolyl-thiazole-4-carboxylic acid methyl ester. LC-MS: t_{R} = 0.83 min; [M+H]⁺ = 234.0.

### 5-(4-ethyl-phenyl)-2-methyl-thiazole-4-carboxylic acid

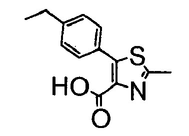

prepared by saponification of 5-(4-ethyl-phenyl)-2-methyl-thiazole-4-carboxylic acid methyl ester. LC-MS: t_{R} = 0.88 min; [M+H]⁺ = 248.0.

### 5-(3-fluoro-phenyl)-2-methyl-thiazole-4-carboxylic acid

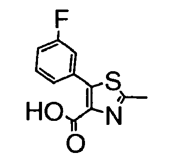

prepared by saponification of 5-(3-fluoro-phenyl)-2-methyl-thiazole-4-carboxylic acid methyl ester. LC-MS: t_{R} = 0.82 min; [M+H]⁺ = 238.1.

### 5-(4-fluoro-phenyl)-2-methyl-thiazole-4-carboxylic acid

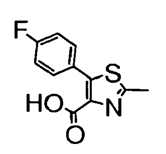

prepared by saponification of 5-(4-fluoro-phenyl)-2-methyl-thiazole-4-carboxylic acid methyl ester. ¹H-NMR (DMSO-d₆): δ = 2.67 (s, 3H); 7.27 (m, 2H); 7.53 (m, 2H); 12.89 (br.s, 1H).

### 2-methyl-5-(4-trifluoromethyl-phenyl)-thiazole-4-carboxylic acid

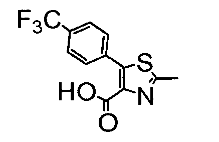

prepared by saponification of 2-methyl-5-(4-trifluoromethyl-phenyl)-thiazole-4-carboxylic acid methyl ester. LC-MS: t_{R} = 0.90 min; [M+H]⁺ = 288.0.

### 2-methyl-5-(3-trifluoromethyl-phenyl)-thiazole-4-carboxylic acid

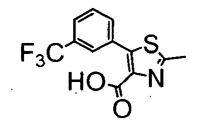

prepared by saponification of 2-methyl-5-(3-trifluoromethyl-phenyl)-thiazole-4-carboxylic acid methyl ester. LC-MS: t_{R} = 0.88 min; [M+H]⁺ = 288.0.

### 2-methyl-5-(2-trifluoromethyl-phenyl)-thiazole-4-carboxylic acid

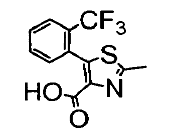

prepared by saponification of 2-methyl-5-(2-trifluoromethyl-phenyl)-thiazole-4-carboxylic acid methyl ester. LC-MS: t_{R} = 0.84 min; [M+H]⁺ = 288.3.

### 5-(2-fluoro-phenyl)-2-methyl-thiazole-4-carboxylic acid

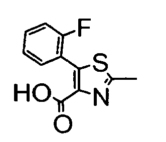

prepared by saponification of 5-(2-fluoro-phenyl)-2-methyl-thiazole-4-carboxylic acid methyl ester. LC-MS: t_{R} = 0.78 min; [M+H]⁺ = 238.3.

### 5-(2-chloro-phenyl)-2-methyl-thiazole-4-carboxylic acid

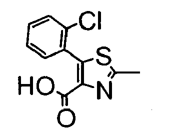

prepared by saponification of 5-(2-chloro-phenyl)-2-methyl-thiazole-4-carboxylic acid methyl ester. LC-MS: t_{R} = 0.82 min; [M+H]⁺ = 253.9.

### 5-(3-chloro-phenyl)-2-methyl-thiazole-4-carboxylic acid

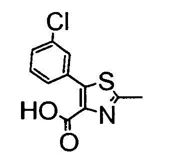

prepared by saponification of 5-(3-chloro-phenyl)-2-methyl-thiazole-4-carboxylic acid methyl ester. LC-MS: t_{R} = 0.84 min; [M+H]⁺ = 254.0.

### 2-methyl-5-o-tolyl-thiazole-4-carboxylic acid

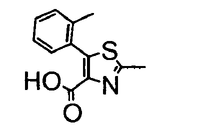

prepared by saponification of 2-methyl-5-o-tolyl-thiazole-4-carboxylic acid methyl ester. LC-MS: t_{R} = 0.80 min; [M+H]⁺ = 234.3.

### 5-(3-methoxy-phenyl)-2-methyl-thiazole-4-carboxylic acid

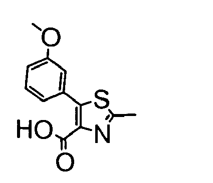

prepared by saponification of 5-(3-methoxy-phenyl)-2-methyl-thiazole-4-carboxylic acid methyl ester. LC-MS: t_{R} = 0.80 min; [M+H]⁺ = 250.0.

### 5-(2-methoxy-phenyl)-2-methyl-thiazole-4-carboxylic acid

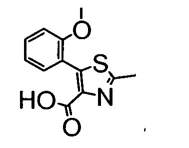

prepared by saponification of 5-(2-methoxy-phenyl)-2-methyl-thiazole-4-carboxylic acid methyl ester. LC-MS: t_{R} = 0.78 min; [M+H]⁺ = 250.0.

### 5-(3,4-dimethyl-phenyl)-2-methyl-thiazole-4-carboxylic acid

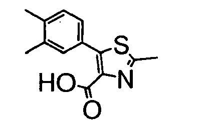

prepared by saponification of 5-(3,4-dimethyl-phenyl)-2-methyl-thiazole-4-carboxylic acid methyl ester. LC-MS: t_{R} = 0.86 min; [M+H]⁺ = 248.3.

### 5-(3-Cyano-phenyl)-2-methyl-thiazole-4-carboxylic acid

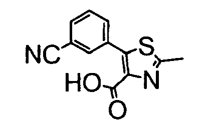

prepared by saponification of 5-(3-cyano-phenyl)-2-methyl-thiazole-4-carboxylic acid methyl ester. LC-MS: t_{R} = 0.76 min; [M+H]⁺ = 245.3.

### 5-[3-(2-Methoxy-ethoxy)-phenyl]-2-methyl-thiazole-4-carboxylic acid

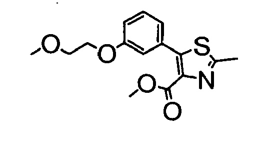

prepared by saponification of 5-[3-(2-methoxy-ethoxy)-phenyl]-2-methyl-thiazole-4-carboxylic acid methyl ester. LC-MS: t_{R} = 0.83 min; [M+H]⁺ = 294.3.

### 2-amino-5-m-tolyl-thiazole-4-carboxylic acid

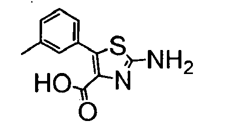

prepared by saponification of 2-amino-5-m-tolyl-thiazole-4-carboxylic acid methyl ester. LC-MS: t_{R} = 0.65 min; [M+H]⁺ = 235.0.

### 2-amino-5-(3-fluoro-phenyl)-thiazole-4-carboxylic acid

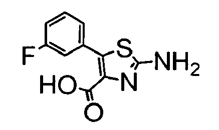

prepared by saponification of 2-amino-5-(3-fluoro-phenyl)-thiazole-4-carboxylic acid methyl ester. LC-MS: t_{R} = 0.62 min; [M+H]⁺ = 239.1.

### 2-amino-5-(3-chloro-phenyl)-thiazole-4-carboxylic acid

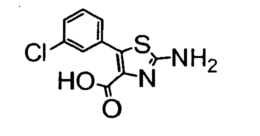

prepared by saponification of 2-amino-5-(3-chloro-phenyl)-thiazole-4-carboxylic acid methyl ester. LC-MS: t_{R} = 0.66 min; [M+H]⁺ = 255.2.

### 2-Bromo-5-m-tolyl-thiazole-4-carboxylic acid

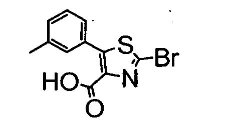

prepared by saponification of 2-Bromo-5-m-tolyl-thiazole-4-carboxylic acid methyl ester. LC-MS (basic): t_{R} = 0.57 min; [M+H]⁺ = 297.8.

### 5-m-Tolyl-thiazole-4-carboxylic acid

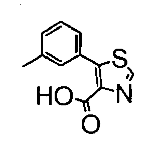

prepared by saponification of 5-m-tolyl-thiazole-4-carboxylic acid methyl ester. LC-MS: t_{R} = 0.86 min; [M+H]⁺ = 220.2.

### A.1.8 Synthesis of 2-methoxy-5-m-tolyl-thiazole-4-carboxylic acid

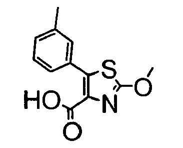

At 0°C under an atmosphere of nitrogen MeOH (0.96 mmol) is added to a suspension of sodium hydride (0.96 mmol) in THF (2.0 mL). After 5 min a solution of 2-bromo-5-m-tolyl-thiazole-4-carboxylic acid methyl ester (0.48 mmol) in DMF (0.2 mL) and THF (1.0 mL) is added dropwise. The mixture is stirred for 16 h at RT, cooled to 0°C and treated with water (0.5 mL) and aq. NaOH solution (1.0 M, 0.5 mL). After 2 h the solvents are removed in vacuo and the residue is dissolved in warm water (1.0 mL). Ether is added, the layers are separated and the aq. layer is concentrated partially in vacuo to remove traces of ether. The mixture is cooled to 0°C and made acidic (pH 4) by addition of hydrochloric acid (2.0 M). The precipitate is filtered off, washed with water and dried in vacuo to give the desired product. LC-MS: t_{R} = 0.88 min; [M+H]⁺ = 250.3.

### A.2 Synthesis of benzo[1,4]oxazine-carboxylic acid derivatives

### A.2.1 Synthesis of 3-amino-2-hydroxy-benzoic acid methyl ester

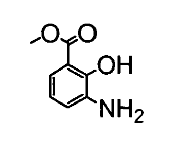

A solution of methyl 3-nitrosalicylate (26.6 mmol) in MeOH (50 mL) is treated with Pd/C (10%, 500 mg) and stirred at RT under a hydrogen atmosphere (1 bar) for 16 h. After filtration through celite and removal of the solvents the desired product is obtained which is used without further purification. LC-MS: t_{R} = 0.51 min; [M+H]⁺ = 168.0.

### A.2.2 Synthesis of 3-oxo-3,4-dihydro-2H-benzo[1,4]oxazine-8-carboxylic acid methyl ester

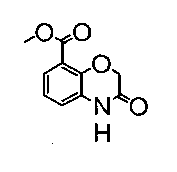

At RT chloro-acetyl chloride (29.0 mmol) is added dropwise to a solution of 3-amino-2-hydroxy-benzoic acid methyl ester (26.4 mmol) in DMF (100 mL). After 20 min K₂CO₃ (126 mmol) is added portionwise, the mixture is stirred for 16h at RT and the solvents are removed in vacuo. Water and DCM are added, the layers are separated and the organic layer is washed with brine and dried over Na₂SO₄. The solvents are removed in vacuo to give a crude product which is used without further purification. LC-MS: t_{R} = 0.68 min; [M+H]⁺ = 208.0.

### A.2.3 Synthesis of 4-methyl-3-oxo-3,4-dihydro-2H-benzo[1,4]oxazine-8-carboxylic acid methyl ester

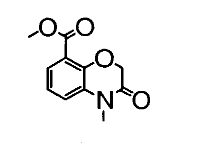

K₂CO₃ (6.66 mmol) is added to a solution of 3-oxo-3,4-dihydro-2H-benzo[1,4]oxazine-8-carboxylic acid methyl ester (2.90 mmol) in DMF (10 mL). After 30 min methyl iodide (5.79 mmol) is added and the mixture is stirred for 2h at 75°C. Cold water and EtOAc are added, the layers are separated and the aq. layer is extracted with EtOAc. The combined organic layers are washed with water and brine, dried over MgSO₄ and concentrated in vacuo to give a crude product which is used without further purification. LC-MS: t_{R} = 0.76 min; [M+H]⁺ = 222.2.

### A.2.4 Synthesis of 3,4-dihydro-2H-benzo[1,4]oxazine-8-carboxylic acid methyl ester

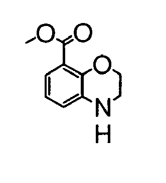

Boron trifluoride diethyl etherate (10.1 mmol) is added dropwise to a mixture of 3-oxo-3,4-dihydro-2H-benzo[1,4]oxazine-8-carboxylic acid methyl ester (4.83 mmol) in THF (12 mL) to keep the temperature below 5°C. After 20 min NaBH₄ (10.1 mmol) is added and the mixture is stirred at 5°C for 60 min. EtOAc (6.0 mL) and hydrochloric acid (1.0 M, 6.0 mL) are added dropwise. The mixture is made basic by addition of sat. aq. NaHCO₃ solution, the layers are separated and the aq. layer is extracted with EtOAc. The combined organic layers are dried over MgSO₄ and concentrated in vacuo to give a crude product which is purified by CC (heptane to heptane/EtOAc 3/7). LC-MS: t_{R} = 0.69 min; [M+H]⁺ = 194.0.

### A.2.5 Synthesis of 4-methyl-3,4-dihydro-2H-benzo[1,4]oxazine-8-carboxylic acid methyl ester

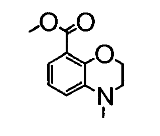

K₂CO₃ (4.76 mmol) is added to a solution of 3,4-dihydro-2H-benzo[1,4]oxazine-8-carboxylic acid methyl ester (2.07 mmol) in DMF (3.0 mL). After 30 min methyl iodide (4.14 mmol) is added and the mixture is stirred for 2h at 75°C. Cold water and EtOAc are added, the layers are separated and the aq. layer is extracted with EtOAc. The combined organic layers are washed with water and brine, dried over MgSO₄ and concentrated in vacuo to give a crude product which is used without further purification. LC-MS: t_{R} = 0.83 min; [M+H]⁺ = 208.1.

### A.2.6 Synthesis of 2-amino-3-hydroxy-benzoic acid methyl ester

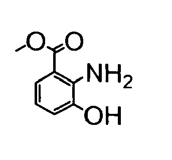

A solution of (trimethylsilyl)diazomethane in hexane (2.0 M, 10.9 mmol) is added dropwise (10 min) to a mixture of 3-hydroxyanthranilic acid (9.93 mmol) in MeOH (10.5 mL) and toluene (42 mL). The mixture is stirred for 16h, concentrated in vacuo, diluted with ether and EtOAc and washed several times with water. The organic layer is dried over MgSO₄ and concentrated under reduced pressure. The residue is purified by CC (heptane to heptane/EtOAc 7/3) to give the desired ester as a brown solid. LC-MS: t_{R} = 0.70 min; [M+H]⁺= 168.0.

### A.2.7 Synthesis of 3-oxo-3,4-dihydro-2H-benzo[1,4]oxazine-5-carboxylic acid methyl ester

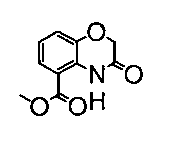

At RT chloro-acetyl chloride (8.06 mmol) is added dropwise to a solution of 2-amino-3-hydroxy-benzoic acid methyl ester (7.33 mmol) in DMF (50 mL). After 20 min K₂CO₃ (34.9 mmol) is added portionwise, the mixture is stirred for 16h at RT and the solvents are removed in vacuo. Water and DCM are added, the layers are separated and the organic layer is washed with brine and dried over Na₂SO₄. The solvents are removed in vacuo to give a crude product which is purified by CC (heptane to heptane/EtOAc 6/4). LC-MS: t_{R} = 0.82 min; [M+CH₃CN+H]⁺ = 249.0.

### A.2.8 Synthesis of 3,4-dihydro-2H-benzo[1,4]oxazine-5-carboxylic acid methyl ester

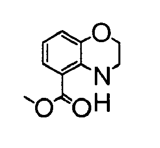

Boron trifluoride diethyl etherate (7.10 mmol) is added dropwise to a mixture of 3-oxo-3,4-dihydro-2H-benzo[1,4]oxazine-5-carboxylic acid methyl ester (3.38 mmol) in THF (10 mL) to keep the temperature below 5°C. After 20 min NaBH₄ (7.10 mmol) is added and the mixture is stirred at 5°C for 90 min. EtOAc (6.0 mL) and hydrochloric acid (1.0 M, 6.0 mL) are added dropwise. The mixture is made basic by addition of aq. Na₂CO₃ solution, the layers are separated and the aq. layer is extracted with EtOAc. The combined organic layers are dried over MgSO₄ and concentrated in vacuo to give a crude product which is purified by CC (heptane to heptane/EtOAc 3/7). LC-MS: t_{R} = 0.90 min; [M+CH₃CN+H]⁺ = 235.3.

### A.2.9 Synthesis of 4-methyl-3,4-dihydro-2H-benzo[1,4]oxazine-5-carboxylic acid methyl ester

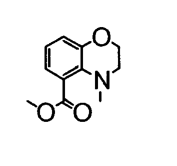

K₂CO₃ (1.79 mmol) is added to a solution of 3,4-dihydro-2H-benzo[1,4]oxazine-5-carboxylic acid methyl ester (0.78 mmol) in DMF (1.0 mL). After 30 min methyl iodide (1.55 mmol) is added and the mixture is stirred for 2h at 75°C. Cold water and EtOAc are added, the layers are separated and the aq. layer is extracted with EtOAc. The combined organic layers are washed with water and brine, dried over MgSO₄ and concentrated in vacuo to give a crude product which is used without further purification. LC-MS: t_{R} = 0.71 min; [M+H]⁺ = 208.1.

### A.2.10 Synthesis of benzo[1,4]oxazine-carboxylic acid derivatives by ester hydrolysis (general procedure)

A solution of NaOH (4.00 mmol) in a mixture of MeOH (3.0 mL) and water (6.8 mL) is added to the respective ester derivative (2.00 mmol). The mixture is stirred at 55°C for 16h, partially concentrated in vacuo to remove MeOH and made acidic by addition of hydrochloric acid (1.0M). The respective carboxylic acid precipitates and is collected by filtration.

### 3,4-dihydro-2H-benzo[1,4]oxazine-8-carboxylic acid

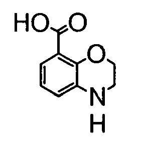

prepared by saponification of 3,4-dihydro-2H-benzo[1,4]oxazine-8-carboxylic acid methyl ester. LC-MS: t_{R} = 0.55 min; [M+H]⁺ = 180.0.

### 4-methyl-3,4-dihydro-2H-benzo[1,4]oxazine-8-carboxylic acid

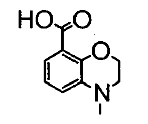

prepared by saponification of 4-methyl-3,4-dihydro-2H-benzo[1,4]oxazine-8-carboxylic acid methyl ester. LC-MS: t_{R}= 0.72 min; [M+H]⁺ = 194.1.

### 3,4-dihydro-2H-benzo[1,4]oxazine-5-carboxylic acid

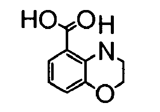

prepared by saponification of 3,4-dihydro-2H-benzo[1,4]oxazine-5-carboxylic acid methyl ester. LC-MS: t_{R} = 0.76 min; [M+H]⁺ = 180.2.

### 4-methyl-3,4-dihydro-2H-benzo[1,4]oxazine-5-carboxylic acid

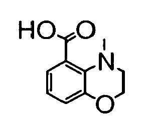

prepared by saponification of 4-methyl-3,4-dihydro-2H-benzo[1,4]oxazine-5-carboxylic acid methyl ester. LC-MS: t_{R} = 0.55 min; [M+H]⁺ = 194.1.

### 3-oxo-3,4-dihydro-2H-benzo[1,4]oxazine-8-carboxylic acid

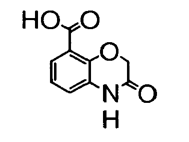

prepared by saponification of 3-oxo-3,4-dihydro-2H-benzo[1,4]oxazine-8-carboxylic acid methyl ester. LC-MS: t_{R} = 0.56 min; [M+CH₃CN+H]⁺ = 235.0.

### 4-methyl-3-oxo-3,4-dihydro-2H-benzo[1,4]oxazine-8-carboxylic acid

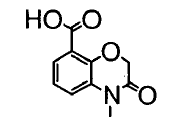

prepared by saponification of 4-methyl-3-oxo-3,4-dihydro-2H-benzo[1,4]oxazine-8-carboxylic acid methyl ester. LC-MS: t_{R} = 0.64 min; [M+CH₃CN+H]⁺ = 249.3.

### 3-Oxo-3,4-dihydro-2H-benzo[1,4]oxazine-5-carboxylic acid

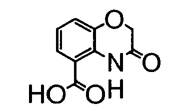

prepared by saponification of 3-oxo-3,4-dihydro-2H-benzo[1,4]oxazine-5-carboxylic acid methyl ester. LC-MS: t_{R} = 0.71 min; [M+CH₃CN+H]⁺ = 235.1.

### A.3 Synthesis of chroman-carboxylic acid derivatives

### A.3.1 Synthesis of 3-prop-2-ynyloxy-benzoic acid methyl ester

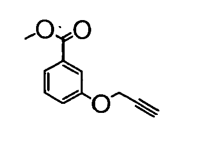

A solution of propargyl bromide in toluene (80%, 68.7 mmol, 7.40 mL) is added to a solution of 3-hydroxy-benzoic acid methyl ester (48.6 mmol) in DMF (45 mL). K₂CO₃ is added and the mixture is stirred at RT for 4h. Water and ether are added, the layers are separated and the organic layer is washed with aq. NaOH solution (5%) and brine. The solvents are removed in vacuo to give the desired ester as a pale yellow solid. ¹H-NMR (CDCl₃): δ = 2.56 (s, 1H); 3.94 (s, 3H); 4.76 (s, 2H); 7.20 (d, J = 8.04 Hz, 1H); 7.39 (t, J = 8.16 Hz, 1H); 7.66 (bs, 1H); 7.71 (d, J = 7.78 Hz, 1H).

### A.3.2 Synthesis of 2H-chromene-5-carboxylic acid methyl ester

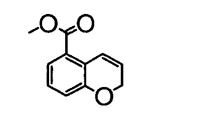

A solution of 3-prop-2-ynyloxy-benzoic acid methyl ester (10.5 mmol) in N,N-diethylaniline (20 mL) is heated to reflux for 15h. The mixture is cooled to RT, diluted with ether and washed with hydrochloric acid (5%) and brine. The solvents are removed in vacuo and the residue is purified by chromatography (silica, heptane to heptane/EtOAc 95/5) to give the desired chromene derivative. ¹H-NMR (CDCl₃): δ = 3.91 (s, 3H); 4.80 (bs, 2H); 5.93-5.98 (m, 1H); 6.99 (d, J = 8.03 Hz, 1H); 7.16 (t, J = 7.66 Hz, 1H); 7.34 (d, J = 10.3 Hz, 1H); 7.50 (d, J = 7.28 Hz, 1H).

### A.3.3 Synthesis of 2H-chromene-5-carboxylic acid

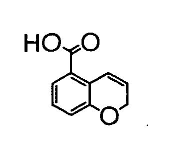

A solution of NaOH (7.26 mmol) in a mixture of MeOH (5.4 mL) and water (12.1 mL) is added to 2H-chromene-5-carboxylic acid methyl ester (4.84 mmol). The mixture is stirred at 55°C for 3h, partially concentrated in vacuo to remove MeOH and made acidic by addition of hydrochloric acid (1.0M). The desired carboxylic acid precipitates and is collected by filtration. ¹H-NMR (DMSO-d₆): δ = 4.75 (bs, 2H); 5.99-6.05 (m, 1H); 6.98 (d, J = 7.78 Hz, 1H); 7.19 (t, J = 7.78 Hz, 1H); 7.25 (d, J = 10.3 Hz, 1H); 7.40 (d, J = 7.78 Hz, 1H); 13.0 (bs, 1H).

### A.3.4 Synthesis of chroman-5-carboxylic acid

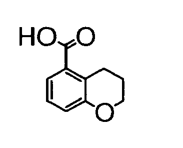

A solution of 2H-chromene-5-carboxylic acid (1.42 mmol) in MeOH (5.0 mL) is treated with Pd/C (10%, 50 mg) and stirred at RT under a hydrogen atmosphere (1 bar) for 16 h. After filtration through celite and removal of the solvents the desired product is obtained which is used without further purification. ¹H-NMR (DMSO-d₆): δ = 1.90 (m, 2H); 2.98 (m, 2H); 4.13 (m, 2H); 6.89-6.94 (m, 1H); 7.11-7.17 (m, 1H); 7.31-7.36 (m, 1H); 12.8 (bs, 1H).

### A.3.5 Synthesis of chroman

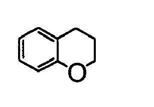

A solution of 4-chromanone (19.6 mmol) in HOAc (30 ml) is added to a suspension of zinc powder (445 mmol) in HOAc (60 ml). The mixture is stirred at 100°C for 4h, cooled to RT, filtered through celite and concentrated in vacuo. EtOAc and aq. NaOH solution (1.0 M) are added, the layers are separated and the aq. layer is extracted twice with EtOAc. The combined organic layers are dried over MgSO₄ and concentrated in vacuo to give the desired product which is used without further purification. ¹H-NMR (CDCl₃): δ = 2.04 (m, 2H); 2.82 (m, 2H); 4.21 (m, 2H); 6.80-6.89 (m, 2H); 7.04-7.14 (m, 2H).

### A.3.6 Synthesis of chroman-8-carboxylic acid

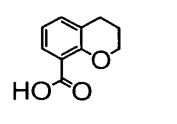

At RT a solution of chroman (17.7 mmol) in ether (15 mL) is added over 10 min to a solution of n-BuLi (19.5 mmol) in a mixture of hexane (12.2 mL) and ether (15 mL). The mixture is stirred at reflux for 150 min, allowed to reach RT and poured into a mixture of dry ice and ether. Ice water is added and the layers are separated. The aq. layer is made acidic and extracted with a mixture of ether and EtOAc. The combined organic layers are washed with water, dried over Na₂SO₄ and concentrated in vacuo to give a crude product which is purified by CC (heptane/EtOAc 9/1 to EtOAc). LC-MS: t_{R} = 0.76 min; [M+CH₃CN+H]⁺ = 220.1.

### A.4 Synthesis of 2,3-dihydro-benzofuran-4-carboxylic acid

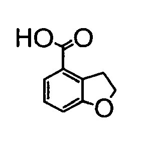

Benzofuran-4-carboxylic acid (30.8 mmol, M.A. Eissenstat et al. J. Med. Chem. 1995, 38, 3094-3105) is added to a suspension of Pd/C (10%, 2.00 g) in EtOH (25 mL). Additional EtOH (75 mL) is added and the mixture is stirred at RT under a hydrogen atmosphere (4 bar) for 16 h. After filtration through celite and removal of the solvents the desired product is obtained which is used without further purification. ¹H-NMR (DMSO-d₆): δ = 3.45 (t, J = 8.79 Hz, 2H); 4.55 (t, J = 8.79 Hz, 2H); 6.99 (d, J = 7.78 Hz, 1H); 7.21 (t, J = 7.91 Hz, 1H); 7.39 (d, J = 7.78 Hz, 1H); 12.9 (bs, 1H).

### A.5 Synthesis of 2,3,6,7-Tetrahydro-benzo[1,2-b;4,5-b']difuran-4-carboxylic acid

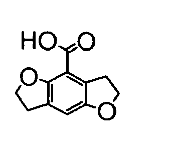

2;3,6,7-Tetrahydro-benzo[1,2-b;4,5-b']difuran-4-carbaldehyde (0.25 mmol; D.E. Nichols et al. J. Med. Chem. 1996, 39, 2953-2961) is added to a suspension of silver oxide (0.38 mmol) in aq. NaOH solution (5%, 2.0 mL). After stirring for 5 h the mixture is filtered and the residue is washed with water (2.0 mL). The filtrate is cooled to 0°C, made acidic by addition of hydrochloric acid (25%) and filtered. The residue is washed with cold water (2.0 mL) and heptane and dried in vacuo to give the desired product. LC-MS (basic): t_{R} = 0.20 min; [M-H]⁻ = 205.2.

### A.6 Synthesis of imidazo[2,1-b]thiazole derivatives

### A.6.1 Synthesis of 2-amino-4-methyl-thiazole-5-carboxylic acid methyl ester

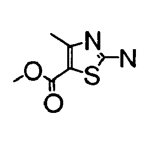

A mixture of thiourea (59.8 mmol) and 2-chloro-3-oxo-butyric acid methyl ester (59.8 mmol) in EtOH (140 mL) is heated at reflux for 14h and concentrated in vacuo. Water and aq. NaHCO₃ are added and the mixture is extracted several times with EtOAc. The combined organic layers are dried and concentrated in vacuo to give the desired amino-thiazole derivative. LC-MS: t_{R} = 0.51 min; [M+H]⁺ = 173.0.

### A.6.2 Synthesis of 3-methyl-imidazo[2,1-b]thiazole-2-carboxylic acid methyl ester

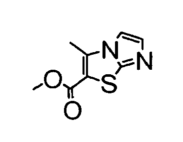

A mixture of bromoacetaldehyde diethyl acetal (29.3 mmol, 1.26eq) in water (200 mL) is treated dropwise with conc. hydrochloric acid (3.0 mL), stirred for 14h at RT and heated for additional 30 min at 80°C. After cooling to RT NaHCO₃ (37.9 mmol) is added carefully and the mixture is stirred for 2h and treated with 2-Amino-4-methyl-thiazole-5-carboxylic acid methyl ester (23.2 mmol, 1.00eq). After 1h dioxane (130 mL) is added and the mixture is stirred at RT for 30 min and at 100°C for 48h. The organic solvents are removed in vacuo and the mixture is extracted several times with DCM and chloroform. The combined organic layers are dried over Na₂SO₄ and concentrated in vacuo to give the desired ester which is used without further purification. LC-MS: t_{R} = 0.55 min; [M+H]⁺ = 197.0.

### A.6.3 Synthesis of 2-bromo-3-oxo-butyric acid ethyl ester

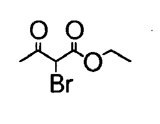

At -5°C trimethylsilyl trifluoromethanesulfonate (36.9 mmol) is added dropwise to a solution of ethyl acetoacetate (30.7 mmol) and NEt₃ (36.9 mmol) in DCM (50 mL). The solution is stirred for 90 min at 0°C and treated over 30 min with a solution of bromine (30.7 mmol) in DCM (20 mL). After 60 min water (100 mL) is added, the layers are separated and the aq. layer is extracted three times with water (100 mL each). The organic layer is dried over MgSO₄ and concentrated under reduced pressure to give the desired product which is used without further purification. ¹H-NMR (CDCl₃): δ = 1.34 (t, J = 7.16 Hz, 3H); 2.46 (s, 3H); 4.31 (q, J = 7.20 Hz, 2H); 4.77 (s, 1H).

### A.6.4 Synthesis of 2,6-dimethyl-imidazo[2,1-b]thiazole-5-carboxylic acid ethyl ester

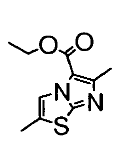

A mixture of 5-methyl-2-aminothiazole (7.09 mmol) and 2-bromo-3-oxo-butyric acid ethyl ester (8.51 mmol) in acetone (17 mL) is stirred for 16h at RT and for additional 7h at reflux. The solvents are removed in vacuo, chloroform and sat aq. NaHCO₃ solution are added, the layers are separated and the aq. layer is extracted with chloroform. The combined organic layers are dried over MgSO₄ and concentrated in vacuo to give a crude product which is purified by CC (heptane to heptane/EtOAc 6/4). LC-MS: t_{R} = 0.80 min; [M+H]⁺ = 225.3.

### A.6.5 Synthesis of 5-methyl-2-thioxo-2,3-dihydro-1H-imidazole-4-carboxylic acid ethyl ester

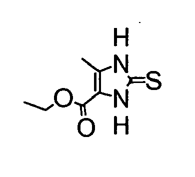

Pd/C (10%, 1.00 g) is added to a solution of 2-hydroxyimino-3-oxo-butyric acid ethyl ester (62.8 mmol) in hydrochloric acid (1.25 M in EtOH, 75 mL) and the mixture is stirred at RT under a hydrogen atmosphere (4 bar) for 48 h. After filtration through celite and removal of the solvents crude 2-amino-3-oxo-butyric acid ethyl ester hydrochloride is obtained which is dissolved in a mixture of water (220 mL), EtOH (30 mL) and conc hydrochloric acid (37%, 2.5 mL). A solution of potassium thiocyanate (49.9 mmol) in water (25 mL) is added and the mixture is stirred for 2h at reflux. By cooling in an ice bath the desired product precipitates and is collected by filtration. LC-MS: t_{R} = 0.59 min; [M+H]⁺ = 187.2.

### A.6.6 Synthesis of 3,5-dimethyl-imidazo[2,1-b]thiazole-6-carboxylic acid ethyl ester

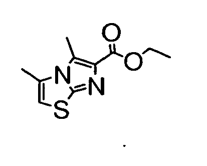

A mixture of 5-methyl-2-thioxo-2,3-dihydro-1H-imidazole-4-carboxylic acid ethyl ester (5.37 mmol) and chloroacetone (6.44 mmol) in EtOH (10 mL) is heated at reflux for 150 min. The solvents are removed in vacuo and a solution of POCl₃ (16.1 mmol) in MeCN (10 mL) is added. The mixture is stirred at reflux for 60 h, concentrated in vacuo and diluted with chloroform. Ice water is added and the mixture is neutralized by addition of Na₂CO₃. The layers are separated and the aq. layer is extracted with chloroform. The combined organic layers are dried over Na₂SO₄ and concentrated in vacuo to give a mixture of two regioisomers (see **A.6.7**) which are separated by CC (heptane to heptane/EtOAc 3/7). LC-MS: t_{R} = 0.71 min; [M+H]⁺ = 225.0.

### A.6.7 Synthesis of 3,6-dimethyl-imidazo[2,1-b]thiazole-5-carboxylic acid ethyl ester

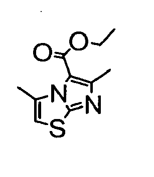

3,6-dimethyl-imidazo[2,1-b]thiazole-5-carboxylic acid ethyl ester is obtained as a side-product in the synthesis of 3,5-dimethyl-imidazo[2,1-b]thiazole-6-carboxylic acid ethyl ester (see **A.6.6**). LC-MS: t_{R} = 0.81 min; [M+H]⁺ = 225.0.

### A.6.8 Synthesis of 3-Methyl-imidazo[2,1-b]thiazole-6-carboxylic acid ethyl ester

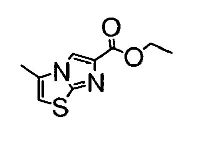

A mixture of 2-thioxo-2,3-dihydro-1H-imidazole-4-carboxylic acid ethyl ester (5.81 mmol) and chloroacetone (6.97 mmol) in EtOH (8.0 mL) is heated at reflux for 120 min. The solvents are removed in vacuo and POCl₃ (87.1 mmol) is added. The mixture is stirred at RT for 16 h and at reflux for 4 h, concentrated in vacuo and diluted with chloroform. Ice water is added and the mixture is neutralized by addition of Na₂CO₃. The layers are separated and the aq. layer is extracted with chloroform. The combined organic layers are dried over Na₂SO₄ and concentrated in vacuo to give a crude product which is purified by CC (heptane/EtOAc 9/1 to heptane/EtOAc 3/7). LC-MS: t_{R} = 0.73 min; [M+H]⁺ = 211.0.

### A.6.9 Synthesis of 2,3,6-trimethyl-imidazo[2,1-b]thiazole-5-carboxylic acid ethyl ester and of 2,3,5-trimethyl-imidazo[2,1-b]thiazole-6-carboxylic acid ethyl ester

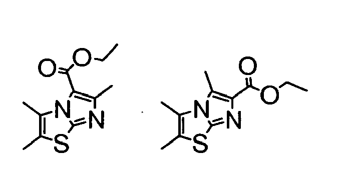

A mixture of 5-methyl-2-thioxo-2,3-dihydro-1H-imidazole-4-carboxylic acid ethyl ester (10.7 mmol) and 3-bromo-2-butanone (10.7 mmol) in EtOH (16 mL) is heated at reflux for 3h. The solvents are removed in vacuo and POCl₃ (161 mmol) is added. The mixture is stirred at reflux for 3h, concentrated in vacuo and diluted with chloroform. Ice water is added and the mixture is neutralized by addition of Na₂CO₃. The layers are separated and the aq. layer is extracted with chloroform. The combined organic layers are dried over Na₂SO₄ and concentrated in vacuo to give a mixture of two regioisomers which are separated by CC (heptane/EtOAc 9/1 to EtOAc). 2,3,6-trimethyl-imidazo[2,1-b]thiazole-5-carboxylic acid ethyl ester is obtained as major isomer. LC-MS: t_{R} = 0.84 min; [M+H]⁺ = 239.0; 2,3,5-trimethyl-imidazo[2,1-b]thiazole-6-carboxylic acid ethyl ester is obtained as minor isomer. LC-MS: t_{R} = 0.76 min; [M+H]⁺ = 239.0.

### A.6.10 Synthesis of 2-(2,2-dialkoxy-ethylsulfanyl)-5-methyl-1H-imidazole-4-carboxylic acid ethyl ester derivatives (general procedure)

A solution of sodium ethoxide (5.37 mmol) in ethanol (3.3 mL) is added to a solution of 5-methyl-2-thioxo-2,3-dihydro-1H-imidazole-4-carboxylic acid ethyl ester (5.37 mmol) in ethanol (7.0 mL). The respective alkyl bromide (5.37 mmmol) is added and the mixture is stirred at reflux for 12h. After cooling to RT the mixture is filtered and concentrated in vacuo to give the desired product which is used without further purification.

### 2-(2,2-diethoxy-ethylsulfanyl)-5-methyl-1H-imidazole-4-carboxylic acid ethyl ester

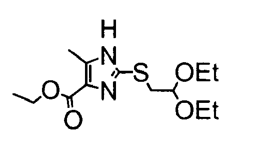

prepared by reaction of 5-methyl-2-thioxo-2,3-dihydro-1H-imidazole-4-carboxylic acid ethyl ester with bromoacetaldehyde diethyl acetal. LC-MS: t_{R} = 0.70 min; [M+H]⁺ = 303.4.

### 2-(2,2-Dimethoxy-1-methyl-ethylsulfanyl)-5-methyl-1H - imidazole-4-carboxylic acid ethyl ester

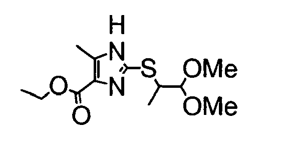

prepared by reaction of 5-methyl-2-thioxo-2,3-dihydro-1H-imidazole-4-carboxylic acid ethyl ester with 2-bromo-1,1-dimethoxy-propane. LC-MS: t_{R} = 0.67 min; [M+H]⁺ = 289.0.

### A.6.11 Synthesis of 3-hydroxy-2,3-dihydro-imidazo[2,1-b]thiazole-6-carboxylic acid ethyl ester derivatives (general procedure)

A mixture of the respective 2-(2,2-dialkoxy-ethylsulfanyl)-5-methyl-1H-imidazole-4-carboxylic acid ethyl ester derivative (10.0 mmol) in hydrochloric acid (15%, 8.0 mL) is stirred for 1h at RT and neutralized by addition of aq. Na₂CO₃ solution. The obtained precipitate is filtered off to give the desired product which is used without further purification.

### 3-hydroxy-5-methyl-2,3-dihydro-imidazo[2,1-b]thiazole-6-carboxylic acid ethyl ester

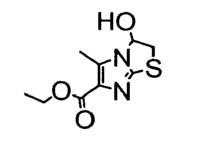

prepared by cyclization of 2-(2,2-diethoxy-ethylsulfanyl)-5-methyl-1H-imidazole-4-carboxylic acid ethyl ester. LC-MS: t_{R} = 0.55 min; [M+H]⁺ = 229.3.

### 3-hydroxy-2,5-dimethyl-2,3-dihydro-imidazo[2,1-b]thiazole-6-carboxylic acid ethyl ester

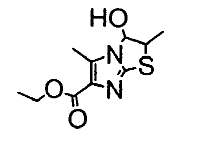

prepared by cyclization of 2-(2,2-Dimethoxy-1-methyl-ethylsulfanyl)-5-methyl-1H-imidazole-4-carboxylic acid ethyl ester. LC-MS: t_{R} = 0.60 min; [M+H]⁺ = 243.2.

### A.6.12 Synthesis of imidazo[2,1-b]thiazole-6-carboxylic acid ethyl ester derivatives (general procedure)

The respective 3-hydroxy-2,3-dihydro-imidazo[2,1-b]thiazole-6-carboxylic acid ethyl ester derivative (4.00 mmol) is added to POCl₃ (9.3 mL), stirred at reflux for 3h (respectively 16h) and concentrated in vacuo. Chloroform and ice-water are added successively and the mixture is neutralized by addition of Na₂CO₃. The layers are separated and the aq. layer is extracted with chloroform. The combined organic layers are dried over Na₂SO₄ and concentrated in vacuo to give the desired product which is purified by CC (heptane/EtOAc 1/1 to EtOAc).

### 5-methyl-imidazo[2,I-b]thiazole-6-carboxylic acid ethyl ester

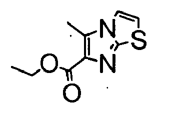

prepared by dehydration of 3-hydroxy-5-methyl-2,3-dihydro-imidazo[2,1-b]thiazole-6-carboxylic acid ethyl ester. LC-MS: t_{R} = 0.66 min; [M+H]⁺ = 211.0.

### 2,5-dimethyl-imidazo[2,1-b]thiazole-6-carboxylic acid ethyl ester

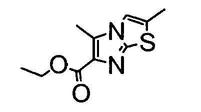

prepared by dehydration of 3-hydroxy-2,5-dimethyl-2,3-dihydro-imidazo[2,1-b]thiazole-6-carboxylic acid ethyl ester. LC-MS: t_{R} = 0.72 min; [M+H]⁺ = 225.0.

### A.6.13 Synthesis of N,N-dimethyl-N'-thiazol-2-yl-formamidine derivatives (general procedure)

N,N-Dimethylformamide dimethyl acetale (89.9 mmol, 2.0eq) is added dropwise to a solution of the respective 2-aminothiazole (44.9 mmol, 1.0eq) in toluene (30 mL). The mixture is heated at reflux for 22h, cooled to RT and concentrated in vacuo. A small amount of hexane is added and the obtained precipitate is filtered off to give the respective formamidine derivative.

### N,N-dimethyl-N'-thiazol-2-yl-formamidine

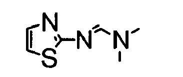

prepared by reaction of 2-aminothiazole with N,N-dimethylformamide dimethyl acetale. LC-MS: t_{R} = 0.40 min; [M+H]⁺ = 156.0.

### N,N-dimethyl-N'-(5-methyl-thiazol-2-yl)-formamidine

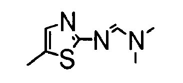

prepared by reaction of 5-methyl-thiazol-2-ylamine with N,N-dimethylformamide dimethyl acetate. LC-MS: t_{R} = 0.52 min; [M+H]⁺ = 170.2.

### N,N-dimethyl-N'-(4-methyl-thiazol-2-yl)-formamidine

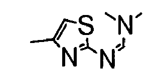

prepared by reaction of 4-methyl-thiazol-2-ylamine with N,N-dimethylformamide dimethyl acetale. LC-MS: t_{R} = 0.51 min; [M+H]⁺ = 170.1.

### N'-(4,5-dimethyl-thiazol-2-yl)-N,N-dimethyl-formamidine

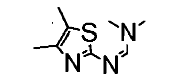

prepared by reaction of 4,5-dimethyl-thiazol-2-ylamine with N,N-dimethylformamide dimethyl acetale. LC-MS: t_{R} = 0.56 min; [M+H]⁺ = 184.1.

### A.6.14 Synthesis of 3-ethoxycarbonylmethyl-thiazol-3-ium bromide derivatives (general procedure)

The respective N,N-dimethyl-N'-thiazol-2-yl-formamidine derivative (45.1 mmol, 1.00eq) is added portionwise to vigorously stirred ethyl bromoacetate (225 mmol, 5.0eq). After 2h toluene (12 mL) is added and the mixture is stirred for 24h. The obtained precipitate is filtered off and the residue is recrystallized from MeCN to give the respective thiazolium bromide.

### 2-(dimethylamino-methyleneamino)-3-ethoxycarbonylmethyl-thiazol-3-ium bromide

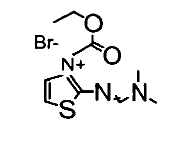

prepared by reaction of ethyl bromoacetate with N,N-dimethyl-N'-thiazol-2-yl-formamidine. LC-MS: t_{R} = 0.58 min; [M+H]⁺ = 242.1.

### 2-(dimethylamino-methyleneamino)-3-ethoxycarbonylmethyl-5-methyl-thiazol-3-ium bromide

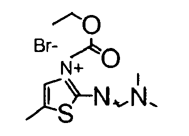

prepared by reaction of ethyl bromoacetate with N,N-dimethyl-N'-(5-methyl-thiazol-2-yl)-formamidine. LC-MS: t_{R} = 0.63 min; [M+H]⁺ = 256.2.

### 2-(dimethylamino-methyleneamino)-3-ethoxycarbonylmethyl-4-methyl-thiazol-3-ium bromide

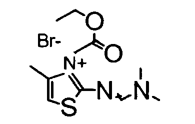

prepared by reaction of ethyl bromoacetate with N,N-dimethyl-N'-(4-methyl-thiazol-2-yl)-formamidine. LC-MS: t_{R} = 0.61 min; [M+H]⁺ = 256.0.

### 2-(dimethylamino-methyleneamino)-3-ethoxycarbonylmethyl-4,5-dimethyl-thiazol-3-ium bromide

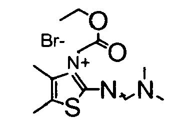

prepared by reaction of ethyl bromoacetate with N'-(4,5-dimethyl-thiazol-2-yl)-N,N-dimethyl-formamidine. LC-MS: t_{R} = 0.67 min; [M+H]⁺ = 270.1.

### A.6.15 Synthesis of imidazo[2,1-b]thiazole-5-carboxylic acid ethyl ester derivatives (general procedure)

DBU (68.9 mmol, 1.58eq) is added to a suspension of the respective thiazolium bromide derivative (43.6 mmol, 1.00eq) in DMF (50 mL). The solution is stirred for 24h and diluted with ice-cold water. The obtained precipitate is filtered off to give the respective imidazo-thiazole derivative.

### imidazo[2,1-b] thiazole-5-carboxylic acid ethyl ester

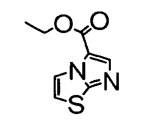

prepared by cyclisation of 2-(dimethylamino-methyleneamino)-3-ethoxycarbonylmethyl-thiazol-3-ium bromide. LC-MS: t_{R} = 0.76 min; [M+H]⁺ = 197.0.

### 2-methyl-imidazo[2,1-b]thiazole-5-carboxylic acid ethyl ester

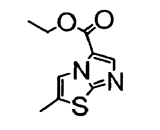

prepared by cyclisation of 2-(dimethylamino-methyleneamino)-3-ethorxycarbonyl-methyl-5-methyl-thiazol-3-ium bromide. LC-MS: t_{R} = 0.83 min; [M+H]⁺ = 211.0.

### 3-methyl-imidazo[2,1-b]thiazole-5-carboxylic acid ethyl ester

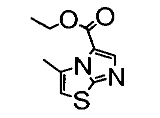

prepared by cyclisation of 2-(dimethylamino-methyleneamino)-3-ethoxycarbonylmethyl-4-methyl-thiazol-3-ium bromide. LC-MS: t_{R} = 0.83 min; [M+H]⁺ = 211.0.

### 2,3-dimethyl-imidazo[2,1-b]thiazole-5-carboxylic acid ethyl ester

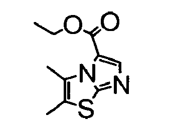

prepared by cyclisation of 2-(dimethylamino-methyleneamino)-3-ethoxycarbonylmethyl-4,5-dimethyl-thiazol-3-ium bromide. LC-MS: t_{R} = 0.88 min; [M+H]⁺ = 225.0.

### A.6.16 Synthesis of imidazo[2,1-b]thiazole-carboxylic acid derivatives (general procedure)

An aq. NaOH solution (1.0M, 23 mL) is added to a solution of the respective carboxylic ester derivative (11.3 mmol) in THF (12 mL) and MeOH (4.0 mL). The mixture is stirred for 16h, the organic volatiles are removed in vacuo and water (10 mL) is added. The mixture is cooled to 0°C and made acidic (pH = 3-4) by addition of hydrochloric acid (1.0 M). The obtained precipitate is filtered off, washed with cold water and dried in vacuo to give the desired acid which is used without further purification.

### 3-methyl-imidazo[2,1-b]thiazole-2-carboxylic acid

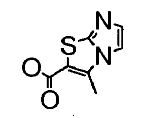

prepared by saponification of 3-methyl-imidazo[2,1-b]thiazole-2-carboxylic acid methyl ester. LC-MS: t_{R} = 0.24 min; [M+H]⁺ = 183.0.

### 2,6-dimethyl-imidazo[2,1-b] thiazole-5-carboxylic acid

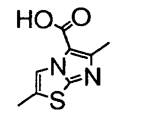

prepared by saponification of 2,6-dimethyl-imidazo[2,1-b]thiazole-5-carboxylic acid ethyl ester. LC-MS: t_{R} = 0.55 min; [M+H]⁺ = 197.3.

### 3,5-dimethyl-imidazo[2,1-b]thiazole-6-carboxylic acid

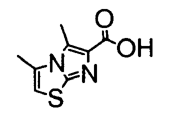

prepared by saponification of 3,5-dimethyl-imidazo[2;1-b]thiazole-6-carboxylic acid ethyl ester. LC-MS: t_{R} = 0.50 min; [M+H]⁺ = 197.0.

### 3,6-dimethyl-imidazo[2,1-b]thiazole-5-carboxylic acid

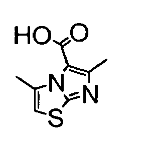

prepared by saponification of 3,6-dimethyl-imidazo[2,1-b]thiazole-5-carboxylic acid ethyl ester. LC-MS: t_{R} = 0.51 min; [M+H]⁺ = 197.0.

### 3-methyl-imidazo[2,1-b]thiazole-6-carboxylic acid

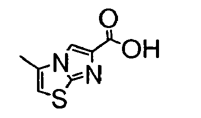

prepared by saponification of 3-methyl-imidazo[2,1-b]thiazole-6-carboxylic acid ethyl ester. LC-MS: t_{R} = 0.46 min; [M+H]⁺ = 183.0.

### 2,3,6-trimethyl-imidazo[2,1-b]thiazole-5-carboxylic acid

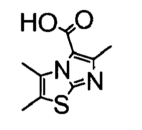

prepared by saponification of 2,3,6-trimethyl-imidazo[2,1-b]thiazole-5-carboxylic acid ethyl ester. LC-MS: t_{R} = 0.56 min; [M+H]⁺ = 211.0.

### 2,3,5-trimethyl-imidazo[2,1-b]thiazole-6-carboxylic acid

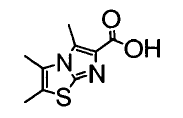

prepared by saponification of 2,3,5-trimethyl-imidazo[2,1-b]thiazole-6-carboxylic acid ethyl ester. LC-MS: t_{R} = 0.57 min; [M+H]⁺ = 211.0.

### 5-methyl-imidazo[2,1-b]thiazole-6-carboxylic acid

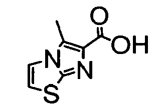

prepared by saponification of 5-methyl-imidazo[2,1-b]thiazole-6-carboxylic acid ethyl ester. LC-MS: t_{R} = 0.39 min; [M+H]⁺ = 183.0.

### 2,5-dimethyl-imidazo[2,1-b]thiazole-6-carboxylic acid

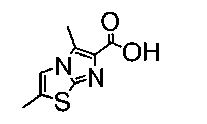

prepared by saponification of 2,5-dimethyl-imidazo[2,1-b]thiazole-6-carboxylic acid ethyl ester. LC-MS: t_{R} = 0.51 min; [M+H]⁺ = 197.0.

### imidazo[2,1-b]thiazole-5-carboxylic acid

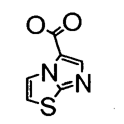

prepared by saponification of imidazo[2,1-b]thiazole-5-carboxylic acid ethyl ester. LC-MS: t_{R} = 0.39 min; [M+H]⁺ = 169.0.

### 2-methyl-imidazo[2,1-b]thiazole-5-carboxylic acid

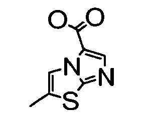

prepared by saponification of 2-methyl-imidazo[2,1-b]thiazole-5-carboxylic acid ethyl ester. LC-MS: t_{R} = 0.51 min; [M+H]⁺ = 183.0.

### 3-methyl-imidazo[2,1-b]thiazole-5-carboxylic acid

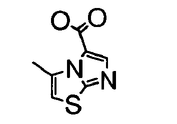

prepared by saponification of 3-methyl-imidazo[2,1-b]thiazole-5-carboxylic acid ethyl ester. LC-MS: t_{R} = 0.53 min; [M+H]⁺ = 183.0.

### 2,3-dimethyl-imidazo[2,1-b]thiazole-5-carboxylic acid

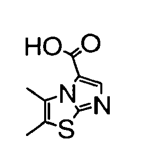

prepared by saponification of 2,3-Dimethyl-imidazo[2,1-b]thiazole-5-carboxylic acid ethyl ester. LC-MS: t_{R} = 0.59 min; [M+H]⁺ = 197.0

### A.6.17 Synthesis of 6-trifluoromethyl-imidazo[2,1-b]thiazole

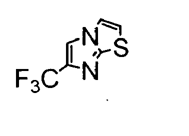

3-Bromo-1,1,1-trifluoroacetone (11.0 mmol) is added to a solution of 2-aminothiazole (10.0 mmol) in acetone (20 mL) and the mixture is stirred at reflux for 20h. The obtained precipitate is filtered off, treated with hydrobromic acid (2.0 M, 40 mL), stirred at reflux for 1h and cooled to RT. The mixture is made basic by addition of ammonium hydroxide solution (15%) and the resulting free base is crystallized from EtOH to give the desired product. LC-MS: t_{R} = 0.78 min; [M+H]⁺ = 192.95.

### A.6.18 Synthesis of 6-trifluoromethyl-imidazo[2,1-b]thiazole-5-carboxylic acid

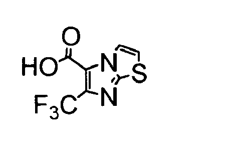

At 0°C POCl₃ (17.1 mmol) is added dropwise to a solution of DMF (20.6 mmol) in chloroform (5.0 mL). A solution of 6-trifluoromethyl-imidazo[2,1-b]thiazole (3.17 mmol) in chloroform (15 mL) is added dropwise at 0°C and the mixture is stirred for 3h at RT. After heating for 2.5d to reflux the mixture is poured into ice, extracted three times with DCM, dried over MgSO₄ and concentrated under reduced pressure. DCM is added, the obtained precipitate is filtered off and the filtrate is concentrated in vacuo to give a crude product which is dissolved in tert.-butanol (19.5 mL). A solution of sodium chlorite (23.0 mmol) and NaH₂PO₄ (17.6 mmol) in water (19.5 mL) is added dropwise and the mixture is stirred for 90 min at RT. The solvents are partially removed in vacuo and the obtained precipitate is filtered off to give the desired product as a white solid. LC-MS: t_{R} = 0.73 min; [M+H]⁺ = 237.2.

### A.6.19 Synthesis of 6-chloro-imidazo[2,1-b]thiazole-5-carboxylic acid

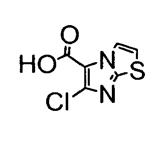

A solution of NaOCl (230 mmol) and NaH₂PO₄ (176 mmol) in water (195 mL) is added dropwise to a solution of 6-chloro-imidazo[2,1-b]thiazole-5-carbaldehyde (26.8 mmol) in tert.-butanol (195 mL) and the mixture is stirred for 8h at RT. The solvents are partially removed in vacuo and the obtained precipitate is filtered off. The filtrate is made acidic and the obtained precipitate is filtered off to give the desired product as a white solid. LC-MS: t_{R} = 0.67 min; [M+H]⁺ = 202.9.

### A.7 Synthesis of benzofuran-4-carboxylic acid derivatives

### A.7.1 Synthesis of 2-methyl-benzofuran-4-carboxylic acid

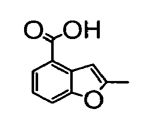

2-Methyl-benzofuran-4-carboxylic acid methyl ester (1.31 mmol, Ishikawa T. et al., Heterocycles, 1994, 39, 371-380) is added to a solution of NaOH (32.5 mmol) in MeOH (24.4 mL) and water (54.4 mL). The mixture is stirred at 55°C for 16 h, partially concentrated in vacuo to remove MeOH and made acidic by addition of hydrochloric acid (1.0 M). The precipitate is filtered off and dried in vacuo to give the desired product. LC-MS: t_{R} = 0.84 min; [M+CH₃CN+H]⁺ = 217.9.

### A.7.2 Synthesis of 2,3-dimethyl-benzofuran-4-carboxylic acid

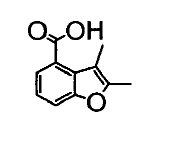

### 3-(1-Methyl-2-oxo-propoxy)-benzoic acid

A solution of methyl-3-hydroxybenzoate (32.2 mmol) and 3-chloro-2-butanone (32.2 mmol) in acetone (60 mL) is treated with K₂CO₃ (96.6 mmol) and KI (8.68 mmol) respectively and stirred at reflux for 16 h. After cooling to RT water and ether are added, the layers are separated and the aq. layer is extracted with ether. The combined organic layers are washed with NaOH solution (1.0 M) and water, dried over Na₂SO₄ and concentrated in vacuo to give the desired product. LC-MS: t_{R} = 0.89 min; [M+CH₃CN+H]⁺ = 264.1.

### 2,3-Dimethyl-benzofuran-4-carboxylic acid methyl ester

Conc. sulfuric acid (3.92 mL, 96%) is added dropwise to 3-(1-methyl-2-oxo-propoxy)-benzoic acid under stirring to keep the temperature below 30°C. After 1 h the mixture is poured into ice-cold water and the obtained precipitate is filtered off and dissolved in ether. The mixture is extracted three times with a sat. NaHCO₃ solution and the organic layer is dried over Na₂SO₄ and concentrated in vacuo to give a crude product which is purified by CC (heptane to heptane/EtOAc 9/1). LC-MS: t_{R} = 0.99 min; [M+H]⁺ = 205.1.

### 2,3-Dimethyl-benzofuran-4-carboxylic acid

2,3-Dimethyl-benzofuran-4-carboxylic acid methyl ester (0.12 mmol) is added to a solution of NaOH (0.18 mmol) in MeOH (0.14 mL) and water (0.14 mL). The mixture is stirred at 55°C for 3 h, partially concentrated in vacuo to remove MeOH and made acidic by addition of hydrochloric acid (1.0 M). The precipitate is filtered off and dried in vacuo to give the desired product. LC-MS: t_{R} = 0.86 min; [M+CH₃CN+H]⁺ = 232.0.

### A.8 Synthesis of 2-aminomethyl-3-aza-bicyclo[3.1.0]hexane-3-carboxylic acid tert-butyl ester

### A.8.1 Synthesis of 3-aza-bicyclo[3.1.0]hexane-2,3-dicarboxylic acid 3-tert-butyl ester

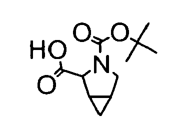

DIPEA (249 mmol, 1.10eq) and a solution of di-tert-butyl dicarbonate (238 mmol, 1.05eq) in DCM (100 mL) are added successively to a suspension of 3-aza-bicyclo[3.1.0]hexane-2-carboxylic acid (226 mmol, 1.0eq) in DCM (400 mL). The mixture is stirred for 22h and concentrated in vacuo to a volume of approximately 100 mL. EtOAc (200 mL) is added and the mixture is made acidic (pH 3) by addition of aq. citric acid solution. The layers are separated and the aq. layer is extracted three times with EtOAc (100 mL each). The combined organic layers are washed with brine, dried over MgSO₄ and concentrated in vacuo to give the desired carboxylic acid as an viscous oil which is used without further purification. LC-MS: t_{R} = 0.75 min; [M+H]⁺ = 228.1.

### A.8.2 Synthesis of 3-aza-bicyclo[3.1.0]hexane-2,3-dicarboxylic acid 3-tert-butyl ester 2-methyl ester

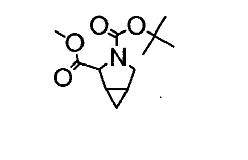

To a solution of 3-aza-bicyclo[3.1.0]hexane-2,3-dicarboxylic acid 3-tert-butyl ester (226 mmol, 1.0eq) in DMF (350 mL) is added Cs₂CO₃ (304 mmol, 1.35eq). Methyl iodide (397 mmol, 1.75eq) is added dropwise and the suspension is stirred for 60 min. The mixture is filtered and the filtrate is diluted with water (200 mL) and TBME (150 mL). The layers are separated and the aq. layer is extracted four times with TBME (150 mL each). The combined organic layers are washed three times with brine, dried over Na₂SO₄ and concentrated in vacuo to give the desired carboxylic ester as an oil which is used without further purification. LC-MS: t_{R} = 0.88 min; [M+H]⁺ = 242.1.

### A.8.3 Synthesis of 2-hydroxymethyl-3-aza-bicyclo[3.1.0]hexane-3-carboxylic acid tert-butyl ester

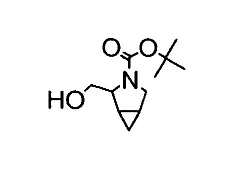

At -78°C a solution of DIBAL in toluene (1.7M, 205 mmol, 2.6eq) is added dropwise to a solution of 3-aza-bicyclo[3.1.0]hexane-2,3-dicarboxylic acid 3-tert-butyl ester 2-methyl ester (78.0 mmol, 1.0eq) in THF (350 mL). After 20 min the solution is allowed to reach RT and poured into a mixture of aq. NaOH solution (1.0M, 400 mL) and ice. The layers are separated and the aq. layer is extracted three times with EtOAc (300 mL each). The combined organic layers are washed with aq. NaOH solution (1.0M) and brine, dried over Na₂SO₄ and concentrated in vacuo to give a crude oil which is purified by CC [gradient: heptane to heptane/EtOAc 1/1, R_{f} = 0.12 (*cy-*hexane/EtOAc 4/1)]. After removal of the solvents the desired alcohol is obtained as a colorless oil. LC-MS: t_{R} = 0.83 min; [M+H]⁺ = 214.0.

### A.8.4 Synthesis of 2-formyl-3-aza-bicyclo[3.1.0]hexane-3-carboxylic acid tert-butyl ester

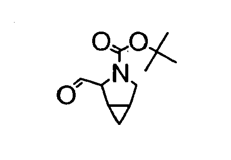

Dess-Martin periodinane (47 mmol, 1.4eq) is added to a solution of 2-hydroxymethyl-3-aza-bicyclo[3.1.0]hexane-3-carboxylic acid tert-butyl ester (34 mmol, 1.0eq) in DCM (500 mL, saturated with water). Additional periodinane is added after 90 min (2.1 mmol), 210 min (4.9 mmol), and 15 h (3.7 mmol). After additional 2 h ether, sat. NaHCO₃ solution and aq. Na₂S₂O₃ solution are added, the layers are separated and the aq. layer is extracted twice with ether. The combined organic layers are washed with sat. NaHCO₃ solution, dried over Na₂SO₄ and concentrated in vacuo to give a crude product which is purified by CC (pentane and pentane/ether 2/1). After removal of the solvents the desired aldehyde is obtained as a colourless oil. LC-MS: t_{R} = 0.87 min; [M+H]⁺ = 212.2.

### A.8.5 Synthesis of (1R*,2S*,5S*)-2-(benzylamino-methyl)-3-aza-bicyclo[3.1.0]hexane-3-carboxylic acid tert-butyl ester

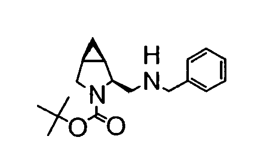

Benzylamine (40 mmol, 1.3eq) is added to a solution of 2-formyl-3-aza-bicyclo[3.1.0]hexane-3-carboxylic acid tert-butyl ester (31 mmol, 1.0eq) in chloroform (62 mL). After 15 min the mixture is treated with sodium triacetoxyborohydride (38 mmol, 1.2eq), stirred for additional 2 h and poured into a sat. aq. NaHCO₃ solution. The layers are separated and the aq. layer is extracted twice with chloroform. The combined organic layers are washed with sat. NaHCO₃ solution, dried over Na₂SO₄ and concentrated in vacuo to give a crude yellow oil which is dissolved in ether (100 mL). A mixture of hydrochloride acid (0.1M) and citric acid (5% in water) is added, the layers are separated and the aq. layer is extracted once with ether. The aq. layer is made basic by addition of solid NaHCO₃ and extracted with ether. After removal of the solvents the desired benzylamine derivative is obtained as a colourless oil. LC-MS: t_{R} = 0.81 min; [M+H]⁺ = 303.2.

### A.8.6 Synthesis of (1R*,2S*,5S*)-2-aminomethyl-3-aza-bicyclo[3.1.0]hexane-3-carboxylic acid tert-butyl ester

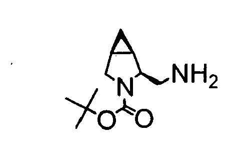

A solution of (1R*,2S*,5S*)-2-(benzylamino-methyl)-3-aza-bicyclo[3.1.0]hexane-3-carboxylic acid tert-butyl ester (36 mmol) in ethanol (65 mL) is treated with Pd/C (950 mg, 50% H₂O) and stirred under a hydrogen atmosphere (1 bar) for 16 h. An additional amount of Pd/C (300 mg) is added and the mixture is stirred for further 16 h. After filtration through celite and removal of the solvents the desired (1R*,2S*,5S*)-2-aminomethyl-3-aza-bicyclo[3.1.0]hexane-3-carboxylic acid tert-butyl ester is obtained which is used without further purification. LC-MS: t_{R} = 0.65 min; [M+H]⁺ = 213.1.

### A.9 Synthesis of N-substituted (1R*,2S*,5S*)-2-(amino-methyl)-3-aza-bicyclo[3.1.0]-hexane derivatives

### A.9.1 Synthesis of (1R*,2S*,5S*)-2-(aroylamino-methyl)-3-aza-bicyclo[3.1.0]-hexane-3-carboxylic acid tert-butyl ester derivatives (general procedure)

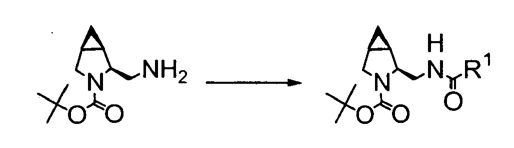

To a solution of the respective carboxylic acid (3.2 mmol, 1.10eq) in DMF (5.0 mL) is added successively DIPEA (8.8 mmol, 3.0eq) and a solution of TBTU (3.7 mmol, 1.25eq) in DMF (5.0 mL). The obtained mixture is added to a solution of (1R*,2S*,5S*)-2-aminomethyl-3-aza-bicyclo[3.1.0]hexane-3-carboxylic acid tert-butyl ester (2.9 mmol, 1.0eq) in DMF (5.0 mL). After 10 min sat. aq. NaHCO₃ solution and ether are added, the layers are separated and the organic layer is washed with sat. NaHCO₃ solution, citric acid (5% in water) and water. After drying over Na₂SO₄ and removal of solvents in vacuo the desired amides are obtained which are used without further purification.

### (1R*,2S*,5S*)-2-[(4-fluoro-benzoylamino)-methyl]-3-aza-bicyclo[3.1.0]hexane-3-carboxylic acid tert-butyl ester

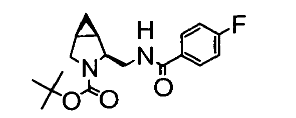

prepared by reaction of (1R*,2S*,5S*)-2-aminomethyl-3-aza-bicyclo[3.1.0]hexane-3-carboxylic acid tert-butyl ester with 4-fluorobenzoic acid. LC-MS: t_{R} = 0.99 min; [M+H]⁺ = 335.1.

### (1R*,2S*,5S*)-2-{[(benzofuran-4-carbonyl)-amino]-methyl}-3-aza-bicyclo[3.1.0]hexane-3-carboxylic acid tert-butyl ester

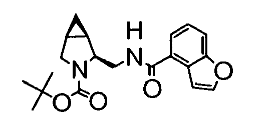

prepared by reaction of (1R*,2S*,5S*)-2-aminomethyl-3-aza-bicyclo[3.1.0]hexane-3-carboxylic acid tert-butyl ester with benzofuran-4-carboxylic acid (M.A. Eissenstat et al. J. Med. Chem. 1995, 38, 3094-3105). LC-MS: t_{R} = 1.01 min; [M+H]⁺ = 357.1.

### (1R*,2S*,5S*)-2-{[(pyridine-2-carbonyl)-amino]-methyl}-3-aza-bicyclo[3.1.0]hexane-3-carboxylic acid tert-butyl ester

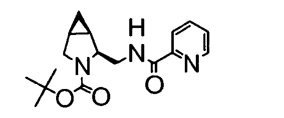

prepared by reaction of (1R*,2S*,5S*)-2-aminomethyl-3-aza-bicyclo[3.1.0]hexane-3-carboxylic acid tert-butyl ester with pyridine-2-carboxylic acid. LC-MS: t_{R} = 0.94 min; [M+H]⁺ = 318.1.

### (1R*,2S*,SS*)-2-{[(6-methyl-imidazo[2,1-b]thiazole-5-carbonyl)-amino]-methyl}-3-aza-bicyclo[3.1.0]hexane-3-carboxylic acid tert-butyl ester

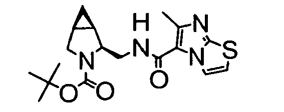

prepared by reaction of (1R*,2S*,5S*)-2-aminomethyl-3-aza-bicyclo[3.1.0]hexane-3-carboxylic acid tert-butyl ester with 6-methyl-imidazo[2,1-b]thiazole-5-carboxylic acid (A. Andreani et al. Eur. J. Med. Chem 1982, 17, 271-274). LC-MS: t_{R} = 0.87 min; [M+H]⁺ = 377.2.

### (1R*,2S*,5S*)-2-{[(2,3-dihydro-benzo[1,4]dioxine-5-carbonyl)-amino]-methyl}-3-aza-bicyclo[3.1.0]hexane-3-carboxylic acid tert-butyl ester

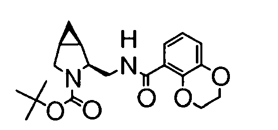

prepared by reaction of (1R*,2S*,5S*)-2-aminomethyl-3-aza-bicyclo[3.1.0]hexane-3-carboxylic acid tert-butyl ester with 2,3-dihydro-benzo[1,4]dioxine-5-carboxylic acid. LC-MS: t_{R} = 0.98 min; [M+H]⁺ = 375.1.

### (1R*,2S*,5S*)-2-{[(imidazo[2,1-b]thiazole-6-carbonyl)-amino]-methyl}-3-aza-bicyclo[3.1.0]hexane-3-carboxylic acid tert-butyl ester

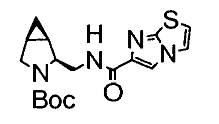

prepared by reaction of (1R*,2S*,5S*)-2-aminomethyl-3-aza-bicyclo[3.1.0]hexane-3-carboxylic acid tert-butyl ester with imidazo[2,1-b]thiazole-6-carboxylic acid. LC-MS (basic): t_{R} = 0.81 min; [M+H]⁺ = 362.8.

### A.9.2 Synthesis of heterocyclyl substituted (1R*,2S*,5S*)-2-(amino-methyl)-3-aza-bicyclo[3.1.0]-hexane-3-carboxylic acid tert-butyl ester derivatives (general procedure)

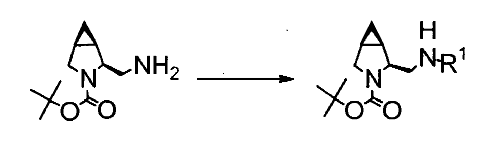

A solution of the respective heterocyclyl halogenide (1.03 mmol, 1.1eq) and of (1R*,2S*,5S*)-2-aminomethyl-3-aza-bicyclo[3.1.0]hexane-3-carboxylic acid tert-butyl ester (0.94 mmol, 1.0eq) in EtOH (2.0 mL) is treated with NEt₃ (1.17 mmol, 1.2eq) and heated under microwave conditions (120°C, 200 W) for 10 min. The crude mixture is purified by prep. HPLC to give the respective products.

### (1R*,2S*,5S*)-2-[(5-bromo-pyrimidin-2-ylamino)-methyl]-3-aza-bicyclo[3.1.0]hexane-3-carboxylic acid tert-butyl ester

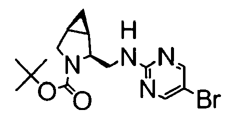

prepared by reaction of (1R*,2S*,5S*)-2-aminomethyl-3-aza-bicyclo[3.1.0]hexane-3-carboxylic acid tert-butyl ester with 5-bromo-2-chloro-pyrimidine. LC-MS: t_{R} = 1.03 min; [M+H]⁺ = 369.1.
Single crystals are obtained by crystallization from chloroform. The relative cis-configuration of the product has been demonstrated.

### (1R*,2S*,5S*)-2-[(6,7-difluoro-quinoxalin-2-ylamino)-methyl]-3-aza-bicyclo[3.1.0]hexane-3-carboxylic acid tert-butyl ester

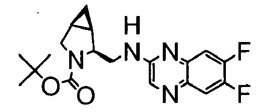

prepared by reaction of (1R*,2S*,5S*)-2-aminomethyl-3-aza-bicyclo[3.1.0]hexane-3-carboxylic acid tert-butyl ester with 2-chloro-6,7-difluoro-quinoxaline (S. Piras, M. Loriga, G. Paglietti Farmaco 2004, 59, 185-194). LC-MS: t_{R} = 1.05 min; [M+H]⁺ = 377.2.

### A.9.3 Synthesis of (1R*,2S*,5S*)-2-(amino-methyl)-3-aza-bicyclo[3.1.0]-hexane derivatives substituted at the side-chain nitrogen atom (general procedure)

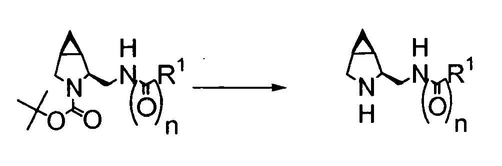

A solution of HCl in dioxane (4.0 M, 4.0 mL) is added to a solution of the respective Boc-protected 3-aza-bicyclo[3.1.0]-hexane derivative (2.9 mmol) in isopropanol or dioxane (2.0 mL). After LC-MS indicated complete reaction (30 min to several hours) the mixture is concentrated in vacuo. The remaining residue is again dissolved in isopropanol (1.0 mL) and concentrated to dryness to give the respective deprotected product which is used without further purification.

### N-[(1R*,2S*,5S*)-3-aza-bicyclo[3.1.0]hex-2-ylmethyl]-4-fluoro-benzamide

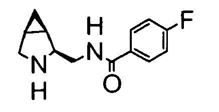

prepared by deprotection of (1R*,2S*,5S*)-2-[(4-fluoro-benzoylamino)-methyl]-3-aza-bicyclo[3.1.0]hexane-3-carboxylic acid tert-butyl ester. LC-MS: t_{R} = 0.57 min; [M+H]⁺ = 235.1.

### benzofuran-4-carboxylic acid [(1R*,2S*,5S*)-3-aza-bicyclo[3.1.0]hex-2-ylmethyl]-amide

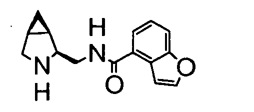

prepared by deprotection of (1R*,2S*,5S*)-2-{[(benzofuran-4-carbonyl)-amino]-methyl}-3-aza-bicyclo[3.1.0]hexane-3-carboxylic acid tert-butyl ester. LC-MS: t_{R} = 0.62 min; [M+H]⁺ = 257.1.

### pyridine-2-carboxylic acid [(1R*,2S*,5S*)-3-aza-bicyclo[3.1.0]hex-2-ylmethyl]-amide

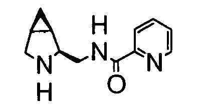

prepared by deprotection of (1R*,2S*,5S*)-2-{[(pyridine-2-carbonyl)-amino]-methyl}-3-aza-bicyclo[3.1.0]hexane-3-carboxylic acid tert-butyl ester. LC-MS: t_{R} = 0.50 min; [M+H]⁺ = 218.0.

### 6-methyl-imidazo[2,1-b]thiazole-5-carboxylic acid [(1R*,2S*,5S*)-3-aza-bicyclo[3.1.0] hex-2-ylmethyl]-amide

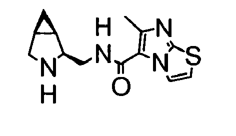

prepared by deprotection of (1R*,2S*,5S*)-2-{[(6-methyl-imidazo[2,1-b]thiazole-5-carbonyl)-amino]-methyl}-3-aza-bicyclo[3.1.0]hexane-3-carboxylic acid tert-butyl ester. LC-MS: t_{R} = 0.62 min; [M+H]⁴= 277.2.

### 2,3-Dihydro-benzo[1,4]dioxine-5-carboxylic acid [(1R*,2S*,5S*)-3-aza-bicyclo[3.1.0] hex-2-ylmethyl]-amide

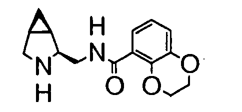

prepared by deprotection of (1R*,2S*,5S*)-2-{[(2,3-dihydro-benzo[1,4]dioxine-5-carbonyl)-amino]-methyl}-3-aza-bicyclo[3.1.0]hexane-3-carboxylic acid tert-butyl ester. LC-MS: t_{R} = 0.60 min; [M+H]⁺ = 275.0.

### Imidazo[2,1-b]thiazole-6-carboxylic acid [(1R*,2S*,5S*)-3-aza-bicyclo[3.1.0]hex-2-ylmethyl]-amide

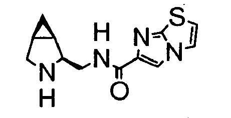

prepared by deprotection of (1R*,2S*,5S*)-2-{[(imidazo[2,1-b]thiazole-6-carbonyl)-amino]-methyl}-3-aza-bicyclo[3.1.0]hexane-3-carboxylic acid tert-butyl ester. LC-MS (basic): t_{R} = 0.59 min; [M+H]⁺ = 263.1.

### (1R*,2S*,5S*)-(3-aza-bicyclo[3.1.0]hex-2-ylmethyl)-(5-bromo-pyrimidin-2-yl)-amine

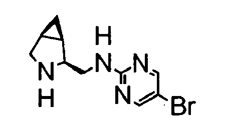

prepared by deprotection of (1R*,2S*,5S*)-2-[(5-bromo-pyrimidin-2-ylamino)-methyl]-3-aza-bicyclo[3.1.0]hexane-3-carboxylic acid tert-butyl ester. LC-MS: t_{R} = 0.58 min; [M+H]⁺ = 268.9.

### (1R*,2S*,5S*)-(3-aza-bicyclo[3,1.0]hex-2-ylmethyl)-(6,7-difluoro-quinoxalin-2-yl)-amine

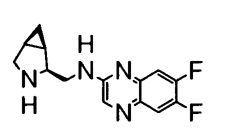

prepared by deprotection of (1R*,2S*,5S*)-2-[(6,7-difluoro-quinoxalin-2-ylamino)-methyl]-3-aza-bicyclo[3.1.0]hexane-3-carboxylic acid tert-butyl ester. LC-MS: t_{R} = 0.68 min; [M+H]⁺ = 277.1.

### A.10 Synthesis of carboxylic amide derivatives (general procedure)

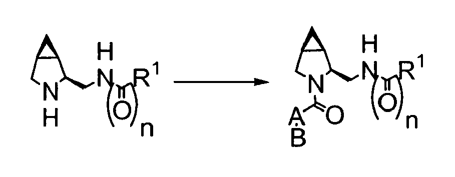

To a solution of the respective carboxylic acid (1.37 mmol, 1.00eq) and DIPEA (2.06 mmol, 1.50eq) in DMF (2.0 mL) is added TBTU (1.47 mmol, 1.05eq). The obtained mixture is treated with a solution of the respective (1R*,2S*,5S*)-3-aza-bicyclo[3.1.0]-hexane derivative (1.37 mmol, 1.00eq) and DIPEA (2.06 mmol, 1.50eq) in DMF (2.0 mL). After 40 min sat. water and TBME are added, the layers are separated and the organic layer is washed with water, hydrochloric acid (0.5 M), aq. NaOH solution (0.5 M) and brine. After drying over Na₂SO₄ and removal of solvents in vacuo the desired amides are obtained which are used without further purification.

### 2,3-Dihydro-benzo[1,4]dioxine-5-carboxylic acid [(1R*,2S*,5S*)-3-(2-bromo-4-methyl-benzoyl)-3-aza-bicyclo[3.1.0]hex-2-ylmethyl]-amide

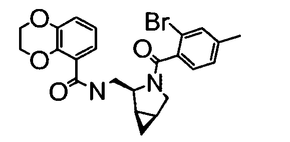

prepared by reaction of 2,3-dihydro-benzo[1,4]dioxine-5-carboxylic acid [(1R*,2S*,5S*)-3-aza-bicyclo[3.1.0]hex-2-ylmethyl]-amide with 2-bromo-4-methylbenzoic acid. LC-MS: t_{R} = 0.89 min; [M+H]⁺ = 471.1.

### 2,3-Dihydro-benzo[1,4]dioxine-5-carboxylic acid [(1R*,2S*,5S*)-3-(2-bromo-5-methyl-benzoyl)-3-aza-bicyclo[3.1.0]hex-2-ylmethyl]-amide

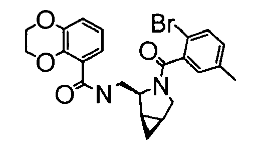

prepared by reaction of 2,3-dihydro-benzo[1,4]dioxine-5-carboxylic acid [(1R*,2S*,5S*)-3-aza-bicyclo[3.1.0]hex-2-ylmethyl]-amide with 2-bromo-5-methylbenzoic acid. LC-MS: t_{R} = 0.89 min; [M+H]⁺ = 471.1.

### 2,3-Dihydro-benzo[1,4]dioxine-5-carboxylic acid [(1R*,2S*,5S*)-3-(2-bromo-3-methyl-benzoyl)-3-aza-bicyclo[3.1.0]hex-2-ylmethyl]-amide

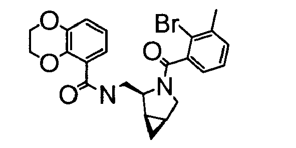

prepared by reaction of 2,3-dihydro-benzo[1,4]dioxine-5-carboxylic acid [(1R*,2S*,5S*)-3-aza-bicyclo[3.1.0]hex-2-ylmethyl]-amide with 2-bromo-3-methylbenzoic acid. LC-MS: t_{R} = 0.88 min; [M+H]⁺ = 471.1.

### A.11 Synthesis of 3-substituted (1R*,2S*,5S*)-2-(amino-methyl)-3-aza-bicyclo[3.1.0]-hexane derivatives

### A.11.1 Synthesis of (1R*,2S*,5S*)-2-[(2,2,2-trifluoro-acetylamino)-methyl]-3-aza-bicyclo[3.1.0]hexane-3-carboxylic acid tert-butyl ester

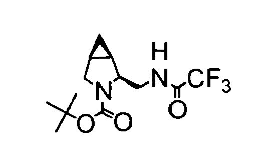

Ethyl trifluoroacetate (4.25 mmol, 1.36eq) is added to a solution of (1R*,2S*,5S*)-2-aminomethyl-3-aza-bicyclo[3.1.0]hexane-3-carboxylic acid tert-butyl ester (3.12 mmol, 1.00eq) in THF (10 mL). After 50 min the solvents are removed in vacuo to give the desired product which is used without further purification in the next step. LC-MS: t_{R} = 0.98 min; [M+H]⁺ = 309.1.

### A.11.2 Synthesis of N-[(1R*,2S*,5S*)-3-aza-bicyclo[3.1.0]hex-2-ylmethyl]-2,2,2-trifluoro-acetamide

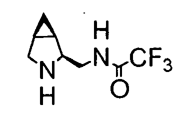

A solution of HCl in dioxane (4 M, 4.0 mL) is added to a solution of (1R*,2S*,5S*)-2-[(2,2,2-trifluoro-acetylamino)-methyl]-3-aza-bicyclo[3.1.0]hexane-3-carboxylic acid tert-butyl ester (3.11 mmol) in THF (4.0 mL) and the mixture is stirred for 5 min at RT and for 30 min at 45°C. The solvents are removed in vacuo and the obtained solid is washed once with a small volume of CHCl₃ to give the desired product which is used without further purification in the next step. LC-MS: t_{R} = 0.63 min; [M+H]⁺ = 209.3.

### A.11.3 Synthesis of 2,2,2-trifluoro-N-[(1R*,2S*,5S*)-3-aza-bicyclo [3.1.0] hex-2-ylmethyl]-acetamide derivatives (general procedure)

A solution of the respective carboxylic acid (1.80 mmol, 1.1eq), DIPEA (4.91 mmol, 3.0eq) and TBTU (1.97 mmol, 1.2eq) in DMF (10 mL) is added to a solution of N-[(1R*,2S*,5S*)-3-aza-bicyclo[3.1.0]hex-2-ylmethyl]-2,2,2-trifluoro-acetamide (1.64 mmol, 1.0eq) in DMF (4.0 mL). The mixture is stirred for 15 - 60 min and poured into a mixture of ice, hydrochloric acid (0.5 M) and TBME. The layers are separated and the aq. layer is extracted with TBME. The combined organic layers are washed twice with sat. aq. NaHCO₃ solution and once with brine. The solvents are removed in vacuo to give the desired product which is either used without further purification or purified by CC [gradient: DCM to DCM/MeOH 98/2].

### 2,2,2-trifluoro-N-[(1R*,2S*,5S*)-3-(2-methyl-5-m-tolyl-thiazole-4-carbonyl)-3-aza-bicyclo[3.1.0]hex-2-ylmethyl]-acetamide

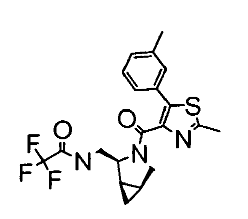

prepared by reaction of N-[(1R*,2S*,5S*)-3-aza-bicyclo[3.1.0]hex-2-ylmethyl]-2,2,2-trifluoro-acetamide with 2-methyl-5-m-tolyl-thiazole-4-carboxylic acid. LC-MS: t_{R} = 0.92 min; [M+H]⁺ = 424.1.

### N-{(1R*,2S*,5S*)-3-[5-(3-Chloro-phenyl)-2-methyl-thiazole-4-carbonyl]-3-aza-bicyclo[3.1.0]hex-2-ylmethyl}-2,2,2-trifluoro-acetamide

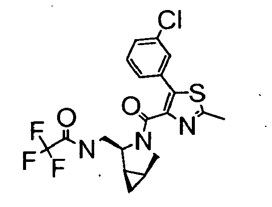

prepared by reaction of N-[(1R*,2S*,5S*)-3-aza-bicyclo[3.1.0]hex-2-ylmethyl]-2,2,2-trifluoro-acetamide with 5-(3-chloro-phenyl)-2-methyl-thiazole-4-carboxylic acid. LC-MS (basic): t_{R} = 0.92 min; [M+H]⁺ = 444.2.

### 2,2,2-Trifluoro-N-{(1R*,2S*,5S*)-3-[5-(3-fluoro-phenyl)-2-methyl-thiazole-4-carbonyl]-3-aza-bicyclo[3.1.0] hex-2-ylmethyl}-acetamide

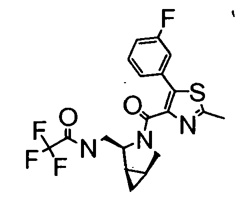

prepared by reaction of N-[(1R*,2S*,5S*)-3-aza-bicyclo[3.1.0]hex-2-ylmethyl]-2,2,2-trifluoro-acetamide with 5-(3-fluoro-phenyl)-2-methyl-thiazole-4-carboxylic acid. LC-MS (basic): t_{R} = 0.90 min; [M+H]⁺ = 428.0.

### N-[(1R*,2S*,5S*)-3-(2-Amino-5-m-tolyl-thiazole-4-carbonyl)-3-aza-bicyclo[3.1.0] hex-2-ylmethyl]-2,2,2-trifluoro-acetamide

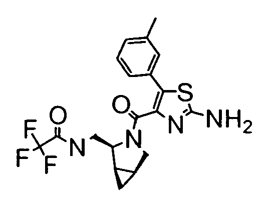

prepared by reaction of N-[(1R*,2S*,5S*)-3-aza-bicyclo[3.l.0]hex-2-ylmethyl]-2,2,2-trifluoro-acetamide with 2-amino-5-m-tolyl-thiazole-4-carboxylic acid. LC-MS (basic): t_{R} = 0.81 min; [M+H]⁺ = 425.2.

### N-{(1R*,2S*,5S*)-3-[2-Amino-5-(3-chloro-phenyl)-thiazole-4-carbonyl]-3-aza-bicyclo[3.1.0] hex-2-ylmethyl}-2,2,2-trifluoro-acetamide

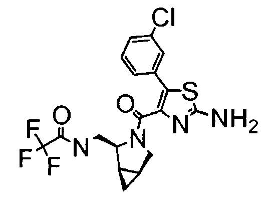

prepared by reaction of N-[(1R*,2S*,5S*)-3-aza-bicyclo[3.1.0]hex-2-ylmethyl]-2,2," trifluoro-acetamide with 2-amino-5-(3-chloro-phenyl)-thiazole-4-carboxylic acid. LC-MS (basic): t_{R} = 0.83 min; [M+H]⁺ = 445.2.

### N-[(1R*,2S*,5S*)-3-(2-Bromo-4-methyl-benzoyl)-3-aza-bicyclo[3.1.0]hex-2-ylmethyl]-2,2,2-trifluoro-acetamide

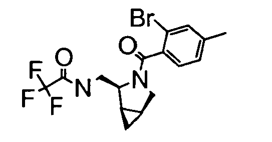

prepared by reaction of N-[(1R*,2S*,5S*)-3-aza-bicyclo[3.1.0]hex-2-ylmethyl]-2,2,2-trifluoro-acetamide with 2-bromo-4-methyl-benzoic acid. LC-MS (basic): t_{R} = 0.92 min; [M+H]⁺ = 404.9.

### A.11.4 Synthesis of [(1R*,2S*,5S*)-2-aminomethyl-3-aza-bicyclo[3.1.0]hex-3-yl]-(hetero)aryl-methanone derivatives (general procedures)

### procedure 1:

A solution of the respective 2,2,2-trifluoro-acetamide derivative (1.42 mmol) in THF (15 mL) is treated with an aq. NaOH solution (2.0 M, 5 mL). The mixture is stirred over night, diluted with MeOH (15 mL), and stirred for additional 16h. Water and EtOAc are added, the layers are separated and the aq. layer is extracted twice with EtOAc. The solvents are removed in vacuo and the residue is purified by prep. HPLC to give the desired amine as a colourless oil.

### procedure 2:

A solution of the respective 2,2,2-trifluoro-acetamide derivative (7.65 mmol) in MeOH (25 mL) is treated with a sat. solution of K₂CO₃ in water (2-20 mL) and stirred at 60°C for 6 h. The mixture is concentrated in vacuo, diluted with citric acid (5%) and washed with TBME. The aq. layer is made basic by addition of aq. NaOH solution (5.0 M) and extracted four times with DCM. The combined organic layers are dried over Na₂SO₄ and concentrated in vacuo to give a crude product which is either used without further purification or purified by prep. HPLC.

### [(1R*,2S*,5S*)-2-Aminomethyl-3-aza-bicyclo [3.1.0] hex-3-yl]-(2-methyl-5-m-tolyl-thiazol-4-yl)-methanone

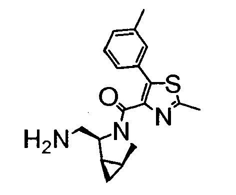

prepared by deprotection of 2,2,2-trifluoro-N-[(1R*,2S*,5S*)-3-(2-methyl-5-m-tolyl-thiazole-4-carbonyl)-3-aza-bicyclo[3.1.0]hex-2-ylmethyl]-acetamide. LC-MS: t_{R} = 0.79 min; [M+H]⁺ = 328.3.

### [(1R*,2S*,5S*)-2-Aminomethyl-3-aza-bicyclo[3.1.0] hex-3-yl]-[5-(3-chloro-phenyl)-2-methyl-thiazol-4-yl]-methanone

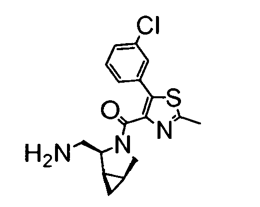

prepared by deprotection of N-{(1R*,2S*,5S*)-3-[5-(3-chloro-phenyl)-2-methylthiazole-4-carbonyl]-3-aza-bicyclo[3.1.0]hex-2-ylmethyl}-2,2,2-trifluoro-acetamide. LC-MS (basic): t_{R} = 0.78 min; [M+H]⁺ = 348.3.

### [(1R*,2S*,5S*)-2-Aminomethyl-3-aza-bicyclo[3.1.0]hex-3-yl]-[S-(3-fluoro-phenyl)-2-methyl-thiazol-4-yl]-methanone

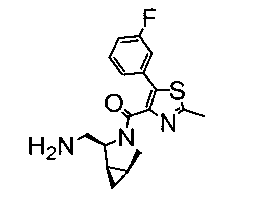

prepared by deprotection of N-{(1R*,2S*,5S*)-3-[5-(3-fluoro-phenyl)-2-methylthiazole-4-carbonyl]-3-aza-bicyclo[3.1.0]hex-2-ylmethyl}-2,2,2-trifluoro-acetamide. LC-MS (basic): t_{R} = 0.77 min; [M+H]⁺ = 332.2.

### [(1R*,2S*,5S*)-2-Aminomethyl-3-aza-bicyclo [3.1.0] hex-3-yl]-(2-amino-5-m-tolyl-thiazol-4-yl)-methanone

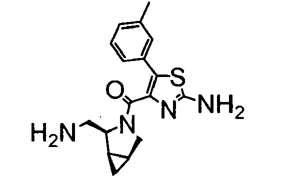

prepared by deprotection of N-[(1R*,2S*,5S*)-3-(2-amino-5-m-tolyl-thiazole-4-carbonyl)-3-aza-bicyclo[3.1.0]hex-2-ylmethyl]-2,2,2-trifluoro-acetamide. LC-MS (basic): t_{R} = 0.69 min; [M+H]⁺ =329.2.

### [2-Amino-5-(3-chloro-phenyl)-thiazol-4-yl]-[(1R*,2S*,5S*)-2-aminomethyl-3-aza-bicyclo[3.1.0]hex-3-yl]-methanone

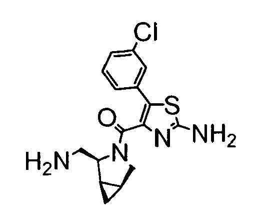

prepared by deprotection of N-{(1R*,2S*,5S*)-3-[2-amino-5-(3-chloro-phenyl)-thiazole-4-carbonyl]-3-aza-bicyclo[3. 1.0]hex-2-ylmethyl}-2,2,2-trifluoro-acetamide. LC-MS (basic): t_{R} = 0.70 min; [M+H]⁺ = 349.3.

### [(1R*,2S*,5S*)-2-Aminomethyl-3-aza-bicyclo [3.1.0] hex-3-yl]-(2-bromo-4-methyl-phenyl)-methanone

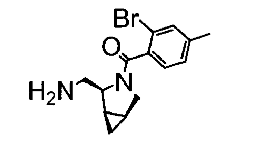

prepared by deprotection of N-[(1R*,2S*,5S*)-3-(2-bromo-4-methyl-benzoyl)-3-aza-bicyclo[3.1.0]hex-2-ylmethyl]-2,2,2-trifluoro-acetamide. LC-MS (basic): t_{R} = 0.88 min; [M+H]⁺ = 309.0.

### A.12 Synthesis of [(1R*,2S*,5S*)-2-aminomethyl-3-aza-bicyclo[3.1.0]hex-3-yl]-(3'-fluoro-5-methyl-biphenyl-2-yl)-methanone

### A.12.1 Synthesis of [(1R*,2S*,5S*)-3-(2-bromo-4-methyl-benzoyl)-3-aza-bicyclo[3.1.0]hex-2-ylmethyl]-carbamic acid tert-butyl ester

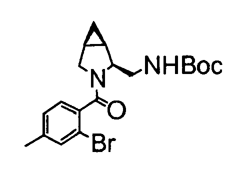

NEt₃ (7.30 mmol, 1.05eq) and a solution of di-tert-butyl dicarbonate (7.09 mmol, 1.02eq) in DCM (15 mL) are added successively to a solution of [(1R*,2S*,5S*)-2-Aminomethyl-3-aza-bicyclo[3.1.0]hex-3-yl]-(2-bromo-4-methyl-phenyl)-methanone (6.95 mmol, 1.0eq) in DCM (10 mL). The mixture is stirred for 15 min and made acidic by addition of aq. citric acid solution (5%). The layers are separated and the organic layer is washed twice with aq. citric acid solution (5%), water and brine. After drying over Na₂SO₄ the mixture is concentrated in vacuo to give the desired product as a white solid which is used without further purification. LC-MS (basic): t_{R} = 0.96 min; [M+H]⁺ = 408.9.

### A.12.2 Synthesis of [(1R*,2S*,5S*)-3-(3'-fluoro-5-methyl-biphenyl-2-carbonyl)-3-aza-bicyclo[3.1.0]hex-2-ylmethyl]-carbamic acid tert-butyl ester

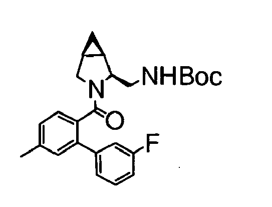

A solution of [(1R*,2S*,5S*)-3-(2-bromo-4-methyl-benzoyl)-3-aza-bicyclo[3.1.0]hex-2-ylmethyl]-carbamic acid tert-butyl ester (2.44 mmol) and 3-fluoro-phenylboronic acid (2.93 mmol) in a mixture of ethanol (7.0 mL) and toluene (7.0 mL) is prepared by gentle heating. An aq. Na₂CO₃ solution (2.0 M) is added and a flow of argon is bubbled through the mixture. After addition of Pd(PPh₃)₄ the mixture is heated to 75°C, stirred for 22 h and cooled to RT. Water (20 mL) is added and the mixture is extracted three times with EtOAc (20 mL each). The combined organic layers are washed three times with water and once with brine, dried over MgSO₄ and concentrated in vacuo to give a crude product which is purified by CC (gradient: DCM to DCM/MeOH 98/2). LC-MS (basic): t_{R} = 1.00 min; [M+H]⁺ = 425.0.

### A.12.3 Synthesis of [(1R*,2S*,5S*)-2-aminomethyl-3-aza-bicyclo[3.1.0]hex-3-yl]-(3'-fluoro-5-methyl-biphenyl-2-yl)-methanone

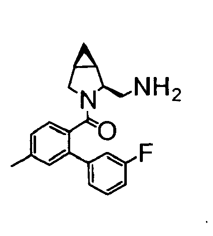

A solution of HCl in dioxane (4.0 M, 7.2 mL) is added to a solution of [(1R*,2S*,5S*)-3-(3'-fluoro-5-methyl-biphenyl-2-carbonyl)-3-aza-bicyclo[3.1.0]hex-2-ylmethyl]-carbamic acid tert-butyl ester (2.24 mmol) in dioxane (3.0 mL). After 1 h the mixture is concentrated in vacuo to give the desired deprotected product which is used without further purification. LC-MS (basic): t_{R} = 0.85 min; [M+H]⁺ = 325.1.

### A.13 Synthesis of pyridine-carboxylic acid derivatives

### A.13.1 Synthesis of 6-chloro-3-formyl-5-methyl-pyridine

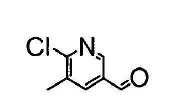

Phosphoroxychloride (183 mL, 2 mol) is heated at 90°C and a mixture of commercially available 2-methyl-2-butennitrile (73 g, 0.9 mol) and DMF (154 mL, 2 mol) is added slowly while keeping the temperature at 100 to 110°C. The mixture is stirred at 110°C for 15 h, cooled to RT and diluted with DCM (500 mL). The mixture is cooled at 0°C and carefully quenched with water (500 mL). The layers are separated and the aq. layer is extracted with DCM (total of 800 mL). The combined organic extracts are dried (Na₂SO₄), filtered and evaporated. The residue is crystallised from cyclohexane to provide 6-chloro-3-formyl-5-methyl-pyridine as slightly yellow crystals; LC-MS: t_{R} = 0.76 min, [M+H]⁺ = 156.1.

### A.13.2 Synthesis of 6-chloro-5-methyl-nicotinic acid

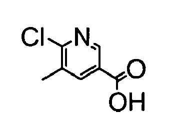

A solution of 6-chloro-3-formyl-5-methyl-pyridine (10 g, 64 mmol) in formic acid (200 mL) is cooled to 0°C and an aq. 50% wt solution of H₂O₂ in water (9.6 mL, 360 mmol) is added at this temperature. The mixture is stirred at 0°C for 15 h, carefully diluted with water (200 mL) and extracted with DCM (8 x 100 mL). The combined organic extracts are washed with 1M aq. HCl (100 mL) (check for remaining peroxide), dried (MgSO₄) and filtered. The residue is concentrated in vacuo to give 6-chloro-5-methyl-nicotinic acid; LC-MS: t_{R} = 0.72 min, [M+H]⁺ = 172.0.

### A.13.3 Synthesis of 6-chloro-5-methyl-nicotinic acid ethyl ester

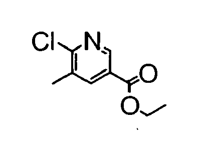

A solution of 6-chloro-5-methyl-nicotinic acid (13.9 g, 80.8 mmol) in dry ethanol (200 mL) containing some drops of concentrated H₂SO₄ is stirred at reflux for 2 days. The solution is cooled to RT, the solvent evaporated, the residue dissolved in EtOAc (200 mL) and washed with a solution of sat. aq. Na₂CO₃ (2 x 80 mL), 1M aq. KHSO₄ (2 x 80 mL) and brine (50 mL). The organic phase is dried over MgSO₄, filtered and evaporated to give 6-chloro-5-methyl-nicotinic acid ethyl ester as a solid; LC-MS: t_{R} = 0.92 min; [M+H]⁺ = 200.1; ¹H NMR (CDCl₃) δ 1.43 (t, *J* = 7.0 Hz, 3 H), 2.46 (s, 3 H), 4.43 (q, *J* = 7.3 Hz, 2 H), 8.16 (m, 1 H), 8.84 (d, *J* = 2.0 Hz, 1 H).

### A.13.4 Synthesis of 2-chloro-6-methyl-isonicotinic acid ethyl ester

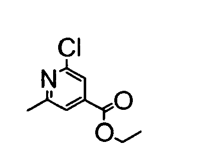

2-chloro-6-methyl-isonicotinic acid ethyl ester is prepared in analogy to 6-chloro-5-methyl-nicotinic acid ethyl ester by esterification of 2-chloro-6-methyl-isonicotinic acid with ethanol; LC-MS: t_{R} = 0.92 min; [M+H]⁺ = 200.0;

### A.13.5 Synthesis of 5-methyl-6-(2-methyl-propenyl)-nicotinic acid ethyl ester

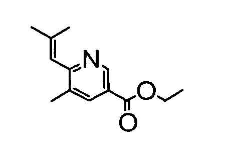

To a solution of 6-chloro-5-methyl-nicotinic acid ethyl ester (4.98 g, 24.9 mmol), 2,4,6-tri-(2-methyl-propenyl)-cycloboroxane pyridine complex (5.74 g, 17.7 mmol, prepared in analogy to a procedure given by F. Kerins, D. F. O'Shea J. Org. Chem. 67 (2002) 4968-4971), and PPh₃ (1.15 g, 4.4 mmol) in DME (60 mL), a solution of 2 M aq. K₂CO₃ (20 mL) is added. The mixture is degassed and flushed with N₂ before Pd(PPh₃)₄ (460 mg, 0.4 mmol) is added. The mixture is stirred at 90°C for 20 h before it is cooled to RT, diluted with EtOAc (150 mL) and washed with sat. aq. NaHCO₃ (2 x 50 mL). The organic extract is dried over MgSO₄, filtered and evaporated. The crude product is purified by FC (SiO₂, heptane/EtOAc) to give 5-methyl-6-(2-methyl-propenyl)-nicotinic acid ethyl ester as an orange oil; LC-MS: t_{R} = 0.72 min, [M+H]⁺ = 220.2.

### A.13.6 Synthesis of 2-methyl-6-(2-methyl-propenyl)-isonicotinic acid ethyl ester

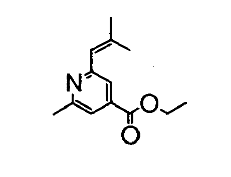

2-Methyl-6-(2-methyl-propenyl)-isonicotinic acid ethyl ester is prepared in analogy to 5-methyl-6-(2-methyl-propenyl)-nicotinic acid ethyl ester by Pd-catalyzed coupling of 2-chloro-6-methyl-isonicotinic acid ethyl ester with 2,4,6-tri-(2-methyl-propenyl)-cycloboroxane pyridine complex; LC-MS: t_{R} = 0.66 min, [M+H]⁺ = 220.4.

### A.13.7 Synthesis of 6-isobutyl-5-methyl-nicotinic acid ethyl ester

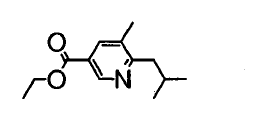

5-Methyl-6-(2-methyl-propenyl)-nicotinic acid ethyl ester (3.98 g, 18.2 mmol) is dissolved in THF (100 mL) and MeOH (100 mL), Pd/C (500 mg, 10% Pd) is added and the mixture is stirred under 1 atm H₂ at RT for 15 h. The catalyst is filtered off and the filtrate is evaporated to give 6-isobutyl-5-methyl-nicotinic acid ethyl ester as a colourless oil; LC-MS: t_{R} = 0.75 min; [M+H]⁺ = 222.2; ¹H NMR (CDCl₃) δ 0.97 (d, *J* = 6.8 Hz, 6 H), 1.42 (t*, J* = 7.3 Hz, 3 H), 2.20 (hept, *J* = 6.8 Hz, 1H), 2.38 (s, 3 H), 2.75 (d, *J* = 7.0 Hz, 2 H), 4.41 (q, *J* = 7.3 Hz, 2 H), 8.03 (d, *J* = 1.8 Hz, 1 H), 9.00 (d, *J*= 2.0 Hz, I H).

### A.13.8 Synthesis of 2-isobutyl-6-methyl-isonicotinic acid ethyl ester

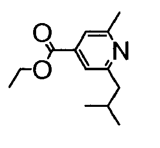

2-Isobutyl-6-methyl-isonicotinic acid ethyl ester is prepared in analogy to 6-isobutyl-5-methyl-nicotinic acid ethyl ester by hydrogenation of 2-methyl-6-(2-methyl-propenyl)-isonicotinic acid ethyl ester; LC-MS: t_{R} = 0.71 min; [M+H]⁺ = 222.1.

### A.13.9 Synthesis of 6-isobutyl-5-methyl-nicotinic acid

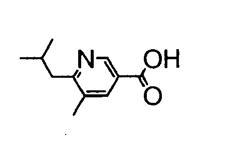

A solution of 6-isobutyl-5-methyl-nicotinic acid ethyl ester (3.75 g, 16.95 mmol) in 12.5% aq. HCl (50 mL) is stirred at 65°C for 24 h before the solvent is evaporated. The residue is dried in vacuo to give 6-isobutyl-5-methyl-nicotinic acid as a white powder; LC-MS: t_{R} = 0.57 min, [M+H]⁺ = 194.3.

### A.13.10 Synthesis of 2-isobutyl-6-methyl-isonicotinic acid

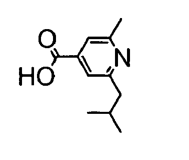

2-Isobutyl-6-methyl-isonicotinic acid is prepared in analogy to 6-isobutyl-5-methyl-nicotinic acid by saponification of 2-isobutyl-6-methyl-isonicotinic acid ethyl ester; LC-MS: t_{R} = 0.52 min; [M+H]⁺ = 194.1.

### B. Preparation of compounds of formula (I):

### B.1 Synthesis of sulfonamide derivatives (general procedure)

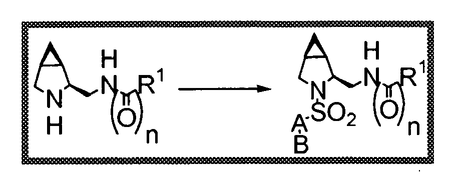

A solution of the respective sulfonyl chloride (0.03 mmol, 1.2eq) in MeCN (0.20 mL) is added to a solution of the respective 3-aza-bicyclo[3.1.0]hexane derivative (0.025 mmol, 1.0eq, hydrochloride salt) and DIPEA (0.06 mmol, 2.5eq) in DMF (0.20 mL). The mixture is shaken over night and purified by prep. HPLC to give the respective sulfonamide derivatives.

### Example 1:

### benzofuran-4-carboxylic acid [(1R*,2S*,5S*)-3-(biphenyl-2-sulfonyl)-3-aza-bicyclo[3.1.0]hex-2-ylmethyl]-amide

prepared by reaction of benzofuran-4-carboxylic acid [(1R*,2S*,5S*)-3-aza-bicyclo[3.1.0]hex-2-ylmethyl]-amide with biphenyl-2-sulfonyl chloride. LC-MS: t_{R} = 1.06 min; [M+H]⁺ = 473.2.

### Example 2:

### benzofuran-4-carboxylic acid [(1R*,2S*,5S*)-3-(2-trifluoromethoxy-benzene-sulfonyl)-3-aza-bicyclo[3.1.0] hex-2-ylmethyl]-amide

prepared by reaction of benzofuran-4-carboxylic acid [(1R*,2S*,5S*)-3-aza-bicyclo[3.1.0]hex-2-ylmethyl]-amide with 2-trifluoromethoxy-benzenesulfonyl chloride. LC-MS: t_{R} = 1.04 min; [M+H]⁺ = 481.1.

### Example 3:

### 6-methyl-imidazo[2,1-b]thiazole-5-carboxylic acid [(1R*,2S*,5S*)-3-(2-trifluoromethoxy-benzenesulfonyl)-3-aza-bicyclo[3.1.0] hex-2-ylmethyl]-amide

prepared by reaction of 6-methyl-imidazo[2,1-b]thiazole-5-carboxylic acid [(1R*,2S*,5S*)-3-aza-bicyclo[3.1.0]hex-2-ylmethyl]-amide with 2-trifluoromethoxy-benzenesulfonyl chloride. LC-MS: t_{R} = 0.87 min; [M+H]⁺ = 501.1.

### Example 4:

### 6-methyl-imidazo[2,1-b]thiazole-5-carboxylic acid [(1R*,2S*,5S*)-3-(naphthalene-1-sulfonyl)-3-aza-bicyclo[3.1.0] hex-2-ylmethyl]-amide

prepared by reaction of 6-methyl-imidazo[2,1-b]thiazole-5-carboxylic acid [(1R*,2S*,5S*)-3-aza-bicyclo[3.1.0]hex-2-ylmethyl]-amide with naphthalene-1-sulfonyl chloride. LC-MS: t_{R} = 0.87 min; [M+H]⁺ = 467.0.

### B.2 Synthesis of carboxylic amide derivatives (general procedure)

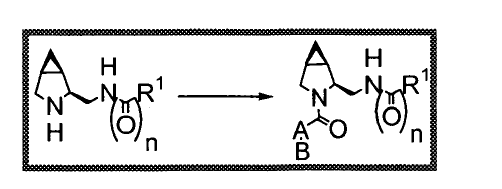

To the respective carboxylic acid (0.030 mmol, 1.2eq) is added successively DIPEA (0.075 mmol, 3.0eq) and a solution of TBTU (0.030 mmol, 1.2eq) in DMF (0.20 mL). The obtained mixture is added to a solution of the respective 3-aza-bicyclo[3.1.0]hexane derivative (0.025 mmol, 1.0eq, free base or hydrochloride salt) in DMF (0.20 mL). The mixture is shaken over night and purified by prep. HPLC to give the respective amide derivatives.

### Example 5:

### 4-fluoro-N-[(1 R*,2S*,5S*)-3-(2-methyl-5-p-tolyl-thiazole-4-carbonyl)-3-aza-bicyclo[3.1.0] hex-2-ylmethyl]-benzamide

prepared by reaction of N-[(1R*,2S*,5S*)-3-aza-bicyclo[3.1.0]hex-2-ylmethyl]-4-fluoro-benzamide with 2-methyl-5-p-tolyl-thiazole-4-carboxylic acid. LC-MS: t_{R} = 1.00 min; [M+H]⁺ = 450.0.

### Example 6:

### N-{(1R*,2S*,5S*)-3-[5-(4-ethyl-phenyl)-2-methyl-thiazole-4-carbonyl]-3-aza-bicyclo[3.1.0] hex-2-ylmethyl}-4-fluoro-benzamide

prepared by reaction of N-[(1R*,2S*,5S*)-3-aza-bicyclo[3.1.0]hex-2-ylmethyl]-4-fluoro-benzamide with 5-(4-ethyl-phenyl)-2-methyl-thiazole-4-carboxylic acid. LC-MS: t_{R} = 1.03 min; [M+H]⁺ = 464.2.

### Example 7:

### N-[(1R*,2S*,5S*)-3-(biphenyl-2-carbonyl)-3-aza-bicyclo[3.1.0]hex-2-ylmethyl]-4-fluoro-benzamide

prepared by reaction of N-[(1R*,2S*,5S*)-3-aza-bicyclo[3.1.0]hex-2-ylmethyl]-4-fluoro-benzamide with biphenyl-2-carboxylic acid. LC-MS: t_{R} = 1.02 min; [M+H]⁺ = 415.1.

### Example 8:

### 4-fluoro-N-{(1R*,2S*,5S*)-3-[5-(3-fluoro-phenyl)-2-methyl-thiazole-4-carbonyl]-3-aza-bicyclo[3.1.0]hex-2-ylmethyl}-benzamide

prepared by reaction of N-[(1R*,2S*,5S*)-3-aza-bicyclo[3.1.0]hex-2-ylmethyl]-4-fluoro-benzamide with 5-(3-fluoro-phenyl)-2-methyl-thiazole-4-carboxylic acid. LC-MS: t_{R} = 0.98 min; [M+H]⁺ = 454.1.

### Example 9:

### 4-fluoro-N-{(1R*,2S*,5S*)-3-[2-methyl-5-(4-trifluoromethyl-phenyl)-thiazole-4-carbonyl]-3-aza-bicyclo[3.1.0] hex-2-ylmethyl}-benzamide

prepared by reaction of N-[(1R*,2S*,5S*)-3-aza-bicyclo[3.1.0]hex-2-ylmethyl]-4-fluoro-benzamide with 2-methyl-5-(4-trifluoromethyl-phenyl)-thiazole-4-carboxylic acid. LC-MS: t_{R} = 1.03 min; [M+H]⁺ = 504.1.

### Example 10:

### 4-fluoro-N-[(1R*,2S*,5S*)-3-(2-methyl-5-m-tolyl-thiazole-4-carbonyl)-3-aza-bicyclo[3.1.0]hex-2-ylmethyl]-benzamide

prepared by reaction of N-[(1R*,2S*,5S*)-3-aza-bicyclo[3.1.0]hex-2-ylmethyl]-4-fluoro-benzamide with 2-methyl-5-m-tolyl-thiazole-4-carboxylic acid. LC-MS: t_{R} = 1.00 min; [M+H]⁺ = 450.1.

### Example 11:

### benzofuran-4-carboxylic acid [(1R*,2S*,5S*)-3-(biphenyl-2-carbonyl)-3-aza-bicyclo[3.1.0]hex-2-ylmethyl]-amide

prepared by reaction of benzofuran-4-carboxylic acid [(1R*,2S*,5S*)-3-aza-bicyclo[3.1.0]hex-2-ylmethyl]-amide with biphenyl-2-carboxylic acid. LC-MS: t_{R} = 1.04 min; [M+H]⁺ = 437.1.

### Example 12:

### benzofuran-4-carboxylic acid [(1R*,2S*,5S*)-3-(2-methyl-5-m-tolyl-thiazole-4-carbonyl)-3-aza-bicyclo[3.1.0]hex-2-ylmethyl]-amide

prepared by reaction of benzofuran-4-carboxylic acid [(1R*,2S*,5S*)-3-aza-bicyclo[3.1.0]hex-2-ylmethyl]-amide with 2-methyl-5-m-tolyl-thiazole-4-carboxylic acid. LC-MS: t_{R} = 1.02 min; [M+H]⁺ = 472.1.

### Example 13:

### benzofuran-4-carboxylic acid {(1R*,2S*,5S*)-3-[5-(4-fluoro-phenyl)-2-methyl-thiazole-4-carbonyl]-3-aza-bicyclo[3.1.0]hex-2-ylmethyl}-amide

prepared by reaction of benzofuran-4-carboxylic acid [(1R*,2S*,5S*)-3-aza-bicyclo[3.1.0]hex-2-ylmethyl]-amide with 5-(4-fluoro-phenyl)-2-methyl-thiazole-4-carboxylic acid. LC-MS: t_{R} = 0.99 min; [M+H]⁺ = 476.1.

### Example 14:

### benzofuran-4-carboxylic acid {(1R*,2S*,5S*)-3-[5-(3-fluoro-phenyl)-2-methyl-thiazole-4-carbonyl]-3-aza-bicyclo[3.1.0]hex-2-ylmethyl}-amide

prepared by reaction of benzofuran-4-carboxylic acid [(1R*,2S*,5S*)-3-aza-bicyclo[3.1.0]hex-2-ylmethyl]-amide with 5-(3-fluoro-phenyl)-2-methyl-thiazole-4-carboxylic acid. LC-MS: t_{R} = 1.00 min; [M+H]⁺ = 476.1.

### Example 15:

### benzofuran-4-carboxylic acid [(1R*,2S*,5S*)-3-(2-methyl-5-p-tolyl-thiazole-4-carbonyl)-3-aza-bicyclo[3.1.0]hex-2-ylmethyl]-amide

prepared by reaction of benzofuran-4-carboxylic acid [(1R*,2S*,5S*)-3-aza-bicyclo[3.1.0]hex-2-ylmethyl]-amide with 2-methyl-5-p-tolyl-thiazole-4-carboxylic acid. LC-MS: t_{R} = 1.02 min; [M+H]⁺ = 472.2.

### Example 16:

### benzofuran-4-carboxylic acid {(1R*,2S*,5S*)-3-[5-(4-ethyl-phenyl)-2-methyl-thiazole-4-carbonyl]-3-aza-bicyclo[3.1.0]hex-2-ylmethyl}-amide

prepared by reaction of benzofuran-4-carboxylic acid [(1R*,2S*,5S*)-3-aza-bicyclo[3.1.0]hex-2-ylmethyl]-amide with 5-(4-ethyl-phenyl)-2-methyl-thiazole-4-carboxylic acid. LC-MS: t_{R} = 1.05 min; [M+H]⁺ = 486.2.

### Example 17:

### pyridine-2-carboxylic acid [(1R*,2S*,5S*)-3-(2-methyl-5-m-tolyl-thiazole-4-carbonyl)-3-aza-bicyclo [3.1.0] hex-2-ylmethyl]-amide

prepared by reaction of pyridine-2-carboxylic acid [(1R*,2S*,5S*)-3-aza-bicyclo[3.1.0]hex-2-ylmethyl]-amide with 2-methyl-5-m-tolyl-thiazole-4-carboxylic acid. LC-MS: t_{R} = 0.96 min; [M+H]⁺ = 433.1.

### Example 18:

### benzofuran-4-carboxylic acid {(1R*,2S*,5S*)-3-[5-(2-fluoro-phenyl)-2-methyl-thiazole-4-carbonyl]-3-aza-bicyclo[3.1.0]hex-2-ylmethyl}-amide

prepared by reaction of benzofuran-4-carboxylic acid [(1R*,2S*,5S*)-3-aza-bicyclo[3.1.0]hex-2-ylmethyl]-amide with 5-(2-fluoro-phenyl)-2-methyl-thiazole-4-carboxylic acid. LC-MS: t_{R} = 0.91 min; [M+H]⁺ = 476.1.

### Example 19:

### benzofuran-4-carboxylic acid {(1R*,2S*,5S*)-3-[5-(2-chloro-phenyl)-2-methyl-thiazole-4-carbonyl]-3-aza-bicyclo[3.1.0]hex-2-ylmethyl}-amide

prepared by reaction of benzofuran-4-carboxylic acid [(1R*,2S*,5S*)-3-aza-bicyclo[3.1.0]hex-2-ylmethyl]-amide with 5-(2-chloro-phenyl)-2-methyl-thiazole-4-carboxylic acid. LC-MS: t_{R} = 0.94 min; [M+H]⁺ = 492.0.

### Example 20:

### benzofuran-4-carboxylic acid {(1R*,2S*,5S*)-3-[5-(3-chloro-phenyl)-2-methyl-thiazole-4-carbonyl]-3-aza-bicyclo[3.1.0]hex-2-ylmethyl}amide

prepared by reaction of benzofuran-4-carboxylic acid [(1R*,2S*,5S*)-3-aza-bicyclo[3.1.0]hex-2-ylmethyl]-amide with 5-(3-chloro-phenyl)-2-methyl-thiazole-4-carboxylic acid. LC-MS: t_{R} = 0.94 min; [M+H]⁺ = 492.0.

### Example 21:

### benzofuran-4-carboxylic acid {(1R*,2S*,5S*)-3-[5-(2-methoxy-phenyl)-2-methyl-thiazole-4-carbonyl]-3-aza-bicyclo[3.1.0]hex-2-ylmethyl}-amide

prepared by reaction of benzofuran-4-carboxylic acid [(1R*,2S*,5S*)-3-aza-bicyclo[3.1.0]hex-2-ylmethyl]-amide with 5-(2-methoxy-phenyl)-2-methyl-thiazole-4-carboxylic acid. LC-MS: t_{R} = 0.90 min; [M+H]⁺ = 488.1.

### Example 22:

### benzofuran-4-carboxylic acid {(1R*,2S*,5S*)-3-[5-(3-methoxy-phenyl)-2-methyl-thiazole-4-carbonyl]-3-aza-bicyclo[3.1.0]hex-2-ylmethyl}-amide

prepared by reaction of benzofuran-4-carboxylic acid [(1R*,2S*,5S*)-3-aza-bicyclo[3.1.0]hex-2-ylmethyl]-amide with 5-(3-methoxy-phenyl)-2-methyl-thiazole-4-carboxylic acid. LC-MS: t_{R} = 0.90 min; [M+H]⁺ = 488.2.

### Example 23:

### benzofuran-4-carboxylic acid {(1R*,2S*,5S*)-3-[2-methyl-5-(3-trifluoromethyl-phenyl)-thiazole-4-carbonyl]-3-aza-bicyclo[3.1.0]hex-2-ylmethyl}-amide

prepared by reaction of benzofuran-4-carboxylic acid [(1R*,2S*,5S*)-3-aza-bicyclo[3.1.0]hex-2-ylmethyl]-amide with 2-methyl-5-(3-trifluoromethyl-phenyl)-thiazole-4-carboxylic acid. LC-MS: t_{R} = 0.95 min; [M+H]⁺ = 526.1.

### Example 24:

### benzofuran-4-carboxylic acid {(1R*,2S*,5S*)-3-[2-methyl-5-(2-trifluoromethyl-phenyl)-thiazole-4-carbonyl]-3-aza-bicyclo[3.1.0]hex-2-ylmethyl}-amide

prepared by reaction of benzofuran-4-carboxylic acid [(1R*,2S*,5S*)-3-aza-bicyclo[3.1.0]hex-2-ylmethyl]-amide with 2-methyl-5-(2-trifluoromethyl-phenyl)-thiazole-4-carboxylic acid. LC-MS: t_{R} = 0.97 min; [M+H]⁺ = 526.1.

### Example 25:

### benzofuran-4-carboxylic acid [(1R*,2S*,5S*)-3-(2-methyl-5-o-tolyl-thiazole-4-carbonyl)-3-aza-bicyclo[3.1.0]hex-2-ylmethyl]-amide

prepared by reaction of benzofuran-4-carboxylic acid [(1R*,2S*,5S*)-3-aza-bicyclo[3.1.0]hex-2-ylmethyl]-amide with 2-methyl-5-o-tolyl-thiazole-4-carboxylic acid. LC-MS: t_{R} = 0.93 min; [M+H]⁺ = 472.2.

### Example 26:

### benzofuran-4-carboxylic acid {(1R*,2S*,5S*)-3-[5-(3,4-dimethyl-phenyl)-2-methyl-thiazole-4-carbonyl]-3-aza-bicyclo[3.1.0]hex-2-ylmethyl}-amide

prepared by reaction of benzofuran-4-carboxylic acid [(1R*,2S*,5S*)-3-aza-bicyclo[3.1.0]hex-2-ylmethyl]-amide with 5-(3,4-dimethyl-phenyl)-2-methyl-thiazole-4-carboxylic acid. LC-MS: t_{R} = 0.97 min; [M+H]⁺ = 486.2.

### Example 27:

### benzofuran-4-carboxylic acid [(1R*,2S*,5S*)-3-(2-amino-5-m-tolyl-thiazole-4-carbonyl)-3-aza-bicyclo[3.1.0]hex-2-ylmethyl]-amide

prepared by reaction of benzofuran-4-carboxylic acid [(1R*,2S*,5S*)-3-aza-bicyclo[3.1.0]hex-2-ylmethyl]-amide with 2-amino-5-m-tolyl-thiazole-4-carboxylic acid. LC-MS: t_{R} = 0.85 min; [M+H]⁺ = 473.1.

### Example 28:

### benzofuran-4-carboxylic acid {(1R*,2S*,5S*)-3-[2-amino-5-(3-fluoro-phenyl)-thiazole-4-carbonyl]-3-aza-bicyclo[3.1.0]hex-2-ylmethyl}-amide

prepared by reaction of benzofuran-4-carboxylic acid [(1R*,2S*,5S*)-3-aza-bicyclo[3.1.0]hex-2-ylmethyl]-amide with 2-amino-5-(3-fluoro-phenyl)-thiazole-4-carboxylic acid. LC-MS: t_{R} = 0.84 min; [M+H]⁺ = 477.1.

### Example 29:

### benzofuran-4-carboxylic acid [(1R*,2S*,5S*)-3-(2-methyl-4-phenyl-pyrimidine-5-carbonyl)-3-aza-bicyclo[3.1.0]hex-2-ylmethyl]-amide

prepared by reaction of benzofuran-4-carboxylic acid [(1R*,2S*,5S*)-3-aza-bicyclo[3.1.0]hex-2-ylmethyl]-amide with 2-methyl-4-phenyl-pyrimidine-5-carboxylic acid. LC-MS: t_{R} = 0.84 min; [M+H]⁺ = 453.2.

### Example 30:

### benzofuran-4-carboxylic acid {(1R*,2S*,5S*)-3-[2-(2-amino-thiazol-4-yl)-benzoyl]-3-aza-bicyclo[3.1.0]hex-2-ylmethyl}-amide

prepared by reaction of benzofuran-4-carboxylic acid [(1R*,2S*,5S*)-3-aza-bicyclo[3.1.0]hex-2-ylmethyl]-amide with 2-(2-amino-thiazol-4-yl)-benzoic acid. LC-MS: t_{R} = 0.82 min; [M+H]⁺ = 458.9.

### Example 31:

### benzofuran-4-carboxylic acid [(1R*,2S*,5S*)-3-(9H-fluorene-4-carbonyl)-3-aza-bicyclo[3.1.0]hex-2-ylmethyl]-amide

prepared by reaction of benzofuran-4-carboxylic acid [(1R*,2S*,5S*)-3-aza-bicyclo[3.1.0]hex-2-ylmethyl]-amide with 9H-fluorene-4-carboxylic acid. LC-MS: t_{R} = 0.96 min; [M+H]⁺ = 449.1.

### Example 32:

### benzofuran-4-carboxylic acid [(1R*,2S*,5S*)-3-(3-phenyl-pyrazine-2-carbonyl)-3-aza-bicyclo[3.1.0]hex-2-ylmethyl]-amide

prepared by reaction of benzofuran-4-carboxylic acid [(1R*,2S*,5S*)-3-aza-bicyclo[3.1.0]hex-2-ylmethyl]-amide with 3-phenyl-pyrazine-2-carboxylic acid. LC-MS: t_{R} = 0.84 min; [M+H]⁺ = 439.1.

### Example 33:

### benzofuran-4-carboxylic acid {(1R*,2S*,5S*)-3-[5-(4-methoxy-phenyl)-oxazole-4-carbonyl]-3-aza-bicyclo[3.1.0]hex-2-ylmethyl}-amide

prepared by reaction of benzofuran-4-carboxylic acid [(1R*,2S*,5S*)-3-aza-bicyclo[3.1.0]hex-2-ylmethyl]-amide with 5-(4-methoxy-phenyl)-oxazole-4-carboxylic acid. LC-MS: t_{R} = 0.87 min; [M+H]⁺ = 457.8.

### Example 34:

### benzofuran-4-carboxylic acid [(1R*,2S*,5S*)-3-(2-pyrazol-1-yl-benzoyl)-3-aza-bicyclo[3.1.0] hex-2-ylmethyl]-amide

prepared by reaction of benzofuran-4-carboxylic acid [(1R*,2S*,5S*)-3-aza-bicyclo[3.1.0]hex-2-ylmethyl]-amide with 2-pyrazol-1-yl-benzoic acid. LC-MS: t_{R} = 0.84 min; [M+H]⁺ = 426.9.

### Example 35:

### benzofuran-4-carboxylic acid [(1R*,2S*,5S*)-3-(2-phenyl-2H-pyrazole-3-carbonyl)-3-aza-bicyclo[3.1.0]hex-2-ylmethyl]-amide

prepared by reaction of benzofuran-4-carboxylic acid [(1R*,2S*,5S*)-3-aza-bicyclo[3.1.0]hex-2-ylmethyl]-amide with 2-phenyl-2H-pyrazole-3-carboxylic acid. LC-MS: t_{R} = 0.85 min; [M+H]⁺ = 427.0.

### Example 36:

### benzofuran-4-carboxylic acid [(1R*,2S*,5S*)-3-(2-methyl-3-phenyl-quinoline-4-carbonyl)-3-aza-bicyclo[3.1.0]hex-2-ylmethyl]-amide

prepared by reaction of benzofuran-4-carboxylic acid [(1R*,2S*,5S*)-3-aza-bicyclo[3.1.0]hex-2-ylmethyl]-amide with 2-methyl-3-phenyl-quinoline-4-carboxylic acid. LC-MS: t_{R} = 0.92 min; [M+H]⁺ = 502.2.

### Example 37:

### benzofuran-4-carboxylic acid [(1R*,2S*,5S*)-3-(5-phenyl-2H-[1,2,3]triazole-4-carbonyl)-3-aza-bicyclo[3.1.0]hex-2-ylmethyl]-amide

prepared by reaction of benzofuran-4-carboxylic acid [(1R*,2S*,5S*)-3-aza-bicyclo[3.1.0]hex-2-ylmethyl]-amide with 5-phenyl-2H-[1,2,3]triazole-4-carboxylic acid. LC-MS: t_{R} = 0.61 min; [M+H]⁺ = 428.1.

### Example 38:

### benzofuran-4-carboxylic acid [(1R*,2S*,5S*)-3-(2'-fluoro-biphenyl-2-carbonyl)-3-aza-bicyclo[3.1.0] hex-2-ylmethyl]-amide

prepared by reaction of benzofuran-4-carboxylic acid [(1R*,2S*,5S*)-3-aza-bicyclo[3.1.0]hex-2-ylmethyl]-amide with 2'-fluoro-biphenyl-2-carboxylic acid. LC-MS: t_{R} = 0.94 min; [M+H]⁺ = 455.1.

### Example 39:

### benzofuran-4-carboxylic acid [(1R*,2S*,5S*)-3-(4'-fluoro-biphenyl-2-carbonyl)-3-aza-bicyclo[3.1.0]hex-2-ylmethyl]-amide

prepared by reaction of benzofuran-4-carboxylic acid [(1R*,2S*,5S*)-3-aza-bicyclo[3.1.0]hex-2-ylmethyl]-amide with 4'-fluoro-biphenyl-2-carboxylic acid. LC-MS: t_{R} = 0.95 min; [M+H]⁺ = 455.1.

### Example 40:

### benzofuran-4-carboxylic acid [(1R*,2S*,5S*)-3-(2'-chloro-biphenyl-2-carbonyl)-3-aza-bicyclo[3.1.0]hex-2-ylmethyl]-amide

prepared by reaction of benzofuran-4-carboxylic acid [(1R*,2S*,5S*)-3-aza-bicyclo[3.1.0]hex-2-ylmethyl]-amide with 2'-chloro-biphenyl-2-carboxylic acid. LC-MS: t_{R} = 0.97 min; [M+H]⁺ = 471.1.

### Example 41:

### benzofuran-4-carboxylic acid [(1R*,2S*,5S*)-3-(3'-chloro-biphenyl-2-carbonyl)-3-aza-bicyclo[3.1.0]hex-2-ylmethyl]-amide

prepared by reaction of benzofuran-4-carboxylic acid [(1R*,2S*,5S*)-3-aza-bicyclo[3.1.0]hex-2-ylmethyl]-amide with 3'-chloro-biphenyl-2-carboxylic acid. LC-MS: t_{R} = 0.98 min; [M+H]⁺ = 471.1.

### Example 42:

### benzofuran-4-carboxylic acid [(1R*,2S*,5S*)-3-(4'-chloro-biphenyl-2-carbonyl)-3-aza-bicyclo[3.1.0]hex-2-ylmethyl]-amide

prepared by reaction of benzofuran-4-carboxylic acid [(1R*,2S*,5S*)-3-aza-bicyclo[3.1.0]hex-2-ylmethyl]-amide with 4'-chloro-biphenyl-2-carboxylic acid. LC-MS: t_{R} = 0.98 min; [M+H]⁺ = 471.1.

### Example 43:

### benzofuran-4-carboxylic acid [(1R*,2S*,5S*)-3-(4'-methyl-biphenyl-2-carbonyl)-3-aza-bicyclo[3.1.0]hex-2-ylmethyl]-amide

prepared by reaction of benzofuran-4-carboxylic acid [(1R*,2S*,5S*)-3-aza-bicyclo[3.1.0]hex-2-ylmethyl]-amide with 4'-methyl-biphenyl-2-carboxylic acid. LC-MS: t_{R} = 0.98 min; [M+H]⁺ = 451.2.

### Example 44:

### benzofuran-4-carboxylic acid [(1R*,2S*,5S*)-3-(3'-methyl-biphenyl-2-carbonyl)-3-aza-bicyclo[3.1.0]hex-2-ylmethyl]-amide

prepared by reaction of benzofuran-4-carboxylic acid [(1R*,2S*,5S*)-3-aza-bicyclo[3.1.0]hex-2-ylmethyl]-amide with 3'-methyl-biphenyl-2-carboxylic acid. LC-MS: t_{R} = 0.98 min; [M+H]⁺ = 451.2.

### Example 45:

### benzofuran-4-carboxylic acid [(1R*,2S*,5S*)3-(3'-methoxy-biphenyl-2-carbonyl)-3-aza-bicyclo[3.1.0]hex-2-ylmethyl]-amide

prepared by reaction of benzofuran-4-carboxylic acid [(1R*,2S*,5S*)-3-aza-bicyclo[3.1.0]hex-2-ylmethyl]-amide with 3'-methoxy-biphenyl-2-carboxylic acid. LC-MS: t_{R} = 0.95 min; [M+H]⁺ = 467.2.

### Example 46:

### benzofuran-4-carboxylic acid [(1R*,2S*,5S*)-3-(4'-methoxy-biphenyl-2-carbonyl)-3-aza-bicyclo[3.1.0]hex-2-ylmethyl]-amide

prepared by reaction of benzofuran-4-carboxylic acid [(1R*,2S*,5S*)-3-aza-bicyclo[3.1.0]hex-2-ylmethyl]-amide with 4'-methoxy-biphenyl-2-carboxylic acid. LC-MS: t_{R} = 0.95 min; [M+H]⁺ = 467.2.

### Example 47:

### benzofuran-4-carboxylic acid [(1R*,2S*,5S*)-3-(2'-rnethoxy-biphenyl-2-carbonyl)-3-aza-bicyclo[3.1.0]hex-2-ylmethyl]-amide

prepared by reaction of benzofuran-4-carboxylic acid [(1R*,2S*,5S*)-3-aza-bicyclo[3.1.0]hex-2-ylmethyl]-amide with 2'-methoxy-biphenyl-2-carboxylic acid. LC-MS: t_{R} = 0.94 min; [M+H]⁺ = 467.2.

### Example 48:

### benzofuran-4-carboxylic acid [(1R*,2S*,5S*)-3-(3'-trifluoromethyl-biphenyl-2-carbonyl)-3-aza-bicyclo[3.1.0]hex-2-ylmethyl]-amide

prepared by reaction of benzofuran-4-carboxylic acid [(1R*,2S*,5S*)-3-aza-bicyclo[3.1.0]hex-2-ylmethyl]-amide with 3'-trifluoromethyl-biphenyl-2-carboxylic acid. LC-MS: t_{R} = 0.99 min; [M+H]⁺ = 505.2.

### Example 49:

### benzofuran-4-carboxylic acid [(1R*,2S*,5S*)-3-(4'-trifluoromethyl-biphenyl-2-carbonyl)-3-aza-bicyclo[3.1.0]hex-2-ylmethyl]-amide

prepared by reaction of benzofuran-4-carboxylic acid [(1R*,2S*,5S*)-3-aza-bicyclo[3.1.0]hex-2-ylmethyl]-amide with 4'-trifluoromethyl-biphenyl-2-carboxylic acid. LC-MS: t_{R} = 0.99 min; [M+H]⁺ = 505.2.

### Example 50:

### benzofuran-4-carboxylic acid [(1R*,2S*,5S*)-3-(3',4'-dimethyl-biphenyl-2-carbonyl)-3-aza-bicyclo[3.1.0]hex-2-ylmethyl]-amide

prepared by reaction of benzofuran-4-carboxylic acid [(1R*,2S*,5S*)-3-aza-bicyclo[3.1.0]hex-2-ylmethyl]-amide with 3',4'-dimethyl-biphenyl-2-carboxylic acid. LC-MS: tₖ = 1.01 min; [M+H]⁺ = 465.3.

### Example 51:

### benzofuran-4-carboxylic acid [(1R*,2S*,5S*)-3-(2-pyridin-3-yl-benzoyl)-3-aza-bicyclo[3.1.0]hex-2-ylmethyl]-amide

prepared by reaction of benzofuran-4-carboxylic acid [3-aza-bicyclo[3.1.0]hex-2-ylmethyl]-amide with 2-pyridin-3-yl-benzoic acid. LC-MS: t_{R} = 0.82 min; [M+H]⁺ = 438.2.

### Example 52:

### 6-methyl-imidazo[2,1-b]thiazole-5-carboxylic acid [(1R*,2S*,5S*)-3-(2-methyl-5-p-tolyl-thiazole-4-carbonyl)-3-aza-bicyclo[3.1.0]hex-2-ylmethyl]-amide

prepared by reaction of 6-methyl-imidazo[2,1-b]thiazole-5-carboxylic acid [(1R*,2S*,5S*)-3-aza-bicyclo[3.1.0]hex-2-ylmethyl]-amide with 2-methyl-5-p-tolyl-thiazole-4-carboxylic acid. LC-MS: t_{R} = 0.87 min; [M+H]⁺ = 492.2.

### Example 53:

### 6-methyl-imidazo[2,1-b]thiazole-5-carboxylic acid [(1R*,2S*,5S*)-3-(2-methyl-5-m-tolyl-thiazole-4-carbonyl)-3-aza-bicyclo[3.1.0]hex-2-ylmethyl]-amide

prepared by reaction of 6-methyl-imidazo[2,1-b]thiazole-5-carboxylic acid [(1R*,2S*,5S*)-3-aza-bicyclo[3.1.0]hex-2-ylmethyl]-amide with 2-methyl-5-m-tolyl-thiazole-4-carboxylic acid. LC-MS: t_{R} = 0.86 min; [M+H]⁺ = 492.1.

### Example 54:

### 6-methyl-imidazo[2,1-b]thiazole-5-carboxylic acid {(1R*,2S*,5S*)-3-[5-(3-fluoro-phenyl)-2-methyl-thiazole-4-carbonyl]-3-aza-bicyclo[3.1.0] hex-2-ylmethyl}-amide

prepared by reaction of 6-methyl-imidazo[2,1-b]thiazole-5-carboxylic acid [(1R*,2S*,5S*)-3-aza-bicyclo[3.1.0]hex-2-ylmethyl]-amide with 5-(3-fluoro-phenyl)-2-methyl-thiazole-4-carboxylic acid. LC-MS: t_{R} = 0.84 min; [M+H]⁺ = 496.1.

### Example 55:

### 6-methyl-imidazo[2,1-b]thiazole-5-carboxylic acid {(1R*,2S*,5S*)-3-[5-(4-ethyl-phenyl)-2-methyl-thiazole-4-carbonyl]-3-aza-bicyclo[3.1.0]hex-2-ylmethyl}-amide

prepared by reaction of 6-methyl-imidazo[2,1-b]thiazole-5-carboxylic acid [(1R*,2S*,5S*)-3-aza-bicyclo[3.1.0]hex-2-ylmethyl]-amide with 5-(4-ethyl-phenyl)-2-methyl-thiazole-4-carboxylic acid. LC-MS: t_{R} = 0.91 min; [M+H]⁺ = 506.0.

### Example 56:

### 6-methyl-imidazo[2,1-b]thiazole-5-carboxylic acid {(1R*,2S*,5S*)-3-[5-(4-fluoro-phenyl)-2-methyl-thiazole-4-carbonyl]-3-aza-bicyclo[3.1.0]hex-2-ylmethyl}-amide

prepared by reaction of 6-methyl-imidazo[2,1-b]thiazole-5-carboxylic acid [(1R*,2S*,5S*)-3-aza-bicyclo[3.1.0]hex-2-ylmethyl]-amide with 5-(4-fluoro-phenyl)-2-methyl-thiazole-4-carboxylic acid. LC-MS: t_{R} = 0.83 min; [M+H]⁺ = 496.1.

### Example 57:

### 6-methyl-imidazo[2,1-b]thiazole-5-carboxylic acid [(1R*,2S*,5S*)-3-(biphenyl-2-carbonyl)-3-aza-bicyclo[3.1.0]hex-2-ylmethyl]-amide

prepared by reaction of 6-methyl-imidazo[2,1-b]thiazole-5-carboxylic acid [(1R*,2S*,5S*)-3-aza-bicyclo[3.1.0]hex-2-ylmethyl]-amide with biphenyl-2-carboxylic acid. LC-MS: t_{R} = 0.88 min; [M+H]⁺ = 457.0.

### Example 58:

### 6-methyl-imidazo[2,1-b]thiazole-5-carboxylic acid [(1R*,2S*,5S*)-3-(3-trifluoromethyl-benzoyl)-3-aza-bicyclo[3.1.0]hex-2-ylmethyl]-amide

prepared by reaction of 6-methyl-imidazo[2,1-b]thiazole-5-carboxylic acid [(1R*,2S*,5S*)-3-aza-bicyclo[3.1.0]hex-2-ylmethyl]-amide with 3-trifluoromethylbenzoic acid. LC-MS: t_{R} = 0.85 min; [M+H]⁺ = 449.1.

### Example 59:

### {(1R*,2S*,5S*)-2-[(6,7-difluoro-quinoxalin-2-ylamino)-methyl]-3-aza-bicyclo[3.1.0]hex-3-yl}-(2-methyl-5-m-tolyl-thiazol-4-yl)-methanone

prepared by reaction of (1R*,2S*,5S*)-(3-aza-bicyclo[3.1.0]hex-2-ylmethyl)-(6,7-difluoro-quinoxalin-2-yl)-amine with 2-methyl-5-m-tolyl-thiazole-4-carboxylic acid. LC-MS: t_{R} = 1.00 min; [M+H]⁺ = 492.1.

### Example 60:

### {(1R*,2S*,5S*)-2-[(6,7-difluoro-quinoxalin-2-ylamino)-methyl]-3-aza-bicyclo[3.1.0]hex-3-yl}-[5-(4-fluoro-phenyl)-2-methyl-thiazol-4-yl]-methanone

prepared by reaction of (1R*,2S*,5S*)-(3-aza-bicyclo[3.1.0]hex-2-ylmethyl)-(6,7-difluoro-quinoxalin-2-yl)-amine with 5-(4-fluoro-phenyl)-2-methyl-thiazole-4-carboxylic acid. LC-MS: t_{R} = 0.96 min; [M+H]⁺ = 496.1.

### Example 61:

### biphenyl-2-yl-{(1R*,2S*,5S*)-2-[(6,7-difluoro-quinoxalin-2-ylamino)-methyl]-3-aza-bicyclo[3.1.0]hex-3-yl}-methanone

prepared by reaction of (1R*,2S*,5S*)-(3-aza-bicyclo[3.1.0]hex-2-ylmethyl)-(6,7-difluoro-quinoxalin-2-yl)-amine with biphenyl-2-carboxylic acid. LC-MS: t_{R} = 1.01 min; [M+H]⁺ = 457.0.

### Example 62:

### {(1R*,2S*,5S*)-2-[(6,7-difluoro-quinoxalin-2-ylamino)-methyl]-3-aza-bicyclo[3.1.0]hex-3-yl}-naphthalen-1-yl-methanone

prepared by reaction of (1R*,2S*,5S*)-(3-aza-bicyclo[3.1.0]hex-2-ylmethyl)-(6,7-difluoro-quinoxalin-2-yl)-amine with naphthalene-1-carboxylic acid. LC-MS: t_{R} = 0.97 min; [M+H]⁺ = 431.1.

### Example 63:

### {(1R*,2S*,5S*)-2-[(5-bromo-pyrimidin-2-ylamino)-methylt]-3-aza-bicyclo[3.1.0]hex-3-yl}-(2-methyl-5-m-tolyl-thiazol-4-yl)-methanone

prepared by reaction of (1R*,2S*,5S*)-(3-aza-bicyclo[3.1.0]hex-2-ylmethyl)-(5-bromo-pyrimidin-2-yl)-amine with 2-methyl-5-m-tolyl-thiazole-4-carboxylic acid. LC-MS: t_{R} = 0.97 min; [M+H]⁺ = 484.0.

### Example 64:

### biphenyl-2-yl-{(1R*,2S*,5S*)-2-[(5-bromo-pyrimidin-2-ylamino)-methyl]-3-aza-bicyclo[3.1.0]hex-3-yl}-methanone

prepared by reaction of (1R*,2S*,5S*)-(3-aza-bicyclo[3.1.0]hex-2-ylmethyl)-(5-bromo-pyrimidin-2-yl)-amine with biphenyl-2-carboxylic acid. LC-MS: t_{R} = 0.99 min; [M+H]⁺ = 449.1.

### B.3 Synthesis of carboxylic amide derivatives (general procedure II)

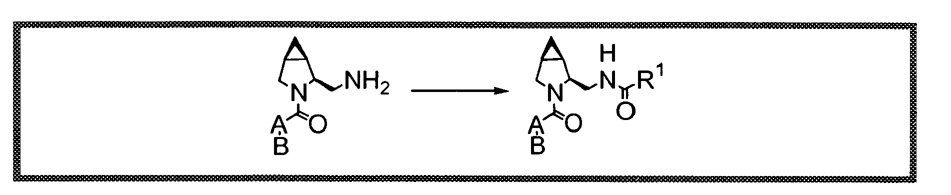

To a solution of the respective carboxylic acid (0.030 mmol, 1.8eq) in DMF (0.25 mL) is added successively a solution of DIPEA (0.075 mmol, 4.4eq) in DMF (0.15 mL) and a solution of TBTU (0.030 mmol, 1.8eq) in DMF (0.15 mL). The obtained mixture is treated with a solution of the respective 3-aza-bicyclo[3.1.0]hexane derivative (0.017 mmol, 1.0eq, free base) in DMF (0.15 mL). The mixture is shaken over night and purified by prep. HPLC to give the respective amide derivatives.

### Example 65:

### imidazo[2,1-b]thiazole-6-carboxylic acid [(1R*,2S*,5S*)-3-(2-methyl-5-m-tolyl-thiazole-4-carbonyl)-3-aza-bicyclo[3.1.0] hex-2-ylmethyl]-amide

prepared by reaction of [(1R*,2S*,5S*)-2-aminomethyl-3-aza-bicyclo[3.1.0]hex-3-yl]-(2-methyl-5-m-tolyl-thiazol-4-yl)-methanone with imidazo[2,1-b]thiazole-6-carboxylic acid. LC-MS: t_{R} = 0.84 min; [M+H]⁺ = 478.1.

### Example 66:

### 3-furan-2-yl-N-[(1R*,2S*,5S*)-3-(2-methyl-5-m-tolyl-thiazole-4-carbonyl)-3-aza-bicyclo[3.1.0] hex-2-ylmethyl]-acrylamide

prepared by reaction of [(1R*,2S*,5S*)-2-aminomethyl-3-aza-bicyclo[3.1.0]hex-3-yl]-(2-methyl-5-m-tolyl-thiazol-4-yl)-methanone with 3-furan-2-yl-acrylic acid. LC-MS: t_{R} = 0.88 min; [M+H]⁺ = 448.2.

### Example 67:

### naphthalene-2-carboxylic acid [(1R*,2S*,5S*)-3-(2-methyl-5-m-tolyl-thiazole-4-carbonyl)-3-aza-bicyclo[3.1.0]hex-2-ylmethyl]-amide

prepared by reaction of [(1R*,2S*,5S*)-2-aminomethyl-3-aza-bicyclo[3.1.0]hex-3-yl]-(2-methyl-5-m-tolyl-thiazol-4-yl)-methanone with naphthalene-2-carboxylic acid. LC-MS: t_{R} = 0.97 min; [M+H]⁺ = 482.2.

### Example 68:

### naphthalene-1-carboxylic acid [(1R*,2S*,5S*)-3-(2-methyl-5-m-tolyl-thiazole-4-carbonyl)-3-aza-bicyclo[3.1.0]hex-2-ylmethyl]-amide

prepared by reaction of [(1R*,2S*,5S*)-2-aminomethyl-3-aza-bicyclo[3.1.0]hex-3-yl]-(2-methyl-5-m-tolyl-thiazol-4-yl)-methanone with naphthalene-1-carboxylic acid. LC-MS: t_{R} = 0.95 min; [M+H]⁺ = 482.2.

### Example 69:

### 2,3-dihydro-benzofuran-7-carboxylic acid [(1R*,2S*,5S*)-3-(2-methyl-5-m-tolyl-thiazole-4-carbonyl)-3-aza-bicyclo[3.1.0]hex-2-ylmethyl]-amide

prepared by reaction of [(1R*,2S*,5S*)-2-aminomethyl-3-aza-bicyclo[3.1.0]hex-3-yl]-(2-methyl-5-m-tolyl-thiazol-4-yl)-methanone with 2,3-dihydro-benzofuran-7-carboxylic acid. LC-MS: t_{R} = 0.92 min; [M+H]⁺ = 474.2.

### Example 70:

### 2-methyl-thiazole-4-carboxylic acid [(1R*,2S*,5S*)-3-(2-methyl-5-m-tolyl-thiazole-4-carbonyl)-3-aza-bicyclo[3.1.0]hex-2-ylmethyl]-amide

prepared by reaction of [(1R*,2S*,5S*)-2-aminomethyl-3-aza-bicyclo[3.1.0]hex-3-yl]-(2-methyl-5-m-tolyl-thiazol-4-yl)-methanone with 2-methyl-thiazole-4-carboxylic acid. LC-MS: t_{R} = 0.88 min; [M+H]⁺ = 453.1.

### Example 71:

### 1H-indole-5-carboxylic acid [(1R*,2S*,5S*)-3-(2-methyl-5-m-tolyl-thiazole-4-carbonyl)-3-aza-bicyclo[3.1.0]hex-2-ylmethyl]-amide

prepared by reaction of [(1R*,2S*,5S*)-2-aminomethyl-3-aza-bicyclo[3.1.0]hex-3-yl]-(2-methyl-5-m-tolyl-thiazol-4-yl)-methanone with 1H-indole-5-carboxylic acid. LC-MS: t_{R} = 0.87 min; [M+H]⁺ = 471.2.

### Example 72:

### 3-hydroxy-N-[(1R*,2S*,5S*)-3-(2-methyl-5-m-tolyl-thiazole-4-carbonyl)-3-aza-bicyclo[3.1.0]hex-2-ylmethyl]-benzamide

prepared by reaction of [(1R*,2S*,5S*)-2-aminomethyl-3-aza-bicyclo[3.1.0]hex-3-yl]-(2-methyl-5-m-tolyl-thiazol-4-yl)-methanone with 3-hydroxy-benzoic acid. LC-MS: t_{R} = 0.78 min; [M+H]⁺ = 448.1.

### Example 73:

### 2-hydroxy-N-[(1R*,2S*,5S*)-3-(2-methyl-5-m-tolyl-thiazole-4-carbonyl)-3-aza-bicyclo[3.1.0]hex-2-ylmethyl]-benzamide

prepared by reaction of [(1R*,2S*,5S*)-2-aminomethyl-3-aza-bicyclo[3.1.0]hex-3-yl]-(2-methyl-5-m-tolyl-thiazol-4-yl)-methanone with 2-hydroxy-benzoic acid. LC-MS: t_{R} = 0.73 min; [M+H]⁺ = 448.2.

### Example 74:

### 2-bromo-4-methyl-thiazole-5-carboxylic acid [(1R*,2S*,5S*)-3-(2-methyl-5-m-tolyl-thiazole-4-carbonyl)-3-aza-bicyclo[3.1.0]hex-2-ylmethyl]-amide

prepared by reaction of [(-1R*,2S*,5S*)-2-aminomethyl-3-aza-bicyclo[3.1.0]hex-3-yl]-(2-methyl-5-m-tolyl-thiazol-4-yl)-methanone with 2-bromo-4-methyl-thiazole-5-carboxylic acid. LC-MS: t_{R} = 0.96 min; [M+H]⁺ = 531.0.

### Example 75:

### furan-3-carboxylic acid [(1R*,2S*,5S*)-3-(2-methyl-5-m-tolyl-thiazole-4-carbonyl)-3-aza-bicyclo[3.1.0]hex-2-ylmethyl]-amide

prepared by reaction of [(1R*,2S*,5S*)-2-aminomethyl-3-aza-bicyclo[3.1.0]hex-3-yl]-(2-methyl-5-m-tolyl-thiazol-4-yl)-methanone with furan-3-carboxylic acid. LC-MS: t_{R} = 0.85 min; [M+H]⁺ = 422.2.

### Example 76:

### N-[(1R*,2S*,5S*)-3-(2-methyl-5-m-tolyl-thiazole-4-carbonyl)-3-aza-bicyclo[3.1.0]hex-2-ylmethyl]-2-(naphthalen-2-yloxy)-acetamide

prepared by reaction of [(1R*,2S*,5S*)-2-aminomethyl-3-aza-bicyclo[3.1.0]hex-3-yl]-(2-methyl-5-m-tolyl-thiazol-4-yl)-methanone with (naphthalen-2-yloxy)-acetic acid. LC-MS: t_{R} = 0.98 min; [M+H]⁺ = 512.2.

### Example 77:

### 3,5-dimethyl-isoxazole-4-carboxylic acid [(1R*,2S*,5S*)-3-(2-methyl-5-m-tolyl-thiazole-4-carbonyl)-3-aza-bicyclo[3.1.0]hex-2-ylmethyl]-amide

prepared by reaction of [(1R*,2S*,5S*)-2-aminomethyl-3-aza-bicyclo[3.1.0]hex-3-yl]-(2-methyl-5-m-tolyl-thiazol-4-yl)-methanone with 3,5-dimethyl-isoxazole-4-carboxylic acid. LC-MS: t_{R} = 0.88 min; [M+H]⁺ = 451.2.

### Example 78:

### 4-oxo-4H-chromene-2-carboxylic acid [(1R*,2S*,5S*)-3-(2-methyl-5-m-tolyl-thiazole-4-carbonyl)-3-aza-bicyclo[3.1.0]hex-2-ylmethyl]-amide

prepared by reaction of [(1R*,2S*,5S*)-2-aminomethyl-3-aza-bicyclo[3.1.0]hex-3-yl]-(2-methyl-5-m-tolyl-thiazol-4-yl)-methanone with 4-oxo-4H-chromene-2-carboxylic acid. LC-MS: t_{R} = 0.90 min; [M+H]⁺ = 500.2.

### Example 79:

### 3,5-dimethoxy-N-[(1R*,2S*,5S*)-3-(2-methyl-5-m-tolyl-thiazole-4-carbonyl)-3-aza-bicyclo[3.1.0] hex-2-ylmethyl]-benzamide

prepared by reaction of [(1R*,2S*,5S*)-2-aminomethyl-3-aza-bicyclo[3.1.0]hex-3-yl]-(2-methyl-5-m-tolyl-thiazol-4-yl)-methanone with 3,5-dimethoxy-benzoic acid. LC-MS: t_{R} = 0.92 min; [M+H]⁺ = 492.1.

### Example 80:

### benzo[1,3]dioxole-5-carboxylic acid [(1R*,2S*,5S*)-3-(2-methyl-5-m-tolyl-thiazole-4-carbonyl)-3-aza-bicyclo[3.1.0]hex-2-ylmethyl]-amide

prepared by reaction of [(1R*,2S*,5S*)-2-aminomethyl-3-aza-bicyclo[3.1.0]hex-3-yl]-(2-methyl-5-m-tolyl-thiazol-4-yl)-methanone with benzo[1,3]dioxole-5-carboxylic acid. LC-MS: t_{R} = 0.89 min; [M+H]⁺ = 476.2.

### Example 81:

### 2,4-dimethoxy-N-[(1R*,2S*,5S*)-3-(2-methyl-5-m-tolyl-thiazole-4-carbonyl)-3-aza-bicyclo[3.1.0]hex-2-ylmethyl]-benzamide

prepared by reaction of [(1R*,2S*,5S*)-2-aminomethyl-3-aza-bicyclo[3.1.0]hex-3-yl]-(2-methyl-5-m-tolyl-thiazol-4-yl)-methanone with 2,4-dimethoxy-benzoic acid. LC-MS: t_{R} = 0.92 min; [M+H]⁺ = 492.2.

### Example 82:

### 2,4-dimethyl-thiazole-5-carboxylic acid [(1R*,2S*,5S*)-3-(2-methyl-5-m-tolyl-thiazole-4-carbonyl)-3-aza-bicyclo[3.1.0]hex-2-ylmethyl]-amide

prepared by reaction of [(1R*,2S*,5S*)-2-aminomethyl-3-aza-bicyclo[3.1.0]hex-3-yl]-(2-methyl-5-m-tolyl-thiazol-4-yl)-methanone with 2,4-dimethyl-thiazole-5-carboxylic acid. LC-MS: t_{R} = 0.86 min; [M+H]⁺ = 467.2.

### Example 83:

### 1-methyl-1H-indole-2-carboxylic acid [(1R*,2S*,5S*)-3-(2-methyl-5-m-tolyl-thiazole-4-carbonyl)-3-aza-bicyclo[3.1.0]hex-2-ylmethyl]-amide

prepared by reaction of [(1R*,2S*,5S*)-2-aminomethyl-3-aza-bicyclo[3.1.0]hex-3-yl]-(2-methyl-5-m-tolyl-thiazol-4-yl)-methanone with 1-methyl-1H-indole-2-carboxylic acid. LC-MS: t_{R} = 0.98 min; [M+H]⁺ = 485.2.

### Example 84:

### 3H-benzoimidazole-5-carboxylic acid [(1R*,2S*,5S*)-3-(2-methyl-5-m-tolyl-thiazole-4-carbonyl)-3-aza-bicyclo[3.1.0]hex-2-ylmethyl]-amide

prepared by reaction of [(1R*,2S*,4S*)-2-aminomethyl-3-aza-bicyclo[3.1.0]hex-3-yl]-(2-methyl-5-m-tolyl-thiazol-4-yl)-methanone with 3H-benzoimidazole-5-carboxylic acid. LC-MS: t_{R} = 0.78 min; [M+H]⁺ = 472.2.

### Example 85:

### 2-(2-methoxy-phenoxy)-N-[(1R*,2S*,5S*)-3-(2-methyl-5-m-tolyl-thiazole-4-carbonyl)-3-aza-bicyclo[3.1.0]hex-2-ylmethyl]-acetamide

prepared by reaction of [(1R*,2S*,5S*)-2-aminomethyl-3-aza-bicyclo[3.1.0]hex-3-yl]-(2-methyl-5-m-tolyl-thiazol-4-yl)-methanone with (2-methoxy-phenoxy)-acetic acid. LC-MS: t_{R} = 0.91 min; [M+H]⁺ = 492.1.

### Example 86:

### benzo[2,1,3]oxadiazole-5-carboxylic acid [(1R*,2S*,5S*)-3-(2-methyl-5-m-tolyl-thiazole-4-carbonyl)-3-aza-bicyclo[3.1.0]hex-2-ylmethyl]-amide

prepared by reaction of [(1R*,2S*,5S*)-2-aminomethyl-3-aza-bicyclo[3.1.0]hex-3-yl]-(2-methyl-5-m-tolyl-thiazol-4-yl)-methanone with benzo[2,1,3]oxadiazole-5-carboxylic acid. LC-MS: t_{R} = 0.93 min; [M+H]⁺ = 474.2.

### Example 87:

### 2,3-dihydro-benzo[1,4]dioxine-5-carboxylic acid [(1R*,2S*,5S*)-3-(2-methyl-5-m-tolyl-thiazole-4-carbonyl)-3-aza-bicyclo[3.1.0]hex-2-ylmethyl]-amide

prepared by reaction of [(1R*,2S*,5S*)-2-aminomethyl-3-aza-bicyclo[3.1.0]hex-3-yl]-(2-methyl-5-m-tolyl-thiazol-4-yl)-methanone with 2,3-dihydro-benzo[1,4]dioxine-5-carboxylic acid. LC-MS: t_{R} = 0.91 min; [M+H]⁺ = 489.8.

### Example 88:

### [1,6]naphthyridine-2-carboxylic acid [(1R*,2S*,5S*)-3-(2-methyl-5-m-tolyl-thiazole-4-carbonyl)-3-aza-bicyclo[3.1.0]hex-2-ylmethyl]-amide

prepared by reaction of [(1R*,2S*,5S*)-2-aminomethyl-3-aza-bicyclo[3.1.0]hex-3-yl]-(2-methyl-5-m-tolyl-thiazol-4-yl)-methanone with [1,6]naphthyridine-2-carboxylic acid. LC-MS: t_{R} = 0.86 min; [M+H]⁺ = 484.2.

### Example 89:

### 2-(3-methoxy-phenoxy)-N-[(1R*,2S*,5S*)-3-(2-methyl-5-m-tolyl-thiazole-4-carbonyl)-3-aza-bicyclo[3.1.0]hex-2-ylmethyl]-acetamide

prepared by reaction of [(1R*,2S*,5S*)-2-aminomethyl-3-aza-bicyclo[3.1.0]hex-3-yl]-(2-methyl-5-m-tolyl-thiazol-4-yl)-methanone with (3-methoxy-phenoxy)-acetic acid. LC-MS: t_{R} = 0.92 min; [M+H]⁺ = 492.2.

### Example 90:

### 2-(2,5-dimethyl-thiazol-4-yl)-N-[(1R*,2S*,5S*)-3-(2-methyl-5-m-tolyl-thiazole-4-carbonyl)-3-aza-bicyclo[3.1.0]hex-2-ylmethyl]-acetamide

prepared by reaction of [(1R*,2S*,5S*)-2-aminomethyl-3-aza-bicyclo[3.1.0]hex-3-yl]-(2-methyl-5-m-tolyl-thiazol-4-yl)-methanone with (2,5-dimethyl-thiazol-4-yl)-acetic acid. LC-MS: t_{R} = 0.86 min; [M+H]⁺ = 481.2.

### Example 91:

### 5-chloro-1,3-dimethyl-1H-pyrazole-4-carboxylic acid [(1R*,2S*,5S*)-3-(2-methyl-5-m-tolyl-thiazole-4-carbonyl)-3-aza-bicyclo[3.1.0]hex-2-ylmethyl]-amide

prepared by reaction of [(1R*,2S*,5S*)-2-aminomethyl-3-aza-bicyclo[3.1.0]hex-3-yl]-: (2-methyl-5-m-tolyl-thiazol-4-yl)-methanone with 5-chloro-1,3-dimethyl-1H-pyrazole-4-carboxylic acid. LC-MS: t_{R} = 0.86 min; [M+H]⁺ = 484.2.

### Example 92:

### benzo[b]thiophene-2-carboxylic acid [(1R*,2S*,5S*)-3-(2-methyl-5-m-tolyl-thiazole-4-carbonyl)-3-aza-bicyclo[3.1.0]hex-2-ylmethyl]-amide

prepared by reaction of [(1R*,2S*,5S*)-2-aminomethyl-3-aza-bicyclo[3.1.0]hex-3-yl]-(2-methyl-5-m-tolyl-thiazol-4-yl)-methanone with benzo[b]thiophene-2-carboxylic acid. LC-MS: t_{R} = 0.98 min; [M+H]⁺ = 488.2.

### Example 93:

### 5-butyl-2-methyl-2H-pyrazole-3-carboxylic acid [(1R*,2S*,5S*)-3-(2-methyl-5-m-tolyl-thiazole-4-carbonyl)-3-aza-bicyclo[3.1.0]hex-2-ylmethyl]-amide

prepared by reaction of [(1R*,2S*,5S*)-2-aminomethyl-3-aza-bicyclo[3.1.0]hex-3-yl]-(2-methyl-5-m-tolyl-thiazol-4-yl)-methanone with 5*-tert-*butyl-2-methyl-2H-pyrazole-3-carboxylic acid. LC-MS: t_{R} = 0.97 min; [M+H]⁺ = 492.2.

### Example 94:

### 1-methyl-1H-indazole-3-carboxylic acid [(1R*,2S*,5S*)-3-(2-methyl-5-m-tolyl-thiazole-4-carbonyl)-3-aza-bicyclo[3.1.0]hex-2-ylmethyl]-amide

prepared by reaction of [(1R*,2S*,5S*)-2-aminomethyl-3-aza-bicyclo[3.1.0]hex-3-yl]-(2-methyl-5-m-tolyl-thiazol-4-yl)-methanone with 1-methyl-1H-indazole-3-carboxylic acid. LC-MS: t_{R} = 0.93 min; [M+H]⁺ = 486.2.

### Example 95:

### pyrazolo[1,5-a]pyrimidine-3-carboxylic acid [(1R*,2S*,5S*)-3-(2-methyl-5-m-tolyl-thiazole-4-carbonyl)-3-aza-bicyclo[3.1.0]hex-2-ylmethyl]-amide

prepared by reaction of [(1R*,2S*,5S*)-2-aminomethyl-3-aza-bicyclo[3.1.0]hex-3-yl]-(2-methyl-5-m-tolyl-thiazol-4-yl)-methanone with pyrazolo[1,5-a]pyrimidine-3-carboxylic acid. LC-MS: t_{R} = 0.81 min; [M+H]⁺ = 473.2.

### Example 96:

### quinoxaline-2-carboxylic acid [(1R*,2S*,5S*)-3-(2-methyl-5-m-tolyl-thiazole-4-carbonyl)-3-aza-bicyclo[3.1.0]hex-2-ylmethyl]-amide

prepared by reaction of [(1R*,2S*,5S*)-2-aminomethyl-3-aza-bicyclo[3.1.0]hex-3-yl]-(2-methyl-5-m-tolyl-thiazol-4-yl)-methanone with quinoxaline-2-carboxylic acid. LC-MS: t_{R} = 0.93 min; [M+H]⁺ = 484.2.

### Example 97:

### 1-methyl-1H-pyrrole-2-carboxylic acid [(1R*,2S*,5S*)-3-(2-methyl-5-m-tolyl-thiazole-4-carbonyl)-3-aza-bicyclo[3.1.0]hex-2-ylmethyl]-amide

prepared by reaction of [(1R*,2S*,5S*)-2-aminomethyl-3-aza-bicyclo[3.1.0]hex-3-yl]-(2-methyl-5-m-tolyl-thiazol-4-yl)-methanone with 1-methyl-1H-pyn-ole-2-carboxylic acid. LC-MS: t_{R} = 0.90 min; [M+H]⁺ = 435.2.

### Example 98:

### 2,8-dimethyl-imidazo[1,2-a]pyridine-3-carboxylic acid [(1R*,2S*,5S*)-3-(2-methyl-5-m-tolyl-thiazole-4-carbonyl)-3-aza-bicyclo[3.1.0]hex-2-ylmethyl]-amide

prepared by reaction of [(1R*,2S*,5S*)-2-aminomethyl-3-aza-bicyclo[3.1.0]hex-3-yl]-(2-methyl-5-m-tolyl-thiazol-4-yl)-methanone with 2,8-dimethyl-imidazo[1,2-a]pyridine-3-carboxylic acid. LC-MS: t_{R} = 0.88 min; [M+H]⁺ = 500.2.

### Example 99:

### 2,6-dimethyl-imidazo[1,2-a]pyridine-3-carboxylic acid [(1R*,2S*,5S*)-3-(2-methyl-5-m-tolyl-thiazole-4-carbonyl)-3-aza-bicyclo[3.1.0]hex-2-ylmethyl]-amide

prepared by reaction of [(1R*,2S*,5S*)-2-aminomethyl-3-aza-bicyclo[3.1.0]hex-3-yl]-(2-methyl-5-m-tolyl-thiazol-4-yl)-methanone with 2,6-dimethyl-imidazo[1,2-a]pyridine-3-carboxylic acid. LC-MS: t_{R} = 0.88 min; [M+H]⁺ = 500.2.

### Example 100:

### 6-isobutyl-5-methyl-N-[(1R*,2S*,5S*)-3-(2-methyl-5-m-tolyl-thiazole-4-carbonyl)-3-aza-bicyclo[3.1.0]hex-2-ylmethyl]-nicotinamide

prepared by reaction of [(1R*,2S*,5S*)-2-aminomethyl-3-aza-bicyclo[3.1.0]hex-3-yl]-(2-methyl-5-m-tolyl-thiazol-4-yl)-methanone with 6-isobutyl-5-methyl-nicotinic acid. LC-MS: t_{R} = 0.95 min; [M+H]⁺ = 503.3.

### Example 101:

### 2-isobutyl-6-methyl-N-[(1R*,2S*,5S*)-3-(2-methyl-5-m-tolyl-thiazole-4-carbonyl)-3-aza-bicyclo[3.1.0]hex-2-ylmethyl]-isonicotinamide

prepared by reaction of [(1R*,2S*,5S*)-2-aminomethyl-3-aza-bicyclo[3.1.0]hex-3-yl]-(2-methyl-5-m-tolyl-thiazol-4-yl)-methanone with 2-isobutyl-6-methyl-isonicotinic acid. LC-MS: t_{R} = 0.95 min; [M+H]⁺ = 503.3.

### Example 102:

### benzo[c]isoxazole-3-carboxylic acid [(1R*,2S*,5S*)-3-(2-methyl-5-m-tolyl-thiazole-4-carbonyl)-3-aza-bicyclo[3.1.0]hex-2-ylmethyl]-amide

prepared by reaction of [(1R*,2S*,5S*)-2-aminomethyl-3-aza-bicyclo[3.1.0]hex-3-yl]-(2-methyl-5-m-tolyl-thiazol-4-yl)-methanone with benzo[c]isoxazole-3-carboxylic acid. LC-MS: t_{R} = 0.94 min; [M+H]⁺ = 473.2.

### Example 103:

### pyrazolo[1,5-a]pyridine-3-carboxylic acid [(1R*,2S*,5S*)-3-(2-methyl-5-m-tolyl-thiazole-4-carbonyl)-3-aza-bicyclo[3.1.0] hex-2-ylmethyl]-amide

prepared by reaction of [(1R*,2S*,5S*)-2-aminomethyl-3-aza-bicyclo[3.1.0]hex-3-yl]-(2-methyl-5-m-tolyl-thiazol-4-yl)-methanone with pyrazolo[1,5-a]pyridine-3-carboxylic acid. LC-MS: t_{R} = 0.85 min; [M+H]⁺ = 472.2.

### Example 104:

### 4-methoxy-N-[(1R*,2S*,5S*)-3-(2-methyl-5-m-tolyl-thiazole-4-carbonyl)-3-aza-bicyclo[3.1.0]hex-2-ylmethyl]-benzamide

prepared by reaction of [(1R*,2S*,5S*)-2-aminomethyl-3-aza-bicyclo[3.1.0]hex-3-yl]-(2-methyl-5-m-tolyl-thiazol-4-yl)-methanone with 4-methoxy-benzoic acid. LC-MS: t_{R} = 0.90 min; [M+H]⁺ = 462.2.

### Examples 105 - 327:

The following examples are prepared in analogy by coupling of the respective 3-aza-bicyclo[3.1.0]hexane derivative with the respective carboxylic acid derivative.

Starting from [(1R*,2S*,5S*)-2-aminomethyl-3-aza-bicyclo[3.1.0]hex-3-yl]-(2-methyl-5-m-tolyl-thiazol-4-yl)-methanone:

| Example | Name | LC-MS | | |
|---|---|---|---|---|
| | | eluent | t_{R} [min] | [M+H]⁺ |
| 105 | Benzo[d]isoxazole-3-carboxylic acid [(1R*,2S*,5S*)-3-(2-methyl-5-m-tolyl-thiazole-4-carbonyl)-3-aza-bicyclo[3.1.0]hex-2-ylmethyl]-amide | basic | 0.94 | 473.3 |
| 106 | 6-Fluoro-4H-benzo[1,3]dioxine-8-carboxylic acid [(1R*,2S*,5S*)-3-(2-methyl-5-m-tolyl-thiazole-4-carbonyl)-3-aza-bicyclo[3.1.0]hex-2-ylmethyl]-amide | basic | 0.92 | 508.4 |
| 107 | 2,3-Dihydro-benzofuran-4-carboxylic acid [(1R*,2S*,5S*)-3-(2-methyl-5-m-tolyl-thiazole-4-carbonyl)-3-aza-bicyclo[3.1.0]hex-2-ylmethyl]-amide | basic | 0.91 | 474.4 |
| 108 | 2,2-Dimethyl-2,3-dihydro-benzofuran-7-carboxylic acid [(1R*,2S*,5S*)-3-(2-methyl-5-m-tolyl-thiazole-4-carbonyl)-3-aza-bicyclo[3.1.0]hex-2-ylmethyl]-amide | basic | 0.99 | 502.4 |
| 109 | Isoquinoline-1-carboxylic acid [(1R*,2S*,5S*)-3-(2-methyl-5-m-tolyl-thiazole-4-carbonyl)-3-aza-bicyclo[3.1.0]hex-2-ylmethyl]-amide | basic | 0.96 | 483.4 |
| 110 | Quinoline-8-carboxylic acid [(1R*,2S*,5S*)-3-(2-methyl-5-m-tolyl-thiazole-4-carbonyl)-3-aza-bicyclo[3.1.0]hex-2-ylmethyl]-amide | basic | 0.92 | 483.4 |
| 111 | Quinoline-2-carboxylic acid [(1R*,2S*,5S*)-3-(2-methyl-5-m-tolyl-thiazole-4-carbonyl)-3-aza-bicyclo[3.1.0]hex-2-ylmethyl]-amide | basic | 0.97 | 483.4 |
| 112 | Imidazo[2,1-b]thiazole-5-carboxylic acid [(1R*,2S*,5S*)-3-(2-methyl-5-m-tolyl-thiazole-4-carbonyl)-3-aza-bicyclo[3.1.0]hex-2-ylmethyl]-amide | basic | 0.81 | 478.3 |
| 113 | 3-Methyl-imidazo[2,1-b]thiazole-2-carboxylic acid [(1R*,2S*,5S***)-3-(2-methyl-5-m-tolyl-thiazole-4-carbonyl)-3-aza-bicyclo[3.1.0]hex-2-ylmethyl]-amide | basic | 0.82 | 492.3 |
| 114 | 1-Methyl-1H-indole-3-carboxylic acid [(1R*,2S*,5S*)-3-(2-methyl-5-m-tolyl-thiazole-4-carbonyl)-3-aza-bicyclo[3.1.0]hex-2-ylmethyl]-amide | basic | 0.91 | 485.4 |
| 115 | 1,2-Dimethyl-1H-indole-3-carboxylic acid [(1R*,2S*,5S*)-3-(2-methyl-5-m-tolyl-thiazole-4-carbonyl)-3-aza-bicyclo[3.1.0]hex-2-ylmethyl]-amide | basic | 0.93 | 499.4 |
| 116 | 1H-Indole-3-carboxylic acid [(1R*,2S*,5S*)-3-(2-methyl-5-m-tolyl-thiazole-4-carbonyl)-3-aza-bicyclo[3.1.0]hex-2-ylmethyl]-amide | basic | 0.86 | 471.4 |
| 117 | 1H-Indazole-3-carboxylic acid [(1R*,2S*,5S*)-3-(2-methyl-5-m-tolyl-thiazole-4-carbonyl)-3-aza-bicyclo[3.1.0]hex-2-y!methyl]-amide | basic | 0.87 | 472.4 |
| 118 | Imidazo[1,2-a]pyridine-3-carboxylic acid [(1R*,2S*,5S*)-3-(2-methyl-5-m-tolyl-thiazole-4-carbonyl)-3-aza-bicyclo[3.1.0]hex-2-ylmethyl]-amide | basic | 0.82 | 472.3 |
| 119 | 5-Fluoro-1-methyl-1H-indole-2-carboxylic acid [(1R*,2S*,5S*)-3-(2-methyl-5-m-tolyl-thiazole-4-carbonyl)-3-aza-bicyclo[3.1.0]hex-2-ylmethyl]-amide | basic | 0.99 | 503.4 |
| 120 | 2,5-Dimethyl-2H-pyrazole-3-carboxylic acid [(1R*,2S*,5S*)-3-(2-methyl-5-m-tolyl-thiazole-4-carbonyl)-3-aza-bicyclo[3.1.0]hex-2-ylmethyl]-amide | basic | 0.84 | 450.3 |
| 121 | 2-Ethyl-5-methyl-2H-pyrazole-3-carboxylic acid [(1R*,2S*,5S*)-3-(2-methyl-5-m-tolyl-thiazole-4-carbonyl)-3-aza-bicyclo[3.1.0]hex-2-ylmethyl]-amide | basic | 0.87 | 464.4 |
| 122 | 1-Ethyl-3-methyl-1H-pyrazole-4-carboxylic acid [(1R*,2S*,5S*)-3-(2-methyl-5-m-tolyl-thiazole-4-carbonyl)-3-aza-bicyclo[3.1.0]hex-2-ylmethyl]-amide | basic | 0.80 | 464.4 |
| 123 | 2,3-Dihydro-thieno[3,4-b][1,4]dioxine-5-carboxylic acid [(1R*,2S*,5S*)-3-(2-methyl-5-m-tolyl-thiazole-4-carbonyl)-3-aza-bicyclo[3.1.0]hex-2-ylmethyl]-amide | basic | 0.88 | 496.3 |
| 124 | 3-Bromo-N-[(1R*,2S*,5S*)-3-(2-methyl-5-m-tolyl-thiazole-4-carbonyl)-3-aza-bicyclo[3.1.0]hex-2-ylmethyl]-benzamide | basic | 0.97 | 510.3 |
| 125 | N-[(1R*,2S*,5S*)-3-(2-Methyl-5-m-tolyl-thiazole-4-carbonyl)-3-aza-bicyclo[3.1.0]hex-2-ylmethyl]-3-trifluoromethyl-benzamide | basic | 0.97 | 500.4 |
| 126 | 3-Methoxy-N-[(1R*,2S*,5S*)-3-(2-methyl-5-m-tolyl-thiazole-4-carbonyl)-3-aza-bicyclo[3.1.0]hex-2-ylmethyl]-benzamide | basic | 0.90 | 462.3 |
| 127 | 3-Fluoro-4-methoxy-N-[(1R*,2S*,5S*)-3-(2-methyl-5-m-tolyl-thiazole-4-carbonyl)-3-aza-bicyclo[3.1.0]hex-2-ylmethyl]-benzamide | basic | 0.90 | 480.1 |
| 128 | 3,4-Dichloro-N-[(1R*,2S*,5S*)-3-(2-methyl-5-m-tolyl-thiazole-4-carbonyl)-3-aza-bicyclo[3.1.0]hex-2-ylmethyl]-benzamide | basic | 1.01 | 500.0 |
| 129 | 2-Chloro-4,5-difluoro-N-[(1R*,2S*,5S*)-3-(2-methyl-5-m-tolyl-thiazole-4-carbonyl)-3-aza-bicyclo[3.1.0]hex-2-ylmethyl]-benzamide | basic | 0.93 | 502.0 |
| 130 | 2-Fluoro-5-methyl-N-[(1R*,2S*,5S*)-3-(2-methyl-5-m-tolyl-thiazole-4-carbonyl)-3-aza-bicyclo[3.1.0]hex-2-ylmethyl]-benzamide | basic | 0.94 | 464.1 |
| 131 | 3-Fluoro-2-methyl-N-[(1R*,2S*,5S*)-3-(2-methyl-5-m-tolyl-thiazole-4-carbonyl)-3-aza-bicyclo[3.1.0]hex-2-ylmethyl]-benzamide | basic | 0.91 | 464.1 |
| 132 | 5-Fluoro-2-methoxy-N-[(1R*,2S*,5S*)-3-(2-methyl-5-m-tolyl-thiazole-4-carbonyl)-3-azabicyclo[3.1.0]hex-2-ylmethyl]-benzamide | basic | 0.93 | 480.1 |
| 133 | 3-Chloro-2-methyl-N-[(1R*,2S*,5S*)-3-(2-methyl-5-m-tolyl-thiazole-4-carbonyl)-3-aza-bicyclo[3.1.0]hex-2-ylmethyl]-benzamide | basic | 0.95 | 480.1 |
| 134 | 2-Chloro-3-fluoro-N-[(1R*,2S*,5S*)-3-(2-methyl-5-m-tolyl-thiazole-4-carbonyl)-3-aza-bicyclo[3.1.0]hex-2-ylmethyl]-benzamide | basic | 0.91 | 484.0 |
| 135 | 2,5-Dimethyl-N-[(1R*,2S*,5S*)-3-(2-methyl-5-m-tolyl-thiazole-4-carbonyl)-3-aza-bicyclo[3.1.0]hex-2-ylmethyl]-benzamide | basic | 0.94 | 460.0 |
| 136 | 3,4-Dimethyl-N-[(1R*,2S*,5S*)-3-(2-methyl-5-m-tolyl-thiazole-4-carbonyl)-3-aza-bicyclo[3.1.0]hex-2-ylmethyl]-benzamide | basic | 0.95 | 460.0 |
| 137 | 2,5-Dimethoxy-N-[(1R*,2S*,5S*)-3-(2-methyl-5-m-tolyl-thiazole-4-carbonyl)-3-aza-bicyclo[3.1.0]hex-2-ylmethyl]-benzamide | basic | 0.91 | 492.0 |
| 138 | 2-Chloro-N-[(1R*,2S*,5S*)-3-(2-methyl-5-m-tolyl-thiazole-4-carbonyl)-3-aza-bicyclo[3.1.0]hex-2-ylmethyl]-5-trifluoromethyl-benzamide | basic | 0.97 | 534.0 |
| 139 | N-[(1R*,2S*,5S*)-3-(2-Methyl-5-m-tolyl-thiazole-4-carbonyl)-3-aza-bicyclo[3.1.0]hex-2-ylmethyl]-isophthalamic acid methyl ester | basic | 0.90 | 489.7 |
| 140 | N-[(1R*,2S*,5S*)-3-(2-Methyl-5-m-tolyl-thiazole-4-carbonyl)-3-aza-bicyclo[3.1.0]hex-2-ylmethyl]-4-trifluoromethyl-benzamide | basic | 0.97 | 500.1 |
| 141 | 2-Chloro-4-fluoro-N-[(1R*,2S*,5S*)-3-(2methyl-5-m-tolyl-thiazole-4-carbonyl)-3-aza- bicyclo[3.1.0]hex-2-ylmethyl]-benzamide | basic | 0.91 | 484.0 |
| 142 | 4-tert-Butyl-N-[(1R*,2S*,5S*)-3-(2-methyl-5-m-tolyl-thiazole-4-carbonyl)-3-azabicyclo[3.1.0]hex-2-ylmethyl]-benzamide | basic | 1.02 | 488.1 |
| 143 | 2-Chloro-3-methyl-N-[(1R*,2S*,5S*)-3-(2-methyl-5-m-tolyl-thiazole-4-carbonyl)-3-aza-bicyclo[3.1.0]hex-2-ylmethyl]-benzamide | basic | 0.92 | 480.0 |
| 144 | 3,5-Dichloro-4-hydroxy-N-[(1R*,2S*,5S*)-3-(2-methyl-5-m-tolyl-thiazole-4-carbonyl)-3-aza-bicyclo[3.1.0]hex-2-ylmethyl]-benzamide | basic | 0.58 | 516.0 |
| 145 | 2,4-Dichloro-N-[(1R*,2S*,5S*)-3-(2-methyl-5-m-tolyl-thiazole-4-carbonyl)-3-aza-bicyclo[3.1.0]hex-2-ylmethyl]-benzamide | basic | 0.95 | 500.0 |
| 146 | 4-Methyl-N-[(1R*,2S*,5S*)-3-(2-methyl-5-m-tolyl-thiazole-4-carbonyl)-3-aza-bicyclo[3.1.0]hex-2-ylmethyl]-3-trifluoromethyl-benzamide | basic | 1.00 | 514.1 |
| 147 | 4-Methoxy-2-methyl-N-[(1R*,2S*,5S*)-3-(2-methyl-5-m-tolyl-thiazole-4-carbonyl)-3-aza-bicyclo[3.1.0]hex-2-ylmethyl]-benzamide | basic | 0.90 | 476.1 |
| 148 | 4-Ethyl-N-[(1R*,2S*,5S*)-3-(2-methyl-5-mtolyl-thiazole-4-carbonyl)-3-aza- bicyclo[3.1.0]hex-2-ylmethyl]-benzamide | basic | 0.96 | 460.0 |
| 149 | 4-Methoxy-3-methyl-N-[(1R*,2S*,5S*)-3-(2-methyl-5-m-tolyl-thiazole-4-carbonyl)-3-aza-bicyclo[3.1.0]hex-2-ylmethyl]-benzamide | basic | 0.93 | 476.1 |
| 150 | 3,5-Dimethyl-N-[(1R*,2S*,5S*)-3-(2-methyl-5-m-tolyl-thiazole-4-carbonyl)-3-aza-bicyclo[3.1.0]hex-2-ylmethyl]-benzamide | basic | 0.96 | 460.0 |
| 151 | 5-Bromo-2-chloro-N-[(1R*,2S*,5S*)-3-(2-methyl-5-m-tolyl-thiazole-4-carbonyl)-3-aza-bicyclo[3.1.0]hex-2-ylmethyl]-benzamide | basic | 0.96 | 543.9 |
| 152 | 3-Cyano-N-[(1R*,2S*,5S*)-3-(2-methyl-5-m-tolyl-thiazole-4-carbonyl)-3-azabicyclo[3.1.0]hex-2-ylmethyl]-benzamide | basic | 0.88 | 456.9 |
| 153 | 4-Cyano-N-[(1R*,2S*,5S*)-3-(2-methyl-5-m-tolyl-thiazole-4-carbonyl)-3-aza-bicyclo[3.1.0]hex-2-ylmethyl]-benzamide | basic | 0.88 | 456.9 |
| 154 | N-[(1R*,2S*,5S*)-3-(2-Methyl-5-m-tolyl-thiazole-4-carbonyl)-3-aza-bicyclo[3.1.0]hex-2-ylmethyl]-2-morpholin-4-yl-benzamide | basic | 0.90 | 517.1 |
| 155 | 2-(4-Methyl-piperazin-1-yl)-N-[(1R*,2S*,5S*)-3-(2-methyl-5-m-tolyl-thiazole-4-carbonyl)-3-aza-bicyclo[3.1.0]hex-2-ylmethyl]-benzamide | basic | 0.86 | 530.1 |
| 156 | 4-Chloro-N-[(1R*,2S*,5S*)-3-(2-methyl-5-m-tolyl-thiazole-4-carbonyl)-3-aza-bicyclo[3.1.0]hex-2-ylmethyl]-benzamide | basic | 0.95 | 466.0 |
| 157 | 2,3-Dimethoxy-N-[(1R*,2S*,5S*)-3-(2-methyl-5-m-tolyl-thiazole-4-carbonyl)-3-aza-bicyclo[3.1.0]hex-2-ylmethyl]-benzamide | basic | 0.91 | 492.0 |
| 158 | 3-Iodo-N-[(1R*,2S*,5S*)-3-(2-methyl-5-m-tolyl-thiazole-4-carbonyl)-3-aza-bicyclo[3.1.0]hex-2-ylmethyl]-benzamide | basic | 0.98 | 558.1 |
| 159 | 4-Methoxy-N-[(1R*,2S*,5S*)-3-(2-methyl-5-m- tolyl-thiazole-4-carbonyl)-3-aza-bicyclo[3.1.0]hex-2-ylmethyl]-3-trifluoromethyl-benzamide | basic | 0.95 | 530.0 |
| 160 | 4-Dimethylamino-N-[(1R*,2S*,5S*)-3-(2-methyl-5-m-tolyl-thiazole-4-carbonyl)-3-aza-bicyclo[3.1.0]hex-2-ylmethyl]-benzamide | basic | 0.90 | 475.1 |
| 161 | 2-Bromo-3-methyl-N-[(1R*,2S*,5S*)-3-(2-methyl-5-m-tolyl-thiazole-4-carbonyl)-3-aza-bicyclo[3.1.0]hex-2-ylmethyl]-benzamide | basic | 0.93 | 523.9 |
| 162 | 2-Chloro-N-[(1R*,2S*,5S*)-3-(2-methyl-5-m-tolyl-thiazole-4-carbonyl)-3-azabicyclo[3.1.0]hex-2-ylmethyl]-benzamide | basic | 0.89 | 466.0 |
| 163 | 2-Bromo-5-methyl-N-[(1R*,2S*,5S*)-3-(2-methyl-5-m-tolyl-thiazole-4-carbonyl)-3-aza-bicyclo[3.1.0]hex-2-ylmethyl]-benzamide | basic | 0.94 | 523.9 |
| 164 | 3,4-Dimethoxy-N-[(1R*,2S*,5S*)-3-(2-methyl-5-m-tolyl-thiazole-4-carbonyl)-3-aza-bicyclo[3.1.0]hex-2-ylmethyl]-benzamide | basic | 0.85 | 492.0 |
| 165 | 5-Methyl-imidazo[2,1-b]thiazole-6-carboxylic acid [(1R*,2S*,5S*)-3-(2-methyl-5-m-tolyl-thiazole-4-carbonyl)-3-aza-bicyclo[3.1.0]hex-2-ylmethyl]-amide | basic | 0.85 | 492.0 |
| 166 | 3,5-Dimethyl-imidazo[2,1-b]thiazole-6-carboxylic acid [(1R*,2S*,5S*)-3-(2-methyl-5-m-tolyl-thiazole-4-carbonyl)-3-aza-bicyclo[3.1.0]hex-2-ylmethyl]-amide | basic | 0.89 | 506.0 |
| 167 | 2,6-Dimethyl-imidazo[2,1-b]thiazole-5-carboxylic acid [(1R*,2S*,5S*)-3-(2-methyl-5-m-tolyl-thiazole-4-carbonyl)-3-aza-bicyclo[3.1.0]hex-2-ylmethyl]-amide | basic | 0.89 | 506.1 |
| 168 | 2,3,5-Trimethyl-imidazo[2,1-b]thiazole-6-carboxylic acid [(1R*,2S*,5S*)-3-(2-methyl-5-m-tolyl-thiazole-4-carbonyl)-3-aza-bicyclo[3.1.0]hex-2-ylmethyl]-amide | basic | 0.94 | 520.1 |
| 169 | 2-Methyl-imidazo[2,1-b]thiazole-5-carboxylic acid [(1R*,2S*,5S*)-3-(2-methyl-5-m-tolyl-thiazole-4-carbonyl)-3-aza-bicyclo[3.1.0]hex-2-ylmethyl]-amide | basic | 0.85 | 492.0 |
| 170 | 6-Trifluoromethyl-imidazo[2,1-b]thiazole-5-carboxylic acid [(1R*,2S*,5S*)-3-(2-methyl-5-m-tolyl-thiazole-4-carbonyl)-3-aza-bicyclo[3.1.0]hex-2-ylmethyl]-amide | basic | 0.94 | 546.0 |
| 171 | 3,6-Dimethyl-imidazo[2,1-b]thiazole-5-carboxylic acid [(1R*,2S*,5S*)-3-(2-methyl-5- m-tolyl-thiazole-4-carbonyl)-3-aza-bicyclo[3.1.0]hex-2-ylmethyl]-amide | basic | 0.82 | 506.0 |
| 172 | 3-Methyl-imidazo[2,1-b]thiazole-5-carboxylic acid [(1R*,2S*,5S*)-3-(2-methyl-5-m-tolyl- thiazole-4-carbonyl)-3-aza-bicyclo[3.1.0]hex-2-ylmethyl]-amide | basic | 0.82 | 492.0 |
| 173 | 6-Chloro-imidazo[2,1-b]thiazole-5-carboxylic acid [(1R*,2S*,5S*)-3-(2-methyl-5-m-tolyl-thiazole-4-carbonyl)-3-aza-bicyclo[3.1.0]hex-2-ylmethyl]-amide | basic | 0.93 | 512.0 |
| 174 | 2,3-Dimethyl-imidazo[2,1-b]thiazole-5- carboxylic acid [(1R*,2S*,5S*)-3-(2-methyl-5-m-tolyl-thiazole-4-carbonyl)-3-aza- bicyclo[3.1.0]hex-2-ylmethyl]-amide | basic | 0.86 | 506.0 |
| 175 | 3-Methyl-imidazo[2,1-b]thiazole-6-carboxylic acid [(1R*,2S*,5S*)-3-(2-methyl-5-m-tolyl-thiazole-4-carbonyl)-3-aza-bicyclo[3.1.0]hex-2-ylmethyl]-amide | basic | 0.85 | 492.0 |
| 176 | 2,5-Dimethyl-imidazo[2,1-b]thiazole-6-carboxylic acid [(1R*,2S*,5S*)-3-(2-methyl-5-m-tolyl-thiazole-4-carbonyl)-3-aza-bicyclo[3.1.0]hex-2-ylmethyl]-amide | basic | 0.90 | 506.1 |
| 177 | 3-Oxo-3,4-dihydro-2H-benzo[1,4]oxazine-8- carboxylic acid [(1R*,2S*,5S*)-3-(2-methyl-5-m-tolyl-thiazole-4-carbonyl)-3-aza-bicyclo[3.1.0]hex-2-ylmethyl]-amide | basic | 0.78 | 503.1 |
| 178 | 2H-Chromene-5-carboxylic acid [(1R*,2S*,5S*)-3-(2-methyl-5-m-tolyl-thiazole-4-carbonyl)-3-aza-bicyclo[3.1.0]hex-2-ylmethyl]-amide | basic | 0.91 | 486.1 |
| 179 | 4-Methyl-3,4-dihydro-2H-benzo[1,4]oxazine-8-carboxylic acid [(1R*,2S*,5S*)-3-(2-methyl-5-m-tolyl-thiazole-4-carbonyl)-3-aza-bicyclo[3.1.0]hex-2-ylmethyl]-amide | basic | 0.91 | 503.1 |
| 180 | Chroman-8-carboxylic acid [(1R*,2S*,5S*)-3-(2-methyl-5-m-tolyl-thiazole-4-carbonyl)-3-aza-bicyclo[3.1.0]hex-2-ylmethyl]-amide | basic | 0.94 | 488.1 |
| 181 | 3-Oxo-3,4-dihydro-2H-benzo[1,4]oxazine-5-carboxylic acid [(1R*,2S*,5S*)-3-(2-methyl-5-m-tolyl-thiazole-4-carbonyl)-3-aza-bicyclo[3.1.0]hex-2-ylmethyl]-amide | basic | 0.89 | 503.1 |
| 182 | Chroman-5-carboxylic acid [(1R*,2S*,5S*)-3-(2-methyl-5-m-tolyl-thiazole-4-carbonyl)-3-aza-bicyclo[3.1.0]hex-2-ylmethyl]-amide | basic | 0.90 | 488.1 |
| 183 | 3,4-Dihydro-2H-benzo[1,4]oxazine-8-carboxylic acid [(1R*,2S*,5S*)-3-(2-methyl-5-m-tolyl-thiazole-4-carbonyl)-3-aza-bicyclo[3.1.0]hex-2-ylmethyl]-amide | basic | 0.84 | 488.9 |
| 184 | 3,4-Dihydro-2H-benzo[1,4]oxazine-5- carboxylic acid [(1R*,2S*,5S*)-3-(2-methyl-5-m-tolyl-thiazole-4-carbonyl)-3-aza-bicyclo[3.1.0]hex-2-ylmethyl]-amide | basic | 0.94 | 488.9 |
| 185 | 4-Methyl-3-oxo-3,4-dihydro-2H- benzo[1,4]oxazine-8-carboxylic acid [(1R*,2S*,5S*)-3-(2-methyl-5-m-tolyl-thiazole- 4-carbonyl)-3-aza-bicyclo[3.1.0]hex-2- ylmethyl]-amide | basic | 0.84 | 517.1 |
| 186 | 4-Methyl-3,4-dihydro-2H-benzo[1,4]oxazine-5- carboxylic acid [(1R*,2S*,5S*)-3-(2-methyl-5- m-tolyl-thiazole-4-carbonyl)-3-aza- bicyclo[3.1.0]hex-2-ylmethyl]-amide | basic | 0.90 | 503.1 |
| 187 | 2,3-Dimethyl-benzofuran-4-carboxylic acid [(1R*,2S*,5S*)-3-(2-methyl-5-m-tolyl-thiazole- 4-carbonyl)-3-aza-bicyclo[3.1.0]hex-2- ylmethyl]-amide | acidic | 1.05 | 500.2 |
| 188 | 2-Methyl-benzofuran-4-carboxylic acid [(1R* ,2S* ,5S*)-3-(2-methyl-5-m-tolyl-thiazole-4-carbonyl)-3-aza-bicyclo[3.1.0]hex-2-ylmethyl]-amide | acidic | 1.05 | 486.2 |

Starting from [(1R*,2S*,5S*)-(2-aminomethyl-3-aza-bicyclo[3.1.0]hex-3-yl)]-[5-(3-chloro-phenyl)-2-methyl-thiazol-4-yl]-methanone:

| Example | Name | LC-MS | | |
|---|---|---|---|---|
| | | eluent | t_{R} [min] | [M+H]⁺ |
| 189 | Benzo[d]isoxazole-3-carboxylic acid {(1R*,2S*,5S*)-3-[5-(3-chloro-phenyl)-2- methyl-thiazole-4-carbonyl]-3-aza-bicyclo[3.1.0]hex-2-ylmethyl}-amide | basic | 0.95 | 493.3 |
| 190 | 2,3-Dihydro-benzo[1,4]dioxine-5-carboxylic acid {(1R*,2S*,5S*)-3-[5-(3-chloro-phenyl)-2-methyl-thiazole-4-carbonyl]-3-aza-bicyclo[3.1.0]hex-2-ylmethyl}-amide | basic | 0.91 | 510.3 |
| 191 | 6-Fluoro-4H-benzo[1,3]dioxine-8-carboxylic acid {(1R*,2S*,5S*)-3-[5-(3-chloro-phenyl)-2-methyl-thiazole-4-carbonyl]-3-aza-bicyclo[3.1.0]hex-2-ylmethyl}-amide | basic | 0.93 | 528.3 |
| 192 | 2,3-Dihydro-benzofuran-4-carboxylic acid {(1R*,2S* ,5S*)-3-[5-(3-chloro-phenyl)-2-methyl-thiazole-4-carbonyl]-3-aza-bicyclo[3.1.0]hex-2-ylmethyl}-amide | basic | 0.92 | 494.3 |
| 193 | 2,2-Dimethyl-2,3-dihydro-benzofuran-7-carboxylic acid {(1R*,2S*,5S*)-3-[5-(3-chloro-phenyl)-2-methyl-thiazole-4-carbonyl]-3-azabicyclo[3.1.0]hex-2-ylmethyl -amide | basic | 1.01 | 522.0 |
| 194 | Isoquinoline-1-carboxylic acid {(1R*,2S*,5S*)-3-[5-(3-chloro-phenyl)-2-methyl-thiazole-4-carbonyl]-3-aza-bicyclo[3.1.0]hex-2-ylmethyl}-amide | basic | 0.97 | 503.3 |
| 195 | Quinoline-8-carboxylic acid {(1R*,2S*,5S*)-3-[5-(3-chloro-phenyl)-2-methyl-thiazole-4-carbonyl]-3-aza-bicyclo[3.1.0]hex-2-ylmethyl}amide | basic | 0.94 | 503.3 |
| 196 | Quinoline-2-carboxylic acid {(1R*,2S*,5S*)-3-[5-(3-chloro-phenyl)-2-methyl-thiazole-4-carbonyl]-3-aza-bicyclo[3.1.0]hex-2-ylmethyl}-amide | basic | 0.98 | 503.3 |
| 197 | Imidazo[2,1-b]thiazole-5-carboxylic acid {(1R*,2S*,5S*)-3-[5-(3-chloro-phenyl)-2-methyl-thiazole-4-carbonyl]-3-aza-bicyclo[3.1.0]hex-2-ylmethyl} -amide | basic | 0.83 | 498.3 |
| 198 | 3-Methyl-imidazo[2,1-b]thiazole-2-carboxylic acid {(1R*,2S*,5S*)-3-[5-(3-chloro-phenyl)-2-methyl-thiazole-4-carbonyl]-3-aza-bicyclo[3.1.0]hex-2-ylmethyl}-amide | basic | 0.84 | 512.3 |
| 199 | 1-Methyl-1H-indole-3-carboxylic acid {(1R*,2S*,5S*)-3-[5-(3-chloro-phenyl)-2-methyl-thiazole-4-carbonyl]-3-aza-bicyclo[3.1.0]hex-2-ylmethyl }-amide | basic | 0.92 | 505.3 |
| 200 | 1,2-Dimethyl-1H-indole-3-carboxylic acid {(1R*,2S*,5S*)-3-[5-(3-chloro-phenyl)-2-methyl-thiazole-4-carbonyl]-3-aza-bicyclo[3.1.0]hex-2-ylmethyl -amide | basic | 0.95 | 519.4 |
| 201 | 1H-Indole-3-carboxylic acid {(1R*,2S*,5S*)-3- [5-(3-chloro-phenyl)-2-methyl-thiazole-4-carbonyl]-3-aza-bicyclo[3.1.0]hex-2-ylmethyl}-amide | basic | 0.87 | 491.2 |
| 202 | 1H-Indazole-3-carboxylic acid {(1R*,2S*,5S*)-3-[5-(3-chloro-phenyl)-2-methyl-thiazole-4-carbonyl]-3-aza-bicyclo[3.1.0]hex-2-ylmethyl}-amide | basic | 0.88 | 492.3 |
| 203 | Imidazo[1,2-a]pyridine-3-carboxylic acid {(1R*,2S*,5S*)-3-[5-(3-chloro-phenyl)-2- methyl-thiazole-4-carbonyl]-3-aza-bicyclo[3.1.0]hex-2-ylmethyl -amide | basic | 0.83 | 492.3 |
| 204 | 5-Fluoro-1-methyl-1H-indole-2-carboxylic acid {(1R*,2S*,5S*)-3-[5-(3-chloro-phenyl)-2- methyl-thiazole-4-carbonyl]-3-aza-bicyclo[3.1.0]hex-2-ylmethyl}-amide | basic | 1.00 | 523.2 |
| 205 | 2,5-Dimethyl-2H-pyrazole-3-carboxylic acid {(1R*,2S*,5S*)-3-[5-(3-chloro-phenyl)-2-methyl-thiazole-4-carbonyl]-3-aza-bicyclo[3.1.0]hex-2-ylmethyl}-amide | basic | 0.85 | 470.3 |
| 206 | 2-Ethyl-5-methyl-2H-pyrazole-3-carboxylic acid {(1R*,2S*,5S*)-3-[5-(3-chloro-phenyl)-2-methyl-thiazole-4-carbonyl]-3-aza-bicyclo[3.1.0]hex-2-ylmethyl}-amide | basic | 0.89 | 484.3 |
| 207 | 1-Ethyl-3-methyl-1H-pyrazole-4-carboxylic acid {(1R*,2S*,5S*)-3-[5-(3-chloro-phenyl)-2-methyl-thiazole-4-carbonyl]-3-aza-bicyclo[3.1.0]hex-2-ylmethyl}-amide | basic | 0.81 | 484.3 |
| 208 | 2,3-Dihydro-thieno[3,4-b][1,4]dioxine-5-carboxylic acid {(1R*,2S*,5S*)-3-[5-(3-chloro-phenyl)-2-methyl-thiazole-4-carbonyl]-3-aza-bicyclo[3.1.0]hex-2-ylmethyl}-amide | basic | 0.89 | 516.3 |
| 209 | 3-Bromo-N-{(1R*,2S*,5S*)-3-[5-(3-chloro-phenyl)-2-methyl-thiazole-4-carbonyl]-3-azabicyclo[3.1.0]hex-2-ylmethyl}-benzamide | basic | 0.98 | 530.2 |
| 210 | N-{(1R*,2S*,5S*)-3-[5-(3-Chloro-phenyl)-2- methyl-thiazolc-4-carbonyl]-3-aza- bicyclo[3.1.0]hex-2-ylmethyl}-3-trifluoromethyl-bcnzamide | basic | 0.98 | 520.3 |
| 211 | N-{(1R*,2S*,5S*)-3-[5-(3-Chloro-phenyl)-2-methyl-thiazolc-4-carbonyl]-3-aza-bicyclo[3.1.0]hex-2-ylmethyl} -3-methoxy-benzamide | basic | 0.91 | - 482.3 |

Starting from [(1R*,2S*,5S*)-(2-aminomethyl-3-aza-bicyclo[3.1.0]hex-3-yl)]-[5-(3-fluoro-phenyl)-2-methyl-thiazol-4-yl]-methanone:

| Example | Name | LC-MS | | |
|---|---|---|---|---|
| | | eluent | t_{R} [min] | [M+H]⁺ |
| 212 | 3-Methyl-imidazo[2,1-b]thiazole-5-carboxylic acid {(1R*,2S*,5S*)-3-[5-(3-fluoro-phenyl)-2-methyl-thiazole-4-carbonyl]-3-aza-bicyclo[3.1.0]hex-2-ylmethyl}-amide | basic | 0.83 | 496.1 |
| 213 | 2-Methyl-imidazo[2,1-b]thiazole-5-carboxylic acid {(1R*,2S*,5S*)-3-[5-(3-fluoro-phenyl)-2-methyl-thiazole-4-carbonyl]-3-aza-bicyclo[3.1.0]hex-2-ylmethyl}-amide | basic | 0.85 | 496.1 |
| 214 | Imidazo[2,1-b]thiazole-5-carboxylic acid {(1R*,2S*,5S*)-3-[5-(3-fluoro-phenyl)-2-methyl-thiazole-4-carbonyl]-3-aza-bicyclo[3.1.0]hex-2-ylmethyl}-amide | basic | 0.81 | 482.1 |
| 215 | 1-Methyl-1H-indole-3-carboxylic acid {(1R*,2S*,5S*)-3-[5-(3-fluoro-phenyl)-2-methyl-thiazole-4-carbonyl]-3-aza-bicyclo[3.1.0]hex-2-ylmethyl}-amide | basic | 0.90 | 489.0 |
| 216 | 3-Methyl-imidazo[2,1-b]thiazole-2-carboxylic acid {(1R*,2S*,5S*)-3-[5-(3-fluoro-phenyl)-2-methyl-thiazole-4-carbonyl]-3-aza-bicyclo[3.1.0]hex-2-ylmethyl}-amide | basic | 0.82 | 496.1 |
| 217 | 1-Ethyl-3-methyl-1H-pyrazole-4-carboxylic acid {(1R*,2S*,5S*)-3-[5-(3-fluoro-phenyl)-2-methyl-thiazole-4-carbonyl]-3-aza-bicyclo[3.1.0]hex-2-ylmethyl}-amide | basic | 0.80 | 468.2 |
| 218 | 5-tert-Butyl-2-methyl-2H-pyrazole-3-carboxylic acid {(1R*,2S*,5S*)-3-[5-(3-fluoro-phenyl)-2-methyl-thiazole-4-carbonyl]-3-aza-bicyclo[3.1.0]hex-2-ylmethy}-amide | basic | 0.95 | 496.2 |
| 219 | Benzothiazole-6-carboxylic acid {(1R*,2S*,5S*)-3-[5-(3-fluoro-phenyl)-2-methyl-thiazole-4-carbonyl]-3-aza-bicyclo[3.1.0]hex-2-ylmethyl -amide | basic | 0.85 | 493.1 |
| 220 | Quinoline-4-carboxylic acid {(1R*,2S*,5S*)-3-[5-(3-fluoro-phenyl)-2-methyl-thiazole-4-carbonyl]-3-aza-bicyclo[3.1.0]hex-2-ylmethyl}-amide | basic | 0.83 | 487.1 |
| 221 | N-{(1R*,2S*,5S*)-3-[5-(3-Fluoro-phenyl)-2-methyl-thiazole-4-carbonyl]-3-aza-bicyclo[3.1.0]hex-2-ylmethyl}-5-methyl-nicotinamide | basic | 0.81 | 451.1 |
| 222 | Isoquinoline-1-carboxylic acid {(1R*,2S*,5S*)-3-[5-(3-fluoro-phenyl)-2-methyl-thiazole-4-carbonyl]-3-aza-bicyclo[3.1.0]hex-2-ylmethyl}-amide | basic | 0.94 | 487.1 |
| 223 | Isoquinoline-3-carboxylic acid {(1R*,2S*,5S*)-3-[5-(3-fluoro-phenyl)-2-methyl-thiazole-4-carbonyl]-3-aza-bicyclo[3.1.0]hex-2-ylmethyl}-amide | basic | 0.93 | 487.1 |
| 224 | 4-Hydroxy-quinoline-2-carboxylic acid {(1R*,2S*,5S*)-3-[5-(3-fluoro-phenyl)-2-methyl-thiazole-4-carbonyl]-3-aza-bicyclo[3.1.0]hex-2-ylmethyl -amide | basic | 0.63 | 503. 1 |
| 225 | Quinoline-3-carboxylic acid {(1R*,2S*,5S*)-3-[5-(3-fluoro-phenyl)-2-methyl-thiazole-4-carbonyl]-3-aza-bicyclo[3.1.0]hex-2-ylmethyl }-amide | basic | 0.85 | 487.1 |
| 226 | Quinoline-5-carboxylic acid {(1R*,2S*,5S*)-3-[5-(3-fluoro-phenyl)-2-methyl-thiazole-4-carbonyl]-3-aza-bicyclo[3.1.0]hex-2-ylmethyl}-amide | basic | 0.82 | 487.1 |
| 227 | 1H-Indazole-3-carboxylic acid {(1R*,2S*,5S*)-3-[5-(3-fluoro-phenyl)-2-methyl-thiazole-4-carbonyl]-3-aza-bicyclo[3.1.0]hex-2-ylmethyl}-amide | basic | 0.86 | 476.1 |
| 228 | 4-Methoxy-quinoline-2-carboxylic acid {(1R*,2S*,5S*)-3-[5-(3-fluoro-phenyl)-2-methyl-thiazole-4-carbonyl]-3-aza-bicyclo[3.1.0]hex-2-ylmethyl}-amide | basic | 0.99 | 517.1 |
| 229 | Quinoline-2-carboxylic acid {(1R*,2S*,5S*)-3-[5-(3-fluoro-phenyl)-2-methyl-thiazole-4-carbonyl]-3-aza-bicyclo[3.1.0]hex-2-ylmethyl}-amide | basic | 0.96 | 487.2 |
| 230 | 5-Methoxy-1H-indole-2-carboxylic acid {(1R*,2S*,5S*)-3-[5-(3-fluoro-phenyl)-2-methyl-thiazole-4-carbonyl]-3-aza-bicyclo[3.1.0]hex-2-ylmethyl}-amide | basic | 0.90 | 505.2 |
| 231 | 1H-Indole-4-carboxylic acid {(1R*,2S*,5S*)-3-[5-(3-fluoro-phenyl)-2-methyl-thiazole-4-carbonyl]-3-aza-bicyclo[3.1.0]hex-2-ylmethyl}-amide | basic | 0.85 | 475.1 |
| 232 | Quinoline-6-carboxylic acid {(1R*,2S*,5S*)-3- [5-(3-fluoro-phenyl)-2-methyl-thiazole-4-carbonyl]-3-aza-bicyclo[3.1.0]hex-2-ylmethyl}-amide | basic | 0.82 | 487.1 |
| 233 | 1H-Indole-3-carboxylic acid {(1R*,2S*,5S*)-3-[5-(3-fluoro-phenyl)-2-methyl-thiazole-4-carbonyl]-3-aza-bicyclo[3.1.0]hex-2-ylmethyl}-amide | basic | 0.85 | 475. |
| 234 | 5,7-Dimethyl-pyrazolo[1,5-a]pyrimidine-3-carboxylic acid {(1R*,2S*,5S*)-3-[5-(3-fluoro-phenyl)-2-methyl-thiazole-4-carbonyl]-3-aza-bicyclo[3.1.0]hex-2-ylmethyl}-amide | basic | 0.85 | 505.2 |
| 235 | N-{(1R*,2S*,5S*)-3-[5-(3-Fluoro-phenyl)-2-methyl-thiazole-4-carbonyl]-3-aza-bicyclo[3.1.0]hex-2-ylmethyl}-2,3-dimethoxy-benzamide | basic | 0.90 | 496.2 |
| 236 | 6-Fluoro-4H-benzo[1,3]dioxine-8-carboxylic acid {(1R*,2S*,5S*)-3-[5-(3-fluoro-phenyl)-2-methyl-thiazole-4-carbonyl]-3-aza-bicyclo[3.1.0]hex-2-ylmethyl} -amide | basic | 0.91 | 512.1 |
| 237 | Isoquinoline-5-carboxylic acid {(1R*,2S*,5S*)-3-[5-(3-fluoro-phenyl)-2-methyl-thiazole-4-carbonyl]-3-aza-bicyclo[3.1.0]hex-2-ylmethyl}-amide | basic | 0.81 | 487.2 |
| 238 | Benzo[1,2,3]thiadiazole-5-carboxylic acid {(1R*,2S*,5S*)-3-[5-(3-fluoro-phenyl)-2-methyl-thiazole-4-carbonyl]-3-aza-bicyclo[3.1.0]hex-2-ylmethyl}-amide | basic | 0.89 | 494.1 |
| 239 | Benzo[d]isoxazole-3-carboxylic acid {(1R*,2S*,5S*)-3-[5-(3-fluoro-phenyl)-2-methyl-thiazole-4-carbonyl]-3-aza-bicyclo[3.1.0]hex-2-ylmethyl}-amide | basic | 0.92 | 477.1 |
| 240 | 2,2-Dimethyl-2,3-dihydro-benzofuran-7-carboxylic acid {(1R*,2S*,5S*)-3-[5-(3-fluoro-phenyl)-2-methyl-thiazole-4-carbonyl]-3-aza-bicyclo[3.1.0]hex-2-ylmethyl}-amide | basic | 0.98 | 506.1 |
| 241 | 2,2-Difluoro-benzo[1,3]dioxole-4-carboxylic acid {(1R*,2S*,5S*)-3-[5-(3-fluoro-phenyl)-2-methyl-thiazole-4-carbonyl]-3-aza-bicyclo[3.1.0]hex-2-ylmethyl}-amide | basic | 0.97 | 516.1 |
| 242 | Benzo[1,3]dioxole-4-carboxylic acid {(1R*,2S*,5S*)-3-[5-(3-fluoro-phenyl)-2-methyl-thiazole-4-carbonyl]-3-aza-bicyclo[3.1.0]hex-2-ylmethyl} -amide | basic | 0.89 | 480.1 |
| 243 | 1-Methyl-1H-indole-5-carboxylic acid {(1R*,2S*,5S*)-3-[5-(3-fluoro-phenyl)-2-methyl-thiazole-4-carbonyl]-3-aza-bicyclo[3.1.0]hex-2-ylmethyl}-amide | basic | 0.89 | 489.0 |
| 244 | 2-Ethyl-5-methyl-2H-pyrazole-3-carboxylic acid {(1R*,2S*,5S*)-3-[5-(3-fluoro-phenyl)-2-methyl-thiazole-4-carbonyl]-3-aza-bicyclo[3.1.0]hex-2-ylmethyl -amide | basic | 0.87 | 468.2 |
| 245 | Imidazo[1,2-a]pyridine-3-carboxylic acid {(1R*,2S*,5S*)-3-[5-(3-fluoro-phenyl)-2-methyl-thiazole-4-carbonyl]-3-aza-bicyclo[3.1.0]hex-2-ylmethyl}-amide | basic | 0.82 | 476.1 |
| 246 | 2-Methyl-2H-indazole-3-carboxylic acid {(1R*,2S*,5S*)-3-[5-(3-fluoro-phenyl)-2-methyl-thiazole-4-carbonyl]-3-aza-bicyclo[3.1.0]hex-2-ylmethyl}-amide | basic | 0.90 | 489.7 |
| 247 | 1-Methyl-5-trifluoromethyl-1H-pyrazole-4-carboxylic acid {(1R*,2S*,5S*)-3-[5-(3-fluoro-phenyl)-2-methyl-thiazole-4-carbonyl]-3-aza-bicyclo[3.1.0]hex-2-ylmethyl}-amide | basic | 0.86 | 508.1 |
| 248 | 1,3,5-Trimethyl-1H-pyrazole-4-carboxylic acid {(1R*,2S*,5S*)-3-[5-(3-fluoro-phenyl)-2-methyl-thiazole-4-carbonyl]-3-aza-bicyclo[3.1.0]hex-2-ylmethyl}-amide | basic | 0.79 | 468.2 |
| 249 | Imidazo[1,2-a]pyridine-6-carboxylic acid {(1R*,2S*,5S*)-3-[5-(3-fluoro-phenyl)-2-methyl-thiazole-4-carbonyl]-3-aza-bicyclo[3.1.0]hex-2-ylmethyl}-amide | basic | 0.77 | 476.3 |
| 250 | 2-tert-Butyl-5-methyl-2H-pyrazole-3-carboxylic acid {(1R*,2S*,5S*)-3-[5-(3-fluoro-phenyl)-2-methyl-thiazole-4-carbonyl]-3-aza-bicyclo[3.1.0]hex-2-ylmethyl}-amid | basic | 0.92 | 496.2 |
| 251 | 1,5-Dimethyl-1H-pyrazole-3-carboxylic acid {(1R*,2S*,5S*)-3-[5-(3-fluoro-phenyl)-2-methyl-thiazole-4-carbonyl]-3-aza-bicyclo[3.1.0]hex-2-ylmethyl}-amide | basic | 0.82 | 454.1 |
| 252 | 2,5-Dimethyl-2H-pyrazole-3-carboxylic acid {(1R*,2S*,5S*)-3-[5-(3-fluoro-phenyl)-2-methyl-thiazole-4-carbonyl]-3-aza-bicyclo[3.1.0]hex-2-ylmethyl}-amide | basic | 0.83 | 454.1 |
| 253 | 2,5-Dimethyl-oxazole-4-carboxylic acid {(1R*,2S*,5S*)-3-[5-(3-fluoro-phenyl)-2-methyl-thiazole-4-carbonyl]-3-aza-bicyclo[3.1.0]hex-2-ylmethyl}-amide | basic | 0.87 | 455.1 |
| 254 | 4-Methyl-thiazole-5-carboxylic acid {(1R*,2S*,5S*)-3-[5-(3-fluoro-phenyl)-2-methyl-thiazole-4-carbonyl]-3-aza-bicyclo[3.1.0]hex-2-ylmethyl}-amide | basic | 0.82 | 456.9 |
| 255 | 3,5-Dimethyl-isoxazole-4-carboxylic acid {(1R*,2S±,5S*)-3-[5-(3-fluoro-phenyl)-2-methyl-thiazole-4-carbonyl]-3-aza-bicyclo[3.1.0]hex-2-ylmethyl}-amide | basic | 0.85 | 455.1 |
| 256 | 2,3-Dihydro-benzo[1,4]dioxine-5-carboxylic acid {(1R*,2S*,5S*)-3-[5-(3-fluoro-phenyl)-2-methyl-thiazole-4-carbonyl]-3-aza-bicyclo[3.1.0]hex-2-ylmethyl}-amide | basic | 0.89 | 494.2 |
| 257 | 2,3-Dihydro-benzofuran-4-carboxylic acid {(1R*,2S*,5S*)-3-[5-(3-fluoro-phenyl)-2-methyl-thiazole-4-carbonyl]-3-aza-bicyclo[3.1.0]hex-2-ylmethyl}-amide | basic | 0.90 | 478.1 |
| 258 | 5-Fluoro-1H-indole-2-carboxylic acid {(1R*,2S*,5S*)-3-[5-(3-fluoro-phenyt)-2-methyl-thiazole-4-carbonyl]-3-aza-bicyclo[3.1.0]hex-2-ylmethyl}-amide | basic | 0.92 | 493.1 |
| 259 | 1,3-Dimethyl-1H-pyrazole-4-carboxylic acid {(1R*,2S*,5S*)-3-[5-(3-fluoro-phenyl)-2-methyl-thiazole-4-carbonyl]-3-aza-bicyclo[3.1.0]hex-2-ylmethyl}-amide | basic | 0.77 | 454.1 |
| 260 | 7-Fluoro-1H-indole-2-carboxylic acid {(1R*,2S*,5S*)-3-[5-(3-fluoro-phenyl)-2-methyl-thiazole-4-carbonyl]-3-aza-bicyclo[3.1.0]hex-2-ylmethyl}-amide | basic | 0.93 | 493.1 |
| 261 | 2-Trifluoromethyl-1H-benzoimidazole-5-carboxylic acid {(1R*,2S*,5S*)-3-[5-(3-fluoro-phenyl)-2-methyl-thiazole-4-carbonyl]-3-aza-bicyclo[3.1.0]hex-2-ylmethyl}-amide | basic | 0.61 | 544.0 |
| 262 | 5-Trifluoromethoxy-1H-indole-2-carboxylic acid {(1R*,2S*,5S*)-3-[5-(3-fluoro-phenyl)-2-methyl-thiazole-4-carbonyl]-3-aza-bicyclo[3.1.0]hex-2-ylmethyl}-amide | basic | 0.98 | 559.1 |
| 263 | 3-Bromo-N-{(1R*,2S*,5S*)-3-[5-(3-fluoro-phenyl)-2-methyl-thiazole-4-carbonyl]-3-aza-bicyclo[3.1.0]hex-2-ylmethyl}-benzamide | basic | 0.94 | 514.2 |
| 264 | N-{(1R*,2S*,5S*)-3-[5-(3-Fluoro-phenyl)-2-methyl-thiazole-4-carbonyl]-3-aza-bicyclo[3.1.0]hex-2-ylmethyl}-3-trifluoromethyl-benzamide | basic | 0.94 | 504.2 |

Starting from [(1R*,2S*,5S*)-(2-aminomethyl-3-aza-bicyclo[3.1.0]hex-3-yl)]-[5-(3-fluoro-phenyl)-2-methyl-thiazol-4-yl]-methanone:

| Example | Name | LC-MS | | |
|---|---|---|---|---|
| | | eluent | t_{R} [min] | [M+H]⁺ |
| 265 | Benzo[d]isoxazole-3-carboxylic acid [(1R*,2S*,5S*)-3-(2-amino-5-m-tolyl-thiazole-4-carbonyl)-3-aza-bicyclo[3.1.0]hex-2-ylmethyl]-amide | basic | 0.84 | 474.3 |
| 266 | 2,3-Dihydro-benzo[1,4]dioxine-5-carboxylic acid [(1R*,2S*,5S*)-3-(2-amino-5-m-tolyl-thiazole-4-carbonyl)-3-aza-bicyclo[3.1.0]hex-2-ylmethyl]-amide | basic | 0.81 | 491.2 |
| 267 | 6-Fluoro-4H-benzo[1,3]dioxine-8-carboxylic acid [(1R*,2S*,5S*)-3-(2-amino-5-m-tolyl-thiazole-4-carbonyl)-3-aza-bicyclo[3.1.0]hex-2-ylmethyl]-amide | basic | 0.83 | 509.4 |
| 268 | 2,3-Dihydro-benzofuran-4-carboxylic acid [(1R*,2S*,5S*)-3-(2-amino-5-m-tolyl-thiazole-4-carbonyl)-3-aza-bicyclo[3.1.0]hex-2-ylmethyl]-amide | basic | 0.81 | 475.4 |
| 269 | 2,2-Dimethyl-2,3-dihydro-benzofuran-7-carboxylic acid [(1R*,2S*,5S*)-3-(2-amino-5-m-tolyl-thiazole-4-carbonyl)-3-aza-bicyclo[3.1.0]hex-2-ylmethyl]-amide | basic | 0.89 | 503.4 |
| 270 | Isoquinoline-1-carboxylic acid [(1R*,2S*,5S*)-3-(2-amino-5-m-tolyl-thiazole-4-carbonyl)-3-aza-bicyclo[3.1.0]hex-2-ylmethyl]-amide | basic | 0.85 | 484.4 |
| 271 | Quinoline-8-carboxylic acid [(1R*,2S*,5S*)-3-(2-amino-5-m-tolyl-thiazole-4-carbonyl)-3-aza-bicyclo[3.1.0]hex-2-ylmethyl]-amide | basic | 0.83 | 484.4 |
| 272 | Quinoline-2-carboxylic acid [(1R*,2S*,5S*)-3-(2-amino-5-m-tolyl-thiazole-4-carbonyl)-3-aza-bicyclo[3.1.0]hex-2-ylmethyl]-amide | basic | 0.87 | 484.4 |
| 273 | Imidazo[2,1-b]thiazole-5-carboxylic acid [(1R*,2S*,5S*)-3-(2-amino-5-m-tolyl-thiazole-4-carbonyl)-3-aza-bicyclo[3.1.0]hex-2-ylmethyl]-amide | basic | 0.74 | 479.3 |
| 274 | 3-Methyl-imidazo[2,1-b]thiazole-2-carboxylic acid[(1R*,2S*,5S*)-3-(2-amino-5-m-tolyl-thiazole-4-carbonyl)-3-aza-bicyclo[3.1.0]hex-2-ylmethyl]-amide | basic | 0.74 | 493.3 |
| 275 | 1-Methyl-1H-indole-3-carboxylic acid [(1R*,2S*,5S*)-3-(2-amino-5-m-tolyl-thiazole-4-carbonyl)-3-aza-bicyclo[3.1.0]hex-2-ylmethyl]-amide | basic | 0.81 | 486.4 |
| 276 | 1,2-Dimethyl-1H-indole-3-carboxylic acid [(1R*,2S*,5S*)-3-(2-amino-5-m-tolyl-thiazole-4-carbonyl)-3-aza-bicyclo[3.1.0]hex-2-ylmethyl]-amide | basic | 0.84 | 500.4 |
| 277 | 1H-Indole-3-carboxylic acid [(1R*,2S*,5S*)-3-(2-amino-5-m-tolyl-thiazole-4-carbonyl)-3-aza-bicyclo[3.1.0]hex-2-ylmethyl]-amide | basic | 0.78 | 472.4 |
| 278 | 1H-Indazole-3-carboxylic acid[(1R*,2S*,5S*)-3-(2-amino-5-m-tolyl-thiazole-4-carbonyl)-3-aza-bicyclo[3.1.0]hex-2-ylmethyl]-amide | basic | 0.79 | 473.4 |
| 279 | Imidazo[1,2-a]pyridine-3-carboxylic acid [(1R*,2S*,5S*)-3-(2-amino-5-m-tolyl-thiazole-4-carbonyl)-3-aza-bicyclo[3.1.0]hex-2-ylmethyl]-amide | basic | 0.74 | 473.3 |
| 280 | 5-Fluoro-1-methyl-1H-indole-2-carboxylic acid [(1R*,2S*,5S*)-3-(2-amino-5-m-tolyl-thiazole- 4-carbonyl)-3-aza-bicyclo[3.1.0]hex-2-ylmethyl]-amide | basic | 0.89 | 504.4 |
| 281 | 2,5-Dimethyl-2H-pyrazole-3-carboxylic acid [(1R*,2S*,5S*)-3-(2-amino-5-m-tolyl-thiazole-4-carbonyl)-3-aza-bicyclo[3.1.0]hex-2-ylmethyl]-amide | basic | 0.75 | 451.3 |
| 282 | 2-Ethyl-5-methyl-2H-pyrazole-3-carboxylic acid[(1R*,2S*,5S*)-3-(2-amino-5-m-tolyl-thiazole-4-carbonyl)-3-aza-bicyclo[3.1.0]hex-2-ylmethyl]-amide | basic | 0.78 | 465.4 |
| 283 | 1-Ethyl-3-methyl-1H-pyrazole-4-carboxylic acid [(1R*,2S*,5S*)-3-(2-amino-5-m-tolyl-thiazole-4-carbonyl)-3-aza-bicyclo[3.1.0]hex-2-ylmethyl]-amide | basic | 0.72 | 465.4 |
| 284 | 2,3-Dihydro-thieno[3,4-b][1,4]dioxine-5- carboxylic acid [(1R*,2S*,5S*)-3-(2-amino-5-m-tolyl-thiazole-4-carbonyl)-3-aza-bicyclo[3.1.0]hex-2-ylmethyl]-amide | basic | 0.79 | 497.3 |
| 285 | N-[(1R*,2S*,5S*)-3-(2-Amino-5-m-tolyl-thiazole-4-carbonyl)-3-aza-bicyclo[3.1.0]hex-2-ylmethyl]-3-bromo-benzamide | basic | 0.87 | 511.3 |
| 286 | N-[(1R*,2S*,5S*)-3-(2-Amino-5-m-tolyl-thiazole-4-carbonyl)-3-aza-bicyclo[3.1.0]hex-2-ylmethyl]-3-trifluoromethyl-benzamide | basic | 0.88 | 501.4 |
| 287 | N-[(1R*,2S*,5S*)-3-(2-Amino-5-m-tolyl-thiazole-4-carbonyl)-3-aza-bicyclo[3.1.0]hex-2- ylmethyl]-3-methoxy-benzamide | basic | 0.81 | 463.3 |

Starting from [2-amino-5-(3-chloro-phenyl)-thiazol-4-yl]-[(1R*,2S*,5S*)-2-aminomethyl-3-aza-bicyclo[3.1.0]hex-3-yl]-methanone:

| Example | Name | LC-MS | | |
|---|---|---|---|---|
| | | eluent | t_{R} [min] | [M+H]⁺ |
| 288 | Benzo[d]isoxazole-3-carboxylic acid {(1R*,2S*,5S*)-3-[2-amino-5-(3-chloro-phenyl)-thiazole-4-carbonyl]-3-aza-bicyclo[3.1.0]hex-2-ylmethyl}-amide | basic | 0.85 | 494.3 |
| 289 | 2,3-Dihydro-benzo[1,4]dioxine-5-carboxylic acid {(1R*,2S*,5S*)-3-[2-amino-5-(3-chloro-phenyl)-thiazole-4-carbonyl]-3-aza-bicyclo[3.1.0]hex-2-ylmethyl} -amide | basic | 0.82 | 511.4 |
| 290 | 6-Fluoro-4H-benzo[1,3]dioxine-8-carboxylic acid {(1R*,2S*,5S*)-3-[2-amino-5-(3-chloro-phenyl)-thiazole-4-carbonyl]-3-aza-bicyclo[3.1.0]hex-2-ylmethyl}-amide | basic | 0.84 | 529.3 |
| 291 | 2,3-Dihydro-benzofuran-4-carboxylic acid {(1R*,2S*,5S*)-3-[2-amino-5-(3-chloro-phenyl)-thiazole-4-carbonyl]-3-aza-bicyclo[3.1.0]hex-2-ylmethyl}-amide | basic | 0.82 | 495.3 |
| 292 | 2,2-Dimethyl-2,3-dihydro-benzofuran-7-carboxylic acid {(1R*,2S*,5S*)-3-[2-amino-5-(3-chloro-phenyl)-thiazole-4-carbonyl]-3-aza- bicyclo[3.1.0]hex-2-ylmethyl}-amide | basic | 0.91 | 523.2 |
| 293 | Isoquinoline-1-carboxylic acid {(1R*,2S*,5S*)-3-[2-amino-5-(3-chloro-phenyl)-thiazole-4-carbonyl]-3-aza-bicyclo[3.1.0]hex-2-ylmethyl}- amide | basic | 0.87 | 504.3 |
| 294 | Quinoline-8-carboxylic acid {(1R*,2S*,5S*)-3- [2-amino-5-(3-chloro-phenyl)-thiazole-4-carbonyl]-3-aza-bicyclo[3.1.0]hex-2-ylmethyl}-amide | basic | 0.85 | 504.3 |
| 295 | Quinoline-2-carboxylic acid{(1R*,2S*,5S*)-3-[2-amino-5-(3-chloro-phenyl)-thiazole-4-carbonyl]-3-aza-bicyclo[3.1.0]hex-2-ylmethyl}-amide | basic | 0.88 | 504.3 |
| 296 | Imidazo[2,1-b]thiazole-5-carboxylic acid {(1R*,2S*,5S*)-3-[2-amino-5-(3-chloro-phenyl)-thiazole-4-carbonyl]-3-aza-bicyclo[3.1.0]hex-2-ylmethyl}-amide | basic | 0.75 | 499.3 |
| 297 | 3-Methyl-imidazo[2,1-b]thiazole-2-carboxylic acid {(1R*,2S*,5S*)-3-[2-amino-5-(3-chloro-phenyl)-thiazole-4-carbonyl]-3-aza-bicyclo[3.1.0]hex-2-ylmethyl}-amide | basic | 0.76 | 513.3 |
| 298 | 1-Methyl-1H-indole-3-carboxylic acid {(1R*,2S*,5S*)-3-[2-amino-5-(3-chloro-phenyl)-thiazole-4-carbonyl]-3-aza-bicyclo[3.1.0]hex-2-ylmethyl}-amide | basic | 0.83 | 506.3 |
| 299 | 1,2-Dimethyl-1H-indole-3-carboxylic acid {(1R*,2S*,5S*)-3-[2-amino-5-(3-chloro-phenyl)-thiazole-4-carbonyl]-3-aza-bicyclo[3.1.0]hex-2-ylmethyl}-amide | basic | 0.85 | 520.4 |
| 300 | 1H-Indole-3-carboxylic acid {(1R*,2S*,5S*)-3-[2-amino-5-(3-chloro-phenyl)-thiazole-4-carbonyl]-3-aza-bicyclo[3.1.0]hex-2-ylmethyl}-amide | basic | 0.79 | 492.3 |
| 301 | 1H-Indazole-3-carboxylic acid {(1R*,2S*,5S*)-3-[2-amino-5-(3-chloro-phenyl)-thiazole-4-carbonyl]-3-aza-bicyclo[3.1.0]hex-2-ylmethyl}-amide | basic | 0.80 | 493.3 |
| 302 | 5-Fluoro-1-methyl-1H-indole-2-carboxylic acid {(1R*,2S*,5S*)-3-[2-amino-5-(3-chloro-phenyl)-thiazole-4-carbonyl]-3-aza-bicyclo[3.1.0]hex-2-ylmethyl}-amide | basic | 0.90 | 524.3 |
| 303 | 2,5-Dimcthyl-2H-pyrazole-3-carboxylic acid {(1R*,2S*,5S*)-3-[2-amino-5-(3-chloro-phenyl)-thiazole-4-carbonyl]-3-aza-bicyclo[3.1.0]hex-2-ylmethyl}-amide | basic | 0.76 | 471.3 |
| 304 | 2-Ethyl-5-methyl-2H-pyrazole-3-carboxylic acid {(1R*,2S*,5S*)-3-[2-amino-5-(3-chloro-phenyl)-thiazole-4-carbonyl]-3-aza-bicyclo[3.1.0]hex-2-ylmethyl}-amide | basic | 0.80 | 485.3 |
| 305 | 1-Ethyl-3-methyl-1H-pyrazole-4-carboxylic acid {(1R*,2S*,5S*)-3-[2-amino-5-(3-chloro-phenyl)-thiazole-4-carbonyl]-3-aza-bicyclo[3.1.0]hex-2-ylmethyl}-amide | basic | 0.73 | 485.3 |
| 306 | 2,3-Dihydro-thieno[3,4-b][1,4]dioxine-5-carboxylic acid {(1R*,2S*,5S*)-3-[2-amino-5-(3-chloro-phenyl)-thiazole-4-carbonyl]-3-aza-bicyclo[3.1.0]hex-2-ylmethyl} -amide | basic | 0.80 | 517.3 |
| 307 | N-{(1R*,2S*,5S*)-3-[2-Amino-5-(3-chloro-phenyl)-thiazole-4-carbonyl]-3-aza-bicyclo[3.1.0]hex-2-ylmethyl}-3-promo-benzamide | basic | 0.88 | 531.3 |
| 308 | N-{(1R*,2S*,5S*)-3-[2-Amino-5-(3-chloro-phenyl)-thiazole-4-carbonyl]-3-aza- bicyclo[3.1.0]hex-2-ylmethyl}-3-methoxy- benzamide | basic | 0.82 | 483.5 |

Starting from [(1R*,2S*,5S*)-2-aminomethyl-3-aza-bicyclo[3.1.0]hex-3-yl]-(3'-fluoro-5-methyl-biphenyl-2-yl)-methanone:

| | | LC-MS | | |
|---|---|---|---|---|
| Example | Name | eluent | t_{R} [min] | [M+H]⁺ |
| 309 | 5-Methylsulfanyl-thiophene-2-carboxylic acid [(1R*,2S*,5S*)-3-(3'-fluoro-5-methyl-biphenyl- 2-carbonyl)-3-aza-bicyclo[3.1.0]hex-2-ylmethyl]-amide | basic | 1.00 | 480.9 |
| 310 | 2,3-Dihydro-thieno[3,4-b][1,4]dioxine-5-carboxylic acid [(1R*,2S*,5S*)-3-(3'-fluoro-5-methyl-biphenyl-2-carbonyl)-3-aza-bicycle[3.1.0]hex-2-ylmethyl]-amide | basic | 0.93 | 492.8 |
| 311 | 2,4-Dimethyl-thiazole-5-carboxylic acid [(1R*,2S*,5S*)-3-(3'-fluoro-5-methyl-biphenyl-2-carbonyl)-3-aza-bicyclo[3.1.0]hex-2-ylmethyl]-amide | basic | 0.92 | 464.0 |
| 312 | Benzofuran-2-carboxylic acid [(1R*,2S*,5S*)-3-(3'-fluoro-5-methyl-biphenyl-2-carbonyl)-3-aza-bicyclo[3.1.0]hex-2-ylmethyl]-amide | basic | 1.00 | 469.0 |
| 313 | 3,4-Dihydro-2H-benzo[1,4]oxazine-5-carboxylic acid [(1R*,2S*,5S*)-3-(3'-fluoro-5-methyl-biphenyl-2-carbonyl)-3-aza-bicyclo[3.1.0]hex-2-ylmethyl]-amide | basic | 1.00 | 486.0 |
| 314 | 1-Methyl-1H-indazole-3-carboxylic acid [(1R*,2S*,5S*)-3-(3'-fluoro-5-methyl-biphenyl-2-carbonyl)-3-aza-bicyclo[3.1.0]hex-2-ylmethyl]-amide | basic | 0.99 | 483.0 |
| 315 | 3-Ethynyl-N-[(1R*,2S*,5S*)-3-(3'-fluoro-5-methyl-biphenyl-2-carbonyl)-3-aza-bicyclo[3.1.0]hex-2-ylmethyl]-benzamide | basic | 0.98 | 453.0 |
| 316 | N-[(1R*,2S*,5S*)-3-(3'-Fluoro-5-methyl-biphenyl-2-carbonyl)-3-aza-bicyclo[3.1.0]hex-2-ylmethyl]-benzamide | basic | 0.96 | 429.1 |
| 317 | N-[(1R*,2S*,5S*)-3-(3'-Fluoro-5-methyl-biphenyl-2-carbonyl)-3-aza-bicyclo[3.1.0]hex-2-ylmethyl]-2,5-dimethoxy-benzamide | basic | 0.97 | 488.8 |
| 318 | 2,3,6,7-Tetrahydro-benzo[1,2-b;4,5-b']difuran- 4-carboxylic acid [(1R*,2S*,5S*)-3-(3'-fluoro- 5-methyl-biphenyl-2-carbonyl)-3-aza-bicyclo[3.1.0]hex-2-ylmethyl]-amide | basic | 0.99 | 513.0 |
| 319 | N-[(1R*,2S*,5S*)-3-(3'-Fluoro-5-methyl-biphenyl-2-carbonyl)-3-aza-bicyclo[3.1.0]hex-2-ylmethyl]-3,5-dimethoxy-benzamide | basic | 0.97 | 488.8 |
| 320 | 6-Methyl-imidazo[2,1-b]thiazole-5-carboxylic acid [(1R* ,2S* ,5S*)-3-(3'-fluoro-5-methyl-biphenyl-2-carbonyl)-3-aza-bicyclo[3.1.0]hex-2-ylmethyl]-amide | basic | 0.92 | 488.8 |
| 321 | Imidazo[1,2-a]pyridine-3-carboxylic acid [(1R*,2S*,5S*)-3-(3'-fluoro-5-methyl-biphenyl-2-carbonyl)-3-aza-bicyclo[3.1.0]hex-2-ylmethyl]-amide | basic | 0.89 | 469.0 |
| 322 | 3-Methyl-benzofuran-2-carboxylic acid [(1R*,2S*,5S*)-3-(3'-fluoro-5-methyl-biphenyl- 2-carbonyl)-3-aza-bicyclo[3.1.0]hex-2-ylmethyl]-amide | basic | 1.05 | 483.0 |
| 323 | Benzofuran-4-carboxylic acid [(1R*,2S*,5S*)-3-(3'-fluoro-5-methyl-biphenyl-2-carbonyl)-3-aza-bicyclo[3.1.0]hex-2-ylmethyl]-amide | basic | 0.99 | 469.0 |
| 324 | 2,3-Dihydro-benzofuran-4-carboxylic acid [(1R*,2S*,5S*)-3-(3'-fluoro-5-methyl-biphenyl-2-carbonyl)-3-aza-bicyclo[3.1.0]hex-2-ylmethyl]-amide | basic | 0.97 | 471.0 |
| 325 | 2,3-Dihydro-benzofuran-7-carboxylic acid [(1R*,2S*,5S*)-3-(3'-fluoro-5-methyl-biphenyl-2-carbonyl)-3-aza-bicyclo[3.1.0]hex-2-ylmethyl]-amide | basic | 0.97 | 471.0 |
| 326 | Quinoline-8-carboxylic acid [(1R*,2S*,5S*)-3-(3'-fluoro-5-methyl-biphcnyl-2-carbonyl)-3-aza-bicyclo[3.1.0]hex-2-ylmethyl]-amide | basic | 0.98 | 480.0 |
| 327 | Benzo[d]isoxazole-3-carboxylic acid [(1R*,2S*,5S*)-3-(3'-fluoro-5-methyl-biphenyl-2-carbonyl)-3-aza-bicyclo[3.1.0]hex-2-ylmethyl]-amide | basic | 1.00 | 470.0 |

### B.4 Synthesis of carboxylic amide derivatives (general procedure III)

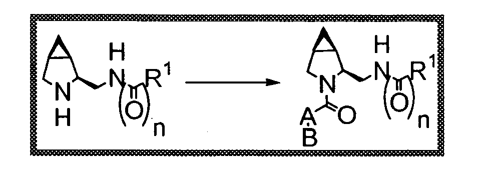

To a solution of the respective carboxylic acid (0.036 mmol) in DMF (0.50 mL) is added successively a solution of DIPEA (0.120 mmol) in DMF (0.20 mL) and a solution of TBTU (0.036 mmol) in DMF (0.30 mL). The obtained mixture is treated with a solution of Imidazo[2,1-b]thiazole-6-carboxylic acid [(1R*,2S*,5S*)-3-aza-bicyclo[3.1.0]hex-2-ylmethyl]-amide (0.030 mmol) in DMF (0.50 mL). The mixture is shaken over night and purified by prep. HPLC to give the respective amide derivative.

| Example | Name | LC-MS | | |
|---|---|---|---|---|
| | | eluent | t_{R} [min] | [M+H]⁺ |
| 328 | Imidazo[2,1-b]thiazole-6-carboxylic acid [(1R*,2S*,5S*)-3-(2-amino-5-m-tolyl-thiazole-4-carbonyl)-3-aza-bicyclo[3.1.0]hex-2-ylmethyl]-amide | basic | 0.74 | 479.1 |
| 329 | Imidazo[2,1-b]thiazole-6-carboxylic acid {(1R* ,2S* ,5S*)-3-[2-amino-5-(3-chloro-phenyl)-thiazole-4-carbonyl]-3-aza-bicyclo[3.1.0]hex-2-ylmethyl}-amide | basic | 0.75 | 498.8 |
| 330 | Imidazo[2,1-b]thiazole-6-carboxylic acid {(1R*,2S*,5S*)-3-[5-(3,4-dimethyl-phenyl)-2-methyl-thiazole-4-carbonyl]-3-aza-bicyclo[3.1.0]hex-2-ylmethyl}-amide | basic | 0.85 | 491.9 |

### B.5 Synthesis of carboxylic amide derivatives (general procedure IV)

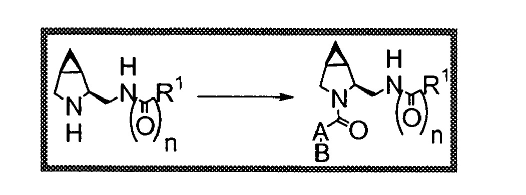

To the respective carboxylic acid (0.135 mmol) is added successively a solution of TBTU (0.150 mmol) in MeCN (0.50 mL) and DIPEA (0.750 mmol). After 30 min a solution of the respective 3-aza-bicyclo[3.1.0]hexane derivative (benzofuran-4-carboxylic acid [(1R*,2S*,5S*)-3-aza-bicyclo[3.1.0]hex-2-ylmethyl]-amide, 6-methyl-imidazo[2,1-b]thiazole-5-carboxylic acid [(1R*,2S*,5S*)-3-aza-bicyclo[3.1.0]hex-2-ylmethyl]-amide or 2,3-dihydro-benzo[1,4]dioxine-5-carboxylic acid [(1R*,2S*,5S*)-3-aza-bicyclo[3.1.0]hex-2-ylmethyl]-amide; 0.150 mmol) in DMF (0.50 mL) is added. The mixture is shaken over night and purified by prep. HPLC to give the respective amide derivative.

| Example | Name | LC-MS | | |
|---|---|---|---|---|
| | | eluent | t_{R} [min] | [M+H]⁺ |
| 331 | 6-Methyl-imidazo[2,1-b]thiazole-5-carboxylic acid ((1R*,2S*,5S*)-3-{5-[3-(2-methoxy-ethoxy)-phenyl]-2-methyl-thiazole-4-carbonyl}-3-aza-bicyclo[3.1.0]hex-2-ylmethyl)-amide | acidic | 0.87 | 552.1 |
| 332 | 6-Methyl-imidazo[2,1-b]thiazole-5-carboxylic acid {(1R*,2S*,5S*)-3-[5-(3-cyano-phenyl)-2-methyl-thiazole-4-carbonyl]-3-aza-bicyclo[3.1.0]hex-2-ylmethyl -amide | acidic | 0.88 | 503.1 |
| 333 | Benzofuran-4-carboxylic acid ((1R*,2S*,5S*)-3-{5-[3-(2-methoxy-ethoxy)-phenyl]-2-methyl-thiazole-4-carbonyl}-3-aza-bicyclo[3.1.0]hex-2-ylmethyl)-amide | acidic | 1.01 | 532.1 |
| 334 | Benzofuran-4-carboxylic acid {(1R*,2S*,5S*)-3-[5-(3-cyano-phenyl)-2-methyl-thiazole-4-carbonyl]-3-aza-bicyclo[3.1.0]hex-2-ylmethyl}-amide | acidic | 1.01 | 483.2 |
| 335 | 2,3-Dihydro-benzo[1,4]dioxine-5-carboxylic acid ((1R*,2S*,5S*)-3-{5-[3-(2-methoxy-ethoxy)-phenyl]-2-methyl-thiazole-4-carbonyl}-3-aza-bicyclo[3.1.0]hex-2-ylmethyl)-amide | acidic | 0.99 | 550.1 |
| 336 | 2,3-Dihydro-benzo[1,4]dioxine-5-carboxylic acid {(1R*,2S*,5S*)-3-[5-(3-cyano-phenyl)-2-methyl-thiazole-4-carbonyl]-3-aza-bicyclo[3.1.0]hex-2-ylmethyl} -amide | acidic | 0.99 | 501.2 |

### B.6 Synthesis of [(1R*,5S*)-3-aza-bicyclo[3.1.0]hex-3-yl]-(2-aryl-phenyl)-methanone- and [(1R*,5S*)-3-aza-bicyclo[3.1.0]hex-3-yl]-(2-heterocyclyl-phenyl)-methanone-derivatives (general procedure)

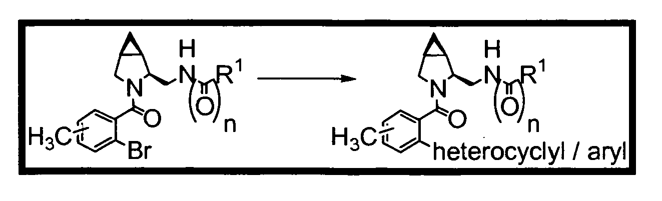

A solution of the respective [(1R*,5S*)-3-aza-bicyclo[3.1.0]hex-3-yl]-(2-bromophenyl)-methanone derivative (0.032 mmol) and the respective aryl-boronic acid (0.048 mmol) in a mixture of ethanol (0.20 mL) and toluene (0.20 mL) is prepared by gentle heating. An aq. Na₂CO₃ solution (2.0 M) is added and a flow of argon is bubbled through the mixture. After addition of Pd(PPh₃)₄ the mixture is heated to 75°C, stirred for 20 h, cooled to RT and purified by prep. HPLC to give the respective biphenyl derivative.
Heterocyclyl-substituted products are prepared in analogy by coupling of the respective aryl bromide with the respective heterocyclyl-boronic acid in DME as solvent.

Starting from 2,3-dihydro-benzo[1,4]dioxine-5-carboxylic acid [(1R*,2S*,5S*)-3-(2-bromo-4-methyl-benzoyl)-3-aza-bicyclo[3.1.0]hex-2-ylmethyl]-amide:

| Example | Name | LC-MS | | |
|---|---|---|---|---|
| | | eluent | t_{R} [min] | [M+H]⁺ |
| 337 | 2,3-Dihydro-benzo[1,4]dioxine-5-carboxylic acid [(1R*,2S*,5S*)-3-(5-methyl-biphenyl-2-carbonyl)-3-aza-bicyclo[3.1.0]hex-2-ylmethyl]-amide | basic | 0.94 | 469.3 |
| 338 | 2,3-Dihydro-benzo[1,4]dioxine-5-carboxylic acid [(1R*,2S*,5S*)-3-(2'-fluoro-5-methyl-biphenyl-2-carbonyl)-3-aza-bicyclo[3.1.0]hex-2-ylmethyl]-amide | basic | 0.94 | 487.3 |
| 339 | 2,3-Dihydro-benzo[1,4]dioxine-5-carboxylic acid [(1R*,2S*,5S*)-3-(5,3'-dimethyl-biphenyl-2-carbonyl)-3-aza-bicyclo[3.1.0]hex-2-ylmethyl]-amide | basic | 0.98 | 483.4 |
| 340 | 2,3-Dihydro-benzo[1,4]dioxine-5-carboxylic acid [(1R*,2S*,5S*)-3-(3'-chloro-5-methyl-biphenyl-2-carbonyl)-3-aza-bicyclo[3.1.0]hex-2-ylmethyl]-amide | basic | 0.98 | 503.3 |
| 341 | 2,3-Dihydro-benzo[1,4]dioxine-5-carboxylic acid [(1R*,2S*,5S*)-3-(3'-methoxy-5-methyl-biphenyl-2-carbonyl)-3-aza-bicyclo[3.1.0]hex-2-ylmethyl]-amide | basic | 0.94 | 499.4 |
| 342 | 2,3-Dihydro-benzo[1,4]dioxine-5-carboxylic acid [(1R*,2S*,5S*)-3-(5-methyl-3'-trifluoromethyl-biphenyl-2-carbonyl)-3-aza-bicyclo[3.1.0]hex-2-ylmethyl]-amide | basic | 0.98 | 537.4 |
| 343 | 2,3-Dihydro-benzo[1,4]dioxine-5-carboxylic acid [(1R*,2S*,5S*)-3-(3'-fluoro-5-methyl-biphenyl-2-carbonyl)-3-aza-bicyclo[3.1.0]hex-2-ylmethyl]-amide | basic | 0.94 | 487.4 |
| 344 | 2,3-Dihydro-benzo[1,4]dioxine-5-carboxylic acid [(1R*,2S*,5S*)-3-(3'-isopropyl-5-methyl-biphenyl-2-carbonyl)-3-aza-bicyclo[3.1.0]hex-2-ylmethyl]-amide | basic | 1.04 | 511.4 |
| 345 | 2,3-Dihydro-benzo[1,4]dioxine-5-carboxylic acid [(1R*,2S*,5S*)-3-(5-methyl-3'-trifluoromethoxy-biphenyl-2-carbonyl)-3-aza-bicyclo[3.1.0]hex-2-ylmethyl]-amide | basic | 1.00 | 553.2 |
| 346 | 2,3-Dihydro-benzo[1,4]dioxine-5-carboxylic acid [(1R*,2S*,5S*)-3-(4'-fluoro-5-methyl-biphenyl-2-carbonyl)-3-aza-bicyclo[3.1.0]hex-2-ylmethyl]-amide | basic | 0.94 | 487.3 |
| 347 | 2,3-Dihydro-benzo[1,4]dioxine-5-carboxylic acid [(1R*,2S*,5S*)-3-(5,4'-dimethyl-biphenyl-2-carbonyl)-3-aza-bicyclo[3.1.0]hex-2-ylmethyl]-amide | basic | 0.98 | 483.4 |
| 348 | 2,3-Dihydro-benzo[1,4]dioxine-5-carboxylic acid [(1R*,2S*,5S*)-3-(5,2',3'-trimethyl-biphenyl-2-carbonyl)-3-aza-bicyclo[3.1.0]hex-2-ylmethyl]-amide | basic | 1.00 | 497.4 |
| 349 | 2,3-Dihydro-benzo[1,4]dioxine-5-carboxylic acid [(1R*,2S*,5S*)-3-(4'-fluoro-5,3'-dimethyl-biphenyl-2-carbonyl)-3-aza-bieyelo[3.1.0]hex-2-ylmethyl]-amide | basic | 0.98 | 501.4 |
| 350 | 2,3-Dihydro-benzo[1,4]dioxine-5-carboxylic acid {(1R*,2S*,5S*)-3-[2-(2,3-dihydro-benzo[1,4]dioxin-6-yl)-4-methyl-benzoyl]-3-aza-bicyclo[3.1.0]hex-2-ylmethyl}-amide | basic | 0.92 | 527.4 |
| 351 | 2,3-Dihydro-benzo[1,4]dioxine-5-carboxylic acid {(1R*,2S*,5S*)-3-[4-methyl-2-(4-methyl-thiophen-2-yl)-benzoyl]-3-aza-bicyclo[3.1.0]hex-2-ylmethyl}-amide | basic | 0.97 | 489.1 |

Starting from 2,3-dihydro-benzo[1,4]dioxine-5-carboxylic acid [(1R*,2S*,5S*)-3-(2-bromo-5-methyl-benzoyl)-3-aza-bicyclo[3.1.0]hex-2-ylmethyl]-amide:

| Example | Name | LC-MS | | |
|---|---|---|---|---|
| | | eluent | t_{R} [min] | [M+H]⁺ |
| 352 | 2,3-Dihydro-benzo[1,4]dioxine-5-carboxylic acid [(1R* ,2S* ,5S*)-3-(4-methyl-biphenyl-2-carbonyl)-3-aza-bicyclo[3.1.0]hex-2-ylmethyl]-amide | basic | 0.94 | 469.1 |
| 353 | 2,3-Dihydro-benzo[1,4]dioxine-5-carboxylic acid [(1R*,2S*,5S*)-3-(2'-fluoro-4-methyl-biphenyl-2-carbonyl)-3-aza-bicyclo[3.1.0]hex-2-ylmethyl]-amide | basic | 0.94 | 487.1 |
| 354 | 2,3-Dihydro-benzo[1,4]dioxine-5-carboxylic acid [(1R*,2S*,5S*)-3-(4,3'-dimethyl-biphenyl-2-carbonyl)-3-aza-bicyclo[3.1.0]hex-2-ylmethyl]-amide | basic | 0.98 | 483.2 |
| 355 | 2,3-Dihydro-benzo[1,4]dioxine-5-carboxylic acid [(1R*,2S*,5S*)-3-(3'-chloro-4-methyl-biphenyl-2-carbonyl)-3-aza-bicyclo[3.1.0]hex-2-ylmethyl]-amide | basic | 0.98 | 503.1 |
| 356 | 2,3-Dihydro-benzo[1,4]dioxinc-5-carboxylic acid [(1R*,2S*,5S*)-3-(3'-methoxy-4-methyl-biphenyl-2-carbonyl)-3-aza-bicyclo[3.1.0]hex-2-ylmethyl]-amide | basic | 0.94 | 499.1 |
| 357 | 2,3-Dihydro-benzo[1,4]dioxine-5-carboxylic acid [(1R*,2S*,5S*)-3-(3'-fluoro-4-mcthyl-biphenyl-2-carbonyl)-3-aza-bicyclo[3.1.0]hex-2-ylmethyl]-amide | basic | 0.94 | 487.1 |
| 358 | 2,3-Dihydro-benzo[1,4]dioxine-5-carboxylic acid [(1R*,2S*,5S*)-3-(4,4'-dimethyl-biphenyl-2-carbonyl)-3-aza-bicyclo[3.1.0]hex-2-ylmethyl]-amide | basic | 0.98 | 483.1 |
| 359 | 2,3-Dihydro-benzo[1,4]dioxine-5-carboxylic acid [(1R*,2S*,5S*)-3-(4'-fluoro-4,3'-dimethyl-biphenyl-2-carbonyl)-3-aza-bicyclo[3.1.0]hex-2-ylmethyl]-amide | basic | 0.98 | 501.1 |
| 360 | 2,3-Dihydro-benzo[1,4]dioxine-5-carboxylic acid {(1R*,2S*,5S*)-3-[2-(2,3-dihydro-benzo[1,4]dioxin-6-yl)-5-methyl-benzoyl]-3-aza-bicyclo[3.1.0]hex-2-ylmethyl}-amid | basic | 0.92 | 527.0 |
| 361 | 2,3-Dihydro-benzo[1,4]dioxine-5-carboxylic acid {(1R*,2S*,5S*)-3-[5-methyl-2-(4-methyl-thiophen-2-yl)-benzoyl]-3-aza-bicyclo[3.1.0]hex-2-ylmethyl}-amide | basic | 0.98 | 488.9 |

Starting from 2,3-dihydro-benzo[1,4]dioxine-5-carboxylic acid [(1R*,2S*,5S*)-3-(2-bromo-3-methyl-benzoyl)-3-aza-bicyclo[3.1.0]hex-2-ylmethyl]-amide:

| Example | Name | LC-MS | | |
|---|---|---|---|---|
| | | eluent | t_{R} [min] | [M+H]⁺ |
| 362 | 2,3-Dihydro-benzo[1,4]dioxine-5-carboxylic acid [(1R*,2S*,5S*)-3-(6-methyl-biphenyl-2-carbonyl)-3-aza-bicyclo[3.1.0]hex-2-ylmethyl]-amide | basic | 0.94 | 469.0 |
| 363 | 2,3-Dihydro-benzo[1,4]dioxine-5-carboxylic acid [(1R*,2S*,5S*)-3-(2'-fluoro-6-methyl-biphenyl-2-carbonyl)-3-aza-bicyclo[3.1.0]hex-2-ylmethyl]-amide | basic | 0.94 | 487.0 |
| 364 | 2,3-Dihydro-benzo[1,4]dioxine-5-carboxylic acid [(1R*,25*,5S*)-3-(6,3'-dimethyl-biphenyl-2-carbonyl)-3-aza-bicyclo[3.1.0]hex-2-ylmethyl]-amide | basic | 0.97 | 483.0 |
| 365 | 2,3-Dihydro-benzo[1,4]dioxine-5-carboxylic acid [(1R*,2S*,5S*)-3-(3'-chloro-6-methyl-biphenyl-2-carbonyl)-3-aza-bicyclo[3.1.0]hex-2-ylmethyl]-amide | basic | 0.97 | 502.9 |
| 366 | 2,3-Dihydro-benzo[1,4]dioxine-5-carboxylic acid [(1R*,2S*,5S*)-3-(3'-methoxy-6-methyl-biphenyl-2-carbonyl)-3-aza-bicyclo[3.1.0]hex-2-ylmethyl]-amide | basic | 0.93 | 499.0 |
| 367 | 2,3-Dihydro-benzo[1,4]dioxine-5-carboxylic acid [(1R*,2S*,5S*)-3-(6-methyl-3'-trifluoromethyl-biphenyl-2-carbonyl)-3-aza-bicyclo[3.1.0]hex-2-ylmethyl]-amide | basic | 0.98 | 536.9 |
| 368 | 2,3-Dihydro-benzo[1,4]dioxine-5-carboxylic acid [(1R*,2S*,5S*)-3-(3'-fluoro-6-methyl-biphenyl-2-carbonyl)-3-aza-bicyclo[3.1.0]hex-2-ylmethyl]-amide | basic | 0.94 | 487.0 |
| 369 | 2,3-Dihydro-benzo[1,4]dioxine-5-carboxylic acid [(1R*,2S*,5S*)-3-(3'-cyano-6-methyl-biphenyl-2-carbonyl)-3-aza-bicyclo[3.1.0]hex-2-ylmethyl]-amide | basic | 0.89 | 494.0 |
| 370 | 2,3-Dihydro-benzo[1,4]dioxine-5-carboxylic acid [(1R*,2S*,5S*)-3-(6-methyl-3'-trifluoromethoxy-biphenyl-2-carbonyl)-3-aza-bicyclo[3.1.0]hex-2-ylmethyl]-amide | basic | 0.99 | 552.9 |
| 371 | 2,3-Dihydro-benzo[1,4]dioxine-5-carboxylic acid [(1R*,2S*,5S*)-3-(4'-fluoro-6-methyl-biphenyl-2-carbonyl)-3-aza-bicyclo[3.1.0]hex-2-ylmethyl]-amide | basic | 0.93 | 487.0 |
| 372 | 2,3-Dihydro-benzo[1,4]dioxine-5-carboxylic acid [(1R*,2S*,5S*)-3-(6,4'-dimethyl-biphenyl-2-carbonyl)-3-aza-bicyclo[3.1.0]hex-2-ylmethyl]-amide | basic | 0.97 | 483.0 |
| 373 | 2,3-Dihydro-benzo[1,4]dioxine-5-carboxylic acid [(1R*,2S*,5S*)-3-(4'-fluoro-6,3'-dimethyl-biphenyl-2-carbonyl)-3-aza-bicyclo[3.1.0]hex-2ylmethyl]-amide | basic | 0.97 | 501.0 |
| 374 | 2,3-Dihydro-benzo[1,4]dioxine-5-carboxylic acid {(1R*,2S*,5S*)-3-[2-(2,3-dihydro-benzo[1,4]dioxin-6-yl)-3-methyl-benzoyl]-3-aza-bicyclo[3.1.0]hex-2-ylmethyl}-amide | basic | 0.92 | 527.0 |
| 375 | 2,3-Dihydro-benzo[1,4]dioxine-5-carboxylic acid [(1R*,2S*,5S*)-3-(3-methyl-2-pyridin-3-yl-benzoyl)-3-aza-bicyclo[3.1.0]hex-2-ylmethyl]-amide | basic | 0.80 | 470.0 |
| 376 | 2,3-Dihydro-benzo[1,4]dioxine-5-carboxylic acid {(1R*,2S*,5S*)-3-[3-methyl-2-(4-methyl-thiophen-2-yl)-benzoyl]-3-aza-bicyclo[3.1.0]hex-2-ylmethyl}-amide | basic | 0.96 | 488.8 |
| 377 | 2,3-Dihydro-benzo[1,4]dioxine-5-carboxylic acid {(1R*,2S*,5S*)-3-[2-(1H-indol-5-yl)-3-methyl-benzoyl]-3-aza-bicyclo[3.1.0]hex-2-ylmethyl}-amide | basic | 0.90 | 508.0 |
| 378 | 2,3-Dihydro-benzo[1,4]dioxine-5-carboxylic acid [(1R*,2S*,5S*)-3-(3-methyl-2-pyrimidin-5-yl-benzoyl)-3-aza-bicyclo[3.1.0]hex-2-ylmethyl]-amide | basic | 0.77 | 471.0 |

### B.7 Synthesis of [(1R*,5S*)-3-Aza-bicyclo[3.1.0]hex-3-yl]-(2-ethynyl-phenyl)-methanone derivatives (general procedure)

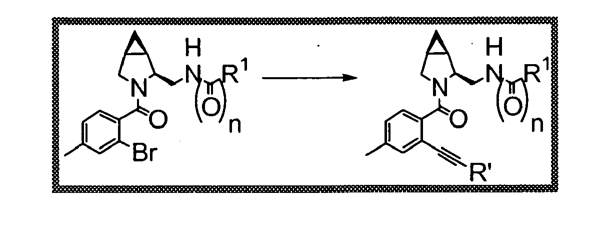

Pd(PPh₃)₂Cl₂ (0.51 mg) is added to a mixture of 2,3-dihydro-benzo[1,4]dioxine-5-carboxylic acid [(1R*,2S*,5S*)-3-(2-bromo-4-methyl-benzoyl)-3-aza-bicyclo[3.1.0]hex-2-ylmethyl]-amide (0.036 mmol), copper(I) iodide (0.20 mg) and NEt₃ (0.30 mL) in THF (0.10 mL) under nitrogen. After 1 min the respective alkyne (0.079 mmol) is added and the mixture is heated to 80°C for 2h. Toluene (0.10 mL), another portion of the respective alkyne (0.108 mmol) and additional Pd(PPh₃)₂Cl₂ (0.51 mg) are added. The mixture is heated to 80°C for 2h, cooled to RT and purified by prep. HPLC to give the respective product.

| Example | Name | LC-MS | | |
|---|---|---|---|---|
| | | eluent | t_{R} [min] | [M+H]⁺ |
| 379 | 2,3-Dihydro-benzo[1,4]dioxine-5-carboxylic acid [(1R*,2S*,5S*)-3-(2-cyclopropylethynyl-4-methyl-benzoyl)-3-aza-bicyclo[3.1.0]hex-2-ylmethyl]-amide | basic | 0.93 | 457.1 |
| 380 | 2,3-Dihydro-benzo[1,4]dioxine-5-carboxylic acid [(1R*,2S*,5S*)-3-(2-but-1-ynyl-4-methyl-benzoyl)-3-aza-bicyclo[3.1.0]hex-2-ylmethyl]-amide | basic | 0.93 | 445.3 |
| 381 | 2,3-Dihydro-benzo[1,4]dioxine-5-carboxylic acid {(1R*,2S*,5S*)-3-[4-methyl-2-(4-methyl-pent-1-ynyl)-benzoyl]-3-aza-bicyclo[3.1.0]hex-2-ylmethyl}-amide | basic | 1.01 | 473.4 |

### B.8 Separation of racemates by chromatography on chiral stationary phases (general procedure)

The respective mixture of enantiomers is separated by chiral HPLC using the respective column and the respective eluent to give the respective product in enantiomerically highly enriched form.

| Example | Name | HPLC | | |
|---|---|---|---|---|
| | | Column | Eluent | t_{R} [min] |
| 382 | Benzofuran-4-carboxylic acid [(1R,2S,5S)-3-(2-methyl-5-m-tolyl-thiazole-4-carbonyl)-3-aza-bicyclo[3.1.0]hex-2-ylmethyl]-amide | ChiralCel OD 20x250 mm 10 µm | hexane/EtOH 80/20 | 13.0 (ent.: 17.9) |
| 383 | Benzofuran-4-carboxylic acid {(1R,2S,5S)-3-[5-(3-fluoro-phenyl)-2-methyl-thiazole-4-carbonyl]-3-aza-bicyclo[3.1.0]hex-2-ylmethyl}-amide | ChiralCel OD 20x250 mm 10 µm | hexane/EtOH 80/20 | 14.5 (ent.: 16.8) |
| 384 | 2,3-Dihydro-benzo[1,4]dioxine-5-carboxylic acid [(1R,2S,5S)-3-(2-methyl-5-m-tolyl-thiazole-4-carbonyl)-3-aza-bicyclo[3.1.0]hex-2-ylmethyl]-amide | ChiralCel OD 4.6x250 mm 10 µm | hexane/EtOH 90/10 | 17.5 (ent.: 21.7) |

### B.9 Example 385:

### 6-Methyl-imidazo[2,1-b]thiazole-5-carboxylic acid [(1R*,2S*,5S*)-3-(2-bromo-5-m-tolyl-thiazole-4-carbonyl)-3-aza-bicyclo[3.1.0]hex-2-ylmethyl]-amide

TBTU (0.19 mmol) is added to a solution of 6-methyl-imidazo[2,1-b]thiazole-5-carboxylic acid [(1R*,2S*,5S*)-3-aza-bicyclo[3.1.0]hex-2-ylmethyl]-amide (0.16 mmol), 2-Bromo-5-m-tolyl-thiazole-4-carboxylic acid (0.16 mmol) and DIPEA (0.40 mmol) in DCM (2.50 mL). The mixture is stirred for 2h at RT, washed twice with water, twice with aq. citric acid solution (10%), once with sat. aq. NaHCO₃ solution and once with water, dried over Na₂SO₄ and concentrated in vacuo to give the crude product which is purified by chromatography (DCM/MeOH 19/1). LC-MS (basic): t_{R} = 0.94 min; [M+H]⁺ = 556.0.

### B.10 Example 386:

### 6-Methyl-imidazo[2,1-b]thiazole-5-carboxylic acid [(1R*,2S*,5S*)-3-(5-m-tolyl-2-trimethylsilanylethynyl-thiazole-4-carbonyl)-3-aza-bicyclo[3.1.0]hex-2-ylmethyl]-amide

Pd(PPh₃)₂Cl₂ (0.65 mg) is added to a mixture of 6-methyl-imidazo[2,1-b]thiazole-5-carboxylic acid [(1R*,2S*,5S*)-3-(2-bromo-5-m-tolyl-thiazole-4-carbonyl)-3-aza-bicyclo[3.1.0]hex-2-ylmethyl]-amide (0.054 mmol), copper(I) iodide (0.26 mg) and NEt₃ (0.60 mL) in THF (0.30 mL) under nitrogen. After 1 min ethynyltrimethylsilane (0.108 mmol) is added. The mixture is heated to 80°C for 3.5 h, concentrated in vacuo and purified by chromatography (DCM/MeOH 19/1) to give the desired product. LC-MS: t_{R} = 1.06 min; [M+H]⁺ = 574.5.

### B.11 Example 387:

### 6-Methyl-imidazo[2,1-b]thiazole-5-carboxylic acid {(1R*,2S*,5S*)-3-[2-(3-hydroxy-prop-1-ynyl)-5-m-tolyl-thiazole-4-carbonyl]-3-aza-bicyclo[3.1.0]hex-2-ylmethyl}-amide

Pd(PPh₃)₂Cl₂ (0.65 mg) is added to a mixture of 6-methyl-imidazo[2,1-b]thiazole-5-carboxylic acid [(1R*,2S*,5S*)-3-(2-bromo-5-m-tolyl-thiazole-4-carbonyl)-3-aza-bicyclo[3.1.0]hex-2-ylmethyl]-amide (0.054 mmol), copper(I) iodide (0.26 mg) and NEt₃ (0.60 mL) in THF (0.30 mL) under nitrogen. After 1 min 2-propyn-1-ol (0.108 mmol) is added. The mixture is heated to 80°C for 3.5 h, concentrated in vacuo and purified by chromatography (DCM/MeOH 19/1) to give the desired product. LC-MS: t_{R} = 0.84 min; [M+H]⁺ = 532.3.

### B.12 Example 388:

### 6-Methyl-imidazo[2,1-b]thiazole-5-carboxylic acid [(1R*,2S*,5S*)-3-(2-ethyl-5-m-tolyl-thiazole-4-carbonyl)-3-aza-bicyclo[3.1.0]hex-2-ylmethyl]-amide

A solution of 6-methyl-imidazo[2,1-b]thiazole-5-carboxylic acid [(1R*,2S*,5S*)-3-(5-m-tolyl-2-trimethylsilanylethynyl-thiazole-4-carbonyl)-3-aza-bicyclo[3.1.0]hex-2-ylmethyl]-amide (0.017 mmol) in EtOH (1.0 mL) is treated successively with tetrabutylammonium fluoride hydrate (0.017 mmol) and Pd/C (10%, 10 mg) and stirred at RT under a hydrogen atmosphere (1 bar) for 16 h. After filtration through celite and removal of the solvents in vacuo a crude product is obtained which is purified by chromatography (EtOAc/heptane 2/1). LC-MS (basic): t_{R} = 0.92 min; [M+H]⁺ = 506.1.

### B.13 Example 389:

### 6-Methyl-imidazo[2,1-b]thiazole-5-carboxylic acid {(1R*,2S*,5S*)-3-[2-(3-hydroxy-propyl)-5-m-tolyl-thiazole-4-carbonyl]-3-aza-bicyclo[3.1.0] hex-2-ylmethyl}-amide

A solution of 6-methyl-imidazo[2,1-b]thiazole-5-carboxylic acid {(1R*,2S*,5S*)-3-[2-(3-hydroxy-prop-1-ynyl)-5-m-tolyl-thiazole-4-carbonyl]-3-aza-bicyclo[3.1.0]hex-2-ylmethyl}-amide (0.033 mmol) in EtOH (1.0 mL) is treated with Pd/C (10%, 10 mg) and stirred at RT under a hydrogen atmosphere (1 bar) for 6 h. Additional Pd/C (10%, 10 mg) is added and the mixture is stirred at RT under a hydrogen atmosphere (1 bar) for 16 h. After filtration through celite and removal of the solvents in vacuo the desired product is obtained. LC-MS (basic): t_{R} = 0.79 min; [M+H]⁺ = 536.1.

### B.14 Example 390:

### 6-Methyl-imidazo[2,1-b]thiazole-5-carboxylic acid [(1R*,2S*,5S*)-3-(5-m-tolyl-thiazole-4-carbonyl)-3-aza-bicyclo[3.1.0]hex-2-ylmethyl]-amide

TBTU (0.058 mmol) is added to a solution of 6-methyl-imidazo[2,1-b]thiazole-5-carboxylic acid [(1R*,2S*,5S*)-3-aza-bicyclo[3.1.0]hex-2-ylmethyl]-amide (0.048 mmol), 5-m-tolyl-thiazole-4-carboxylic acid (0.048 mmol) and DIPEA (0.12 mmol) in DCM (1.00 mL). The mixture is stirred for 2h at RT, washed twice with water, twice with aq. citric acid solution (10%), once with sat. aq. NaHCO₃ solution and once with water, dried over Na₂SO₄ and concentrated in vacuo to give the crude product which is purified by chromatography (DCM/MeOH 19/1). LC-MS (basic): tR = 0.83 min; [M+H]⁺ = 478.1.

### B.15 Example 391:

### 6-Methyl-imidazo[2,1-b]thiazole-5-carboxylic acid [(1R*,2S*,5S*)-3-(2-methoxy-5-m-tolyl-thiazole-4-carbonyl)-3-aza-bicyclo [3.1.0] hex-2-ylmethyl]-amide

TBTU (0.058 mmol) is added to a solution of 6-methyl-imidazo[2,1-b]thiazole-5-carboxylic acid [(1R*,2S*,5S*)-3-aza-bicyclo[3.1.0]hex-2-ylmethyl]-amide (0.048 mmol), 2-methoxy-5-m-tolyl-thiazole-4-carboxylic acid (0.048 mmol) and DIPEA (0.12 mmol) in DCM (1.00 mL). The mixture is stirred for 2h at RT, washed twice with water, twice with aq. citric acid solution (10%), once with sat. aq. NaHCO₃ solution and once with water, dried over Na₂SO₄ and concentrated in vacuo to give the crude product which is purified by chromatography (DCM/MeOH 19/1). LC-MS (basic): t_{R} = 0.91 min; [M+H]⁺ = 508.2.

### II. Biological assays

### In vitro assay

The orexin receptor antagonistic activity of the compounds of formula (I) is determined in accordance with the following experimental method.

### Experimental method:

Intracellular calcium measurements:
Chinese hamster ovary (CHO) cells expressing the human orexin-1 receptor and the human orexin-2 receptor, respectively, are grown in culture medium (Ham F-12 with L-Glutamine) containing 300 µg/ml G418, 100 U/ml penicillin, 100 µg/ml streptomycin and 10 % inactivated fetal calf serum (FCS). The cells are seeded at 80'000 cells / well into 96-well black clear bottom sterile plates (Costar) which have been precoated with 1% gelatine in Hanks' Balanced Salt Solution (HBSS). All reagents are from Gibco BRL. The seeded plates are incubated overnight at 37°C in 5% CO₂.
Human orexin-A as an agonist is prepared as 1 mM stock solution in MeOH: water (1:1), diluted in HBSS containing 0.1 % bovine serum albumin (BSA) and 2 mM HEPES for use in the assay at a final concentration of 10 nM.
Antagonists are prepared as 10 mM stock solution in DMSO, then diluted in 96-well plates, first in DMSO, then in HBSS containing 0.1% bovine serum albumin (BSA) and 2 mM HEPES.
On the day of the assay, 100 µl of loading medium (HBSS containing 1% FCS, 2 mM HEPES, 5 mM probenecid (Sigma) and 3 µM of the fluorescent calcium indicator fluo-3 AM (1 mM stock solution in DMSO with 10% pluronic acid) (Molecular Probes) is added to each well.
The 96-well plates are incubated for 60 min at 37° C in 5% CO₂. The loading solution is then aspirated and cells are washed 3 times with 200 µl HBSS containing 2.5 mM probenecid, 0.1% BSA, 2 mM HEPES. 100 µl of that same buffer is left in each well.

Within the Fluorescent Imaging Plate Reader (FLIPR, Molecular Devices), antagonists are added to the plate in a volume of 50 µl, incubated for 20 min and finally 100 µl of agonist is added. Fluorescence is measured for each well at 1 second intervals, and the height of each fluorescence peak is compared to the height of the fluorescence peak induced by 10 nM orexin-A with buffer in place of antagonist. For each antagonist, IC₅₀ value (the concentration of compound needed to inhibit 50 % of the agonistic response) is determined. Antagonistic activities (IC₅₀ values) of all exemplified compounds arc below 1000 nM with respect to the OX₁ and/or the OX₂ receptor. IC₅₀ values of 365 exemplified compounds are in the range of 5-9992 nM with an average of 728 nM with respect to the OX₁ receptor. IC₅₀ values of all exemplified compounds are in the range of 2-4055 nM with an average of 187 nM with respect to the OX₂ receptor. Antagonistic activities of selected compounds are displayed in *Table 1.*

**Table I**

| Compound | OX₁ IC₅₀ (nM) | OX₂ IC₅₀ (nM) |
|---|---|---|
| 3 | 158 | 348 |
| 8 | 994 | 96 |
| 16 | 143 | 33 |
| 30 | 1215 | 32 |
| 31 | 240 | 51 |
| 32 | 2964 | 91 |
| 33 | 2173 | 94 |
| 42 | 237 | 50 |
| 60 | 596 | 76 |
| 64 | 162 | 242 |
| 74 | 99 | 16 |
| 82 | 864 | 50 |
| 100 | 1296 | 80 |
| 108 | 131 | 101 |
| 114 | 93 | 124 |
| 125 | 98 | 102 |
| 141 | 76 | 14 |
| 143 | 507 | 17 |
| 146 | 54 | 55 |
| 154 | 201 | 237 |
| 178 | 43 | 33 |
| 179 | 237 | 76 |
| 181 | 90 | 154 |
| 182 | 241 | 196 |
| 203 | 42 | 92 |
| 214 | 37 | 17 |
| 242 | 963 | 59 |
| 256 | 111 | 14 |
| 274 | 31 | 87 |
| 284 | 33 | 15 |
| 318 | 196 | 114 |
| 325 | 346 | 40 |
| 333 | 954 | 172 |
| 336 | 141 | 48 |
| 350 | 110 | 194 |
| 376 | 194 | 158 |
| 379 | 373 | 61 |
| 383 | 22 | 12 |
| 387 | 23 | 85 |
| 389 | 19 | 17 |

## Claims

1. A compound of formula (I) 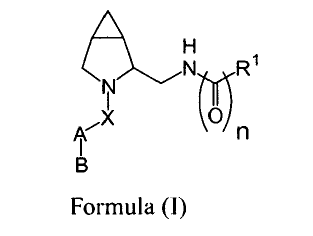 wherein
X represents C(O) or SO₂;
A represents aryl or heterocyclyl, wherein the aryl or heterocyclyl is unsubstituted or independently mono- or di-substituted, wherein the substituents are independently selected from the group consisting of (C₁₋₄)alkyl, (C₃₋₆)cycloalkyl, (C₂₋₆)alkynyl, hydroxy-(C₁₋₄)alkyl, hydroxy-(C₂₋₆)alkynyl, trimethylsilyl-ethynyl, (C₃₋₆)cycloalkyl-ethynyl, (C₁₋₄)alkoxy, trifluoromethyl, trifluoromethoxy, NR²R³, N(R²)C(O)R³, C(O)NR²R³, and halogen;
B represents a hydrogen atom or an aryl- or heterocyclyl-group, wherein the aryl or heterocyclyl is unsubstituted or independently mono-, di-, or trisubstituted, wherein the substituents are independently selected from the group consisting of (C₁₋₄)alkyl, (C₃₋₆)cycloalkyl, (C₁₋₄)alkoxy, methoxy-(C₁₋₄)alkoxy, cyano, trifluoromethyl, trifluoromethoxy, NR²R³, N(R²)C(O)R³, C(O)NR²R³, and halogen; or B represents a 2,3-dihydro-benzo[1,4]dioxinyl group;
or A and B together represent a tricyclic group;
n represents 0 or 1;
R¹ represents aryl or heterocyclyl, wherein the aryl or heterocyclyl is unsubstituted or independently mono-, di-, or trisubstituted wherein the substituents are independently selected from the group consisting of (C₁₋₄)alkyl, (C₂₋₆)alkynyl, (C₃₋₆)cycloalkyl, (C₁₋₄)alkoxy, (C₁₋₄)alkylthio, halogen, hydroxy, cyano, trifluoromethyl, trifluoromethoxy, NR²R³, N(R²)C(O)R³, COOR², and C(O)NR²R³; or R¹ represents a heterocyclyl-ethenyl-, a heterocyclyl-(C₁₋₄)alkyl or an aryloxy-(C₁₋₄)alkyl-group, which groups are unsubstituted or independently mono- or di-substituted at the aryl- or heterocyclyl-part wherein the substituents are independently selected from the group consisting of (C₁₋₄)alkyl, (C₁₋₄)alkoxy, halogen, trifluoromethyl, trifluoromethoxy, and NR²R³; or R¹ represents a 2,3-dihydro-benzofuranyl-, a benzo[1,3]dioxolyl-, a 2,3-dihydro-benzo[1,4]dioxinyl-, a 4-oxo-4*H-*chromenyl-, a 2*H-*chromenyl, a chromanyl-, a 4*H-*benzo[1,3]dioxinyl-, a 2,3-dihydro-thieno[3,4-b][1,4]dioxinyl-, a morpholin-4-yl-phenyl-, a piperazin-1-yl-phenyl-, a 3,4-dihydro-2*H-*benzo[1,4]oxazinyl-, a 3-oxo-3,4-dihydro-2*H-*benzo[1,4]oxazinyl- or a 2,3,6,7-tetrahydro-benzo[1,2-b;4,5-b']difuranyl-group, wherein said groups are unsubstituted or independently mono- or di-substituted wherein the substituents are independently selected from the group consisting of (C₁₋₄)alkyl, (C₁₋₄)alkoxy, and halogen;
R² represents hydrogen or (C₁₋₄)alkyl; and
R³ represents hydrogen or (C₁₋₄)alkyl;
or a pharmaceutically acceptable salt thereof.

2. A compound of general formula (Ia) according to claim 1, wherein the stereogenic centers are in a relative cis-configuration 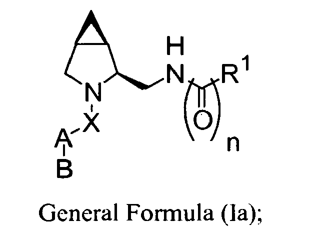 or a pharmaceutically acceptable salt thereof.

3. A compound according to claim 1 or 2, wherein
X represents C(O) or SO₂;
A represents aryl or heterocyclyl, wherein the aryl or heterocyclyl is unsubstituted or independently mono- or di-substituted, wherein the substituents are independently selected from the group consisting of (C₁₋₄)alkyl, (C₃₋₆)cycloalkyl, (C₁₋₄)alkoxy, trifluoromethyl, trifluoromethoxy, NR²R³, N(R²)C(O)R³, C(O)NR²R³, and halogen; B represents a hydrogen atom or an aryl- or heterocyclyl-group, wherein the aryl or heterocyclyl is unsubstituted or independently mono-, di-, or trisubstituted, wherein the substituents are independently selected from the group consisting of (C₁₋₄)alkyl, (C₃₋₆)cycloalkyl, (C₁₋₄)alkoxy, trifluoromethyl, trifluoromethoxy, NR²R³, N(R²)C(O)R³, C(O)NR²R³, and halogen;
or A and B together represent a tricyclic group;
n represents 0 or 1;
R¹ represents aryl or heterocyclyl, wherein the aryl or heterocyclyl is unsubstituted or independently mono-, di-, or trisubstituted wherein the substituents are independently selected from the group consisting of (C₁₋₄)alkyl, (C₃₋₆)cycloalkyl, (C₁₋₄)alkoxy, halogen, hydroxy, cyano, trifluoromethyl, trifluoromethoxy, NR²R³, N(R²)C(O)R³, and C(O)NR²R³; or R¹ represents a heterocyclyl-ethenyl-, a heterocyclyl-(C₁₋₄)alkyl or an aryloxy-(C₁₋₄)alkyl-group, which groups are unsubstituted or independently mono- or di-substituted at the aryl- or heterocyclyl-part wherein the substituents are independently selected from the group consisting of (C₁₋₄)alkyl, (C₁₋₄)alkoxy, halogen, trifluoromethyl, trifluoromethoxy, and NR²R³; or R¹ represents a 2,3-dihydro-benzofuranyl-, a benzo[1,3]dioxolyl-, a 2,3-dihydro-benzo[1,4]dioxinyl- or a 4-oxo-4*H-*chromenyl group, wherein said groups are unsubstituted or mono-substituted at the aromatic ring with substituents independently selected from the group consisting of (C₁₋₄)alkyl, (C₁₋₄)alkoxy, and halogen;
R² represents hydrogen or (C₁₋₄)alkyl; and
R³ represents hydrogen or (C₁₋₄)alkyl;
or a pharmaceutically acceptable salt thereof.

4. Compounds according to any one of claims 1 to 3, wherein
A represents heterocyclyl, wherein the heterocyclyl is unsubstituted or mono-substituted, wherein the substituent is selected from the group consisting of (C₁₋₄)alkyl, and NR²R³;
B represents aryl, wherein the aryl is unsubstituted or independently mono-, di- or trisubstituted, wherein the substituents are independently selected from the group consisting of (C₁₋₄)alkyl, (C₁₋₄)alkoxy, trifluoromethyl, and halogen; and
R¹ represents aryl or heterocyclyl, wherein the aryl or heterocyclyl is unsubstituted or independently mono-, di-, or trisubstituted wherein the substituents are independently selected from the group consisting of (C₁₋₄)alkyl, (C₁₋₄)alkoxy, and halogen; or R¹ represents a 2,3-dihydro-benzofuranyl-, a benzo[1,3]dioxolyl- or a 2,3-dihydro-benzo[1,4]dioxinyl-group;
or a pharmaceutically acceptable salt thereof.

5. Compounds according to any one of claims 1 to 4, wherein
A represents an oxazolyl, a thiazolyl, a pyrimidyl or a pyrazinyl group, wherein said groups are unsubstituted or mono-substituted, wherein the substituent is selected from the group consisting of (C₁₋₄)alkyl, and NR²R³;
B represents phenyl, wherein the phenyl is unsubstituted or independently mono- or di-substituted, wherein the substituents are independently selected from the group consisting of (C₁₋₄)alkyl, (C₁₋₄)alkoxy, trifluoromethyl, and halogen; and
R¹ represents a phenyl, a naphthyl, a benzofuranyl, a imidazo[2,1-b]thiazolyl, a imidazo[1,2-a]pyridyl, a pyrazolo[1,5-a]pyridyl, a thiazolyl, a isoxazolyl, a pyrazolyl, an indolyl, an indazolyl, a benzimidazolyl or a benzothiophenyl group, wherein said groups are unsubstituted or independently mono- or di-substituted wherein the substituents are independently selected from the group consisting of (C₁₋₄)alkyl, (C₁₋₄)alkoxy, and halogen; or R¹ represents a 2,3-dihydro-benzofuranyl-, a benzo[1,3]dioxolyl- or a 2,3-dihydro-benzo[1,4]dioxinyl-group;
or a pharmaceutically acceptable salt thereof.

6. A compound according to any one of claims 1 to 5, wherein
X represents C(O) ;
or a pharmaceutically acceptable salt thereof.

7. A compound according to any one of claims 1 to 6, wherein
n represents 1;
or a pharmaceutically acceptable salt thereof.

8. A compound according to any one of claims 1, 2, 6, or 7, wherein
A represents aryl or heterocyclyl, wherein the aryl or heterocyclyl is unsubstituted or mono-substituted, wherein the substituent is selected from the group consisting of (C₁₋₄)alkyl, (C₃₋₆)cycloalkyl, (C₂₋₆)alkynyl, hydroxy-(C₁₋₄)alkyl, hydroxy-(C₂₋₆)alkynyl, (C₃₋₆)cycloalkyl-ethynyl, (C₁₋₄)alkoxy, NR²R³, and halogen;
or a pharmaceutically acceptable salt thereof.

9. A compound according to any one of claims 1, 2, or 6 to 8, wherein
B represents aryl or heterocyclyl, wherein the aryl or heterocyclyl is unsubstituted or independently mono-, di-, or trisubstituted, wherein the substituents are independently selected from the group consisting of (C₁₋₄)alkyl, (C₁₋₄)alkoxy, cyano, trifluoromethyl, NR²R³, and halogen;
or a pharmaceutically acceptable salt thereof.

10. A compound according to any one of claims 1, 2, or 6 to 9, wherein
R¹ represents aryl or heterocyclyl, wherein the aryl or heterocyclyl is unsubstituted or independently mono-, di-, or trisubstituted wherein the substituents are independently selected from the group consisting of (C₁₋₄)alkyl, (C₁₋₄)alkoxy, halogen, hydroxy, cyano, trifluoromethyl, and COOR²; or R¹ represents a 2,3-dihydro-benzofuranyl-, a benzo[1,3]dioxolyl-, a 2,3-dihydro-benzo[1,4]dioxinyl-, a 2*H-*chromenyl, a chromanyl-, a 4*H-*benzo[1,3]dioxinyl-, a 2,3-dihydro-thieno[3,4-b][1,4]dioxinyl-, a 3,4-dihydro-2*H-*benzo[1,4]oxazinyl- or a 2,3,6,7-tetrahydro-benzo[1,2-b;4,5-b'] difuranyl-group, wherein said groups are unsubstituted or independently mono- or di-substituted wherein the substituents are independently selected from the group consisting of (C₁₋₄)alkyl, (C₁₋₄)alkoxy, and halogen;
or a pharmaceutically acceptable salt thereof.

11. A compound according to any one of claims 1, 2, or 6 to 10, wherein
A represents aryl, wherein the aryl is unsubstituted or mono-substituted, wherein the substituent is selected from the group consisting of (C₁₋₄)alkyl, (C₃₋₆)cycloalkyl, (C₃₋₆)cycloalkyl-ethynyl, or halogen;
or a pharmaceutically acceptable salt thereof.

12. A compound according to any one of claims 1, 2, or 6 to 10, wherein
A represents heterocyclyl, wherein the heterocyclyl is unsubstituted or mono-substituted, wherein the substituent is selected from the group consisting of (C₁₋₄)alkyl, (C₃₋₆)cycloalkyl, hydroxy-(C₁₋₄)alkyl, hydroxy-(C₂₋₆)alkynyl, (C₁₋₄)alkoxy, NR²R³, and halogen;
or a pharmaceutically acceptable salt thereof.

13. A compound according to any one of claims 1 to 4, or 6 to 12, wherein
B represents phenyl, wherein the phenyl is unsubstituted or independently mono-, di-, or trisubstituted, wherein the substituents are independently selected from the group consisting of (C₁₋₄)alkyl, (C₁₋₄)alkoxy, trifluoromethyl, and halogen;
or a pharmaceutically acceptable salt thereof.

14. A compound according to any one of claims 1 to 3, or 6 to 13, wherein
R¹ represents heterocyclyl, wherein the heterocyclyl is unsubstituted or independently mono-, di-, or trisubstituted wherein the substituents are independently selected from the group consisting of (C₁₋₄)alkyl, (C₁₋₄)alkoxy, halogen, and trifluoromethyl;
or a pharmaceutically acceptable salt thereof.

15. A compound according to any one of claims 1 to 3, or 6 to 13, wherein
R¹ represents aryl, wherein the aryl is unsubstituted or independently mono-, di-, or trisubstituted wherein the substituents are independently selected from the group consisting of (C₁₋₄)alkyl, (C₁₋₄)alkoxy, halogen, hydroxy, cyano, and trifluoromethyl; or a pharmaceutically acceptable salt thereof.

16. A compound according to any one of claims 1, 2, or 6 to 13, wherein
R¹ represents a 2,3-dihydro-benzofuranyl-, a 2,3-dihydro-benzo[1,4]dioxinyl-, a chromanyl-, a 2,3-dihydro-thieno[3,4-b][1,4]dioxinyl- or a 3,4-dihydro-2*H-*benzo[1,4]oxazinyl-group, wherein said groups are unsubstituted or mono-substituted wherein the substituent is selected from the group consisting of (C₁₋₄)alkyl, and halogen;
or a pharmaceutically acceptable salt thereof.

17. A compound according to any one of claims 1 to 3, or 6 to 14, wherein, in case R¹ represents heterocyclyl, said heterocyclyl is an imidazo[2,1-b]thiazolyl or an imidazo[1,2-a]pyridyl group, wherein said groups are unsubstituted or mono-substituted, wherein the substituent is selected from the group consisting of (C₁₋₄)alkyl, halogen, and trifluoromethyl;
or a pharmaceutically acceptable salt thereof.

18. A compound according to any one of claims 1, 2, 6 to 10, or 12 to 17, wherein, in case A represents heterocyclyl, said heterocyclyl is a thiazole group, which is unsubstituted or mono-substituted, wherein the substituent is selected from the group consisting of (C₁₋₄)alkyl, hydroxy-(C₁₋₄)alkyl, hydroxy-(C₂₋₆)alkynyl, (C₁₋₄)alkoxy, NR²R³, and halogen;
or a pharmaceutically acceptable salt thereof.

19. A compound according to claim 1 selected from the group consisting of:
4-fluoro-N-{(1R*,2S*,5S*)-3-[5-(3-fluoro-phenyl)-2-methyl-thiazole-4-carbonyl]-3-aza-bicyclo[3.1.0]hex-2-ylmethyl}-benzamide;
benzofuran-4-carboxylic acid [(1R*,2S*,5S*)-3-(biphenyl-2-carbonyl)-3-aza-bicyclo[3.1.0]hex-2-ylmethyl]-amide;
benzofuran-4-carboxylic acid [(1R*,2S*,5S*)-3-(2-methyl-5-m-tolyl-thiazole-4-carbonyl)-3-aza-bicyclo[3.1.0]hex-2-ylmethyl]-amide;
benzofuran-4-carboxylic acid {(1R*,2S*,5S*)-3-[5-(4-fluoro-phenyl)-2-methylthiazole-4-carbonyl]-3-aza-bicyclo[3.1.0]hex-2-ylmethyl}-amide;
benzofuran-4-carboxylic acid {(1R*,2S*,5S*)-3-[5-(3-fluoro-phenyl)-2-methylthiazole-4-carbonyl]-3-aza-bicyclo[3.1.0]hex-2-ylmethyl}-amide;
benzofuran-4-carboxylic acid [(1R*,2S*,5S*)-3-(2-methyl-5-p-tolyl-thiazole-4-carbonyl)-3-aza-bicyclo[3.1.0]hex-2-ylmethyl]-amide;
benzofuran-4-carboxylic acid {(1R*,2S*,5S*)-3-[5-(4-ethyl-phenyl)-2-methylthiazole-4-carbonyl]-3-aza-bicyclo[3.1.0]hex-2-ylmethyl}-amide;
benzofuran-4-carboxylic acid {(1R*,2S*,5S*)-3-[5-(2-fluoro-phenyl)-2-methylthiazole-4-carbonyl]-3-aza-bicyclo[3.1.0]hex-2-ylmethyl}-amide;
benzofuran-4-carboxylic acid {(1R*,2S*,5S*)-3-[5-(2-chloro-phenyl)-2-methylthiazole-4-carbonyl]-3-aza-bicyclo[3.1.0]hex-2-ylmethyl}-amide;
benzofuran-4-carboxylic acid {(1R*,2S*,5S*)-3-[5-(3-chloro-phenyl)-2-methylthiazole-4-carbonyl]-3-aza-bicyclo[3.1.0]hex-2-ylmethyl}-amide;
benzofuran-4-carboxylic acid {(1R*,2S*,5S*)-3-[5-(2-methoxy-phenyl)-2-methylthiazole-4-carbonyl]-3-aza-bicyclo[3.1.0]hex-2-ylmethyl}-amide;
benzofuran-4-carboxylic acid {(1R*,2S*,5S*)-3-[5-(3-methoxy-phenyl)-2-methylthiazole-4-carbonyl]-3-aza-bicyclo[3.1.0]hex-2-ylmethyl}-amide;
benzofuran-4-carboxylic acid {(1R*,2S*,5S*)-3-[2-methyl-5-(3-trifluoromethyl-phenyl)-thiazole-4-carbonyl]-3-aza-bicyclo[3.1.0]hex-2-ylmethyl}-amide;
benzofuran-4-carboxylic acid {(1R*,2S*,5S*)-3-[2-methyl-5-(2-trifluoromethyl-phenyl)-thiazole-4-carbonyl]-3-aza-bicyclo[3.1.0]hex-2-ylmethyl}-amide;
benzofuran-4-carboxylic acid [(1R*,2S*,5S*)-3-(2-methyl-5-o-tolyl-thiazole-4-carbonyl)-3-aza-bicyclo[3.1.0]hex-2-ylmethyl]-amide;
benzofuran-4-carboxylic acid {(1R*,2S*,5S*)-3-[5-(3,4-dimethyl-phenyl)-2-methylthiazole-4-carbonyl]-3-aza-bicyclo[3.1.0]hex-2-ylmethyl}-amide;
benzofuran-4-carboxylic acid [(1R*,2S*,5S*)-3-(2-amino-5-m-tolyl-thiazole-4-carbonyl)-3-aza-bicyclo[3.1.0]hex-2-ylmethyl]-amide;
benzofuran-4-carboxylic acid {(1R*,2S*,5S*)-3-[2-amino-5-(3-fluoro-phenyl)-thiazole-4-carbonyl]-3-aza-bicyclo[3.1.0]hex-2-ylmethyl}-amide;
benzofuran-4-carboxylic acid [(1R*,2S*,5S*)-3-(2-methyl-4-phenyl-pyrimidine-5-carbonyl)-3-aza-bicyclo[3.1.0]hex-2-ylmethyl]-amide;
benzofuran-4-carboxylic acid {(1R*,2S*,5S*)-3-[2-(2-amino-thiazol-4-yl)-benzoyl]-3-aza-bicyclo[3.1.0]hex-2-ylmethyl}-amide;
benzofuran-4-carboxylic acid [(1R*,2S*,5S*)-3-(9H-fluorene-4-carbonyl)-3-aza-bicyclo[3.1.0]hex-2-ylmethyl]-amide;
benzofuran-4-carboxylic acid [(1R*,2S*,5S*)-3-(3-phenyl-pyrazine-2-carbonyl)-3-aza-bicyclo[3.1.0]hex-2-ylmethyl]-amide;
benzofuran-4-carboxylic acid {(1R*,2S*,5S*)-3-[5-(4-methoxy-phenyl)-oxazole-4-carbonyl]-3-aza-bicyclo[3.1.0]hex-2-ylmethyl}-amide;
benzofuran-4-carboxylic acid [(1R*,2S*,5S*)-3-(2'-fluoro-biphenyl-2-carbonyl)-3-aza-bicyclo[3.1.0]hex-2-ylmethyl]-amide;
benzofuran-4-carboxylic acid [(1R*,2S*,5S*)-3-(4'-fluoro-biphenyl-2-carbonyl)-3-aza-bicyclo[3.1.0]hex-2-ylmethyl]-amide;
benzofuran-4-carboxylic acid [(1R*,2S*,5S*)-3-(2'-chloro-biphenyl-2-carbonyl)-3-aza-bicyclo[3.1.0]hex-2-ylmethyl]-amide;
benzofuran-4-carboxylic acid [(1R*,2S*,5S*)-3-(3'-chloro-biphenyl-2-carbonyl)-3-aza-bicyclo[3.1.0]hex-2-ylmethyl]-amide;
benzofuran-4-carboxylic acid [(1R*,2S*,5S*)-3-(4'-chloro-biphenyl-2-carbonyl)-3-aza-bicyclo[3.1.0]hex-2-ylmethyl]-amide;
benzofuran-4-carboxylic acid [(1R*,2S*,5S*)-3-(4'-methyl-biphenyl-2-carbonyl)-3-aza-bicyclo[3.1.0]hex-2-ylmethyl]-amide;
benzofuran-4-carboxylic acid [(1R*,2S*,5S*)-3-(3'-methyl-biphenyl-2-carbonyl)-3-aza-bicyclo[3.1.0]hex-2-ylmethyl]-amide;
benzofuran-4-carboxylic acid [(1R*,2S*,5S*)-3-(3'-methoxy-biphenyl-2-carbonyl)-3-aza-bicyclo[3.1.0]hex-2-ylmethyl]-amide;
benzofuran-4-carboxylic acid [(1R*,2S*,5S*)-3-(4'-methoxy-biphenyl-2-carbonyl)-3-aza-bicyclo[3.1.0]hex-2-ylmethyl]-amide;
benzofuran-4-carboxylic acid [(1R*,2S*,5S*)-3-(3'-trifluoromethyl-biphenyl-2-carbonyl)-3-aza-bicyclo[3.1.0]hex-2-ylmethyl]-amide;
benzofuran-4-carboxylic acid [(1R*,2S*,5S*)-3-(2-pyridin-3-yl-benzoyl)-3-aza-bicyclo[3.1.0]hex-2-ylmethyl]-amide;
6-methyl-imidazo[2,1-b]thiazole-5-carboxylic acid [(1R*,2S*,5S*)-3-(2-methyl-5-p-tolyl-thiazole-4-carbonyl)-3-aza-bicyclo[3.1.0]hex-2-ylmethyl]-amide;
6-methyl-imidazo[2,1-b]thiazole-5-carboxylic acid [(1R*,2S*,5S*)-3-(2-methyl-5-m-tolyl-thiazole-4-carbonyl)-3-aza-bicyclo[3.1.0]hex-2-ylmethyl]-amide;
6-methyl-imidazo[2,1-b]thiazole-5-carboxylic acid {(1R*,2S*,5S*)-3-[5-(3-fluorophenyl)-2-methyl-thiazole-4-carbonyl]-3-aza-bicyclo[3.1.0]hex-2-ylmethyl}-amide;
6-methyl-imidazo[2, 1-b]thiazole-5-carboxylic acid {(1R*,2S*,5S*)-3-[5-(4-ethylphenyl)-2-methyl-thiazole-4-carbonyl]-3-aza-bicyclo[3.1.0]hex-2-ylmethyl}-amide;
6-methyl-imidazo[2,1-b]thiazole-5-carboxylic acid {(1R*,2S*,5S*)-3-[5-(4-fluorophenyl)-2-methyl-thiazole-4-carbonyl]-3-aza-bicyclo[3.1.0]hex-2-ylmethyl}-amide;
6-methyl-imidazo[2,1-b]thiazole-5-carboxylic acid [(1R*,2S*,5S*)-3-(biphenyl-2-carbonyl)-3-aza-bicyclo[3.1.0]hex-2-ylmethyl]-amide;
{(1R*,2S*,5S*)-2-[(6,7-difluoro-quinoxalin-2-ylamino)-methyl]-3-aza-bicyclo[3.1.0]hex-3-yl}-(2-methyl-5-m-tolyl-thiazol-4-yl)-methanone;
{(1R*,2S*,5S*)-2-[(6,7-difluoro-quinoxalin-2-ylamino)-methyl]-3-aza-bicyclo[3.1.0]hex-3-yl}-[5-(4-fluoro-phenyl)-2-methyl-thiazol-4-yl]-methanone;
{(1R*,2S*,5S*)-2-[(5-bromo-pyrimidin-2-ylamino)-methyl]-3-aza-bicyclo[3.1.0]hex-3-yl}-(2-methyl-5-m-tolyl-thiazol-4-yl)-methanone;
naphthalene-2-carboxylic acid [(1R*,2S*,5S*)-3-(2-methyl-5-m-tolyl-thiazole-4-carbonyl)-3-aza-bicyclo[3.1.0]hex-2-ylmethyl]-amide;
naphthalene-1-carboxylic acid [(1R*,2S*,5S*)-3-(2-methyl-5-m-tolyl-thiazole-4-carbonyl)-3-aza-bicyclo[3.1.0]hex-2-ylmethyl]-amide; 2,3-dihydro-benzofuran-7-carboxylic acid [(1R*,2S*,5S*)-3-(2-methyl-5-m-tolyl-thiazole-4-carbonyl)-3-aza-bicyclo[3.1.0]hex-2-ylmethyl]-amide; 1H-indole-5-carboxylic acid [(1R*,2S*,5S*)-3-(2-methyl-5-m-tolyl-thiazole-4-carbonyl)-3-aza-bicyclo[3.1.0]hex-2-ylmethyl]-amide;
2-hydroxy-N-[(1R*,2S*,5S*)-3-(2-methyl-5-m-tolyl-thiazole-4-carbonyl)-3-aza bicyclo[3.1.0]hex-2-ylmethyl]-benzamide;
2-bromo-4-methyl-thiazole-5-carboxylic acid [(1R*,2S*,5S*)-3-(2-methyl-5-m-tolyl-thiazole-4-carbonyl)-3-aza-bicyclo[3.1.0]hex-2-ylmethyl]-amide;
furan-3-carboxylic acid [(1R*,2S*,5S*)-3-(2-methyl-5-m-tolyl-thiazole-4-carbonyl)-3-aza-bicyclo[3.1.0]hex-2-ylmethyl]-amide;
3,5-dimethyl-isoxazole-4-carboxylic acid [(1R*,2S*,5S*)-3-(2-methyl-5-m-tolyl-thiazole-4-carbonyl)-3-aza-bicyclo[3.1.0]hex-2-ylmethyl]-amide; 3,5-dimethoxy-N-[(1R*,2S*,5S*)-3-(2-methyl-5-m-tolyl-thiazole-4-carbonyl)-3-aza-bicyclo[3.1.0]hex-2-ylmethyl]-benzamide;
benzo[1,3]dioxole-5-carboxylic acid [(1R*,2S*,5S*)-3-(2-methyl-5-m-totyl-thiazole-4-carbonyl)-3-aza-bicyclo[3.1.0]hex-2-ylmethyl]-amide; 2,4-dimethoxy-N-[(1R*,2S*,5S*)-3-(2-methyl-5-m-tolyl-thiazole-4-carbonyl)-3-aza-bicyclo[3.1.0]hex-2-ylmethyl]-benzamide;
2,4-dimethyl-thiazole-5-carboxylic acid [(1R*,2S*,5S*)-3-(2-methyl-5-m-tolyl-thiazole-4-carbonyl)-3-aza-bicyclo[3.1.0]hex-2-ylmethyl]-amide; 1-methyl-1H-indole-2-carboxylic acid [(1R*,2S*,5S*)-3-(2-methyl-5-m-tolyl-thiazole-4-carbonyl)-3-aza-bicyclo[3.1.0]hex-2-ylmethyl]-amide;
3H-benzoimidazole-5-carboxylic acid [(1R*,2S*,5S*)-3-(2-methyl-5-m-tolyl-thiazole-4-carbonyl)-3-aza-bicyclo[3.1.0]hex-2-ylmethyl]-amide;
benzo[2,1,3]oxadiazole-5-carboxylic acid [(1R*,2S*,5S*)-3-(2-methyl-5-m-tolyl-thiazole-4-carbonyl)-3-aza-bicyclo[3.1.0]hex-2-ylmethyl]-amide; 2,3-dihydro-benzo[1,4]dioxine-5-carboxylic acid [(1R*,2S*,5S*)-3-(2-methyl-5-m-tolyl-thiazole-4-carbonyl)-3-aza-bicyclo[3.1.0]hex-2-ylmethyl]-amide; 5-chloro-1,3-dimethyl-1H-pyrazole-4-carboxylic acid [(1R*,2S*,5S*)-3-(2-methyl-5-m-tolyl-thiazole-4-carbonyl)-3-aza-bicyclo[3.1.0]hex-2-ylmethyl]-amide; benzo[b]thiophene-2-carboxylic acid [(1R*,2S*,5S*)-3-(2-methyl-5-m-tolyl-thiazole-4-carbonyl)-3-aza-bicyclo[3.1.0]hex-2-ylmethyl]-amide; 5-tert-butyl-2-methyl-2H-pyrazole-3-carboxylic acid [(1R*,2S*,5S*)-3-(2-methyl-5-m-tolyl-thiazole-4-carbonyl)-3-aza-bicyclo[3.1.0]hex-2-ylmethyl]-amide; 1-methyl-1H-indazole-3-carboxylic acid [(1R*,2S*,5S*)-3-(2-methyl-5-m-tolyl-thiazole-4-carbonyl)-3-aza-bicyclo[3.1.0]hex-2-ylmethyl]-amide; 1-methyl-1H-pyrrole-2-carboxylic acid [(1R*,2S*,5S*)-3-(2-methyl-5-m-tolyl-thiazole-4-carbonyl)-3-aza-bicyclo[3.1.0]hex-2-ylmethyl]-amide; 2,8-dimethyl-imidazo[1,2-a]pyridine-3-carboxylic acid [(1R*,2S*,5S*)-3-(2-methyl-5-m-tolyl-thiazole-4-carbonyl)-3-aza-bicyclo[3.1.0]hex-2-ylmethyl]-amide; 6-isobutyl-5-methyl-N-[(1R*,2S*,5S*)-3-(2-methyl-5-m-tolyl-thiazole-4-carbonyl)-3-aza-bicyclo[3.1.0]hex-2-ylmethyl]-nicotinamide; pyrazolo[1,5-a]pyridine-3-carboxylic acid [(1R*,2S*,5S*)-3-(2-methyl-5-m-tolyl-thiazole-4-carbonyl)-3-aza-bicyclo[3.1.0]hex-2-ylmethyl]-amide; benzo[d]isoxazole-3-carboxylic acid [(1R*,2S*,5S*)-3-(2-methyl-5-m-tolyl-thiazole-4-carbonyl)-3-aza-bicyclo[3.1.0]hex-2-ylmethyl]-amide; 6-fluoro-4H-benzo[1,3]dioxine-8-carboxylic acid [(1R*,2S*,5S*)-3-(2-methyl-5-m-tolyl-thiazole-4-carbonyl)-3-aza-bicyclo[3.1.0]hex-2-ylmethyl]-amide; 2,3-dihydro-benzofuran-4-carboxylic acid [(1R*,2S*,5S*)-3-(2-methyl-5-m-tolyl-thiazole-4-carbonyl)-3-aza-bicyclo[3.1.0]hex-2-ylmethyl]-amide; isoquinoline-1-carboxylic acid [(1R*,2S*,5S*)-3-(2-methyl-5-m-tolyl-thiazole-4-carbonyl)-3-aza-bicyclo[3.1.0]hex-2-ylmethyl]-amide;
quinoline-8-carboxylic acid [(1R*,2S*,5S*)-3-(2-methyl-5-m-tolyl-thiazole-4-carbonyl)-3-aza-bicyclo[3.1.0]hex-2-ylmethyl]-amide;
quinoline-2-carboxylic acid [(1R*,2S*,5S*)-3-(2-methyl-5-m-tolyl-thiazole-4-carbonyl)-3-aza-bicyclo[3.1.0]hex-2-ylmethyl]-amide; imidazo[2,1-b]thiazole-5-carboxylic acid [(1R*,2S*,5S*)-3-(2-methyl-5-m-tolyl-thiazole-4-carbonyl)-3-aza-bicyclo[3.1.0]hex-2-ylmethyl]-amide; 3-methyl-imidazo[2,1-b]thiazole-2-carboxylic acid [(1R*,2S*,5S*)-3-(2-methyl-5-m-tolyl-thiazole-4-carbonyl)-3-aza-bicyclo[3.1.0]hex-2-ylmethyl]-amide;
1-methyl-1H-indole-3-carboxylic acid [(1R*,2S*,5S*)-3-(2-methyl-5-m-tolyl-thiazole-4-carbonyl)-3-aza-bicyclo[3.1.0]hex-2-ylmethyl]-amide;
1H-indole-3-carboxylic acid [(1R*,2S*,5S*)-3-(2-methyl-5-m-tolyl-thiazole-4-carbonyl)-3-aza-bicyclo[3.1.0]hex-2-ylmethyl]-amide;
1H-indazole-3-carboxylic acid [(1R*,2S*,5S*)-3-(2-methyl-5-m-tolyl-thiazole-4-carbonyl)-3-aza-bicyclo[3.1.0]hex-2-ylmethyl]-amide; imidazo[1,2-a]pyridine-3-carboxylic acid [(1R*,2S*,5S*)-3-(2-methyl-5-m-tolyl-thiazole-4-carbonyl)-3-aza-bicyclo[3.1.0]hex-2-ylmethyl]-amide; 2,3-dihydro-thieno[3,4-b][1,4]dioxine-5-carboxylic acid [(1R*,2S*,5S*)-3-(2-methyl-5-m-tolyl-thiazole-4-carbonyl)-3-aza-bicyclo[3.1.0]hex-2-ylmethyl]-amide; 3-bromo-N-[(1R*,2S*,5S*)-3-(2-methyl-5-m-tolyl-thiazole-4-carbonyl)-3-aza-bicyclo[3.1.0]hex-2-ylmethyl]-benzamide;
N-[(1R*,2S*,5S*)-3-(2-methyl-5-m-tolyl-thiazole-4-carbonyl)-3-aza-bicyclo[3.1.0]hex-2-ylmethyl]-3-trifluoromethyl-benzamide;
3-methoxy-N-[(1R*,2S*,5S*)-3-(2-methyl-5-m-tolyl-thiazole-4-carbonyl)-3-aza-bicyclo[3.1.0]hex-2-ylmethyl]-benzamide; 3-fluoro-4-methoxy-N-[(1R*,2S*,5S*)-3-(2-methyl-5-m-tolyl-thiazole-4-carbonyl)-3-aza-bicyclo[3.1.0]hex-2-ylmethyl]-benzamide;
3,4-dichloro-N-[(1R*,2S*,5S*)-3-(2-methyl-5-m-tolyl-thiazole-4-carbonyl)-3-aza-bicyclo[3.1.0]hex-2-ylmethyl]-benzamide; 2-chloro-4,5-difluoro-N-[(1R*,2S*,5S*)-3-(2-methyl-5-m-tolyl-thiazole-4-carbonyl)-3-aza-bicyclo[3.1.0]hex-2-ylmethyl]-benzamide;
2-fluoro-5-methyl-N-[(1R*,2S*,5S*)-3-(2-methyl-5-m-tolyl-thiazole-4-carbonyl)-3-aza-bicyclo[3.1.0]hex-2-ylmethyl]-benzamide;
3-fluoro-2-methyl-N-[(1R*,2S*,5S*)-3-(2-methyl-5-m-tolyl-thiazole-4-carbonyl)-3-aza-bicyclo[3.1.0]hex-2-ylmethyl]-benzamide;
5-fluoro-2-methoxy-N-[(1R*,2S*,5S*)-3-(2-methyl-5-m-tolyl-thiazole-4-carbonyl)-3-aza-bicyclo[3.1.0]hex-2-ylmcthyl]-benzamide;
2-chloro-3-fluoro-N-[(1R*,2S*,5S*)-3-(2-methyl-5-m-tolyl-thiazole-4-carbonyl)-3-aza-bicyclo[3.1.0]hex-2-ylmethyl]-benzamide;
2,5-dimethyl-N-[(1R*,2S*,5S*)-3-(2-methyl-5-m-tolyl-thiazole-4-carbonyl)-3-aza-bicyclo[3.1.0]hex-2-ylmethyl]-benzamide;
3,4-dimethyl-N-[(1R*,2S*,5S*)-3-(2-methyl-5-m-tolyl-thiazole-4-carbonyl)-3-aza-bicyclo[3.1.0]hex-2-ylmethyl]-benzamide;
2,5-dimethoxy-N-[(1R*,2S*,5S*)-3-(2-methyl-5-m-tolyl-thiazole-4-carbonyl)-3-aza-bicyclo[3.1.0]hex-2-ylmethyl]-benzamide;
N-[(1R*,2S*,5S*)-3-(2-methyl-5-m-tolyl-thiazole-4-carbonyl)-3-aza-bicyclo[3.1.0]hex-2-ylmethyl]-isophthalamic acid methyl ester;
2-chloro-4-fluoro-N-[(1R*,2S*,5S*)-3-(2-methyl-5-m-tolyl-thiazole-4-carbonyl)-3-aza-bicyclo[3.1.0]hex-2-ylmethyl]-benzamide; 2-chloro-3-methyl-N-[(1R*,2S*,5S*)-3-(2-methyl-5-m-tolyl-thiazole-4-carbonyl)-3-aza-bicyclo[3.1.0]hex-2-ylmethyl]-benzamide;
3,5-dichloro-4-hydroxy-N-[(1R*,2S*,5S*)-3-(2-methyl-5-m-tolyl-thiazole-4-carbonyl)-3-aza-bicyclo[3.1.0]hex-2-ylmethyl]-benzamide;
2,4-dichloro-N-[(1R*,2S*,5S*)-3-(2-methyl-5-m-tolyl-thiazole-4-carbonyl)-3-aza-bicyclo[3.1.0]hex-2-ylmethyl]-benzamide;
4-methyl-N-[(1R*,2S*,5S*)-3-(2-methyl-5-m-tolyl-thiazole-4-carbonyl)-3-aza-bicyclo[3.1.0]hex-2-ylmethyl]-3-trifluoromethyl-benzamide; 4-methoxy-2-methyl-N-[( 1R*,2S*,5S*)-3-(2-methyl-5-m-tolyl-thiazole-4-carbonyl)-3-aza-bicyclo[3.1.0]hex-2-ylmethyl]-benzamide;
4-ethyl-N-[(1R*,2S*,5S*)-3-(2-methyl-5-m-tolyl-thiazole-4-carbonyl)-3-aza-bicyclo[3.1.0]hex-2-ylmethyl]-benzamide;
4-methoxy-3-methyl-N-[(1R*,2S*,5S*)-3-(2-methyl-5-m-tolyl-thiazole-4-carbonyl)-3-aza-bicyclo[3.1.0]hex-2-ylmethyl]-benzamide;
3,5-dimethyl-N-[(1R*,2S*,5S*)-3-(2-methyl-5-m-tolyl-thiazole-4-carbonyl)-3-aza-bicyclo[3.1.0]hex-2-ylmethyl]-benzamide;
5-bromo-2-chloro-N-[(1R*,2S*,5S*)-3-(2-methyl-5-m-tolyl-thiazole-4-carbonyl)-3-aza-bicyclo[3.1.0]hex-2-ylmethyl]-benzamide;
3-cyano-N-[(1R*,2S*,5S*)-3-(2-methyl-5-m-tolyl-thiazole-4-carbonyl)-3-aza-bicyclo[3.1.0]hex-2-ylmethyl]-benzamide; 4-cyano-N-[(1R*,2S*,5S*)-3-(2-methyl-5-m-tolyl-thiazole-4-carbonyl)-3-aza-bicyclo[3.1.0]hex-2-ylmethyl]-benzamide; 4-chloro-N-[(1R*,2S*,5S*)-3-(2-methyl-5-m-tolyl-thiazole-4-carbonyl)-3-aza-bicyclo[3.1.0]hex-2-ylmethyl]-benzamide; 3-iodo-N-[(1R*,2S*,5S*)-3-(2-methyl-5-m-tolyl-thiazole-4-carbonyl)-3-aza-bicyclo[3.1.0]hex-2-ylmethyl]-benzamide; 2-bromo-3-methyl-N-[(1R*,2S*,5S*)-3-(2-methyl-5-m-tolyl-thiazole-4-carbonyl)-3-aza-bicyclo[3.1.0]hex-2-ylmethyl]-benzamide; 5-methyl-imidazo[2,1-b]thiazole-6-carboxylic acid [(1R*,2S*,5S*)-3-(2-methyl-5-m-tolyl-thiazole-4-carbonyl)-3-aza-bicyclo[3.1.0]hex-2-ylmethyl]-amide; 3,5-dimethyl-imidazo[2,1-b]thiazole-6-carboxylic acid [(1R*,2S*,5S*)-3-(2-methyl-5-m-tolyl-thiazole-4-carbonyl)-3-aza-bicyclo[3.1.0]hex-2-ylmethyl]-amide; 2,6-dimethyl-imidazo[2,1-b]thiazole-5-carboxylic acid [(1R*,2S*,5S*)-3-(2-methyl-5-m-tolyl-thiazole-4-carbonyl)-3-aza-bicyclo[3.1.0]hex-2-ylmethyl]-amide; 2,3,5-trimethyl-imidazo[2,1-b]thiazole-6-carboxylic acid [(1R*,2S*,5S*)-3-(2-methyl-5-m-tolyl-thiazole-4-carbonyl)-3-aza-bicyclo[3.1.0]hex-2-ylmethyl]-amide; 2-methyl-imidazo[2,1-b]thiazole-5-carboxylic acid [(1R*,2S*,5S*)-3-(2-methyl-5-m-tolyl-thiazole-4-carbonyl)-3-aza-bicyclo[3.1.0]hex-2-ylmethyl]-amide; 6-trifluoromethyl-imidazo[2,1-b]thiazole-5-carboxylic acid [(1R*,2S*,5S*)-3-(2-methyl-5-m-tolyl-thiazole-4-carbonyl)-3-aza-bicyclo[3.1.0]hex-2-ylmethyl]-amide; 3,6-dimethyl-imidazo[2,1-b]thiazole-5-carboxylic acid [(1R*,2S*,5S*)-3-(2-methyl-5-m-tolyl-thiazole-4-carbonyl)-3-aza-bicyclo[3.1.0]hex-2-ylmethyl]-amide; 3-methyl-imidazo[2,1-b]thiazole-5-carboxylic acid [(1R*,2S*,5S*)-3-(2-methyl-5-m-tolyl-thiazole-4-carbonyl)-3-aza-bicyclo[3.1.0]hex-2-ylmethyl]-amide; 6-chloro-imidazo[2,1-b]thiazole-5-carboxylic acid [(1R*,2S*,5S*)-3-(2-methyl-5-m-tolyl-thiazole-4-carbonyl)-3-aza-bicyclo[3.1.0]hex-2-ylmethyl]-amide; 2H-chromene-5-carboxylic acid [(1R*,2S*,5S*)-3-(2-methyl-5-m-tolyl-thiazole-4-carbonyl)-3-aza-bicyclo[3.1.0]hex-2-ylmethyl]-amide; 4-methyl-3,4-dihydro-2H-benzo[1,4]oxazine-8-carboxylic acid [(1R*,2S*,5S*)-3-(2-methyl-5-m-tolyl-thiazole-4-carbonyl)-3-aza-bicyclo[3.1.0]hex-2-ylmethyl]-amide;
chroman-8-carboxylic acid [(1R*,2S*,5S*)-3-(2-methyl-5-m-tolyl-thiazole-4-carbonyl)-3-aza-bicyclo[3.1.0]hex-2-ylmethyl]-amide; 3-oxo-3,4-dihydro-2H-benzo[1,4]oxazine-5-carboxylic acid [(1R*,2S*,5S*)-3-(2-methyl-5-m-tolyl-thiazole-4-carbonyl)-3-aza-bicyclo[3.1.0]hex-2-ylmethyl]-amide; 3,4-dihydro-2H-benzo[1,4]oxazine-8-carboxylic acid [(1R*,2S*,5S*)-3-(2-methyl-5-m-tolyl-thiazole-4-carbonyl)-3-aza-bicyclo[3.1.0]hex-2-ylmethyl]-amide; 3,4-dihydro-2H-benzo[1,4]oxazine-5-carboxylic acid [(1R*,2S*,5S*)-3-(2-methyl-5-m-tolyl-thiazole-4-carbonyl)-3-aza-bicyclo[3.1.0]hex-2-ylmethyl]-amide; 2-methyl-benzofuran-4-carboxylic acid [(1R*,2S*,5S*)-3-(2-methyl-5-m-tolyl-thiazole-4-carbonyl)-3-aza-bicyclo[3.1.0]hex-2-ylmethyl]-amide; benzo[d]isoxazole-3-carboxylic acid {(1R*,2S*,5S*)-3-[5-(3-chloro-phenyl)-2-methyl-thiazole-4-carbonyl]-3-aza-bicyclo[3.1.0]hex-2-ylmethyl}-amide; 2,3-dihydro-benzo[1,4]dioxine-5-carboxylic acid {(1R*,2S*,5S*)-3-[5-(3-chlorophenyl)-2-methyl-thiazole-4-carbonyl]-3-aza-bicyclo[3.1.0]hex-2-ylmethyl}-amide; 6-fluoro-4H-benzo[1,3]dioxine-8-carboxylic acid {(1R*,2S*,5S*)-3-[5-(3-chlorophenyl)-2-methyl-thiazole-4-carbonyl]-3-aza-bicyclo[3.1.0]hex-2-ylmethyl}-amide; 2,3-dihydro-benzofuran-4-carboxylic acid {(1R*,2S*,5S*)-3-[5-(3-chloro-phenyl)-2-methyl-thiazole-4-carbonyl]-3-aza-bicyclo[3.1.0]hex-2-ylmethyl -amide; isoquinoline-1-carboxylic acid {(1R*,2S*,5S*)-3-[5-(3-chloro-phenyl)-2-methylthiazole-4-carbonyl]-3-aza-bicyclo[3.1.0]hex-2-ylmethyl}-amide; quinoline-8-carboxylic acid {(1R*,2S*,5S*)-3-[5-(3-chloro-phenyl)-2-methylthiazole-4-carbonyl]-3-aza-bicyclo[3.1.0]hex-2-ylmethyl}-amide; quinoline-2-carboxylic acid {(1R*,2S*,5S*)-3-[5-(3-chloro-phenyl)-2-methylthiazole-4-carbonyl]-3-aza-bicyclo[3.1.0]hex-2-ylmethyl}-amide; imidazo[2,1-b]thiazole-5-carboxylic acid {(1R*,2S*,5S*)-3-[5-(3-chloro-phenyl)-2-methyl-thiazole-4-carbonyl]-3-aza-bicyclo[3.1.0]hex-2-ylmethyl}-amide; 3-methyl-imidazo[2,1-b]thiazole-2-carboxylic acid {(1R*,2S*,5S*)-3-[5-(3-chlorophenyl)-2-methyl-thiazole-4-carbonyl]-3-aza-bicyclo[3.1.0]hex-2-ylmethyl}-amide; 1-methyl-1H-indole-3-carboxylic acid {(1R*,2S*,5S*)-3-[5-(3-chloro-phenyl)-2-methyl-thiazole-4-carbonyl]-3-aza-bicyclo[3.1.0]hex-2-ylmethyl -amide; 1,2-dimethyl-1H-indole-3-carboxylic acid {(1R*,2S*,5S*)-3-[5-(3-chloro-phenyl)-2-methyl-thiazole-4-carbonyl]-3-aza-bicyclo[3.1.0]hex-2-ylmethyl} -amide;
1H-indole-3-carboxylic acid {(1R*,2S*,5S*)-3-[5-(3-chloro-phenyl)-2-methylthiazole-4-carbonyl]-3-aza-bicyclo[3.1.0]hex-2-ylmethyl}-amide; 1H-indazole-3-carboxylic acid {(1R*,2S*,5S*)-3-[5-(3-chloro-phenyl)-2-methylthiazole-4-carbonyl]-3-aza-bicyclo[3.1.0]hex-2-ylmethyl}-amide; imidazo[1,2-a]pyridine-3-carboxylic acid {(1R*,2S*,5S*)-3-[5-(3-chloro-phenyl)-2-methyl-thiazole-4-carbonyl]-3-aza-bicyclo[3.1.0]hex-2-ylmethyl}-amide; 2,5-dimethyl-2H-pyrazole-3-carboxylic acid {(1R*,2S*,5S*)-3-[5-(3-chloro-phenyl)-2-methyl-thiazole-4-carbonyl]-3-aza-bicyclo[3.1.0]hex-2-ylmethyl}-amide; 2-ethyl-5-methyl-2H-pyrazole-3-carboxylic acid {(1R*,2S*,5S*)-3-[5-(3-chlorophenyl)-2-methyl-thiazole-4-carbonyl]-3-aza-bicyclo[3.1.0]hex-2-ylmethyl}-amide; 2,3-dihydro-thieno[3,4-b][1,4]dioxine-5-carboxylic acid {(1R*,2S*,5S*)-3-[5-(3-chloro-phenyl)-2-methyl-thiazole-4-carbonyl]-3-aza-bicyclo[3.1.0]hex-2-ylmethyl}-amide;
3-bromo-N-{(1R*,2S*,5S*)-3-[5-(3-chloro-phenyl)-2-methyl-thiazole-4-carbonyl]-3-aza-bicyclo[3.1.0]hex-2-ylmethyl}-benzamide; N- {(1R*,2S*,5S*)-3-[5-(3-chloro-phenyl)-2-methyl-thiazole-4-carbonyl]-3-aza-bicyclo[3.1.0]hex-2-ylmethyl}-3-trifluoromethyl-benzamide;
N-{(1R*,2S*,5S*)-3-[5-(3-chloro-phenyl)-2-methyl-thiazole-4-carbonyl]-3-aza-bicyclo[3.1.0]hex-2-ylmethyl}-3-methoxy-benzamide;
3-Methyl-imidazo[2,1-b]thiazole-5-carboxylic acid {(1R*,2S*,5S*)-3-[5-(3-fluorophenyl)-2-methyl-thiazole-4-carbonyl]-3-aza-bicyclo[3.1.0]hex-2-ylmethyl}-amide;
2-methyl-imidazo[2,1-b]thiazole-5-carboxylic acid {(1R*,2S*,5S*)-3-[5-(3-fluorophenyl)-2-methyl-thiazole-4-carbonyl]-3-aza-bicyclo[3.1.0]hex-2-ylmethyl} -amide;
imidazo[2,1-b]thiazole-5-carboxylic acid {(1R*,2S*,5S*)-3-[5-(3-fluoro-phenyl)-2-methyl-thiazole-4-carbonyl]-3-aza-bicyclo[3.1.0]hex-2-ylmethyl}-amide;
1-methyl-1H-indole-3-carboxylic acid {(1R*,2S*,5S*)-3-[5-(3-fluoro-phenyl)-2-methyl-thiazole-4-carbonyl]-3-aza-bicyclo[3.1.0]hex-2-ylmethyl}-amide;
3-methyl-imidazo[2,1-b]thiazole-2-carboxylic acid {(1R*,2S*,5S*)-3-[5-(3-fluorophenyl)-2-methyl-thiazole-4-carbonyl]-3-aza-bicyclo[3.1.0]hex-2-ylmethyl}-amide;
1-ethyl-3-methyl-1H-pyrazole-4-carboxylic acid {(1R*,2S*,5S*)-3-[5-(3-fluorophenyl)-2-methyl-thiazole-4-carbonyl]-3-aza-bicyclo[3.1.0]hex-2-ylmethyl} -amide;
5-tert-butyl-2-methyl-2H-pyrazole-3-carboxylic acid {(1R*,2S*,5S*)-3-[5-(3-fluorophenyl)-2-methyl-thiazole-4-carbonyl]-3-aza-bicyclo[3.1.0]hex-2-ylmethyl}-amide; isoquinoline-1-carboxylic acid {(1R*,2S*,5S*)-3-[5-(3-fluoro-phenyl)-2-methylthiazole-4-carbonyl]-3-aza-bicyclo[3.1.0]hex-2-ylmethyl}-amide; 1H-indazole-3-carboxylic acid {(1R*,2S*,5S*)-3-[5-(3-fluoro-phenyl)-2-methylthiazole-4-carbonyl]-3-aza-bicyclo[3.1.0]hex-2-ylmethyl}-amide; 4-methoxy-quinoline-2-carboxylic acid {(1R*,2S*,5S*)-3-[5-(3-fluoro-phenyl)-2-methyl-thiazole-4-carbonyl]-3-aza-bicyclo[3.1.0]hex-2-ylmethyl}-amide; quinoline-2-carboxylic acid {(1R*,2S*,5S*)-3-[5-(3-fluoro-phenyl)-2-methylthiazole-4-carbonyl]-3-aza-bicyclo[3.1.0]hex-2-ylmethyl}-amide; 1H-indole-3-carboxylic acid {(1R*,2S*,5S*)-3-[5-(3-fluoro-phenyl)-2-methylthiazole-4-carbonyl]-3-aza-bicyclo[3.1.0]hex-2-ylmethyl}-amide; 6-fluoro-4H-benzo[1,3]dioxine-8-carboxylic acid {(1R*,2S*,5S*)-3-[5-(3-fluorophenyl)-2-methyl-thiazole-4-carbonyl]-3-aza-bicyclo[3.1.0]hex-2-ylmethyl -amide; benzo[d]isoxazole-3-carboxylic acid {(1R*,2S*,5S*)-3-[5-(3-fluoro-phenyl)-2-methyl-thiazole-4-carbonyl]-3-aza-bicyclo[3.1.0]hex-2-ylmethyl}-amide; benzo[1,3]dioxole-4-carboxylic acid {(1R*,2S*,5S*)-3-[5-(3-fluoro-phenyl)-2-methyl-thiazole-4-carbonyl]-3-aza-bicyclo[3.1.0]hex-2-ylmethyl -amide; 2-ethyl-5-methyl-2H-pyrazole-3-carboxylic acid {(1R*,2S*,5S*)-3-[5-(3-fluorophenyl)-2-methyl-thiazole-4-carbonyl]-3-aza-bicyclo[3.1.0]hex-2-ylmethyl}-amide; imidazo[1,2-a]pyridine-3-carboxylic acid {(1R*,2S*,5S*)-3-[5-(3-fluoro-phenyl)-2-methyl-thiazole-4-carbonyl]-3-aza-bicyclo[3.1.0]hex-2-ylmethyl}-amide; 1-methyl-5-trifluoromethyl-1H-pyrazole-4-carboxylic acid {(1R*,2S*,5S*)-3-[5-(3-fluoro-phenyl)-2-methyl-thiazole-4-carbonyl]-3-aza-bicyclo[3.1.0]hex-2-ylmethyl}-amide;
1,5-dimethyl-1H-pyrazole-3-carboxylic acid {(1R*,2S*,5S*)-3-[5-(3-fluoro-phenyl)-2-methyl-thiazole-4-carbonyl]-3-aza-bicyclo[3.1.0]hex-2-ylmethyl}-amide; 2,5-dimethyl-2H-pyrazole-3-carboxylic acid {(1R*,2S*,5S*)-3-[5-(3-fluoro-phenyl)-2-methyl-thiazole-4-carbonyl]-3-aza-bicyclo[3.1.0]hex-2-ylmethyl}-amide; 2,5-dimethyl-oxazole-4-carboxylic acid {(1R*,2S*,5S*)-3-[5-(3-fluoro-phenyl)-2-methyl-thiazole-4-carbonyl]-3-aza-bicyclo[3.1.0]hex-2-ylmethyl}-amide;
2,3-dihydro-benzo[1,4]dioxine-5-carboxylic acid {(1R*,2S*,5S*)-3-[5-(3-fluorophenyl)-2-methyl-thiazole-4-carbonyl]-3-aza-bicyclo[3.1.0]hex-2-ylmethyl}-amide;
2,3-dihydro-benzofuran-4-carboxylic acid {(1R*,2S*,5S*)-3-[5-(3-fluoro-phenyl)-2-methyl-thiazole-4-carbonyl]-3-aza-bicyclo[3.1.0]hex-2-ylmethyl}-amide;
1,3-dimethyl-1H-pyrazole-4-carboxylic acid {(1R*,2S*,5S*)-3-[5-(3-fluoro-phenyl)-2-methyl-thiazole-4-carbonyl]-3-aza-bicyclo[3.1.0]hex-2-ylmethyl}-amide;
7-fluoro-1H-indole-2-carboxylic acid {(1R*,2S*,5S*)-3-[5-(3-fluoro-phenyl)-2-methyl-thiazole-4-carbonyl]-3-aza-bicyclo[3.1.0]hex-2-ylmethyl}-amide;
2-trifluoromethyl-1H-benzoimidazole-5-carboxylic acid {(1R*,2S*,5S*)-3-[5-(3-fluoro-phenyl)-2-methyl-thiazole-4-carbonyl]-3-aza-bicyclo[3.1.0]hex-2-ylmethyl}-amide;
3-bromo-N-{(1R*,2S*,5S*)-3-[5-(3-fluoro-phenyl)-2-methyl-thiazole-4-carbonyl]-3-aza-bicyclo [3.1.0]hex-2-ylmethyl}-benzamide;
N- {(1R*,2S*,5S*)-3-[5-(3-fluoro-phenyl)-2-methyl-thiazole-4-carbonyl]-3-aza-bicyclo[3.1.0]hex-2-ylmethyl}-3-trifluoromethyl-benzamide;
benzo[d]isoxazole-3-carboxylic acid [(1R*,2S*,5S*)-3-(2-amino-5-m-tolyl-thiazole-4-carbonyl)-3-aza-bicyclo[3.1.0]hex-2-ylmethyl]-amide;
2,3-dihydro-benzo[1,4]dioxine-5-carboxylic acid [(1R*,2S*,5S*)-3-(2-amino-5-m-tolyl-thiazole-4-carbonyl)-3-aza-bicyclo[3.1.0]hex-2-ylmethyl]-amide;
6-fluoro-4H-benzo[1,3]dioxine-8-carboxylic acid [(1R*,2S*,5S*)-3-(2-amino-5-m-tolyl-thiazole-4-carbonyl)-3-aza-bicyclo[3.1.0]hex-2-ylmethyl]-amide;
2,3-dihydro-benzofuran-4-carboxylic acid [(1R*,2S*,5S*)-3-(2-amino-5-m-tolyl-thiazole-4-carbonyl)-3-aza-bicyclo[3.1.0]hex-2-ylmethyl]-amide;
2,2-dimethyl-2,3-dihydro-benzofuran-7-carboxylic acid [(1R*,2S*,5S*)-3-(2-amino-5-m-tolyl-thiazole-4-carbonyl)-3-aza-bicyclo[3.1.0]hex-2-ylmethyl]-amide;
isoquinoline-1-carboxylic acid [(1R*,2S*,5S*)-3-(2-amino-5-m-tolyl-thiazole-4-carbonyl)-3-aza-bicyclo[3.1.0]hex-2-ylmethyl]-amide;
quinoline-8-carboxylic acid [(1R*,2S*,5S*)-3-(2-amino-5-m-tolyl-thiazole-4-carbonyl)-3-aza-bicyclo[3.1.0]hex-2-ylmethyl]-amide;
imidazo[2,1-b]thiazole-5-carboxylic acid [(1R*,2S*,5S*)-3-(2-amino-5-m-tolyl-thiazole-4-carbonyl)-3-aza-bicyclo[3.1.0]hex-2-ylmethyl]-amide;
3-methyl-imidazo[2,1-b]thiazole-2-carboxylic acid [(1R*,2S*,5S*)-3-(2-amino-5-m-tolyl-thiazole-4-carbonyl)-3-aza-bicyclo[3.1.0]hex-2-ylmethyl]-amide;
1-methyl-1H-indole-3-carboxylic acid [(1R*,2S*,5S*)-3-(2-amino-5-m-tolyl-thiazole-4-carbonyl)-3-aza-bicyclo[3.1.0]hex-2-ylmethyl]-amide;
1H-indole-3-carboxylic acid [(1R*,2S*,5S*)-3-(2-amino-5-m-tolyl-thiazole-4-carbonyl)-3-aza-bicyclo[3.1.0]hex-2-ylmethyl]-amide;
1H-indazole-3-carboxylic acid [(1R*,2S*,5S*)-3-(2-amino-5-m-tolyl-thiazole-4-carbonyl)-3-aza-bicyclo[3.1.0]hex-2-ylmethyl]-amide;
imidazo[1,2-a]pyridine-3-carboxylic acid [(1R*,2S*,5S*)-3-(2-amino-5-m-tolyl-thiazole-4-carbonyl)-3-aza-bicyclo[3.1.0]hex-2-ylmethyl]-amide;
2-ethyl-5-methyl-2H-pyrazole-3-carboxylic acid [(1R*,2S*,5S*)-3-(2-amino-5-m-tolyl-thiazole-4-carbonyl)-3-aza-bicyclo[3.1.0]hex-2-ylmethyl]-amide;
1-ethyl-3-methyl-1H-pyrazole-4-carboxylic acid [(1R*,2S*,5S*)-3-(2-amino-5-m-tolyl-thiazole-4-carbonyl)-3-aza-bicyclo[3.1.0]hex-2-ylmethyl]-amide;
2,3-dihydro-thieno[3,4-b][1,4]dioxine-5-carboxylic acid [(1R*,2S*,5S*)-3-(2-amino-5-m-tolyl-thiazole-4-carbonyl)-3-aza-bicyclo[3.1.0]hex-2-ylmethyl]-amide;
N-[(1R*,2S*,5S*)-3-(2-amino-5-m-tolyl-thiazole-4-carbonyl)-3-aza-bicyclo[3.1.0]hex-2-ylmethyl]-3-bromo-benzamide;
N-[(1R*,2S*,5S*)-3-(2-amino-5-m-tolyl-thiazole-4-carbonyl)-3-aza-bicyclo[3.1.0]hex-2-ylmethyl]-3-methoxy-benzamide;
benzo[d]isoxazole-3-carboxylic acid {(1R*,2S*,5S*)-3-[2-amino-5-(3-chlorophenyl)-thiazole-4-carbonyl]-3-aza-bicyclo[3.1.0]hex-2-ylmethyl}-amide;
2,3-dihydro-benzo[1,4]dioxine-5-carboxylic acid {(1R*,2S*,5S*)-3-[2-amino-5-(3-chloro-phenyl)-thiazole-4-carbonyl]-3-aza-bicyclo[3.1.0]hex-2-ylmethyl}-amide;
6-fluoro-4H-benzo[1,3]dioxine-8-carboxylic acid {(1R*,2S*,5S*)-3-[2-amino-5-(3-chloro-phenyl)-thiazole-4-carbonyl]-3-aza-bicyclo[3.1.0]hex-2-ylmethyl}-amide;
2,3-dihydro-benzofuran-4-carboxylic acid {(1R*,2S*,5S*)-3-[2-amino-5-(3-chlorophenyl)-thiazole-4-carbonyl]-3-aza-bicyclo[3.1.0]hex-2-ylmethyl}-amide;
isoquinoline-1-carboxylic acid {(1R*,2S*,5S*)-3-[2-amino-5-(3-chloro-phenyl)-thiazole-4-carbonyl]-3-aza-bicyclo[3.1.0]hex-2-ylmethyl}-amide;
quinoline-8-carboxylic acid {(1R*,2S*,5S*)-3-[2-amino-5-(3-chloro-phenyl)-thiazole-4-carbonyl]-3-aza-bicyclo[3.1.0]hex-2-ylmethyl}-amide;
imidazo[2,1-b]thiazole-5-carboxylic acid {(1R*,2S*,5S*)-3-[2-amino-5-(3-chlorophenyl)-thiazole-4-carbonyl]-3-aza-bicyclo[3.1.0]hex-2-ylmethyl}-amide; 1-methyl-1H-indole-3-carboxylic acid {(1R*,2S*,5S*)-3-[2-amino-5-(3-chlorophenyl)-thiazole-4-carbonyl]-3-aza-bicyclo[3.1.0]hex-2-ylmethyl}-amide;
1H-indole-3-carboxylic acid {(1R*,2S*,5S*)-3-[2-amino-5-(3-chloro-phenyl)-thiazole-4-carbonyl]-3-aza-bicyclo[3.1.0]hex-2-ylmethyl}-amide; 1H-indazole-3-carboxylic acid {(1R*,2S*,5S*)-3-[2-amino-5-(3-chloro-phenyl)-thiazole-4-carbonyl]-3-aza-bicyclo[3.1.0]hex-2-ylmethyl -amide; 2,5-dimethyl-2H-pyrazole-3-carboxylic acid {(1R*,2S*,5S*)-3-[2-amino-5-(3-chlorophenyl)-thiazole-4-carbonyl]-3-aza-bicyclo[3.1.0]hex-2-ylmethyl}-amide; 2-ethyl-5-methyl-2H-pyrazole-3-carboxylic acid {(1R*,2S*,5S*)-3-[2-amino-5-(3-chloro-phenyl)-thiazole-4-carbonyl]-3-aza-bicyclo[3.1.0]hex-2-ylmethyl -amide; 2,3-dihydro-thieno[3,4-b][1,4]dioxine-5-carboxylic acid {(1R*,2S*,5S*)-3-[2-amino-5-(3-chloro-phenyl)-thiazole-4-carbonyl]-3-aza-bicyclo[3.1.0]hex-2-ylmethyl}-amide; N-{(1R*,2S*,5S*)-3-[2-amino-5-(3-chloro-phenyl)-thiazole-4-carbonyl]-3-aza-bicyclo[3.1.0]hex-2-ylmethyl}-3-bromo-benzamide; N-{(1R*,2S*,5S*)-3-[2-amino-5-(3-chloro-phenyl)-thiazole-4-carbonyl]-3-aza-bicyclo[3.1.0]hex-2-ylmethyl}-3-methoxy-benzamide; 6-methyl-imidazo[2,1-b]thiazole-5-carboxylic acid [(1R*,2S*,5S*)-3-(3'-fluoro-5-methyl-biphenyl-2-carbonyl)-3-aza-bicyclo[3.1.0]hex-2-ylmethyl]-amide;
imidazo[1,2-a]pyridine-3-carboxylic acid [(1R*,2S*,5S*)-3-(3'-fluoro-5-methylbiphenyl-2-carbonyl)-3-aza-bicyclo[3.1.0]hex-2-ylmethyl]-amide; 2,3-dihydro-benzofuran-4-carboxylic acid [(1R*,2S*,5S*)-3-(3'-fluoro-5-methylbiphenyl-2-carbonyl)-3-aza-bicyclo[3.1.0]hex-2-ylmethyl]-amide; 2,3-dihydro-benzofuran-7-carboxylic acid [(1R*,2S*,5S*)-3-(3'-fluoro-5-methylbiphenyl-2-carbonyl)-3-aza-bicyclo[3.1.0]hex-2-ylmethyl]-amide; 6-methyl-imidazo[2,1-b]thiazole-5-carboxylic acid {(1R*,2S*,5S*)-3-[5-(3-cyanophenyl)-2-methyl-thiazole-4-carbonyl]-3-aza-bicyclo[3.1.0]hex-2-ylmethyl}-amide; benzofuran-4-carboxylic acid {(1R*,2S*,5S*)-3-[5-(3-cyano-phenyl)-2-methylthiazole-4-carbonyl]-3-aza-bicyclo[3.1.0]hex-2-ylmethyl}-amide; 2,3-dihydro-benzo[1,4]dioxine-5-carboxylic acid {(1R*,2S*,5S*)-3-[5-(3-cyanophenyl)-2-methyl-thiazole-4-carbonyl]-3-aza-bicyclo[3.1.0]hex-2-ylmethyl}-amide;
2,3-dihydro-benzo[1,4]dioxine-5-carboxylic acid [(1R*,2S*,5S*)-3-(5-methylbiphenyl-2-carbonyl)-3-aza-bicyclo[3.1.O]hex-2-ylmethyl]-amide;
2,3-dihydro-benzo[1,4]dioxine-5-carboxylic acid [(1R*,2S*,5S*)-3-(2'-fluoro-5-methyl-biphenyl-2-carbonyl)-3-aza-bicyclo[3.1.0]hex-2-ylmcthyl]-amide;
2,3-dihydro-benzo[1,4]dioxine-5-carboxylic acid [(1R*,2S*,5S*)-3-(5,3'-dimethylbiphenyl-2-carbonyl)-3-aza-bicyclo[3.1.0]hex-2-ylmethyl]-amide;
2,3-dihydro-benzo[1,4]dioxine-5-carboxylic acid [(1R*,2S*,5S*)-3-(3'-chloro-5-methyl-biphenyl-2-carbonyl)-3-aza-bicyclo[3.1.0]hex-2-ylmethyl]-amide;
2,3-dihydro-benzo[1,4]dioxine-5-carboxylic acid [(1R*,2S*,5S*)-3-(3'-methoxy-5-methyl-biphenyl-2-carbonyl)-3-aza-bicyclo[3.1.0]hex-2-ylmethyl]-amide;
2,3-dihydro-benzo[1,4]dioxine-5-carboxylic acid [(1R*,2S*,5S*)-3-(5-methyl-3'-trifluoromethyl-biphenyl-2-carbonyl)-3-aza-bicyclo[3.1.O]hex-2-ylmethyl]-amide;
2,3-dihydro-benzo[ 1,4]dioxine-5-carboxylic acid [(1R*,25*,5S*)-3-(3'-fluoro-5-methyl-biphenyl-2-carbonyl)-3-aza-bicyclo[3.1.0]hex-2-ylmethyl]-amide;
2,3-dihydro-benzo[1,4]dioxine-5-carboxylic acid [(1R*,2S*,5S*)-3-(5,4'-dimethylbiphenyl-2-carbonyl)-3-aza-bicyclo[3.1.0]hex-2-ylmethyl]-amide;
2,3-dihydro-benzo[1,4]dioxine-5-carboxylic acid [(1R*,2S*,5S*)-3-(4'-fluoro-5,3'-dimethyl-biphenyl-2-carbonyl)-3-aza-bicyclo[3.1.0]hex-2-ylmethyl]-amide;
2,3-dihydro-benzo[1,4]dioxine-5-carboxylic acid {(1R*,2S*,5S*)-3-[4-methyl-2-(4-methyl-thiophen-2-yl)-benzoyl]-3-aza-bicyclo[3.1.0]hex-2-ylmethyl }-amide;
2,3-dihydro-benzo[1,4]dioxine-5-carboxylic acid [(1R*,2S*,5S*)-3-(4'-fluoro-4,3'-dimethyl-biphenyl-2-carbonyl)-3-aza-bicyclo[3.1.0]hex-2-ylmethyl]-amide;
2,3-dihydro-benzo[1,4]dioxine-5-carboxylic acid [(1R*,2S*,5S*)-3-(6-methylbiphenyl-2-carbonyl)-3-aza-bicyclo[3.1.0]hex-2-ylmethyl]-amide;
2,3-dihydro-benzo[1,4]dioxine-5-carboxylic acid [(1R*,2S*,5S*)-3-(3'-fluoro-6-methyl-biphenyl-2-carbonyl)-3-aza-bicyclo[3.1.0]hex-2-ylmethyl]-amide;
2,3-dihydro-benzo[1,4]dioxine-5-carboxylic acid [(1R*,2S*,5S*)-3-(2-cyclopropylethynyl-4-methyl-benzoyl)-3-aza-bicyclo[3.1.0]hex-2-ylmethyl]-amide; benzofuran-4-carboxylic acid [(1R,2S,5S)-3-(2-methyl-5-m-tolyl-thiazole-4-carbonyl)-3-aza-bicyclo[3.1.0]hex-2-ylmethyl]-amide;
benzofuran-4-carboxylic acid {(1R,2S,5S)-3-[5-(3-fluoro-phenyl)-2-methyl-thiazole-4-carbonyl]-3-aza-bicyclo[3.1.0]hex-2-ylmethyl}-amide;
2,3-dihydro-benzo[1,4]dioxine-5-carboxylic acid [(1R,2S,5S)-3-(2-methyl-5-m-tolyl-thiazole-4-carbonyl)-3-aza-bicyclo[3.1.0]hex-2-ylmethyl]-amide;
6-methyl-imidazo[2,1-b]thiazole-5-carboxylic acid [(1R*,2S*,SS*)-3-(2-bromo-5-m-tolyl-thiazole-4-carbonyl)-3-aza-bicyclo[3.1.0]hex-2-ylmethyl]-amide;
6-methyl-imidazo[2,1-b]thiazole-5-carboxylic acid [(1R*,2S*,5S*)-3-(5-m-tolyl-2-trimethylsilanylethynyl-thiazole-4-carbonyl)-3-aza-bicydo[3.1.0]hex-2-ylmethyl]-amide;
6-methyl-imidazo[2,1-b]thiazole-5-carboxylic acid {(1R*,2S*,5S*)-3-[2-(3-hydroxy-prop-1-ynyl)-5-m-tolyl-thiazole-4-carbonyl]-3-aza-bicyclo[3.1.0]hex-2-ylmethyl}-amide;
6-methyl-imidazo[2,1-b]thiazole-5-carboxylic acid [(1R*,2S*,5S*)-3-(2-ethyl-5-m-tolyl-thiazole-4-carbonyl)-3-aza-bicyclo[3.1.0]hex-2-ylmethyl]-amide;
6-methyl-imidazo[2,1-b]thiazole-5-carboxylic acid {(1R*,2S*,5S*)-3-[2-(3-hydroxypropyl)-5-m-tolyl-thiazole-4-carbonyl]-3-aza-bicyclo[3.1.0]hex-2-ylmethyl}-amide;
6-methyl-imidazo[2,1-b]thiazole-5-carboxylic acid [(1R*,2S*,5S*)-3-(5-m-tolyl-thiazole-4-carbonyl)-3-aza-bicyclo[3.1.0]hex-2-ylmethyl]-amide; and
6-methyl-imidazo[2,1-b]thiazole-5-carboxylic acid [(1R*,2S*,5S*)-3-(2-methoxy-5-m-tolyl-thiazole-4-carbonyl)-3-aza-bicyclo[3.1.0]hex-2-ylmethyl]-amide;
or a pharmaceutically acceptable salt of such a compound.

20. A compound of any one of claims 1 to 19 for use as medicament.

21. Use of a compound according to any of claims 1 to 19 for the preparation of a medicament for the prevention or treatment of diseases selected from the group consisting of dysthymic, mood, psychotic and anxiety disorders; diabetes and appetite, taste, eating, or drinking disorders; hypothalamic diseases; disturbed biological and circadian rhythms; all types of sleep disorders; sleep disturbances associated with diseases such as neurological disorders including neuropathic pain and restless leg syndrome; insomnias related to psychiatric disorders; sleep apnea; narcolepsy; idiopathic insomnias; parasomnias; stress-related syndromes; benign prostatic hypertrophy; all types of psychoactive substance use and abuse; all dementias and cognitive dysfunctions in the healthy population and in psychiatric and neurologic disorders; and other diseases related to general orexin system dysfunctions.

22. Use according to claim 21 wherein said sleep disorders comprise all types of insomnias, narcolepsy and other disorders of excessive sleepiness, sleep-related dystonias, restless leg syndrome, sleep apneas, jet-lag syndrome, shift-work syndrome, delayed or advanced sleep phase syndrome or insomnias related to psychiatric disorders.

23. Use according to claim 21 wherein said cognitive dysfunctions comprise deficits in all types of attention, learning and memory functions occurring transiently or chronically in the normal, healthy, young, adult or aging population, and also occurring transiently or chronically in psychiatric, neurologic, cardiovascular and immune disorders.

24. Use according to claim 21 wherein said eating disorders comprise metabolic dysfunction; dysregulated appetite control; compulsive obesities; emeto-bulimia or anorexia nervosa.

25. Use according to claim 21 wherein said psychoactive substance use and abuse comprise all types of psychological or physical addictions and their related tolerance and dependence components.

## Patentansprüche

1. Verbindung der Formel (I) 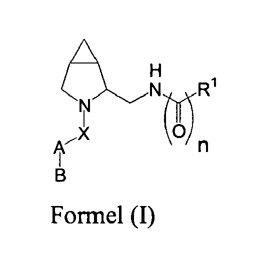 wobei
X C(O) oder SO₂ repräsentiert;
A Aryl oder Heterocyclyl repräsentiert, wobei das Aryl oder Heterocyclyl unsubstituiert oder unabhängig mono- oder disubstituiert ist, wobei die Substituenten unabhängig ausgewählt sind aus der Gruppe bestehend aus (C₁₋₄)Alkyl, (C₃₋₆)Cycloalkyl, (C₂₋₆)Alkynyl, Hydroxy-(C₁₋₄)alkyl, Hydroxy-(C₂₋₆)alkynyl, Trimethylsilyl-ethynyl, (C₃₋₆)Cycloalkyl-ethynyl, (C₁₋₄)Alkoxy, Trifluormethyl, Trifluormethoxy, NR²R³, N(R²)C(O)R³, C(O)NR²R³ und Halogen;
B ein Wasserstoffatom oder eine Aryl- oder Heterocyclyl-Gruppe repräsentiert, wobei das Aryl oder Heterocyclyl unsubstituiert oder unabhängig mono-, di- oder trisubstituiert ist, wobei die Substituenten unabhängig ausgewählt sind aus der Gruppe bestehend aus (C₁₋₄)Alkyl, (C₃₋₆)Cycloalkyl, (C₁₋₄)Alkoxy, Methoxy-(C₁₋₄)alkoxy, Cyano, Trifluormethyl, Trifluormethoxy, NR²R³, N(R²)C(O)R³, C(O)NR²R³ und Halogen; oder B eine 2,3-Dihydro-benzo[1,4]dioxinyl-Gruppe repräsentiert;
oder A und B zusammen eine trizyklische Gruppe repräsentieren;
n 0 oder 1 repräsentiert;
R¹ Aryl oder Heterocyclyl repräsentiert, wobei das Aryl oder Heterocyclyl unsubstituiert oder unabhängig mono-, di- oder trisubstituiert ist, wobei die Substituenten unabhängig ausgewählt sind aus der Gruppe bestehend aus (C₁₋₄)Alkyl, (C₂₋₆)Alkynyl, (C₃₋₆)Cycloalkyl, (C₁₋₄)Alkoxy, (C₁₋₄)Alkylthio, Halogen, Hydroxy, Cyano, Trifluormethyl, Trifluormethoxy, NR²R³, N(R²)C(O)R³, COOR² und C(O)NR²R³; oder R¹ eine Heterocyclyl-ethenyl-, eine Heterocyclyl-(C₁₋₄)alkyl- oder eine Aryloxy-(C₁₋₄)alkyl-Gruppe repräsentiert, wobei die Gruppen unsubstituiert oder unabhängig mono- oder disubstituiert sind am Aryl- oder Heterocyclyl-Teil, wobei die Substituenten unabhängig ausgewählt sind aus der Gruppe bestehend aus (C₁₋₄)Alkyl, (C₁₋₄)Alkoxy, Halogen, Trifluormethyl, Trifluormethoxy und NR²R³; oder R¹ eine 2,3-Dihydro-benzofuranyl-, eine Benzo[1,3]dioxolyl-, eine 2,3-Dihydro-benzo[1,4]dioxinyl-, eine 4-Oxo-4*H*-chromenyl-, eine 2*H*-Chromenyl-, eine Chromanyl-, eine 4*H*-Benzo[1,3]dioxinyl-, eine 2,3-Dihydro-thieno[3,4-b][1,4]dioxinyl-, eine Morpholin-4-yl-phenyl-, eine Piperazin-1-yl-phenyl-, eine 3,4-Dihydro-2*H*-benzo[1,4]oxazinyl-, eine 3-Oxo-3,4-dihydro-2*H*-benzo[1,4]oxazinyl- oder eine 2,3,6,7-Tetrahydro-benzo[1,2-b;4,5-b']difuranyl-Gruppe repräsentiert, wobei die genannten Gruppen unsubstituiert oder unabhängig mono- oder disubstituiert sind, wobei die Substituenten unabhängig ausgewählt sind aus der Gruppe bestehend aus (C₁₋₄)Alkyl, (C₁₋₄)Alkoxy und Halogen;
R² Wasserstoff oder (C₁₋₄)Alkyl repräsentiert; und
R³ Wasserstoff oder (C₁₋₄)Alkyl repräsentiert;
oder ein pharmazeutisch akzeptables Salz davon.

2. Verbindung der allgemeinen Formel (Ia) nach Anspruch 1, wobei die stereogenen Zentren in einer relativen cis-Konfiguration vorliegen 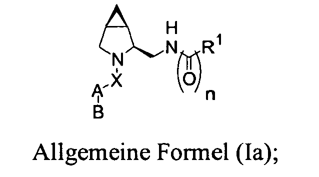 oder ein pharmazeutisch akzeptables Salz davon.

3. Verbindung nach Anspruch 1 oder 2, wobei
X C(O) oder SO₂ repräsentiert;
A Aryl oder Heterocyclyl repräsentiert, wobei das Aryl oder Heterocyclyl unsubstituiert oder unabhängig mono- oder disubstituiert ist, wobei die Substituenten unabhängig ausgewählt sind aus der Gruppe bestehend aus (C₁₋₄)Alkyl, (C₃₋₆)Cycloalkyl, (C₁₋₄)Alkoxy, Trifluormethyl, Trifluormethoxy, NR²R³, N(R²)C(O)R³, C(O)NR²R³ und Halogen;
B ein Wasserstoffatom oder eine Aryl- oder Heterocyclyl-Gruppe repräsentiert, wobei das Aryl oder Heterocyclyl unsubstituiert oder unabhängig mono-, di- oder trisubstituiert ist, wobei die Substituenten unabhängig ausgewählt sind aus der Gruppe bestehend aus (C₁₋₄)Alkyl, (C₃₋₆)Cycloalkyl, (C₁₋₄)Alkoxy, Trifluormethyl, Trifluormethoxy, NR²R³, N(R²)C(O)R³, C(O)NR²R³ und Halogen;
oder A und B zusammen eine trizyklische Gruppe repräsentieren;
n 0 oder 1 repräsentiert;
R¹ Aryl oder Heterocyclyl repräsentiert, wobei das Aryl oder Heterocyclyl unsubstituiert oder unabhängig mono-, di- oder trisubstituiert ist, wobei die Substituenten unabhängig ausgewählt sind aus der Gruppe bestehend aus (C₁₋₄)Alkyl, (C₃₋₆)Cycloalkyl, (C₁₋₄)Alkoxy, Halogen, Hydroxy, Cyano, Trifluormethyl, Trifluormethoxy, NR²R³, N(R²)C(O)R³ und C(O)NR²R³; oder R¹ eine Heterocyclyl-ethenyl-, eine Heterocyclyl-(C₁₋₄)alkyl oder eine Aryloxy-(C₁₋₄)alkyl-Gruppe repräsentiert, wobei die Gruppen unsubstituiert oder unabhängig mono- oder disubstituiert sind am Aryl- oder Heterocyclyl-Teil, wobei die Substituenten unabhängig ausgewählt sind aus der Gruppe bestehend aus (C₁₋₄)Alkyl, (C₁₋₄)Alkoxy, Halogen, Trifluormethyl, Trifluormethoxy und NR²R³; oder R¹ eine 2,3-Dihydro-benzofuranyl-, eine Benzo[1,3]dioxolyl-, eine 2,3-Dihydro-benzo[1,4]dioxinyl- oder eine 4-Oxo-4*H*-chromenyl-Gruppe repräsentiert, wobei die genannten Gruppen unsubstituiert oder monosubstituiert sind am aromatischen Ring durch Substituenten, die unabhängig ausgewählt sind aus der Gruppe bestehend aus (C₁₋₄)Alkyl, (C₁₋₄)Alkoxy und Halogen;
R² Wasserstoff oder (C₁₋₄)Alkyl repräsentiert; und
R³ Wasserstoff oder (C₁₋₄)Alkyl repräsentiert;
oder ein pharmazeutisch akzeptables Salz davon.

4. Verbindungen nach einem der Ansprüche 1 bis 3, wobei
A Heterocyclyl repräsentiert, wobei das Heterocyclyl unsubstituiert oder monosubstituiert ist, wobei der Substituent ausgewählt ist aus der Gruppe bestehend aus (C₁₋₄)Alkyl und NR²R³;
B Aryl repräsentiert, wobei das Aryl unsubstituiert oder unabhängig mono-, di- oder trisubstituiert ist, wobei die Substituenten unabhängig ausgewählt sind aus der Gruppe bestehend aus (C₁₋₄)Alkyl, (C₁₋₄)Alkoxy, Trifluormethyl und Halogen; und
R¹ Aryl oder Heterocyclyl repräsentiert, wobei das Aryl oder Heterocyclyl unsubstituiert oder unabhängig mono-, di- oder trisubstituiert ist, wobei die Substituenten unabhängig ausgewählt sind aus der Gruppe bestehend aus (C₁₋₄)Alkyl, (C₁₋₄)Alkoxy und Halogen; oder R¹ eine 2,3-Dihydro-benzofuranyl-, eine Benzo[1,3]dioxolyl- oder eine 2,3-Dihydro-benzo[1,4]dioxinyl-Gruppe repräsentiert; oder ein pharmazeutisch akzeptables Salz davon.

5. Verbindungen nach einem der Ansprüche 1 bis 4, wobei
A eine Oxazolyl-, eine Thiazolyl-, eine Pyrimidyl- oder eine Pyrazinylgruppe repräsentiert, wobei die genannten Gruppen unsubstituiert oder monosubstituiert sind, wobei der Substituent ausgewählt ist aus der Gruppe bestehend aus (C₁₋₄)Alkyl und NR²R³;
B Phenyl repräsentiert, wobei das Phenyl unsubstituiert oder unabhängig mono- oder disubstituiert ist, wobei die Substituenten unabhängig ausgewählt sind aus der Gruppe bestehend aus (C₁₋₄)Alkyl, (C₁₋₄)Alkoxy, Trifluormethyl und Halogen; und
R¹ eine Phenyl-, eine Naphthyl-, eine Benzofuranyl-, eine Imidazo[2,1-b]thiazolyl-, eine Imidazo[1,2-a]pyridyl-, eine Pyrazolo[1,5-a]pyridyl-, eine Thiazolyl-, eine Isoxazolyl-, eine Pyrazolyl-, eine Indolyl-, eine Indazolyl-, eine Benzimidazolyl- oder eine Benzothiophenyl-Gruppe repräsentiert, wobei die genannten Gruppen unsubstituiert oder unabhängig mono- oder disubstituiert sind, wobei die Substituenten unabhängig ausgewählt sind aus der Gruppe bestehend aus (C₁₋₄)Alkyl, (C₁₋₄)Alkoxy und Halogen; oder R¹ eine 2,3-Dihydro-benzofuranyl-, eine Benzo[1,3]dioxolyl- oder eine 2,3-Dihydro-benzo[1,4]dioxinyl-Gruppe repräsentiert; oder ein pharmazeutisch akzeptables Salz davon.

6. Verbindung nach einem der Ansprüche 1 bis 5, wobei
X C(O) repräsentiert;
oder ein pharmazeutisch akzeptables Salz davon.

7. Verbindung nach einem der Ansprüche 1 bis 6, wobei
n 1 repräsentiert;
oder ein pharmazeutisch akzeptables Salz davon.

8. Verbindung nach einem der Ansprüche 1, 2, 6 oder 7, wobei A Aryl oder Heterocyclyl repräsentiert, wobei das Aryl oder Heterocyclyl unsubstituiert oder monosubstituiert ist, wobei der Substituent ausgewählt ist aus der Gruppe bestehend aus (C₁₋₄)Alkyl, (C₃₋₆)Cycloalkyl, (C₂₋₆)Alkynyl, Hydroxy-(C₁₋₄)alkyl, Hydroxy-(C₂₋₆)alkynyl, (C₃₋₆)Cycloalkyl-ethynyl, (C₁₋₄)Alkoxy, NR²R³ und Halogen;
oder ein pharmazeutisch akzeptables Salz davon.

9. Verbindung nach einem der Ansprüche 1, 2 oder 6 bis 8, wobei B Aryl oder Heterocyclyl repräsentiert, wobei das Aryl oder Heterocyclyl unsubstituiert oder unabhängig mono-, di- oder trisubstituiert ist, wobei die Substituenten unabhängig ausgewählt sind aus der Gruppe bestehend aus (C₁₋₄)Alkyl, (C₁₋₄)Alkoxy, Cyano, Trifluormethyl, NR²R³ und Halogen;
oder ein pharmazeutisch akzeptables Salz davon.

10. Verbindung nach einem der Ansprüche 1, 2 oder 6 bis 9, wobei R¹ Aryl oder Heterocyclyl repräsentiert, wobei das Aryl oder Hetercyclyl unsubstituiert oder unabhängig mono-, di- oder trisubstituiert ist, wobei die Substituenten unabhängig ausgewählt sind aus der Gruppe bestehend aus (C₁-4)Alkyl, (C₁₋₄)Alkoxy, Halogen, Hydroxy, Cyano, Trifluormethyl und COOR²; oder R¹ eine 2,3-Dihydro-benzofuranyl-, eine Benzo[1,3]dioxolyl-, eine 2,3-Dihydro-benzo[1,4]dioxinyl-, eine 2*H*-Chromenyl, eine Chromanyl-, eine 4*H*-Benzo[1,3]dioxinyl-, eine 2,3-Dihydro-thieno[3,4-b][1,4]dioxinyl-, eine 3,4-Dihydro-2*H*-benzo[1,4]oxazinyl- oder eine 2,3,6,7-Tetrahydro-benzo[1,2-b;4,5-b']difuranyl-Gruppe repräsentiert, wobei die genannten Gruppen unsubstituiert oder unabhängig mono- oder disubstituiert sind, wobei die Substituenten unabhängig ausgewählt sind aus der Gruppe bestehend aus (C₁₋₄)Alkyl, (C₁₋₄)Alkoxy und Halogen;
oder ein pharmazeutisch akzeptables Salz davon.

11. Verbindung nach einem der Ansprüche 1, 2 oder 6 bis 10, wobei A Aryl repräsentiert, wobei das Aryl unsubstituiert oder monosubstituiert ist, wobei der Substituent ausgewählt ist aus der Gruppe bestehend aus (C₁₋₄)Alkyl, (C₃₋₆)Cycloalkyl, (C₃₋₆)Cycloalkyl-ethynyl oder Halogen;
oder ein pharmazeutisch akzeptables Salz davon.

12. Verbindung nach einem der Ansprüche 1, 2 oder 6 bis 10, wobei A Heterocyclyl repräsentiert, wobei das Heterocyclyl unsubstituiert oder monosubstituiert ist, wobei der Substituent ausgewählt ist aus der Gruppe bestehend aus (C₁₋₄)Alkyl, (C₃₋₆)Cycloalkyl, Hydroxy-(C₁₋₄)alkyl, Hydroxy-(C₂₋₆)alkynyl, (C₁₋₄)Alkoxy, NR²R³ und Halogen;
oder ein pharmazeutisch akzeptables Salz davon.

13. Verbindung nach einem der Ansprüche 1 bis 4 oder 6 bis 12, wobei B Phenyl repräsentiert, wobei das Phenyl unsubstituiert oder unabhängig mono-, di- oder trisubstituiert ist, wobei die Substituenten unabhängig ausgewählt sind aus der Gruppe bestehend aus (C₁₋₄)Alkyl, (C₁₋₄)Alkoxy, Trifluormethyl und Halogen;
oder ein pharmazeutisch akzeptables Salz davon.

14. Verbindung nach einem der Ansprüche 1 bis 3 oder 6 bis 13, wobei R¹ Heterocyclyl repräsentiert, wobei das Heterocyclyl unsubstituiert oder unabhängig mono-, di- oder trisubstituiert ist, wobei die Substituenten unabhängig ausgewählt sind aus der Gruppe bestehend aus (C₁₋₄)Alkyl, (C₁₋₄)Alkoxy, Halogen, und Trifluormethyl;
oder ein pharmazeutisch akzeptables Salz davon.

15. Verbindung nach einem der Ansprüche 1 bis 3 oder 6 bis 13, wobei R¹ Aryl repräsentiert, wobei das Aryl unsubstituiert oder unabhängig mono-, di- oder trisubstituiert ist, wobei die Substituenten unabhängig ausgewählt sind aus der Gruppe bestehend aus (C₁₋₄)Alkyl, (C₁₋₄)Alkoxy, Halogen, Hydroxy, Cyano und Trifluormethyl;
oder ein pharmazeutisch akzeptables Salz davon.

16. Verbindung nach einem der Ansprüche 1, 2 oder 6 bis 13, wobei R¹ eine 2,3-Dihydro-benzofuranyl-, eine 2,3-Dihydro-benzo[1,4]dioxinyl-, eine Chromanyl-, eine 2,3-Dihydro-thieno[3,4-b][1,4]dioxinyl- oder eine 3,4-Dihydro-2*H*-benzo[1,4]oxazinyl-Gruppe repräsentiert, wobei die genannten Gruppen unsubstituiert oder monosubstituiert sind, wobei der Substituent ausgewählt ist aus der Gruppe bestehend aus (C₁₋₄)Alkyl und Halogen;
oder ein pharmazeutisch akzeptables Salz davon.

17. Verbindung nach einem der Ansprüche 1 bis 3 oder 6 bis 14, wobei in dem Fall, dass R¹ Heterocyclyl repräsentiert, das genannte Heterocyclyl ein Imidazo[2,1-b]thiazolyl oder eine Imidazo[1,2-a]Pyridyl-Gruppe ist, wobei die genannten Gruppen unsubstituiert oder monosubstituiert sind, wobei der Substituent ausgewählt ist aus der Gruppe bestehend aus (C₁₋₄)Alkyl, Halogen und Trifluormethyl;
oder ein pharmazeutisch akzeptables Salz davon.

18. Verbindung nach einem der Ansprüche 1, 2, 6 bis 10 oder 12 bis 17, wobei in dem Fall, dass A Heterocyclyl repräsentiert, das genannte Heterocyclyl eine Thiazolgruppe ist, die unsubstituiert oder monosubstituiert ist, wobei der Substituent ausgewählt ist aus der Gruppe bestehend aus (C₁₋₄)Alkyl, Hydroxy-(C₁₋₄)alkyl, Hydroxy-(C₂₋₆)alkynyl, (C₁₋₄)Alkoxy, NR²R³ und Halogen;
oder ein pharmazeutisch akzeptables Salz davon.

19. Verbindung nach Anspruch 1, die ausgewählt ist aus der Gruppe bestehend aus:
4-Fluor-N-{(1R*,2S*,5S*)-3-[5-(3-fluor-phenyl)-2-methyl-thiazol-4-carbonyl]-3-aza-b icyclo[3.1.0]hex-2-ylmethyll-benzamid;
Benzofuran-4-carbonsäure-[(1R*,2S*,5S*)-3-(biphenyl-2-carbonyl)-3-aza-bicyclo[3.1. 0]hex-2-ylmethyl]-amid;
Benzofuran-4-carbonsäure-[(1R*,2S*,5S*)-3-(2-methyl-5-m-tolyl-thiazol-4-carbonyl)-3-aza-bicyclo[3.1.0]hex-2-ylmethyl]-amid;
Benzofuran-4-carbonsäure-{(1R*,2S*,5S*)-3-[5-(4-fluor-phenyl)-2-methyl-thiazol-4-c arbonyl]-3-aza-bicyclo[3.1.0]hex-2-ylmethyl}-amid;
Benzofuran-4-carbonsäure- {(1R*,2S*,5S*)-3-[5-(3-fluor-phenyl)-2-methyl-thiazol-4-c arbonyl]-3-aza-bicyclo [3.1.0]hex-2-ylmethyl}-amid;
Benzofuran-4-carbonsäure-[(1R*,2S*,5S*)-3-(2-methyl-5-p-tolyl-thiazol-4-carbonyl)-3 -aza-bicyclo[3.1.0]hex-2-ylmethyl]-amid;
Benzofuran-4-carbonsäure-{(1R*,2S*,5S*)-3-[5-(4-ethyl-phenyl)-2-methyl-thiazol-4-c arbonyl]-3-aza-bicyclo[3.1.0]hex-2-ylmethyl}-amid;
Benzofuran-4-carbonsäure-{(1R*,2S*,5S*)-3-[5-(2-fluor-phenyl)-2-methyl-thiazol-4-c arbonyl]-3-aza-bicyclo[3.1.0]hex-2-ylmethyl}-amid;
Benzofuran-4-carbonsäure-{(1R*,2S*,5S*)-3-[5-(2-chlor-phenyl)-2-methyl-thiazol-4-c arbonyl]-3-aza-bicyclo[3.1.0]hex-2-ylmethyl }-amid;
Benzofuran-4-carbonsäure-{(1R*,2S*,5S*)-3-[5-(3-chlor-phenyl)-2-methyl-thiazol-4-c arbonyl]-3-aza-bicyclo[3.1.0]hex-2-ylmethyl}-amid;
Benzofuran-4-carbonsäure- {(1R*,2S*,5S*)-3-[5-(2-methoxy-phenyl)-2-methyl-thiazol -4-carbonyl]-3-aza-bicyclo[3.1.0]hex-2-ylmethyl}-amid;
Benzofuran-4-carbonsäure-{(1R*,2S*,5S*)-3-[5-(3-methoxy-phenyl)-2-methyl-thiazol -4-carbonyl]-3-aza-bicyclo[3.1.0]hex-2-ylmethyl}-amid;
Benzofuran-4-carbonsäure-{(1R*,2S*,5S*)-3-[2-methyl-5-(3-trifluormethyl-phenyl)-th iazol-4-carbonyl]-3-aza-bicyclo[3.1.0]hex-2-ylmethyl}-amid;
Benzofuran-4-carbonsäure-{(1R*,2S*,5S*)-3-[2-methyl-5-(2-trifluormethyl-phenyl)-th iazol-4-carbonyl]-3-aza-bicyclo[3.1.0]hex-2-ylmethyl}-amid;
Benzofuran-4-carbonsäure-[(1R*,2S*,5S*)-3-(2-methyl-5-o-tolyl-thiazol-4-carbonyl)-3 -aza-bicyclo[3.1.0]hex-2-ylmethyl]-amid;
Benzofuran-4-carbonsäure- {(1R*,2S*,5S*)-3-[5-(3,4-dimethyl-phenyl)-2-methyl-thiaz ol-4-carbonyl]-3-aza-bicyclo [3.1.0]hex-2-ylmethyl}-amid;
Benzofuran-4-carbonsäure-[(1R*,2S*,5S*)-3-(2-amino-5-m-tolyl-thiazol-4-carbonyl)-3 -aza-bicyclo[3.1.0]hex-2-ylmethyl]-amid;
Benzofuran-4-carbonsäure-{(1R*,2S*,5S*)-3-[2-amino-5-(3-fluor-phenyl)-thiazol-4-ca rbonyl]-3-aza-bicyclo[3.1.0]hex-2-ylmethyl}-amid;
Benzofuran-4-carbonsäure-[(1R*,2S*,5S*)-3-(2-methyl-4-phenyl-pyrimidin-5-carbony 1)-3-aza-bicyclo[3.1.0]hex-2-ylmethyl]-amid;
Benzofuran-4-carbonsäure-{(1R*,2S*,5S*)-3-[2-(2-amino-thiazol-4-yl)-benzoyl]-3-aza -bicyclo[3.1.0]hex-2-ylmethyl}-amid;
Benzofuran-4-carbonsäure-[(1R*,2S*,5S*)-3-(9H-fluoren-4-carbonyl)-3-aza-bicyclo[3. 1.0]hex-2-ylmethyl]-amid;
Benzofuran-4-carbonsäure-[(1R*,2S*,5S*)-3-(3-phenyl-pyrazin-2-carbonyl)-3-aza-bic yclo[3.1.0]hex-2-ylmethyl]-amid;
Benzofuran-4-carbonsäure-{(1R*,2S*,5S*)-3-[5-(4-methoxy-phenyl)-oxazol-4-carbon yl]-3-aza-bicyclo[3.1.0]hex-2-ylmethyl}-amid;
Benzofuran-4-carbonsäure-[(1R*,2S*,5S*)-3-(2'-fluor-biphenyl-2-carbonyl)-3-aza-bicy clo[3.1.0]hex-2-ylmethyl]-amid;
Benzofuran-4-carbonsäure-[(1R*,2S*,5S*)-3-(4'-fluor-biphenyl-2-carbonyl)-3-aza-bicy clo[3.1.0]hex-2-ylmethyl]-amid;
Benzofuran-4-carbonsäure-[(1R*,2S*,5S*)-3-(2'-chlor-biphenyl-2-carbonyl)-3-aza-bic yclo[3.1.0]hex-2-ylmethyl]-amid;
Benzofuran-4-carbonsäure-[(1R*,2S*,5S*)-3-(3'-chlor-biphenyl-2-carbonyl)-3-aza-bic yclo[3.1.0]hex-2-ylmethyl]-amid;
Benzofuran-4-carbonsäure-[(1R*,2S*,5S*)-3-(4'-chlor-biphenyl-2-carbonyl)-3-aza-bic yclo[3.1.0]hex-2-ylmethyl]-amid;
Benzofuran-4-carbonsäure-[(1R*,2S*,5S*)-3-(4'-methyl-biphenyl-2-carbonyl)-3-aza-bi cyclo[3.1.0]hex-2-ylmethyl]-amid;
Benzofuran-4-carbonsäure-[(1R*,2S*,5S*)-3-(3'-methyl-biphenyl-2-carbonyl)-3-aza-bi cyclo[3.1.0]hex-2-ylmethyl]-amid;
Benzofuran-4-carbonsäure-[(1R*,2S*,5S*)-3-(3'-methoxy-biphenyl-2-carbonyl)-3-aza-bicyclo[3.1.0]hex-2-ylmethyl]-amid;
Benzofuran-4-carbonsäure-[(1R*,2S*,5S*)-3-(4'-methoxy-biphenyl-2-carbonyl)-3-aza-bicyclo[3.1.0]hex-2-ylmethyl]-amid;
Benzofuran-4-carbonsäure-[(1R*,2S*,5S*)-3-(3'-trifluormethyl-biphenyl-2-carbonyl)-3 -aza-bicyclo[3.1.0]hex-2-ylmethyl]-amid;
Benzofuran-4-carbonsäure-[(1R*,2S*,5S*)-3-(2-pyridin-3-yl-benzoyl)-3-aza-bicyclo[3. 1.0]hex-2-ylmethyl]-amid;
6-Methyl-imidazo[2,1-b]thiazol-5-carbonsäure-[(1R*,2S*,5S*)-3-(2-methyl-5-p-tolyl-t hiazol-4-carbonyl)-3-aza-bicyclo[3.1.0]hex-2-ylmethyl]-amid;
6-Methyl-imidazo[2,1-b]thiazol-5-carbonsäure-[(1R*,2S*,5S*)-3-(2-methyl-5-m-tolyl-thiazol-4-carbonyl)-3-aza-bicyclo[3. 1.0]hex-2-ylmethyl]-amid;
6-Methyl-imidazo[2,1-b]thiazol-5-carbonsäure-{(1R*,2S*,5S*)-3-[5-(3-fluor-phenyl)-2-methyl-thiazol-4-carbonyl]-3-aza-bicyclo[3.1.0]hex-2-ylmethyl}-amid;
6-Methyl-imidazo[2,1-b]thiazol-5-carbonsäure-{(1R*,2S*,5S*)-3 -[5-(4-ethyl-phenyl)-2-methyl-thiazol-4-carbonyl]-3-aza-bicyclo[3.1.0]hex-2-ylmethyl}-amid;
6-Methyl-imidazo[2,1-b]thiazol-5-carbonsäure-{(1R*,2S*,5S*)-3-[5-(4-fluor-phenyl)-2-methyl-thiazol-4-carbonyl]-3-aza-bicyclo[3.1.0]hex-2-ylmethyl}-amid;
6-Methyl-imidazo[2,1-b]thiazol-5-carbonsäure-[(1R*,2S*,5S*)-3-(biphenyl-2-carbonyl )-3-aza-bicyclo[3.1.0]hex-2-ylmethyl]-amid;
{(1R*,2S*,5S*)-2-[(6,7-Difluor-chinoxalin-2-ylamino)-methyl]-3-aza-bicyclo[3.1.0]he x-3-yl}-(2-methyl-5-m-tolyl-thiazol-4-yl)-methanon;
{(1R*,2S*,5S*)-2-[(6,7-Difluor-chinoxalin-2-ylamino)-methyl]-3-aza-bicyclo[3.1.0]he x-3-yl}-[5-(4-fluor-phenyl)-2-methyl-thiazol-4-yl]-methanon;
{(1R*,2S*,5S*)-2-[(5-Brom-pyrimidin-2-ylamino)-methyl]-3-aza-bicyclo[3.1.0]hex-3-yl}-(2-methyl-5-m-tolyl-thiazol-4-yl)-methanon;
Naphthalen-2-carbonsäure-[(1R*,2S*,5S*)-3-(2-methyl-5-m-tolyl-thiazol-4-carbonyl)-3-aza-bicyclo[3.1.0]hex-2-ylmethyl]-amid;
Naphthalen-1-carbonsäure-[(1R*,2S*,5S*)-3-(2-methyl-5-m-tolyl-thiazol-4-carbonyl)-3-aza-bicyclo[3.1.0]hex-2-ylmethyl]-amid; 2,3-Dihydro-benzofuran-7-carbonsäure-[(1R*,2S*,5S*)-3-(2-methyl-5-m-tolyl-thiazol-4-carbonyl)-3-aza-bicyclo[3.1.0]hex-2-ylmethyl]-amid;
1H-Indol-5-carbonsäure-[(1R*,2S*,5S*)-3-(2-methyl-5-m-tolyl-thiazol-4-carbonyl)-3-aza-bicyclo[3.1.0]hex-2-ylmethyl]-amid;
2-Hydroxy-N-[(1R*,2S*,5S*)-3-(2-methyl-5-m-tolyl-thiazol-4-carbonyl)-3-aza-bicyclo [3.1.0]hex-2-ylmethyl]-benzamid; 2-Brom-4-methyl-thiazol-5-carbonsäure-[(1R*,2S*,5S*)-3-(2-methyl-5-m-tolyl-thiazol -4-carbonyl)-3-aza-bicyclo[3.1.0]hex-2-ylmethyl]-amid;
Furan-3-carbonsäure-[(1R*,2S*,5S*)-3-(2-methyl-5-m-tolyl-thiazol-4-carbonyl)-3-aza-bicyclo[3.1.0]hex-2-ylmethyl]-amid;
3,5-Dimethyl-isoxazol-4-carbonsäure-[(1R*,2S*,5S*)-3-(2-methyl-5-m-tolyl-thiazol-4-carbonyl)-3-aza-bicyclo[3.1.0]hex-2-ylmethyl]-amid;
3,5-Dimethoxy-N-[(1R*,2S*,5S*)-3-(2-methyl-5-m-tolyl-thiazol-4-carbonyl)-3-aza-bic yclo[3.1.0]hex-2-ylmethyl]-benzamid;
Benzo[1,3]dioxol-5-carbonsäure-[(1R*,2S*,5S*)-3-(2-methyl-5-m-tolyl-thiazol-4-carb onyl)-3-aza-bicyclo[3.1.0]hex-2-ylmethyl]-amid;
2,4-Dimethoxy-N-[(1R*,2S*,5S*)-3-(2-methyl-5-m-tolyl-thiazol-4-carbonyl)-3-aza-bic yclo[3.1.0]hex-2-ylmethyl]-benzamid;
2,4-Dimethyl-thiazol-5-carbonsäure-[(1R*,2S*,5S*)-3-(2-methyl-5-m-tolyl-thiazol-4-c arbonyl)-3-aza-bicyclo[3.1.0]hex-2-ylmethyl]-amid;
1-Methyl- 1H-indol-2-carbonsäure-[(1R*,2S*,5S*)-3-(2-methyl-5-m-tolyl-thiazol-4-car bonyl)-3-aza-bicyclo[3.1.0]hex-2-ylmethyl]-amid;
3H-Benzoimidazol-5-carbonsäure-[(1R*,2S*,5S*)-3-(2-methyl-5-m-tolyl-thiazol-4-car bonyl)-3-aza-bicyclo[3.1.0]hex-2-ylmethyl]-amid;
Benzo[2,1,3]oxadiazol-5-carbonsäure-[(1R*,2S*,5S*)-3-(2-methyl-5-m-tolyl-thiazol-4 -carbonyl)-3-aza-bicyclo[3.1.0]hex-2-ylmethyl]-amid;
2,3-Dihydro-benzo[1,4]dioxin-5-carbonsäure-[(1R*,2S*,5S*)-3-(2-methyl-5-m-tolyl-th iazol-4-carbonyl)-3-aza-bicyclo[3.1.0]hex-2-ylmethyl]-amid;
5-Chlor-1,3-dimethyl-1H-pyrazol-4-carbonsäure-[(1R*,2S*,5S*)-3-(2-methyl-5-m-toly 1-thiazol-4-carbonyl)-3-aza-bicyclo[3.1.0]hex-2-ylmethyl]-amid;
Benzo[b]thiophen-2-carbonsäure-[(1R*,2S*,5S*)-3-(2-methyl-5-m-tolyl-thiazol-4-carb onyl)-3-aza-bicyclo[3.1.0]hex-2-ylmethyl]-amid;
5-*tert*-Butyl-2-methyl-2H-pyrazol-3-carbonsäure-[(1R*,2S*,5S*)-3-(2-methyl-5-m-tol yl-thiazol-4-carbonyl)-3-aza-bicyclo[3.1.0]hex-2-ylmethyl]-amid;
1-Methyl-1H-indazol-3-carbonsäure-[(1R*,2S*,5S*)-3-(2-methyl-5-m-tolyl-thiazol-4-c arbonyl)-3-aza-bicyclo[3.1.0]hex-2-ylmethyl]-amid;
1-Methyl-1H-pyrrol-2-carbonsäure-[(1R*,2S*,5S*)-3-(2-methyl-5-m-tolyl-thiazol-4-ca rbonyl)-3-aza-bicyclo[3.1.0]hex-2-ylmethyl]-amid;
2,8-Dimethyl-imidazo[1,2-a]pyridin-3-carbonsäure-[(1R*,2S*,5S*)-3-(2-methyl-5-m-t olyl-thiazol-4-carbonyl)-3-aza-bicyclo[3.1.0]hex-2-ylmethyl]-amid;
6-Isobutyl-5-methyl-N-[(1R*,2S*,5S*)-3-(2-methyl-5-m-tolyl-thiazol-4-carbonyl)-3-az a-bicyclo[3.1.0]hex-2-ylmethyl]-nicotinamid;
Pyrazolo[1,5-a]pyridin-3-carbonsäure-[(1R*,2S*,5S*)-3-(2-methyl-5-m-tolyl-thiazol-4 -carbonyl)-3-aza-bicyclo[3.1.0]hex-2-ylmethyl]-amid;
Benzo[d]isoxazol-3-carbonsäure-[(1R*,2S*,5S*)-3-(2-methyl-5-m-tolyl-thiazol-4-car bonyl)-3-aza-bicyclo[3.1.0]hex-2-ylmethyl]-amid;
6-Fluor-4H-benzo[1,3]dioxin-8-carbonsäure-[(1R*,2S*,5S*)-3-(2-methyl-5-m-tolyl-t hiazol-4-carbonyl)-3-aza-bicyclo[3.1.0]hex-2-ylmethyl]-amid; 2,3-Dihydro-benzofuran-4-carbonsäure-[(1R*,2S*,5S*)-3-(2-methyl-5-m-tolyl-thiazol -4-carbonyl)-3-aza-bicyclo[3.1.0]hex-2-ylmethyl]-amid;
Isochinolin-1-carbonsäure-[(1R*,2S*,5S*)-3-(2-methyl-5-m-tolyl-thiazol-4-carbonyl) -3-aza-bicyclo[3.1.0]hex-2-ylmethyl]-amid;
Chinolin-8-carbonsäure-[(1R*,2S*,5S*)-3-(2-methyl-5-m-tolyl-thiazol-4-carbonyl)-3-aza-bicyclo[3.1.0]hex-2-ylmethyl]-amid;
Chinolin-2-carbonsäure-[(1R*,2S*,5S*)-3-(2-methyl-5-m-tolyl-thiazol-4-carbonyl)-3-aza-bicyclo[3.1.0]hex-2-ylmethyl]-amid;
Imidazo[2,1-b]thiazol-5-carbonsäure-[(1R*,2S*,5S*)-3-(2-methyl-5-m-tolyl-thiazol-4 -carbonyl)-3-aza-bicyclo[3.1.0]hex-2-ylmethyl]-amid;
3-Methyl-imidazo[2,1-b]thiazol-2-carbonsäure-[(1R*,2S*,5S*)-3-(2-methyl-5-m-tolyl -thiazol-4-carbonyl)-3-aza-bicyclo[3.1.0]hex-2-ylmethyl]-amid;
1-Methyl- 1H-indol-3-carbonsäure-[(1R*,2S*,5S*)-3 -(2-methyl-5-m-tolyl-thiazol-4-ca rbonyl)-3-aza-bicyclo[3.1.0]hex-2-ylmethyl]-amid;
1H-Indol-3-carbonsäure-[(1R*,2S*,5S*)-3-(2-methyl-5-m-tolyl-thiazol-4-carbonyl)-3 -aza-bicyclo[3.1.0]hex-2-ylmethyl]-amid;
1H-Indazol-3-carbonsäure-[(1R*,2S*,5S*)-3-(2-methyl-5-m-tolyl-thiazol-4-carbonyl) -3-aza-bicyclo[3.1.0]hex-2-ylmethyl]-amid;
Imidazo[1,2-a]pyridin-3-carbonsäure-[(1R*,2S*,5S*)-3-(2-methyl-5-m-tolyl-thiazol-4 -carbonyl)-3-aza-bicyclo[3.1.0]hex-2-ylmethyl]-amid;
2,3-Dihydro-thieno[3,4-b][1,4]dioxin-5-carbonsäure-[(1R*,25*,5S*)-3-(2-methyl-5-m -tolyl-thiazol-4-carbonyl)-3-aza-bicyclo[3.1.0]hex-2-ylmethyl]-amid;
3-Brom-N-[(1R*,2S*,5S*)-3-(2-methyl-5-m-tolyl-thiazol-4-carbonyl)-3-aza-bicyclo[ 3.1.0]hex-2-ylmethyl]-benzamid;
N-[(1R*,2S*,5S*)-3-(2-Methyl-5-m-tolyl-thiazol-4-carbonyl)-3-aza-bicyclo[3.1.0]hex -2-ylmethyl]-3-trifluormethyl-benzamid;
3-Methoxy-N-[(1R*,2S*,5S*)-3-(2-methyl-5-m-tolyl-thiazol-4-carbonyl)-3-aza-bicyc lo[3.1.0]hex-2-ylmethyl]-benzamid;
3-Fluor-4-methoxy-N-[(1R*,2S*,5S*)-3-(2-methyl-5-m-tolyl-thiazol-4-carbonyl)-3-a za-bicyclo[3.1.0]hex-2-ylmethyl]-benzamid;
3,4-Dichlor-N-[(1R*,2S*,5S*)-3-(2-methyl-5-m-tolyl-thiazol-4-carbonyl)-3-aza-bicyc lo[3.1.0]hex-2-ylmethyl]-benzamid;
2-Chlor-4,5-difluor-N-[(1R*,2S*,5S*)-3-(2-methyl-5-m-tolyl-thiazol-4-carbonyl)-3-a za-bicyclo[3.1.0]hex-2-ylmethyl]-benzamid;
2-Fluor-5-methyl-N-[(1R*,2S*,5S*)-3-(2-methyl-5-m-tolyl-thiazol-4-carbonyl)-3-aza
-bicyclo[3.1.0]hex-2-ylmethyl]-benzamid;
3-Fluor-2-methyl-N-[(1R*,2S*,5S*)-3-(2-methyl-5-m-tolyl-thiazol-4-carbonyl)-3-aza
-bicyclo[3.1.0]hex-2-ylmethyl]-benzamid;
5-Fluor-2-methoxy-N-[(1R*,2S*,5S*)-3-(2-methyl-5-m-tolyl-thiazol-4-carbonyl)-3-a za-bicyclo[3.1.0]hex-2-ylmethyl]-benzamid;
2-Chlor-3-fluor-N-[(1R*,2S*,5S*)-3-(2-methyl-5-m-tolyl-thiazol-4-carbonyl)-3-aza-b icyclo[3.1.0]hex-2-ylmethyl]-benzamid;
2,5-Dimethyl-N-[(1R*,2S*,5S*)-3-(2-methyl-5-m-tolyl-thiazol-4-carbonyl)-3-aza-bic yclo[3.1.0]hex-2-ylmethyl]-benzamid;
3,4-Dimethyl-N-[( 1R*,2S*,5S*)-3-(2-methyl-5-m-tolyl-thiazol-4-carbonyl)-3-aza-bic yclo[3.1.0]hex-2-ylmethyl]-benzamid;
2,5-Dimethoxy-N-[(1R*,2S*,5S*)-3-(2-methyl-5-m-tolyl-thiazol-4-carbonyl)-3-aza-bi cyclo[3.1.0]hex-2-ylmethyl]-benzamid;
N-[(1R*,2S*,5S*)-3-(2-Methyl-5-m-tolyl-thiazol-4-carbonyl)-3-aza-bicyclo[3.1.0]hex -2-ylmethyl]-isophthalaminsäuremethylester;
2-Chlor-4-fluor-N-[(1R*,2S*,5S*)-3-(2-methyl-5-m-tolyl-thiazol-4-carbonyl)-3-aza-b icyclo[3.1.0]hex-2-ylmethyl]-benzamid;
2-Chlor-3-methyl-N-[(1R*,2S*,5S*)-3-(2-methyl-5-m-tolyl-thiazol-4-carbonyl)-3-aza -bicyclo[3.1.0]hex-2-ylmethyl]-benzamid;
3,5-Dichlor-4-hydroxy-N-[(1R*,2S*,5S*)-3-(2-methyl-5-m-tolyl-thiazol-4-carbonyl)-3-aza-bicyclo[3.1.0]hex-2-ylmethyl]-benzamid;
2,4-Dichlor-N-[(1R*,2S*,5S*)-3-(2-methyl-5-m-tolyl-thiazol-4-carbonyl)-3-aza-bicyc lo[3.1.0]hex-2-ylmethyl]-benzamid;
4-Methyl-N-[(1R*,2S*,5S*)-3-(2-methyl-5-m-tolyl-thiazol-4-carbonyl)-3-aza-bicyclo [3.1.0]hex-2-ylmethyl]-3-trifluormethyl-benzamid;
4-Methoxy-2-methyl-N-[(1R*,2S*,5S*)-3-(2-methyl-5-m-tolyl-thiazol-4-carbonyl)-3-aza-bicyclo[3.1.0]hex-2-ylmethyl]-benzamid;
4-Ethyl-N-[(1R*,2S*,5S*)-3-(2-methyl-5-m-tolyl-thiazol-4-carbonyl)-3-aza-bicyclo[3 .1.0]hex-2-ylmethyl]-benzamid;
4-Methoxy-3-methyl-N-[(1R*,2S*,5S*)-3-(2-methyl-5-m-tolyl-thiazol-4-carbonyl)-3-aza-bicyclo[3.1.0]hex-2-ylmethyl]-benzamid;
3,5-Dimethyl-N-[(1R*,2S*,5S*)-3-(2-methyl-5-m-tolyl-thiazol-4-carbonyl)-3-aza-bic yclo[3.1.0]hex-2-ylmethyl]-benzamid;
5-Brom-2-chlor-N-[(1R*,2S*,5S*)-3-(2-methyl-5-m-tolyl-thiazol-4-carbonyl)-3-aza-b icyclo[3.1.0]hex-2-ylmethyl]-benzamid;
3-Cyano-N-[(1R*,2S*,5S*)-3-(2-methyl-5-m-tolyl-thiazol-4-carbonyl)-3-aza-bicyclo[ 3.1.0]hex-2-ylmethyl]-benzamid;
4-Cyano-N-[(1R*,2S*,5S*)-3-(2-methyl-5-m-tolyl-thiazol-4-carbonyl)-3-aza-bicyclo[ 3.1.0]hex-2-ylmethyl]-benzamid;
4-Chlor-N-[(1R*,2S*,5S*)-3-(2-methyl-5-m-tolyl-thiazol-4-carbonyl)-3-aza-bicyclo[ 3.1.0]hex-2-ylmethyl]-benzamid;
3-Iod-N-[(1R*,2S*,5S*)-3-(2-methyl-5-m-tolyl-thiazol-4-carbonyl)-3-aza-bicyclo[3.1 .0]hex-2-ylmethyl]-benzamid;
2-Brom-3-methyl-N-[(1R*,2S*,5S*)-3-(2-methyl-5-m-tolyl-thiazol-4-carbonyl)-3-aza -bicyclo[3.1.0]hex-2-ylmethyl]-benzamid;
5-Methyl-imidazo[2,1-b]thiazol-6-carbonsäure-[(1R*,2S*,5S*)-3-(2-methyl-5-m-tolyl -thiazol-4-carbonyl)-3-aza-bicyclo[3.1.0]hex-2-ylmethyl]-amid;
3,5-Dimethyl-imidazo[2,1-b]thiazol-6-carbonsäure-[(1R*,2S*,5S*)-3-(2-methyl-5-m-tolyl-thiazol-4-carbonyl)-3-aza-bicyclo[3.1.0]hex-2-ylmethyl]-amid;
2,6-Dimethyl-imidazo[2,1-b]thiazol-5-carbonsäure-[(1R*,2S*,5S*)-3-(2-methyl-5-m-tolyl-thiazol-4-carbonyl)-3-aza-bicyclo[3.1.0]hex-2-ylmethyl]-amid;
2,3,5-Trimethyl-imidazo[2,1-b]thiazol-6-carbonsäure-[(1R*,2S*,5S*)-3-(2-methyl-5-m-tolyl-thiazol-4-carbonyl)-3-aza-bicyclo[3.1.0]hex-2-ylmethyl]-amid;
2-Methyl-imidazo[2,1-b]thiazol-5-carbonsäure-[(1R*,2S*,5S*)-3-(2-methyl-5-m-tolyl -thiazol-4-carbonyl)-3-aza-bicyclo[3.1.0]hex-2-ylmethyl]-amid;
6-Trifluormethyl-imidazo[2,1-b]thiazol-5-carbonsäure-[(1R*,2S*,5S*)-3-(2-methyl-5 -m-tolyl-thiazol-4-carbonyl)-3-aza-bicyclo[3.1.0]hex-2-ylmethyl]-amid;
3,6-Dimethyl-imidazo[2,1-b]thiazol-5-carbonsäure-[(1R*,2S*,5S*)-3-(2-methyl-5-m-tolyl-thiazol-4-carbonyl)-3-aza-bicyclo[3.1.0]hex-2-ylmethyl]-amid;
3-Methyl-imidazo[2,1-b]thiazol-5-carbonsäure-[(1R*,2S*,5S*)-3-(2-methyl-5-m-tolyl -thiazol-4-carbonyl)-3-aza-bicyclo[3.1.0]hex-2-ylmethyl]-amid;
6-Chlor-imidazo[2,1-b]thiazol-5-carbonsäure-[(1R*,25*,5S*)-3-(2-methyl-5-m-tolyl-t hiazol-4-carbonyl)-3-aza-bicyclo[3.1.0]hex-2-ylmethyl]-amid;
2H-Chromen-5-carbonsäure-[(1R*,2S*,5S*)-3-(2-methyl-5-m-tolyl-thiazol-4-carbony 1)-3-aza-bicyclo[3.1.0]hex-2-ylmethyl]-amid;
4-Methyl-3,4-dihydro-2H-benzo[1,4]oxazin-8-carbonsäure-[(1R*,2S*,5S*)-3-(2-meth yl-5-m-tolyl-thiazol-4-carbonyl)-3-aza-bicyclo[3.1.0]hex-2-ylmethyl]-amid;
Chroman-8-carbonsäure-[(1R*,2S*,5S*)-3-(2-methyl-5-m-tolyl-thiazol-4-carbonyl)-3 -aza-bicyclo[3.1.0]hex-2-ylmethyl]-amid;
3-Oxo-3,4-dihydro-2H-benzo[1,4]oxazin-5-carbonsäure-[(1R*,2S*,5S*)-3-(2-methyl-5-m-tolyl-thiazol-4-carbonyl)-3-aza-bicyclo[3.1.0]hex-2-ylmethyl]-amid;
3,4-Dihydro-2H-benzo [1,4]oxazin-8-carbonsäure-[(1R*,2S*,5S*)-3-(2-methyl-5-m-to lyl-thiazol-4-carbonyl)-3-aza-bicyclo[3.1.0]hex-2-ylmethyl]-amid;
3,4-Dihydro-2H-benzo[1,4]oxazin-5-carbonsäure-[(1R*,2S*,5S*)-3-(2-methyl-5-m-to lyl-thiazol-4-carbonyl)-3-aza-bicyclo[3.1.0]hex-2-ylmethyl]-amid;
2-Methyl-benzofuran-4-carbonsäure-[(1R*,2S*,5S*)-3-(2-methyl-5-m-tol yl-thiazol-4-carbonyl)-3-aza-bicyclo[3.1.0]hex-2-ylmethyl]-amid;
Benzo[d]isoxazol-3-carbonsäure-{(1R*,2S*,5S*)-3-[5-(3-chlor-phenyl)-2-methyl-thia zol-4-carbonyl]-3-aza-bicyclo[3.1.0]hex-2-ylmethyl}-amid;
2,3-Dihydro-benzo[1,4]dioxin-5-carbonsäure-{(1R*,2S*,5S*)-3-[5-(3-chlor-phenyl)-2-methyl-thiazol-4-carbonyl]-3-aza-bicyclo[3.1.0]hex-2-ylmethyl }-amid;
6-Fluor-4H-benzo[1,3]dioxin-8-carbonsäure-{(1R*,2S*,5S*)-3-[5-(3-chlor-phenyl)-2-methyl-thiazol-4-carbonyl]-3-aza-bicyclo[3.1.0]hex-2-ylmethyl}-amid;
2,3-Dihydro-benzofuran-4-carbonsäure-{(1R*,2S*,5S*)-3-[5-(3-chlor-phenyl)-2-meth yl-thiazol-4-carbonyl]-3-aza-bicyclo [3.1.0]hex-2-ylmethyl -amid;
Isochinolin-1-carbonsäure-{(1R*,2S*,5S*)-3-[5-(3-chlor-phenyl)-2-methyl-thiazol-4-carbonyl]-3-aza-bicyclo[3.1.0]hex-2-ylmethyl}-amid;
Chinolin-8-carbonsäure-{(1R*,2S*,5S*)-3-[5-(3-chlor-phenyl)-2-methyl-thiazol-4-car bonyl]-3-aza-bicyclo[3.1.0]hex-2-ylmethyl}-amid;
Chinolin-2-carbonsäure-{(1R*,2S*,5S*)-3-[5-(3-chlor-phenyl)-2-methyl-thiazol-4-car bonyl]-3-aza-bicyclo[3.1.0]hex-2-ylmethyl}-amid;
Imidazo[2,1-b]thiazol-5-carbonsäure-{(1R*,2S*,5S*)-3-[5-(3-chlor-phenyl)-2-methyl
-thiazol-4-carbonyl]-3-aza-bicyclo[3.1.0]hex-2-ylmethyl-amid; 3-Methyl-imidazo[2,1-b]thiazol-2-carbonsäure-{(1R*,2S*,5S*)-3-[5-(3-chlor-phenyl) -2-methyl-thiazol-4-carbonyl]-3-aza-bicyclo[3.1.0]hex-2-ylmethyl}-amid; 1-Methyl-1H-indol-3-carbonsäure-{(1R*,2S*,5S*)-3-[5-(3-chlor-phenyl)-2-methyl-th iazol-4-carbonyl]-3-aza-bicyclo[3.1.0]hex-2-ylmethyl}-amid;
1,2-Dimethyl-1H-indol-3-carbonsäure-{(1R*,2S*,5S*)-3-[5-(3-chlor-phenyl)-2-meth yl-thiazol-4-carbonyl]-3-aza-bicyclo[3.1.0]hex-2-ylmethyl}-amid; 1H-Indol-3-carbonsäure-{(1R*,2S*,5S*)-3-[5-(3-chlor-phenyl)-2-methyl-thiazol-4-ca rbonyl]-3-aza-bicyclo[3.1.0]hex-2-ylmethyl}-amid;
1H-Indazol-3-carbonsäure-{(1R*,2S*,5S*)-3-[5-(3-chlor-phenyl)-2-methyl-thiazol-4-carbonyl]-3-aza-bicyclo[3.1.0]hex-2-ylmethyl}-amid;
Imidazo[1,2-a]pyridin-3-carbonsäure-{(1R*,2S*,5S*)-3-[5-(3-chlor-phenyl)-2-methyl -thiazol-4-carbonyl]-3-aza-bicyclo[3. 1.0]hex-2-ylmethyll-amid;
2,5-Dimethyl-2H-pyrazol-3-carbonsäure-{(1R*,2S*,5S*)-3-[5-(3-chlor-phenyl)-2-met hyl-thiazol-4-carbonyl]-3-aza-bicyclo[3.1.0]hex-2-ylmethyl -amid;
2-Ethyl-5-methyl-2H-pyrazol-3-carbonsäure-{(1R*,2S*,5S*)-3-[5-(3-chlor-phenyl)-2 -methyl-thiazol-4-carbonyl]-3-aza-bicylo[3.1.0]hex-2-ylmethyl}-amid;
2,3-Dihydro-thieno[3,4-b][1,4]dioxin-5-carbonsäure-{(1R*,2S*,5S*)-3-[5-(3-chlor-ph enyl)-2-methyl-thiazol-4-carbonyl]-3-aza-bicyclo[3.1.0]hex-2-ylmethyl }-amid;
3-Brom-N-{(1R*,2S*,5S*)-3-[5-(3-chlor-phenyl)-2-methyl-thiazol-4-carbonyl]-3-aza -bicyclo[3.1.0]hex-2-ylmethyl}-benzamid;
N-{(1R*,2S*,5S*)-3-[5-(3-chlor-phenyl)-2-methyl-thiazol-4-carbonyl]-3-aza-bicyclo[ 3.1.0]hex-2-ylmethyl }-3-trifluormethyl-benzamid;
N-{(1R*,2S*,5S*)-3-[5-(3-Chlor-phenyl)-2-methyl-thiazol-4-carbonyl]-3-aza-bicyclo [3.1.0]hex-2-ylmethyl}-3-methoxy-benzamid;
3-Methyl-imidazo[2,1-b]thiazol-5-carbonsäure-{(1R*,2S*,5S*)-3-[5-(3-fluor-phenyl) -2-methyl-thiazol-4-carbonyl]-3-aza-bicyclo[3.1.0]hex-2-ylmethyl}-amid;
2-Methyl-imidazo[2,1-b]thiazol-5-carbonsäure-{(1R*,2S*,5S*)-3-[5-(3-fluor-phenyl) -2-methyl-thiazol-4-carbonyl]-3-aza-bicyclo[3.1.0]hex-2-ylmethyl }-amid;
Imidazo[2,1-b]thiazol-5-carbonsäure-{(1R*,2S*,5S*)-3-[5-(3-fluor-phenyl)-2-methyl-thiazol-4-carbonyl]-3-aza-bicyclo[3.1.0]hex-2-ylmethyl}-amid;
1-Methyl-1H-indol-3-carbonsäure-{(1R*,2S*,5S*)-3-[5-(3-fluor-phenyl)-2-methyl-thiazol-4-carbonyl]-3-aza-bicyclo[3.1.0]hex-2-ylmethyl}-amid; 3-Methyl-imidazo[2,1-b] thiazol-2-carbonsäure-{(1R*,2S*,5S*)-3-[5-(3-fluor-phenyl) -2-methyl-thiazol-4-carbonyl]-3-aza-bicyclo[3.1.0]hex-2-ylmethyl}-amid;
1-Ethyl-3-methyl-1H-pyrazol-4-carbonsäure-{(1R*,2S*,5S*)-3-[5-(3-fluor-phenyl)-2-methyl-thiazol-4-carbonyl]-3-aza-bicyclo[3.1.0]hex-2-ylmethyl}-amid; 5-tert-Butyl-2-methyl-2H-pyrazol-3-carbonsäure-{(1R*,2S*,5S*)-3-[5-(3-fluor-pheny 1)-2-methyl-thiazol-4-carbonyl]-3-aza-bicyclo[3.1.0]hex-2-ylmethyl}-amid; Isochinolin-1-carbonsäure-{(1R*,2S*,5S*)-3-[5-(3-fluor-phenyl)-2-methyl-thiazol-4-carbonyl]-3-aza-bicyclo[3.1.0]hex-2-ylmethyl}-amid;
1H-Indazol-3-carbonsäure-{(1R*,2S*,5S*)-3-[5-(3-fluor-phenyl)-2-methyl-thiazol-4-carbonyl]-3-aza-bicyclo[3.1.0]hex-2-ylmethyl}-amid; 4-Methoxy-chinolin-2-carbonsäure-{(1R*,2S*,5S*)-3-[5-(3-fluor-phenyl)-2-methyl-t hiazol-4-carbonyl]-3-aza-bicyclo[3.1.0]hex-2-ylmethyl }-amid; Chinolin-2-carbonsäure-{(1R*,2S*,5S*)-3-[5-(3-fluor-phenyl)-2-methyl-thiazol-4-car bonyl]-3-aza-bicyclo[3.1.0]hex-2-ylmethyl}-amid;
1H-Indol-3-carbonsäure-{(1R*,2S*,5S*)-3-[5-(3-fluor-phenyl)-2-methyl-thiazol-4-ca rbonyl]-3-aza-bicydo[3.1.0]hex-2-ylmethyl}-amid; 6-Fluor-4H-benzo[1,3]dioxin-8-carbonsäure-{(1R*,2S*,5S*)-3-[5-(3-fluor-phenyl)-2-methyl-thiazol-4-carbonyl]-3-aza-bicyclo[3.1.0]hex-2-ylmethyl}-amid; Benzo[d]isoxazol-3-carbonsäure-{(1R*,2S*,5S*)-3-[5-(3-fluor-phenyl)-2-methyl-thia zol-4-carbonyl]-3-aza-bicyclo [3.1.0]hex-2-ylmethyl }-amid;
Benzo[1,3]dioxol-4-carbonsäure-{(1R*,2S*,5S*)-3-[5-(3-fluor-phenyl)-2-methyl-thia zol-4-carbonyl]-3-aza-bicyclo[3.1.0]hex-2-ylmethyl}-amid; 2-Ethyl-5-methyl-2H-pyrazol-3-carbonsäure-{(1R*,2S*,5S*)-3-[5-(3-fluor-phenyl)-2-methyl-thiazol-4-carbonyl]-3-aza-bicyclo[3.1.0]hex-2-ylmethyl}-amid; Imidazo[1,2-a]pyridin-3-carbonsäure-{(1R*,2S*,5S*)-3-[5-(3-fluor-phenyl)-2-methyl -thiazol-4-carbonyl]-3-aza-bicydo[3.1.0]hex-2-ylmethyl}-amid;
1-Methyl-5-trifluormethyl-1H-pyrazol-4-carbonsäure-{(1R*,2S*,5S*)-3-[5-(3-fluor-p henyl)-2-methyl-thiazol-4-carbonyl]-3-aza-bicyclo[3.1.0]hex-2-ylmethyl }-amid; 1,5-Dimethyl-1H-pyrazol-3-carbonsäure-{(1R*,2S*,5S*)-3-[5-(3-fluor-phenyl)-2-met hyl-thiazol-4-carbonyl]-3-aza-bicyclo[3.1.0]hex-2-ylmethyl}-amid;
2,5-Dimethyl-2H-pyrazol-3-carbonsäure-{(1R*,2S*,5S*)-3-[5-(3-fluor-phenyl)-2-met hyl-thiazol-4-carbonyl]-3-aza-bicyclo[3.1.0]hex-2-ylmethyl}-amid; 2,5-Dimethyl-oxazol-4-carbonsäure-{(1R*,2S*,5S*)-3-[5-(3-fluor-phenyl)-2-methyl-t hiazol-4-carbonyl]-3-aza-bicyclo [3.1.0]hex-2-ylmethyl }-amid;
2,3-Dihydro-benzo[1,4]dioxin-5-carbonsäure-{(1R*,2S*,5S*)-3-[5-(3-fluor-phenyl)-2 -methyl-thiazol-4-carbonyl]-3-aza-bicyclo[3.1.0]hex-2-ylmethyl}-amid; 2,3-Dihydro-benzofuran-4-carbonsäure-{(1R*,2S*,5S*)-3-[5-(3-fluor-phenyl)-2-meth yl-thiazol-4-carbonyl]-3-aza-bicyclo [3.1.0]hex-2-ylmethyl }-amid;
1,3-Dimethyl-1H-pyrazol-4-carbonsäure-{(1R*,2S*,5S*)-3-[5-(3-fluor-phenyl)-2-met hyl-thiazol-4-carbonyl]-3-aza-bicyclo[3.1.0]hex-2-ylmethyl}-amid;
7-Fluor-1H-indol-2-carbonsäure-{(1R*,2S*,5S*)-3-[5-(3-fluor-phenyl)-2-methyl-thia zol-4-carbonyl]-3-aza-bicyclo[3.1.0]hex-2-ylmethyl}-amid; 2-Trifluormethyl-1H-benzoimidazol-5-carbonsäure-{(1R*,2S*,5S*)-3-[5-(3 -fluor-phe nyl)-2-methyl-thiazol-4-carbonyl]-3-aza-bicyclo[3.1.0]hex-2-ylmethyl}-amid; 3-Brom-N-{(1R*,2S*,5S*)-3-[5-(3-fluor-phenyl)-2-methyl-thiazol-4-carbonyl]-3-aza-bicyclo[3.1.0]hex-2-ylmethyl}-benzamid; N-{(1R*,2S*,5S*)-3-[5-(3-Fluor-phenyl)-2-methyl-thiazol-4-carbonyl]-3-aza-bicyclo [3.1.0]hex-2-ylmethyl}-3-trifluormethyl-benzamid;
Benzo[d] isoxazol-3-carbonsäure-[(1R*,2S*,5S*)-3-(2-amino-5-m-tolyl-thiazol-4-carb onyl)-3-aza-bicyclo[3.1.0]hex-2-ylmethyl]-amid;
2,3-Dihydro-benzo[1,4]dioxin-5-carbonsäure-[(1R*,2S*,5S*)-3-(2-amino-5-m-tolyl-t hiazol-4-carbonyl)-3-aza-bicyclo[3.1.0]hex-2-ylmethyl]-amid;
6-Fluor-4H-benzo[1,3]dioxin-8-carbonsäure-[(1R*,2S*,5S*)-3-(2-amino-5-m-tolyl-thiazol-4-carbonyl)-3-aza-bicyclo[3.1.0]hex-2-ylmethyl]-amid; 2,3-Dihydro-benzofuran-4-carbonsäure-[(1R*,2S*,5S*)-3-(2-amino-5-m-tolyl-thiazol -4-carbonyl)-3-aza-bicyclo[3.1.0]hex-2-ylmethyl]-amid;
2,2-Dimethyl-2,3-dihydro-benzofuran-7-carbonsäure-[(1R*,2S*,5S*)-3-(2-amino-5-m -tolyl-thiazol-4-carbonyl)-3-aza-bicyclo[3.1.0]hex-2-ylmethyl]-amid;
Isochinolin-1-carbonsäure-[(1R*,2S*,5S*)-3-(2-amino-5-m-tolyl-thiazol-4-carbonyl)-3-aza-bicyclo[3.1.0]hex-2-ylmethyl]-amid;
Chinolin-8-carbonsäure-[(1R*,2S*,5S*)-3-(2-amino-5-m-tolyl-thiazol-4-carbonyl)-3-aza-bicyclo[3.1.0]hex-2-ylmethyl]-amid;
Imidazo[2,1-b]thiazol-5-carbonsäure-[(1R*,2S*,5S*)-3-(2-amino-5-m-tolyl-thiazol-4-carbonyl)-3-aza-bicyclo[3.1.0]hex-2-ylmethyl]-amid; 3-Methyl-imidazo[2,1-b]thiazol-2-carbonsäure-[(1R*,2S*,5S*)-3-(2-amino-5-m-tolyl-thiazol-4-carbonyl)-3-aza-bicyclo[3.1.0]hex-2-ylmethyl]-amid;
1-Methyl-1H-indol-3-carbonsäure-[(1R*,2S*,5S*)-3-(2-amino-5-m-tolyl-thiazol-4-ca rbonyl)-3-aza-bicyclo[3.1.0]hex-2-ylmethyl]-amid;
1H-Indol-3-carbonsäure-[(1R*,2S*,5S*)-3-(2-amino-5-m-tolyl-thiazol-4-carbonyl)-3-aza-bicyclo[3.1.0]hex-2-ylmethyl]-amid;
1H-Indazol-3-carbonsäure-[(1R*,2S*,5S*)-3-(2-amino-5-m-tolyl-thiazol-4-carbonyl)-3-aza-bicyclo[3.1.0]hex-2-ylmethyl]-amid;
Imidazo[1,2-a]pyridin-3-carbonsäure-[(1R*,2S*,5S*)-3-(2-amino-5-m-tolyl-thiazol-4-carbonyl)-3-aza-bicyclo[3.1.0]hex-2-ylmethyl]-amid; 2-Ethyl-5-methyl-2H-pyrazol-3-carbonsäure-[(1R*,2S*,5S*)-3-(2-amino-5-m-tolyl-th iazol-4-carbonyl)-3-aza-bicyclo[3.1.0]hex-2-ylmethyl]-amid;
1-Ethyl-3-methyl-1H-pyrazol-4-carbonsäure-[(1R*,2S*,5S*)-3-(2-amino-5-m-tolyl-thiazol-4-carbonyl)-3-aza-bicyclo[3.1.0]hex-2-ylmethyl]-amid; 2,3-Dihydro-thieno[3,4-b][1,4] dioxin-5-carbonsäure-[(1R*,2S*,5S*)-3-(2-amino-5-m -tolyl-thiazol-4-carbonyl)-3-aza-bicyclo[3.1.0]hex-2-ylmethyl]-amid; N-[(1R*,2S*,5S*)-3-(2-Amino-5-m-tolyl-thiazol-4-carbonyl)-3-aza-bicyclo[3.1.0]hex -2-ylmethyl]-3-brom-benzamid; N-[(1R*,2S*,5S*)-3-(2-Amino-5-m-tolyl-thiazol-4-carbonyl)-3-aza-bicyclo[3.1.0]hex -2-ylmethyl]-3-methoxy-benzamid; Benzo[d]isoxazol-3-carbonsäure-{(1R*,2S*,5S*)-3-[2-amino-5-(3 -chlor-phenyl)-thia zol-4-carbonyl]-3-aza-bicyclo[3.1.0]hex-2-ylmethyl}-amid;
2,3-Dihydro-benzo[1,4]dioxin-5-carbonsäure-{(1R*,2S*,5S*)-3-[2-amino-5-(3-chlorphenyl)-thiazol-4-carbonyl]-3-aza-bicyclo[3.1.0]hex-2-ylmethyl }-amid; 6-Fluor-4H-benzo[1,3]dioxin-8-carbonsäure-{(1R*,2S*,5S*)-3-[2-amino-5-(3-chlor-phenyl)-thiazol-4-carbonyl]-3-aza-bicyclo[3.1.0]hex-2-ylmethyl}-amid; 2,3-Dihydro-benzofuran-4-carbonsäure-{(1R*,2S*,5S*)-3-[2-amino-5-(3-chlor-pheny 1)-thiazol-4-carbonyl]-3-aza-bicyclo[3.1.0]hex-2-ylmethyl}-amid; Isochinolin-1-carbonsäure-{(1R*,2S*,5S*)-3-[2-amino-5-(3-chlor-phenyl)-thiazol-4-c arbonyl]-3-aza-bicyclo[3.1.0]hex-2-ylmethyl}-amid;
Chinolin-8-carbonsäure-{(1R*,2S*,5S *)-3-[2-amino-5-(3-chlor-phenyl)-thiazol-4-car bonyl]-3-aza-bicyclo[3.1.0]hex-2-ylmethyl}-amid; Imidazo[2,1-b]thiazol-5-carbonsäure-{(1R*,2S*,5S*)-3-[2-amino-5-(3-chlor-phenyl)-thiazol-4-carbonyl]-3-aza-bicyclo[3.1.0]hex-2-ylmethyl}-amid;
1-Methyl-1H-indol-3-carbonsäure-{(1R*,2S*,5S*)-3-[2-amino-5-(3-chlor-phenyl)-thiazol-4-carbonyl]-3-aza-bicyclo[3.1.0]hex-2-ylmethyl}-amid;
1H-Indol-3-carbonsäure-{(1R*,2S*,5S*)-3-[2-amino-5-(3-chlor-phenyl)-thiazol-4-carbonyl]-3-aza-bicyclo[3.1.0]hex-2-ylmethyl}-amid;
1H-Indazol-3-carbonsäure-{(1R*,2S*,5S*)-3-[2-amino-5-(3-chlor-phenyl)-thiazol-4-carbonyl]-3-aza-bicyclo[3.1.0]hex-2-ylmethyl}-amid; 2,5-Dimethyl-2H-pyrazol-3-carbonsäure-{(1R*,2S*,5S*)-3-[2-amino-5-(3-chlor-phenyl)-thiazol-4-carbonyl]-3-aza-bicyclo[3.1.0]hex-2-ylmethyl}-amid; 2-Ethyl-5-methyl-2H-pyrazol-3-carbonsäure-{(1R*,2S*,5S*)-3-[2-amino-5-(3-chlorphenyl)-thiazol-4-carbonyl]-3-aza-bicyclo[3.1.0]hex-2-ylmethyl}-amid; 2,3-Dihydro-thieno[3,4-b][1,4]dioxin-5-carbonsäure-{(1R*,2S*,5S*)-3-[2-amino-5-(3-chlor-phenyl)-thiazol-4-carbonyl]-3-aza-bicydo[3.1.0]hex-2-ylmethyl}-amid;
N-{(1R*,2S*,5S*)-3-[2-Amino-5-(3-chlor-phenyl)-thiazol-4-carbonyl]-3-aza-bicyclo[3.1.0]hex-2-ylmethyl}-3-brom-benzamid;
N-{(1R*,2S*,5S*)-3-[2-Amino-5-(3-chlor-phenyl)-thiazol-4-carbonyl]-3-aza-bicyclo[3.1.0]hex-2-ylmethyl}-3-methoxy-benzamid; 6-Methyl-imidazo[2,1-b]thiazol-5-carbonsäure-[(1R*,2S*,5S*)-3-(3'-fluor-5-methyl-biphenyl-2-carbonyl)-3-aza-bicyclo[3.1.0]hex-2-ylmethyl]-amid; Imidazo[1,2-a]pyridin-3-carbonsäure-[(1R*,2S*,5S*)-3-(3'-fluor-5-methyl-biphenyl-2-carbonyl)-3-aza-bicyclo[3.1.0]hex-2-ylmethyl]-amid;
2,3-Dihydro-benzofuran-4-carbonsäure-[(1R*,2S*,5S*)-3-(3'-fluor-5-methyl-biphenyl-2-carbonyl)-3-aza-bicyclo[3.1.0]hex-2-ylmethyl]-amid;
2,3-Dihydro-benzofuran-7-carbonsäure-[(1R*,2S*,5S*)-3-(3'-fluor-5-methyl-biphenyl-2-carbonyl)-3-aza-bicyclo[3.1.0]hex-2-ylmethyl]-amid;
6-Methyl-imidazo[2,1-b]thiazol-5-carbonsäure-{(1R*,2S*,5S*)-3-[5-(3-cyano-phenyl)-2-methyl-thiazol-4-carbonyl]-3-aza-bicyclo[3.1.0]hex-2-ylmethyl}-amid; Benzofuran-4-carbonsäure-{(1R*,2S*,5S*)-3-[5-(3-cyano-phenyl)-2-methyl-thiazol-4 -carbonyl]-3-aza-bicyclo[3.1.0]hex-2-ylmethyl}-amid;
2,3-Dihydro-benzo[1,4]dioxin-5-carbonsäure-{(1R*,2S*,5S*)-3-[5-(3-cyano-phenyl)-2-methyl-thiazol-4-carbonyl]-3-aza-bicyclo[3.1.0]hex-2-ylmethyl}-amid; 2,3-Dihydro-benzo[1,4] dioxin-5-carbonsäure-[(1R*,2S*,5S*)-3-(5-methyl-biphenyl-2-carbonyl)-3-aza-bicyclo[3.1.0]hex-2-ylmethyl]-amid;
2,3-Dihydro-benzo[1,4]dioxin-5-carbonsäure-[(1R*,2S*,5S*)-3-(2'-fluor-5-methyl-biphenyl-2-carbonyl)-3-aza-bicyclo[3.1.0]hex-2-ylmethyl]-amid;
2,3-Dihydro-benzo[1,4] dioxin-5-carbonsäure-[(1R*,2S*,5S*)-3-(5,3'-dimethyl-biphenyl-2-carbonyl)-3-aza-bicyclo[3.1.0]hex-2-ylmethyl]-amid; 2,3-Dihydro-benzo[1,4]dioxin-5-carbonsäure-[(1R*,2S*,5S*)-3-(3'-chlor-5-methyl-biphenyl-2-carbonyl)-3-aza-bicyclo[3.1.0]hex-2-ylmethyl]-amid;
2,3-Dihydro-benzo[1,4]dioxin-5-carbonsäure-[(1R*,2S*,5S*)-3-(3'-methoxy-5-methy1-biphenyl-2-carbonyl)-3-aza-bicyclo[3.1.0]hex-2-ylmethyl]-amid; 2,3-Dihydro-benzo[1,4]dioxin-5-carbonsäure-[(1R*,2S*,5S*)-3-(5-methyl-3'-trifluormethyl-biphenyl-2-carbonyl)-3-aza-bicyclo[3. 1.0]hex-2-ylmethyl]-amid; 2,3-Dihydro-benzo[1,4]dioxin-5-carbonsäure-[(1R*,2S*,5S*)-3-(3'-fluor-5-methyl-biphenyl-2-carbonyl)-3-aza-bicyclo[3.1.0]hex-2-ylmethyl]-amid;
2,3-Dihydro-benzo[1,4]dioxin-5-carbonsäure-[(1R*,2S*,5S*)-3-(5,4'-dimethyl-biphenyl-2-carbonyl)-3-aza-bicyclo[3.1.0]hex-2-ylmethyl]-amid;
2,3-Dihydro-benzo[1,4]dioxin-5-carbonsäure-[(1R*,2S*,5S*)-3-(4'-fluor-5,3'-dimethyl-biphenyl-2-carbonyl)-3-aza-bicyclo[3.1.0]hex-2-ylmethyl]-amid; 2,3-Dihydro-benzo[1,4]dioxin-5-carbonsäure-{(1R*,2S*,5S*)-3-[4-methyl-2-(4-methyl-thiophen-2-yl)-benzoyl]-3-aza-bicyclo[3.1.0]hex-2-ylmethyl}-amid; 2,3-Dihydro-benzo[1,4]dioxin-5-carbonsäure-[(1R*,2S*,5S*)-3-(4'-fluor-4,3'-dimethyl-biphenyl-2-carbonyl)-3-aza-bicyclo[3.1.0]hex-2-ylmethyl]-amid; 2,3-Dihydro-benzo[1,4]dioxine-5-carbonsäure-[(1R*,2S*,5S*)-3-(6-methyl-biphenyl-2-carbonyl)-3-aza-bicyclo[3.1.0]hex-2-ylmethyl]-amid;
2,3-Dihydro-benzo[1,4]dioxin-5-carbonsäure-[(1R*,2S*,5S*)-3-(3'-fluor-6-methyl-biphenyl-2-carbonyl)-3-aza-bicyclo[3.1.0]hex-2-ylmethyl]-amid;
2,3-Dihydro-benzo[1,4]dioxin-5-carbonsäure-[(1R*,2S*,5S*)-3-(2-cyclopropylethyny1-4-methyl-benzoyl)-3-aza-bicyclo[3.1.0]hex-2-ylmethyl]-amid; Benzofuran-4-carbonsäure-[(1R,2S,5S)-3-(2-methyl-5-m-tolyl-thiazol-4-carbonyl)-3-aza-bicyclo[3.1.0]hex-2-ylmethyl]-amid;
Benzofuran-4-carbonsäure-{(1R,2S,5S)-3-[5-(3-fluor-phenyl)-2-methyl-thiazol-4-carb onyl]-3-aza-bicyclo[3.1.0]hex-2-ylmethyl}-amid;
2,3-Dihydro-benzo[1,4]dioxin-5-carbonsäure-[(1R,2S,5S)-3-(2-methyl-5-m-tolyl-thia zol-4-carbonyl)-3-aza-bicyclo[3.1.0]hex-2-ylmethyl]-amid; 6-Methyl-imidazo[2,1-b]thiazol-5-carbonsäure-[(1R*,2S*,5S*)-3-(2-brom-5-m-tolyl-thiazol-4-carbonyl)-3-aza-bicyclo[3.1.0]hex-2-ylmethyl]-amid; 6-Methyl-imidazo[2,1-b]thiazol-5-carbonsäure-[(1R*,2S*,5S*)-3-(5-m-tolyl-2-trimet hylsilanylethynyl-thiazol-4-carbonyl)-3-aza-bicyclo[3.1.0]hex-2-ylmethyl]-amid; 6-Methyl-imidazo[2,1-b]thiazol-5-carbonsäure-{(1R*,2S*,5S*)-3-[2-(3-hydroxy-prop-1-ynyl)-5-m-tolyl-thiazol-4-carbonyl]-3-aza-bicyclo[3.1.0]hex-2-ylmethyl}-amid;
6-Methyl-imidazo[2,1-b]thiazol-5-carbonsäure-[(1R*,2S*,5S*)-3-(2-ethyl-5-m-tolyl-thiazol-4-carbonyl)-3-aza-bicyclo[3.1.0]hex-2-ylmethyl]-amid; 6-Methyl-imidazo[2,1-b]thiazol-5-carbonsäure-{(1R*,2S*,5S*)-3-[2-(3-hydroxy-prop yl)-5-m-tolyl-thiazol-4-carbonyl]-3-aza-bicyclo[3.1.0]hex-2-ylmethyl}-amid; 6-Methyl-imidazo[2,1-b]thiazol-5-carbonsäure-[(R*,2S*,5S*)-3-(5-m-tolyl-thiazol-4 -carbonyl)-3-aza-bicyclo[3.1.0]hex-2-ylmethyl]-amid; und
6-Methyl-imidazo[2,1-b]thiazol-5-carbonsäure-[(1R*,2S*,5S*)-3-(2-methoxy-5-m-tol yl-thiazol-4-carbonyl)-3-aza-bicyclo[3.1.0]hex-2-ylmethyl]-amid;
oder ein pharmazeutisch akzeptables Salz einer solchen Verbindung.

20. Verbindung nach einem der Ansprüche 1 bis 19 zur Verwendung als Medikament.

21. Verwendung einer Verbindung nach einem der Ansprüche 1 bis 19 zur Herstellung eines Medikaments zur Vorbeugung gegen oder Behandlung von Krankheiten, die ausgewählt sind aus der Gruppe bestehend aus dysthymischen, Gemütszustands-, psychotischen und Angststörungen; Diabetes und Appetit-, Geschmacks-, Ess- oder Trinkstörungen; Hypothalamuserkrankungen; gestörten biologischen und zirkadianen Rhythmen; allen Arten von Schlafstörungen; Schlafbeeinträchtigungen im Zusammenhang mit Krankheiten wie neurologischen Störungen wie Nervenschmerzen und Restless-Legs-Syndrom; Insomnien in Verbindung mit psychiatrischen Störungen; Schlafapnoe; Narkolepsie; idiopathischen Insomnien; Parasomnien; stressbedingten Syndromen; benigner Prostatahypertrophie;
allen Arten des Psychotropikumgebrauchs und -missbrauchs; allen Demenzen und kognitiven Funktionsstörungen bei der gesunden Bevölkerung und bei psychiatrischen und neurologischen Störungen; und anderen Krankheiten in Verbindung mit allgemeinen Funktionsstörungen des Orexinsystems.

22. Verwendung nach Anspruch 21, wobei die genannten Schlafstörungen alle Arten von Insomnien, Narkolepsie und andere exzessive Schläfrigkeitsstörungen, schlafbedingte Dystonien, das Restless-Legs-Syndrom, Schlafapnoen, das Jet-Lag-Syndrom, Schichtarbeiter-Syndrom, verzögerte oder vorgelagerte Schlafphasen-Syndrom oder Insomien im Zusammenhang mit psychiatrischen Störungen umfassen.

23. Verwendung nach Anspruch 21, wobei die genannten kognitiven Funktionsstörungen Defizite bei allen Arten von Aufmerksamkeits-, Lern- und Gedächtnisfunktionen umfassen, die vorübergehend oder chronisch bei der normalen, gesunden, jungen, erwachsenen oder alternden Bevölkerung auftreten und auch vorübergehend oder chronisch bei psychiatrischen, neurologischen, Herz-Kreislauf- und Immunstörungen auftreten.

24. Verwendung nach Anspruch 21, wobei die genannten Essstörungen Stoffwechselfunktionsstörungen; fehlregulierte Appetitkontrolle; kompulsive Fettleibigkeit; Emeto-Bulimie oder Anorexia nervosa umfassen.

25. Verwendung nach Anspruch 21, wobei der genannte Psychotropikumgebrauch und -missbrauch alle Arten der psychologischen oder physischen Sucht und ihre verwandten Toleranz- und Abhängigkeitskomponenten umfassen.

## Revendications

1. Composé de formule (I) 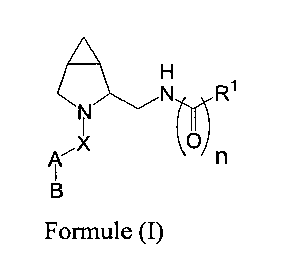 où
X représente C(O) ou SO₂ ;
A représente un groupement aryle ou hétérocyclyle, où le groupement aryle ou hétérocyclyle est non substitué ou indépendamment mono- ou disubstitué par des substituants indépendamment sélectionnés dans le groupe consistant en un alkyle en (C₁₋₄), cycloalkyle en (C₃₋₆), alkynyle en (C₂₋₆), hydroxy-alkyle en (C₁₋₄), hydroxy-alkynyle en (C₂₋₆), triméthylsilyl-éthynyle, cycloalkyle en (C₃₋₆)-éthynyle, alkoxy en (C₁₋₄), trifluorométhyle, trifluorométhoxy, NR²R³, N(R²)C(O)R³, C(O)NR²R³ et halogène ;
B représente un atome d'hydrogène ou un groupement aryle ou hétérocyclyle, où le groupement aryle ou hétérocyclyle est non substitué ou indépendamment mono-, di- ou trisubstitué par des substituants indépendamment sélectionnés dans le groupe consistant en un alkyle en (C₁₋₄), cycloalkyle en (C₃₋₆), alkoxy en (C₁₋₄), méthoxy-alkoxy en (C₁₋₄), cyano, trifluorométhyle, trifluorométhoxy, NR²R³, N(R²)C(O)R³, C(O)NR²R³ et halogène; ou B représente un groupement 2,3-dihydro-benzo[1,4]dioxinyle ;
ou A et B représentent ensemble un groupement tricyclique ;
n représente 0 ou 1 ;
R¹ représente un groupement aryle ou hétérocyclyle, où le groupement aryle ou hétérocyclyle est non substitué ou indépendamment mono-, di- ou trisubstitué par des substituants indépendamment sélectionnés dans le groupe consistant en un alkyle en (C₁₋₄), alkynyle en (C₂₋₆), cycloalkyle en (C₃₋₆), alkoxy en (C₁₋₄), alkyle en (C₁₋₄)-thio, halogène, hydroxy, cyano, trifluorométhyle, trifluorométhoxy, NR²R³, N(R²)C(O)R³, COOR² et C(O)NR²R³ ; ou R¹ représente un groupement hétérocyclyl-éthényle, hétérocyclyl-alkyle en (C₁₋₄) ou aryloxy-alkyle en (C₁₋₄), lesdits groupements étant non substitués ou indépendamment mono- ou disubstitués sur le fragment aryle ou hétérocyclyle par des substituants indépendamment sélectionnés dans le groupe consistant en un alkyle en (C₁₋₄), alkoxy en (C₁₋₄), halogène, trifluorométhyle, trifluorométhoxy et NR²R³ ; ou R¹ représente un groupement 2,3-dihydro-benzofuranyle, benzo[1,3]dioxolyle, 2,3-dihydro-benzo[1,4]dioxinyle, 4-oxo-4*H-*chroményle, 2*H*-chroményle, chromanyle, 4*H*-benzo[1,3]dioxinyle, 2,3-dihydro-thiéno[3,4-b][1,4]dioxinyle, morpholin-4-yl-phényle, pipérazin-1-yl-phényle, 3,4-dihydro-2*H*-benzo[1,4]oxazinyle, 3-oxo-3,4-dihydro-2*H*-benzo[1,4]oxazinyle ou 2,3,6,7-tétrahydro-benzo[1,2-b;4,5-b']difuranyle, lesdits groupements étant non substitués ou indépendamment mono- ou disubstitués par des substituants indépendamment sélectionnés dans le groupe consistant en un alkyle en (C₁₋₄), alkoxy en (C₁₋₄) et halogène ;
R² représente un hydrogène ou un alkyle en (C₁₋₄) ; et
R³ représente un hydrogène ou un alkyle en (C₁₋₄) ;
ou sel pharmaceutiquement acceptable de celui-ci.

2. Composé de formule générale (Ia) selon la revendication 1, où les centres stéréogènes sont en configuration relative *cis.* 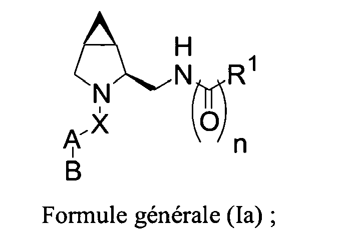 ou sel pharmaceutiquement acceptable de celui-ci.

3. Composé selon la revendication 1 ou 2, où
X représente C(O) ou SO₂ ;
A représente un groupement aryle ou hétérocyclyle, où le groupement aryle ou hétérocyclyle est non substitué ou indépendamment mono- ou disubstitué par des substituants indépendamment sélectionnés dans le groupe consistant en un alkyle en (C₁₋₄), cycloalkyle en (C₃₋₆), alkoxy en (C₁₋₄), trifluorométhyle, trifluorométhoxy, NR²R³, N(R²)C(O)R³, C(O)NR²R³ et halogène ;
B représente un atome d'hydrogène ou un groupement aryle ou hétérocyclyle, où le groupement aryle ou hétérocyclyle est non substitué ou indépendamment mono-, di- ou trisubstitué par des substituants indépendamment sélectionnés dans le groupe consistant en un alkyle en (C₁₋₄), cycloalkyle en (C₃₋₆), alkoxy en (C₁₋₄), trifluorométhyle, trifluorométhoxy, NR²R³, N(R²)C(O)R³, C(O)NR²R³ et halogène ; ou A et B représentent ensemble un groupement tricyclique ;
n représente 0 ou 1 ;
R¹ représente un groupement aryle ou hétérocyclyle, où le groupement aryle ou hétérocyclyle est non substitué ou indépendamment mono-, di- ou trisubstitué par des substituants indépendamment sélectionnés dans le groupe consistant en un alkyle en (C₁₋₄), cycloalkyle en (C₃₋₆), alkoxy en (C₁₋₄), halogène, hydroxy, cyano, trifluorométhyle, trifluorométhoxy, NR²R³, N(R²)C(O)R³ et C(O)NR²R³ ; ou R¹ représente un groupement hétérocyclyl-éthényle, hétérocyclyl-alkyle en (C₁₋₄) ou aryloxy-alkyle en (C₁₋₄), lesdits groupements étant non substitués ou indépendamment mono- ou disubstitués sur le fragment aryle ou hétérocyclyle par des substituants indépendamment sélectionnés dans le groupe consistant en un alkyle en (C₁₋₄), alkoxy en (C₁₋₄), halogène, trifluorométhyle, trifluorométhoxy et NR²R³ ; ou R¹ représente un groupement 2,3-dihydro-benzofuranyle, benzo[1,3]dioxolyle, 2,3-dihydro-benzo[1,4]dioxinyle ou 4-oxo-4*H*-chroményle, lesdits groupements étant non substitués ou monosubstitués sur le noyau aromatique par des substituants indépendamment sélectionnés dans le groupe consistant en un alkyle en (C₁₋₄), alkoxy en (C₁₋₄) et halogène ;
R² représente un hydrogène ou un alkyle en (C₁₋₄) ; et
R³ représente un hydrogène ou un alkyle en (C₁₋₄) ;
ou sel pharmaceutiquement acceptable de celui-ci.

4. Composés selon l'une quelconque des revendications 1 à 3, où A représente un groupement hétérocyclyle, où le groupement hétérocyclyle est non substitué ou monosubstitué par un substituant sélectionné dans le groupe consistant en un alkyle en (C₁₋₄) et NR²R³ ;
B représente un groupement aryle, où le groupement aryle est non substitué ou indépendamment mono-, di- ou trisubstitué par des substituants indépendamment sélectionnés dans le groupe consistant en un alkyle en (C₁₋₄), alkoxy en (C₁₋₄), trifluorométhyle et halogène ; et
R¹ représente un groupement aryle ou hétérocyclyle, où le groupement aryle ou hétérocyclyle est non substitué ou indépendamment mono-, di- ou trisubstitué par des substituants indépendamment sélectionnés dans le groupe consistant en un alkyle en (C₁₋₄), alkoxy en (C₁₋₄) et halogène ; ou R¹ représente un groupement 2,3-dihydro-benzofuranyle, benzo[1,3]dioxolyle ou 2,3-dihydro-benzo[1,4]dioxinyle ;
ou sel pharmaceutiquement acceptable de ceux-ci.

5. Composés selon l'une quelconque des revendications 1 à 4, où A représente un groupement oxazolyle, thiazolyle, pyrimidyle ou pyrazinyle, où lesdits groupements sont non substitués ou monosubstitués par un substituant sélectionné dans le groupe consistant en un alkyle en (C₁₋₄) et NR²R³ ;
B représente un groupement phényle, où le groupement phényle est non substitué ou indépendamment mono- ou disubstitué par des substituants indépendamment sélectionnés dans le groupe consistant en un alkyle en (C₁₋₄), alkoxy en (C₁₋₄), trifluorométhyle et halogène ; et
R¹ représente un groupement phényle, naphtyle, benzofuranyle, imidazo[2,1-b]thiazolyle, imidazo[1,2-a]pyridyle, pyrazolo[1,5-a]pyridyle, thiazolyle, isoxazolyle, pyrazolyle, indolyle, indazolyle, benzimidazolyle ou benzothiophényle, où lesdits groupements sont non substitués ou indépendamment mono- ou disubstitués par des substituants indépendamment sélectionnés dans le groupe consistant en un alkyle en (C₁₋₄), alkoxy en (C₁₋₄) et halogène ; ou R¹ représente un groupement 2,3-dihydro-benzofuranyle, benzo[1,3]dioxolyle ou 2,3-dihydro-benzo[1,4]dioxinyle ;
ou sel pharmaceutiquement acceptable de ceux-ci.

6. Composé selon l'une quelconque des revendications 1 à 5, où X représente C(O) ;
ou sel pharmaceutiquement acceptable de celui-ci.

7. Composé selon l'une quelconque des revendications 1 à 6, où n représente 1 ;
ou sel pharmaceutiquement acceptable de celui-ci.

8. Composé selon l'une quelconque des revendications 1, 2, 6 ou 7, où A représente un groupement aryle ou hétérocyclyle, où le groupement aryle ou hétérocyclyle est non substitué ou monosubstitué par un substituant sélectionné dans le groupe consistant en un alkyle en (C₁₋₄), cycloalkyle en (C₃₋₆), alkynyle en (C₂₋₆), hydroxy-alkyle en (C₁₋₄), hydroxy-alkynyle en (C₂₋₆), cycloalkyle en (C₃₋₆)-éthynyle, alkoxy en (C₁₋₄), NR²R³ et halogène ;
ou sel pharmaceutiquement acceptable de celui-ci.

9. Composé selon l'une quelconque des revendications 1, 2 ou 6 à 8, où B représente un groupement aryle ou hétérocyclyle, où le groupement aryle ou hétérocyclyle est non substitué ou indépendamment mono-, di- ou trisubstitué par des substituants indépendamment sélectionnés dans le groupe consistant en un alkyle en (C₁₋₄), alkoxy en (C₁₋₄), cyano, trifluorométhyle, NR²R³ et halogène ;
ou sel pharmaceutiquement acceptable de celui-ci.

10. Composé selon l'une quelconque des revendications 1, 2 ou 6 à 9, où R¹ représente un groupement aryle ou hétérocyclyle, où le groupement aryle ou hétérocyclyle est non substitué ou indépendamment mono-, di- ou trisubstitué par des substituants indépendamment sélectionnés dans le groupe consistant en un alkyle en (C₁₋₄), alkoxy en (C₁₋₄), halogène, hydroxy, cyano, trifluorométhyle et COOR² ; ou R¹ représente un groupement 2,3-dihydro-benzofuranyle, benzo[1,3]dioxolyle, 2,3-dihydro-benzo[1,4]dioxinyle, 2*H*-chroményle, chromanyle, 4*H-*benzo[1,3]dioxinyle, 2,3-dihydro-thiéno[3,4-b][1,4]dioxinyle, 3,4-dihydro-2*H-*benzo[1,4]oxazinyle ou 2,3,6,7-tétrahydro-benzo[1,2-b;4,5-b'] difuranyle, où lesdits groupements sont non substitués ou indépendamment mono- ou disubstitués par des substituants indépendamment sélectionnés dans le groupe consistant en un alkyle en (C₁₋₄), alkoxy en (C₁₋₄) et halogène ;
ou sel pharmaceutiquement acceptable de celui-ci.

11. Composé selon l'une quelconque des revendications 1, 2 ou 6 à 10, où A représente un groupement aryle, où le groupement aryle est non substitué ou monosubstitué par un substituant sélectionné dans le groupe consistant en un alkyle en (C₁₋₄), cycloalkyle en (C₃₋₆), cycloalkyle en (C₃₋₆)-éthynyle ou halogène ;
ou sel pharmaceutiquement acceptable de celui-ci.

12. Composé selon l'une quelconque des revendications 1, 2 ou 6 à 10, où A représente un groupement hétérocyclyle, où le groupement hétérocyclyle est non substitué ou monosubstitué par un substituant sélectionné dans le groupe consistant en un alkyle en (C₁₋₄), cycloalkyle en (C₃₋₆), hydroxy-alkyle en (C₁₋₄), hydroxy-alkynyle en (C₂₋₆), alkoxy en (C₁₋₄), NR²R³ et halogène ;
ou sel pharmaceutiquement acceptable de celui-ci.

13. Composé selon l'une quelconque des revendications 1 à 4 ou 6 à 12, où B représente un groupement phényle, où le groupement phényle est non substitué ou indépendamment mono-, di- ou trisubstitué par des substituants indépendamment sélectionnés dans le groupe consistant en un alkyle en (C₁₋₄), alkoxy en (C₁₋₄), trifluorométhyle et halogène ;
ou sel pharmaceutiquement acceptable de celui-ci.

14. Composé selon l'une quelconque des revendications 1 à 3 ou 6 à 13, où R¹ représente un groupement hétérocyclyle, où le groupement hétérocyclyle est non substitué ou indépendamment mono-, di- ou trisubstitué par des substituants indépendamment sélectionnés dans le groupe consistant en un alkyle en (C₁₋₄), alkoxy en (C₁₋₄), halogène et trifluorométhyle ;
ou sel pharmaceutiquement acceptable de celui-ci.

15. Composé selon l'une quelconque des revendications 1 à 3 ou 6 à 13, où R¹ représente un groupement aryle, où le groupement aryle est non substitué ou indépendamment mono-, di- ou trisubstitué par des substituants indépendamment sélectionnés dans le groupe consistant en un alkyle en (C₁₋₄), alkoxy en (C₁₋₄), halogène, hydroxy, cyano et trifluorométhyle ;
ou sel pharmaceutiquement acceptable de celui-ci.

16. Composé selon l'une quelconque des revendications 1, 2 ou 6 à 13, où R¹ représente un groupement 2,3-dihydro-benzofuranyle, 2,3-dihydro-benzo[1,4]dioxinyle, chromanyle, 2,3-dihydro-thiéno[3,4-b][1,4]dioxinyle ou 3,4-dihydro-2*H-*benzo[1,4]oxazinyle, où lesdits groupements sont non substitués ou monosubstitués par un substituant sélectionné dans le groupe consistant en un alkyle en (C₁₋₄) et un halogène ;
ou sel pharmaceutiquement acceptable de celui-ci.

17. Composé selon l'une quelconque des revendications 1 à 3 ou 6 à 14, où quand R¹ représente un groupement hétérocyclyle, ledit groupement hétérocyclyle est un groupement imidazo[2,1-b]thiazolyle ou imidazo[1,2-a]pyridyle, où lesdits groupements sont non substitués ou monosubstitués par un substituant sélectionné dans le groupe consistant en un alkyle en (C₁₋₄), halogène et trifluorométhyle ;
ou sel pharmaceutiquement acceptable de celui-ci.

18. Composé selon l'une quelconque des revendications 1, 2, 6 à 10 ou 12 à 17, où quand A représente un groupement hétérocyclyle, ledit groupement hétérocyclyle est un groupement thiazole qui est non substitué ou monosubstitué par un substituant sélectionné dans le groupe consistant en un alkyle en (C₁₋₄), hydroxy-alkyle en (C₁₋₄), hydroxy-alkynyle en (C₂₋₆), alkoxy en (C₁₋₄), NR²R³ et halogène ;
ou sel pharmaceutiquement acceptable de celui-ci.

19. Composé selon la revendication 1, qui est sélectionné dans le groupe consistant en les suivants :
4-fluoro-*N*- {(1R*,2S*,5S*)-3-[5-(3-fluoro-phényl)-2-méthyl-thiazole-4-carbonyl]-3-aza-bicyclo[3.1.0]hex-2-ylméthyl}-benzamide ;
[(1R*,2S*,5S*)-3-(biphényl-2-carbonyl)-3-aza-bicyclo[3.1.0]hex-2-ylméthyl]-amide de l'acide benzofuran-4-carboxylique ;
[(1R*,2S*,5S*)-3-(2-méthyl-5-*m*-tolyl-thiazole-4-carbonyl)-3-aza-bicyclo[3.1.0]hex-2-ylméthyl]-amide de l'acide benzofuran-4-carboxylique ;
{(1R*,2S*,5S*)-3-[5-(4-fluoro-phényl)-2-méthyl-thiazole-4-carbonyl]-3-aza-bicyclo[3.1.0]hex-2-ylméthyl}-amide de l'acide benzofuran-4-carboxylique ;
{(1R*,2S*,5S*)-3-[5-(3-fluoro-phényl)-2-méthyl-thiazole-4-carbonyl]-3-aza-bicyclo[3.1.0]hex-2-ylméthyl}-amide de l'acide benzofuran-4-carboxylique ; [(1R*,2S*,5S*)-3-(2-méthyl-5-*p*-tolyl-thiazole-4-carbonyl)-3-aza-bicyclo[3.1.0]hex-2-ylméthyl]-amide de l'acide benzofuran-4-carboxylique ;
{(1R*,2S*,5S*)-3-[5-(4-éthyl-phényl)-2-méthyl-thiazole-4-carbonyl]-3-aza-bicyclo[3.1.0]hex-2-ylméthyl}-amide de l'acide benzofuran-4-carboxylique ;
{(1R*,2S*,5S*)-3-[5-(2-fluoro-phényl)-2-méthyl-thiazole-4-carbonyl]-3-aza-bicyclo[3.1.0]hex-2-ylméthyl}-amide de l'acide benzofuran-4-carboxylique ;
{(1R*,2S*,5S*)-3-[5-(2-chloro-phényl)-2-méthyl-thiazole-4-carbonyl]-3-aza-bicyclo[3.1.0]hex-2-ylméthyl}-amide de l'acide benzofuran-4-carboxylique ;
{(1R*,2S*,5S*)-3-[5-(3-chloro-phényl)-2-méthyl-thiazole-4-carbonyl]-3-aza-bicyclo[3.1.0]hex-2-ylméthyl}-amide de l'acide benzofuran-4-carboxylique ;
{(1R*,2S*,5S*)-3-[5-(2-méthoxy-phényl)-2-méthyl-thiazole-4-carbonyl]-3-aza-bicyclo[3.1.0]hex-2-ylméthyl}-amide de l'acide benzofuran-4-carboxylique ;
{(1R*,2S*,5S*)-3-[5-(3-méthoxy-phényl)-2-méthyl-thiazole-4-carbonyl]-3-aza-bicyclo[3.1.0]hex-2-ylméthyl}-amide de l'acide benzofuran-4-carboxylique ;
{(1R*,2S*,5S*)-3-[2-méthyl-5-(3-trifluorométhyl-phényl)-thiazole-4-carbonyl]-3-aza-bicyclo[3.1.0]hex-2-ylméthyl}-amide de l'acide benzofuran-4-carboxylique ;
{(1R*,2S*,5S*)-3-[2-méthyl-5-(2-trifluorométhyl-phényl)-thiazole-4-carbonyl]-3-aza-bicyclo[3.1.0]hex-2-ylméthyl}-amide de l'acide benzofuran-4-carboxylique ; [(1R*,2S*,5S*)-3-(2-méthyl-5-o-tolyl-thiazole-4-carbonyl)-3-aza-bicyclo[3.1.0]hex-2-ylméthyl]-amide de l'acide benzofuran-4-carboxylique ;
{(1R*,2S*,5S*)-3-[5-(3,4-diméthyl-phényl)-2-méthyl-thiazole-4-carbonyl]-3-aza-bicyclo[3.1.0]hex-2-ylméthyl}-amide de l'acide benzofuran-4-carboxylique ; [(1R*,2S*,5S*)-3-(2-amino-5-*m*-tolyl-thiazole-4-carbonyl)-3-aza-bicyclo[3.1.0]hex-2-ylméthyl]-amide de l'acide benzofuran-4-carboxylique ; {(1R*,2S*,5S*)-3-[2-amino-5-(3-fluoro-phényl)-thiazole-4-carbonyl]-3-aza-bicyclo[3.1.0]hex-2-ylméthyl}-amide de l'acide benzofuran-4-carboxylique ;
[(1R*,2S*,5S*)-3-(2-méthyl-4-phényl-pyrimidine-5-carbonyl)-3-aza-bicyclo[3.1.0]hex-2-ylméthyl]-amide de l'acide benzofuran-4-carboxylique ;
{(1R*,2S*,5S*)-3-[2-(2-amino-thiazol-4-yl)-benzoyl]-3-aza-bicyclo[3.1.0]hex-2-ylméthyl}-amide de l'acide benzofuran-4-carboxylique ; [(1R*,2S*,5S*)-3-(9*H*-fluorène-4-carbonyl)-3-aza-bicyclo[3.1.0]hex-2-ylméthyl]-amide de l'acide benzofuran-4-carboxylique ;
[(1R*,2S*,5S*)-3-(3-phényl-pyrazine-2-carbonyl)-3-aza-bicyclo[3.1.0]hex-2-ylméthyl]-amide de l'acide benzofuran-4-carboxylique ; {(1R*,2S*,5S*)-3-[5-(4-méthoxy-phényl)-oxazole-4-carbonyl]-3-aza-bicyclo[3.1.0]hex-2-ylméthyl}-amide de l'acide benzofuran-4-carboxylique ; [(1R*,2S*,5S*)-3-(2'-fluoro-biphényl-2-carbonyl)-3-aza-bicyclo[3.1.0]hex-2-ylméthyl]-amide de l'acide benzofuran-4-carboxylique ; [(1R*,2S*,5S*)-3-(4'-fluoro-biphényl-2-carbonyl)-3-aza-bicyclo[3.1.0]hex-2-ylméthyl]-amide de l'acide benzofuran-4-carboxylique ; [(1R*,2S*,5S*)-3-(2'-chloro-biphényl-2-carbonyl)-3-aza-bicyclo[3.1.0]hex-2-ylméthyl]-amide de l'acide benzofuran-4-carboxylique ; [(1R*,2S*,5S*)-3-(3'-chloro-biphényl-2-carbonyl)-3-aza-bicyclo[3.1.0]hex-2-ylméthyl]-amide de l'acide benzofuran-4-carboxylique ; [(1R*,2S*,5S*)-3-(4'-chloro-biphényl-2-carbonyl)-3-aza-bicyclo[3.1.0]hex-2-ylméthyl]-amide de l'acide benzofuran-4-carboxylique ; [(1R*,2S*,5S*)-3-(4'-méthyl-biphényl-2-carbonyl)-3-aza-bicyclo[3.1.0]hex-2-ylméthyl]-amide de l'acide benzofuran-4-carboxylique ; [(1R*,2S*,5S*)-3-(3'-méthyl-biphényl-2-carbonyl)-3-aza-bicyclo[3.1.0]hex-2-ylméthyl]-amide de l'acide benzofuran-4-carboxylique ; [(1R*,2S*,5S*)-3-(3'-méthoxy-biphényl-2-carbonyl)-3-aza-bicyclo[3.1.0]hex-2-ylméthyl]-amide de l'acide benzofuran-4-carboxylique ;
[(1R*,2S*,5S*)-3-(4'-méthoxy-biphényl-2-carbonyl)-3-aza-bicyclo[3.1.0]hex-2-ylméthyl]-amide de l'acide benzofuran-4-carboxylique ; [(1R*,2S*,5S*)-3-(3'-trifluorométhyl-biphényl-2-carbonyl)-3-aza-bicyclo[3.1.0]hex-2-ylméthyl]-amide de l'acide benzofuran-4-carboxylique ; [(1R*,2S*,5S*)-3-(2-pyridin-3-yl-benzoyl)-3-aza-bicyclo[3.1.0]hex-2-ylméthyl]-amide de l'acide benzofuran-4-carboxylique ;
[(1R*,2S*,5S*)-3-(2-méthyl-5-*p*-tolyl-thiazole-4-carbonyl)-3-aza-bicyclo[3.1.0]hex-2-ylméthyl]-amide de l'acide 6-méthyl-imidazo[2,1-b]thiazole-5-carboxylique ;
[(1R*,2S*,5S*)-3-(2-méthyl-5-*m*-tolyl-thiazole-4-carbonyl)-3-aza-bicyclo[3.1.0]hex-2-ylméthyl]-amide de l'acide 6-méthyl-imidazo[2,1-b]thiazole-5-carboxylique ;
{(1R*,2S*,5S*)-3-[5-(3-fluoro-phényl)-2-méthyl-thiazole-4-carbonyl]-3-aza-bicyclo[3.1.0]hex-2-ylméthyl}-amide de l'acide 6-méthyl-imidazo[2,1-b]thiazole-5-carboxylique ; {(1R*,2S*,5S*)-3-[5-(4-éthyl-phényl)-2-méthyl-thiazole-4-carbonyl]-3-aza-bicyclo[3.1.0]hex-2-ylméthyl}-amide de l'acide 6-méthyl-imidazo[2,1-b]thiazole-5-carboxylique ;
{(1R*,2S*,5S*)-3-[5-(4-fluoro-phényl)-2-méthyl-thiazole-4-carbonyl]-3-aza-bicyclo[3.1.0]hex-2-ylméthyl}-amide de l'acide 6-méthyl-imidazo[2,1-b]thiazole-5-carboxylique ;
[(1R*,2S*,5S*)-3-(biphényl-2-carbonyl)-3-aza-bicyclo[3.1.0]hex-2-ylméthyl]-amide de l'acide 6-méthyl-imidazo[2,1-b]thiazole-5-carboxylique ;
{(1R*,2S*,5S*)-2-[(6,7-difluoro-quinoxalin-2-ylamino)-méthyl]-3-aza-bicyclo[3.1.0]hex-3-yl}-(2-méthyl-5-*m*-tolyl-thiazol-4-yl)-méthanone ;
{(1R*,2S*,5S*)-2-[(6,7-difluoro-quinoxalin-2-ylamino)-méthyl]-3-aza-bicyclo[3.1.0]hex-3-yl}-[5-(4-fluoro-phényl)-2-méthyl-thiazol-4-yl]-méthanone ;
{(1R*,2S*,5S*)-2-[(5-bromo-pyrimidin-2-ylamino)-méthyl]-3-aza-bicyclo[3.1.0]hex-3-yl}-(2-méthyl-5-*m*-tolyl-thiazol-4-yl)-méthanone ;
[(1R*,2S*,5S*)-3-(2-méthyl-5-*m*-tolyl-thiazole-4-carbonyl)-3-aza-bicyclo[3.1.0]hex-2-ylméthyl]-amide de l'acide naphtalène-2-carboxylique ;
[(1R*,2S*,5S*)-3-(2-méthyl-5-*m*-tolyl-thiazole-4-carbonyl)-3-aza-bicyclo[3.1.0]hex-2-ylméthyl]-amide de l'acide naphtalène-1-carboxylique ;
[(1R*,2S*,5S*)-3-(2-méthyl-5-*m*-tolyl-thiazole-4-carbonyl)-3-aza-bicyclo[3.1.0]hex-2-ylméthyl]-amide de l'acide 2,3-dihydro-benzofuran-7-carboxylique ;
[(1R*,2S*,5S*)-3-(2-méthyl-5-*m*-tolyl-thiazole-4-carbonyl)-3-aza-bicyclo[3.1.0]hex-2-ylméthyl]-amide de l'acide 1*H*-indole-5-carboxylique ; 2-hydroxy-*N*-[(1R*,2S*,5S*)-3-(2-méthyl-5-*m*-tolyl-thiazole-4-carbonyl)-3-aza-bicyclo[3.1.0]hex-2-ylméthyl]-benzamide ;
[(1R*,2S*,5S*)-3-(2-méthyl-5-*m*-tolyl-thiazole-4-carbonyl)-3-aza-bicyclo[3.1.0]hex-2-ylméthyl]-amide de l'acide 2-bromo-4-méthyl-thiazole-5-carboxylique ; [(1R*,2S*,5S*)-3-(2-méthyl-5-*m*-tolyl-thiazole-4-carbonyl)-3-aza-bicyclo[3.1.0]hex-2-ylméthyl]-amide de l'acide furan-3-carboxylique ; [(1R*,2S*,5S*)-3-(2-méthyl-5-*m*-tolyl-thiazole-4-carbonyl)-3-aza-bicyclo[3.1.0]hex-2-ylméthyl]-amide de l'acide 3,5-diméthyl-isoxazole-4-carboxylique ; 3,5-diméthoxy-*N*-[(1R*,2S*,5S*)-3-(2-méthyl-5-*m*-tolyl-thiazole-4-carbonyl)-3-aza-bicyclo[3.1.0]hex-2-ylméthyl]-benzamide ;
[(1R*,2S*,5S*)-3-(2-méthyl-5-*m*-tolyl-thiazole-4-carbonyl)-3-aza-bicyclo[3.1.0]hex-2-ylméthyl]-amide de l'acide benzo[1,3]dioxole-5-carboxylique ; 2,4-diméthoxy-*N*-[(1R*,2S*,5S*)-3-(2-méthyl-5-*m*-tolyl-thiazole-4-carbonyl)-3-aza-bicyclo[3.1.0]hex-2-ylméthyl]-benzamide ;
[(1R*,2S*,5S*)-3-(2-méthyl-5-*m*-tolyl-thiazole-4-carbonyl)-3-aza-bicyclo[3.1.0]hex-2-ylméthyl]-amide de l'acide 2,4-diméthyl-thiazole-5-carboxylique ;
[(1R*,2S*,5S*)-3-(2-méthyl-5-*m*-tolyl-thiazole-4-carbonyl)-3-aza-bicyclo[3.1.0]hex-2-ylméthyl]-amide de l'acide 1-méthyl-1*H*-indole-2-carboxylique ; [(1R*,2S*,5S*)-3-(2-méthyl-5-*m*-tolyl-thiazole-4-carbonyl)-3-aza-bicyclo [3.1.0]hex-2-ylméthyl]-amide de l'acide 3*H*-benzoimidazole-5-carboxylique; [(1R*,2S*,5S*)-3-(2-méthyl-5-*m*-tolyl-thiazole-4-carbonyl)-3-aza-bicyclo[3.1.0]hex-2-ylméthyl]-amide de l'acide benzo[2,1,3]oxadiazole-5-carboxylique ; [(1R*,2S*,5S*)-3-(2-méthyl-5-*m*-tolyl-thiazole-4-carbonyl)-3-aza-bicyclo[3.1.0]hex-2-ylméthyl]-amide de l'acide 2,3-dihydro-benzo[1,4]dioxine-5-carboxylique ; [(1R*,2S*,5S*)-3-(2-méthyl-5-*m*-tolyl-thiazole-4-carbonyl)-3-aza-bicyclo[3.1.0]hex-2-ylméthyl]-amide de l'acide 5-chloro-1,3-diméthyl-1*H*-pyrazole-4-carboxylique ;
[(1R*,2S*,5S*)-3-(2-méthyl-5-*m*-tolyl-thiazole-4-carbonyl)-3-aza-bicyclo[3.1.0]hex-2-ylméthyl]-amide de l'acide benzo[b]thiophène-2-carboxylique ; [(1R*,2S*,5S*)-3-(2-méthyl-5-*m*-tolyl-thiazole-4-carbonyl)-3-aza-bicyclo[3.1.0]hex-2-ylméthyl]-amide de l'acide 5-*tert*-butyl-2-méthyl-2*H*-pyrazole-3-carboxylique; [(1R*,2S*,5S*)-3-(2-méthyl-5-*m*-tolyl-thiazole-4-carbonyl)-3-aza-bicyclo[3.1.0]hex-2-ylméthyl]-amide de l'acide 1-méthyl-1*H*-indazole-3-carboxylique ; [(1R*,2S*,5S*)-3-(2-méthyl-5-*m*-tolyl-thiazole-4-carbonyl)-3-aza-bicyclo[3.1.0]hex-2-ylméthyl]-amide de l'acide 1-méthyl-1*H*-pyrrole-2-carboxylique ;
[(1R*,2S*,5S*)-3-(2-méthyl-5-*m*-tolyl-thiazole-4-carbonyl)-3-aza-bicyclo[3.1.0]hex-2-ylméthyl]-amide de l'acide 2,8-diméthyl-imidazo[1,2-a]pyridine-3-carboxylique ; 6-isobutyl-5-méthyl-*N*-[(1R*,2S*,5S*)-3-(2-méthyl-5-*m*-tolyl-thiazole-4-carbonyl)-3-aza-bicyclo[3.1.0]hex-2-ylméthyl]-nicotinamide ; [(1R*,2S*,5S*)-3-(2-méthyl-5-*m*-tolyl-thiazole-4-carbonyl)-3-aza-bicyclo[3.1.0]hex-2-ylméthyl]-amide de l'acide pyrazolo[1,5-a]pyridine-3-carboxylique ; [(1R*,2S*,5S*)-3-(2-méthyl-5-*m*-tolyl-thiazole-4-carbonyl)-3-aza-bicyclo[3.1.0]hex-2-ylméthyl]-amide de l'acide benzo[d]isoxazole-3-carboxylique ; [(1R*,2S*,5S*)-3-(2-méthyl-5-*m*-tolyl-thiazole-4-carbonyl)-3-aza-bicyclo[3.1.0]hex-2-ylméthyl]-amide de l'acide 6-fluoro-4*H*-benzo[1,3]dioxine-8-carboxylique; [(1R*,2S*,5S*)-3-(2-méthyl-5-*m*-tolyl-thiazole-4-carbonyl)-3-aza-bicyclo[3.1.0]hex-2-ylméthyl]-amide de l'acide 2,3-dihydro-benzofuran-4-carboxylique ; [(1R*,2S*,5S*)-3-(2-méthyl-5-*m*-tolyl-thiazole-4-carbonyl)-3-aza-bicyclo[3.1.0]hex-2-ylméthyl]-amide de l'acide isoquinoline-1-carboxylique ; [(1R*,2S*,5S*)-3-(2-méthyl-5-*m*-tolyl-thiazole-4-carbonyl)-3-aza-bicyclo[3.1.0]hex-2-ylméthyl]-amide de l'acide quinoline-8-carboxylique ; [(1R*,2S*,5S*)-3-(2-méthyl-5-*m*-tolyl-thiazole-4-carbonyl)-3-aza-bicyclo[3.1.0]hex-2-ylméthyl]-amide de l'acide quinoline-2-carboxylique ; [(1R*,2S*,5S*)-3-(2-méthyl-5-*m*-tolyl-thiazole-4-carbonyl)-3-aza-bicyclo[3.1.0]hex-2-ylméthyl]-amide de l'acide imidazo[2,1-b]thiazole-5-carboxylique ; [(1R*,2S*,5S*)-3-(2-méthyl-5-*m*-tolyl-thiazole-4-carbonyl)-3-aza-bicyclo [3.1.0]hex-2-ylméthyl]-amide de l'acide 3-méthyl-imidazo[2,1-b]thiazole-2-carboxylique ; [(1R*,2S*,5S*)-3-(2-méthyl-5-*m*-tolyl-thiazole-4-carbonyl)-3-aza-bicyclo[3.1.0]hex-2-ylméthyl]-amide de l'acide 1-méthyl-1*H*-indole-3-carboxylique ; [(1R*,2S*,5S*)-3-(2-méthyl-5-*m*-tolyl-thiazole-4-carbonyl)-3-aza-bicyclo[3.1.0]hex-2-ylméthyl]-amide de l'acide 1*H*-indole-3-carboxylique ; [(1R*,2S*,5S*)-3-(2-méthyl-5-*m*-tolyl-thiazole-4-carbonyl)-3-aza-bicyclo[3.1.0]hex-2-ylméthyl]-amide de l'acide 1*H*-indazole-3-carboxylique ; [(1R*,2S*,5S*)-3-(2-méthyl-5-*m*-tolyl-thiazole-4-carbonyl)-3-aza-bicyclo[3.1.0]hex-2-ylméthyl]-amide de l'acide imidazo[1,2-a]pyridine-3-carboxylique ; [(1R*,2S*,5S*)-3-(2-méthyl-5-*m*-tolyl-thiazole-4-carbonyl)-3-aza-bicyclo[3.1.0]hex-2-ylméthyl]-amide de l'acide 2,3-dihydro-thiéno[3,4-b][1,4]dioxine-5-carboxylique ;
3-bromo-*N*-[(1R*,2S*,5S*)-3-(2-méthyl-5-*m*-tolyl-thiazole-4-carbonyl)-3-aza-bicyclo[3.1.0]hex-2-ylméthyl]-benzamide ; *N*-[(1R*,2S*,5S*)-3-(2-méthyl-5-*m*-tolyl-thiazole-4-carbonyl)-3-aza-bicyclo[3.1.0]hex-2-ylméthyl]-3-trifluorométhyl-benzamide ; 3-méthoxy-*N*-[(1R*,2S*,5S*)-3-(2-méthyl-5-*m*-tolyl-thiazole-4-carbonyl)-3-aza-bicyclo[3.1.0]hex-2-ylméthyl]-benzamide ;
3-fluoro-4-méthoxy-*N*-[(1R*,2S*,5S*)-3-(2-méthyl-5-*m*-tolyl-thiazole-4-carbonyl)-3-aza-bicyclo[3.1.0]hex-2-ylméthyl]-benzamide ; 3,4-dichloro-*N*-[(1R*,2S*,5S*)-3-(2-méthyl-5-*m*-tolyl-thiazole-4-carbonyl)-3-aza-bicyclo[3.1.0]hex-2-ylméthyl]-benzamide ; 2-chloro-4,5-difluoro-*N*-[(1R*,2S*,5S*)-3-(2-méthyl-5-*m*-tolyl-thiazole-4-carbonyl)-3-aza-bicyclo[3.1.0]hex-2-ylméthyl]-benzamide ; 2-fluoro-5-méthyl-*N*-[(1R*,2S*,5S*)-3-(2-méthyl-5-*m*-tolyl-thiazole-4-carbonyl)-3-aza-bicyclo[3.1.0]hex-2-ylméthyl]-benzamide ; 3-fluoro-2-méthyl-*N*-[(1R*,2S*,5S*)-3-(2-méthyl-5-*m*-tolyl-thiazole-4-carbonyl)-3-aza-bicyclo[3.1.0]hex-2-ylméthyl]-benzamide ;
5-fluoro-2-méthoxy-*N*-[(1R*,2S*,5S*)-3-(2-méthyl-5-*m*-tolyl-thiazole-4-carbonyl)-3-aza-bicyclo[3.1.0]hex-2-ylméthyl]-benzamide ; 2-chloro-3-fluoro-*N*-[(1R*,2S*,5S*)-3-(2-méthyl-5-*m*-tolyl-thiazole-4-carbonyl)-3-aza-bicyclo[3.1.0]hex-2-ylméthyl]-benzamide ; 2,5-diméthyl-*N*-[(1R*,2S*,5S*)-3-(2-méthyl-5-*m*-tolyl-thiazole-4-carbonyl)-3-aza-bicyclo[3.1.0]hex-2-ylméthyl]-benzamide ; 3,4-diméthyl-*N*-[(1R*,2S*,5S*)-3-(2-méthyl-5-*m*-tolyl-thiazole-4-carbonyl)-3-aza-bicyclo[3.1.0]hex-2-ylméthyl]-benzamide ; 2,5-diméthoxy-*N*-[(1R*,2S*,5S*)-3-(2-méthyl-5-*m*-tolyl-thiazole-4-carbonyl)-3-aza-bicyclo[3.1.0]hex-2-ylméthyl]-benzamide ;
ester méthylique de l'acide *N*-[(1R*,2S*,5S*)-3-(2-méthyl-5-*m*-tolyl-thiazole-4-carbonyl)-3-aza-bicyclo[3.1.0]hex-2-ylméthyl]-isophtalamique ; 2-chloro-4-fluoro-*N*-[(1R*,2S*,5S*)-3-(2-méthyl-5-*m*-tolyl-thiazole-4-carbonyl)-3-aza-bicyclo[3.1.0]hex-2-ylméthyl]-benzamide ; 2-chloro-3-méthyl-*N*-[(1R*,2S*,5S*)-3-(2-méthyl-5-*m*-tolyl-thiazole-4-carbonyl)-3-aza-bicyclo[3.1.0]hex-2-ylméthyl]-benzamide ;
3,5-dichloro-4-hydroxy-*N-*[(1R*,2S*,5S*)-3-(2-méthyl-5-*m*-tolyl-thiazole-4-carbonyl)-3-aza-bicyclo[3.1.0]hex-2-ylméthyl]-benzamide ; 2,4-dichloro-*N*-[(1R*,2S*,5S*)-3-(2-méthyl-5-*m*-tolyl-thiazole-4-carbonyl)-3-aza-bicyclo[3.1.0]hex-2-ylméthyl]-benzamide ; 4-méthyl-*N*-[(1R*,2S*,5S*)-3-(2-méthyl-5-*m*-tolyl-thiazole-4-carbonyl)-3-aza-bicyclo[3.1.0]hex-2-ylméthyl]-3-trifluorométhyl-benzamide ; 4-méthoxy-2-méthyl-*N*-[(1R*,2S*,5S*)-3-(2-méthyl-5-*m*-tolyl-thiazole-4-carbonyl)-3-aza-bicyclo[3.1.0]hex-2-ylméthyl]-benzamide ;
4-éthyl-*N*-[(1R*,2S*,5S*)-3-(2-méthyl-5-*m*-tolyl-thiazole-4-carbonyl)-3-aza-bicyclo[3.1.0]hex-2-ylméthyl]-benzamide ; 4-méthoxy-3-méthyl-*N*-[(1R*,2S*,5S*)-3-(2-méthyl-5-*m*-tolyl-thiazole-4-carbonyl)-3-aza-bicyclo[3.1.0]hex-2-ylméthyl]-benzamide ; 3,5-diméthyl-*N*-[(1R*,2S*,5S*)-3-(2-méthyl-5-*m*-tolyl-thiazole-4-carbonyl)-3-aza-bicyclo[3.1.0]hex-2-ylméthyl]-benzamide ; 5-bromo-2-chloro-*N*-[(1R*,2S*,5S*)-3-(2-méthyl-5-*m*-tolyl-thiazole-4-carbonyl)-3-aza-bicyclo[3.1.0]hex-2-ylméthyl]-benzamide ; 3-cyano-*N*-[(1R*,2S*,5S*)-3-(2-méthyl-5-*m*-tolyl-thiazole-4-carbonyl)-3-aza-bicyclo[3.1.0]hex-2-ylméthyl]-benzamide ; 4-cyano-*N*-[(1R*,2S*,5S*)-3-(2-méthyl-5-*m*-tolyl-thiazole-4-carbonyl)-3-aza-bicyclo[3.1.0]hex-2-ylméthyl]-benzamide ; 4-chloro-*N*-[(1R*,2S*,5S*)-3-(2-méthyl-5-*m*-tolyl-thiazole-4-carbonyl)-3-aza-bicyclo[3.1.0]hex-2-ylméthyl]-benzamide ; 3-iodo-*N*-[(1R*,2S*,5S*)-3-(2-méthyl-5-*m*-tolyl-thiazole-4-carbonyl)-3-aza-bicyclo[3.1.0]hex-2-ylméthyl]-benzamide ; 2-bromo-3-méthyl-*N*-[(1R*,2S*,5S*)-3-(2-méthyl-5-*m*-tolyl-thiazole-4-carbonyl)-3-aza-bicyclo[3.1.0]hex-2-ylméthyl]-benzamide ;
[(1R*,2S*,5S*)-3-(2-méthyl-5-*m*-tolyl-thiazole-4-carbonyl)-3-aza-bicyclo[3.1.0]hex-2-ylméthyl]-amide de l'acide 5-méthyl-imidazo[2,1-b]thiazole-6-carboxylique ;
[(1R*,2S*,5S*)-3-(2-méthyl-5-*m*-tolyl-thiazole-4-carbonyl)-3-aza-bicyclo [3.1.0]hex-2-ylméthyl]-amide de l'acide 3,5-diméthyl-imidazo[2,1-b]thiazole-6-carboxylique ;
[(1R*,2S*,5S*)-3-(2-méthyl-5-*m*-tolyl-thiazole-4-carbonyl)-3-aza-bicyclo[3.1.0]hex-2-ylméthyl]-amide de l'acide 2,6-diméthyl-imidazo[2,1-b]thiazole-5-carboxylique ;
[(1R*,2S*,5S*)-3-(2-méthyl-5-*m*-tolyl-thiazole-4-carbonyl)-3-aza-bicyclo[3.1.0]hex-2-ylméthyl]-amide de l'acide 2,3,5-triméthyl-imidazo[2,1-b]thiazole-6-carboxylique ; [(1R*,2S*,5S*)-3-(2-méthyl-5-*m*-tolyl-thiazole-4-carbonyl)-3-aza-bicyclo[3.1.0]hex-2-ylméthyl]-amide de l'acide 2-méthyl-imidazo[2,1-b]thiazole-5-carboxylique ;
[(1R*,2S*,5S*)-3-(2-méthyl-5-*m*-tolyl-thiazole-4-carbonyl)-3-aza-bicyclo [3.1.O]hex-2-ylméthyl]-amide de l'acide 6-trifluorométhyl-imidazo[2,1-b]thiazole-5-carboxylique ;
[(1R*,2S*,5S*)-3-(2-méthyl-5-*m*-tolyl-thiazole-4-carbonyl)-3-aza-bicyclo [3.1.0]hex-2-ylméthyl]-amide de l'acide 3,6-diméthyl-imidazo[2,1-b]thiazole-5-carboxylique ; [(1R*,2S*,5S)-3-(2-méthyl-5-*m*-tolyl-thiazole-4-carbonyl)-3-aza-bicyclo[3.1.0]hex-2-ylméthyl]-amide de l'acide 3-méthyl-imidazo[2,1-b]thiazole-5-carboxylique ; [(1R*,2S*,5S*)-3-(2-méthyl-5-*m*-tolyl-thiazole-4-carbonyl)-3-aza-bicyclo[3.1.0]hex-2-ylméthyl]-amide de l'acide 6-chloro-imidazo[2,1-b]thiazole-5-carboxylique ;
[(1R*,2S*,5S*)-3-(2-méthyl-5-*m*-tolyl-thiazole-4-carbonyl)-3-aza-bicyclo[3.1.0]hex-2-ylméthyl]-amide de l'acide 2*H*-chromène-5-carboxylique; [(1R*,2S*,5S*)-3-(2-méthyl-5-*m*-tolyl-thiazole-4-carbonyl)-3-aza-bicyclo[3.1.0]hex-2-ylméthyl]-amide de l'acide 4-méthyl-3,4-dihydro-2*H*-benzo[1,4]oxazine-8-carboxylique ;
[(1R*,2S*,5S*)-3-(2-méthyl-5-*m*-tolyl-thiazole-4-carbonyl)-3-aza-bicyclo[3.1.0]hex-2-ylméthyl]-amide de l'acide chroman-8-carboxylique ;
[(1R*,2S*,5S*)-3-(2-méthyl-5-*m*-tolyl-thiazole-4-carbonyl)-3-aza-bicyclo[3.1.0]hex-2-ylméthyl]-amide de l'acide 3-oxo-3,4-dihydro-2*H*-benzo[1,4]oxazine-5-carboxylique ;
[(1R*,2S*,5S*)-3-(2-méthyl-5-*m*-tolyl-thiazole-4-carbonyl)-3-aza-bicyclo[3.1.0]hex-2-ylméthyl]-amide de l'acide 3,4-dihydro-2*H*-benzo[1,4]oxazine-8-carboxylique; [(1R*,2S*,5S*)-3-(2-méthyl-5-*m*-tolyl-thiazole-4-carbonyl)-3-aza-bicyclo[3.1.0]hex-2-ylméthyl]-amide de l'acide 3,4-dihydro-2*H*-benzo[1,4] oxazine-5-carboxylique; [(1R*,2S*,5S*)-3-(2-méthyl-5-*m*-tolyl-thiazole-4-carbonyl)-3-aza-bicyclo[3.1.0]hex-2-ylméthyl]-amide de l'acide 2-méthyl-benzofuran-4-carboxylique ;
{(1R*,2S*,5S*)-3-[5-(3-chloro-phényl)-2-méthyl-thiazole-4-carbonyl]-3-aza-bicyclo[3.1.0]hex-2-ylméthyl}-amide de l'acide benzo[d]isoxazole-3-carboxylique ;
{(1R*,2S*,5S*)-3-[5-(3-chloro-phényl)-2-méthyl-thiazole-4-carbonyl]-3-aza-bicyclo[3.1.0]hex-2-ylméthyl}-amide de l'acide 2,3-dihydro-benzo[1,4]dioxine-5-carboxylique ; {(1R*,2S*,5S*)-3-[5-(3-chloro-phényl)-2-méthyl-thiazole-4-carbonyl]-3-aza-bicyclo[3.1.0]hex-2-ylméthyl}-amide de l'acide 6-fluoro-4*H*-benzo[1,3]dioxine-8-carboxylique ;
{(1R*,2S*,5S*)-3-[5-(3-chloro-phényl)-2-méthyl-thiazole-4-carbonyl]-3-aza-bicyclo[3.1.0]hex-2-ylméthyl}-amide de l'acide 2,3-dihydro-benzofuran-4-carboxylique ;
{(1R*,2S*,5S*)-3-[5-(3-chloro-phényl)-2-méthyl-thiazole-4-carbonyl]-3-aza-bicyclo[3.1.0]hex-2-ylméthyl}-amide de l'acide isoquinoline-1-carboxylique ;
{(1R*,2S*,5S*)-3-[5-(3-chloro-phényl)-2-méthyl-thiazole-4-carbonyl]-3-aza-bicyclo[3.1.0]hex-2-ylméthyl}-amide de l'acide quinoline-8-carboxylique ;
{(1R*,2S*,5S*)-3-[5-(3-chloro-phényl)-2-méthyl-thiazole-4-carbonyl]-3-aza-bicyclo[3.1.0]hex-2-ylméthyl}-amide de l'acide quinoline-2-carboxylique ;
{(1R*,2S*,5S*)-3-[5-(3-chloro-phényl)-2-méthyl-thiazole-4-carbonyl]-3-aza-bicyclo[3.1.0]hex-2-ylméthyl}-amide de l'acide imidazo[2,1-b]thiazole-5-carboxylique ;
{(1R*,2S*,5S*)-3-[5-(3-chloro-phényl)-2-méthyl-thiazole-4-carbonyl]-3-aza-bicyclo[3.1.0]hex-2-ylméthyl}-amide de l'acide 3-méthyl-imidazo[2,1-b]thiazole-2-carboxylique ;
{(1R*,2S*,5S*)-3-[5-(3-chloro-phényl)-2-méthyl-thiazole-4-carbonyl]-3-aza-bicyclo[3.1.0]hex-2-ylméthyl}-amide de l'acide 1-méthyl-1*H*-indole-3-carboxylique; {(1R*,2S*,5S*)-3-[5-(3-chloro-phényl)-2-méthyl-thiazole-4-carbonyl]-3-aza-bicyclo[3.1.0]hex-2-ylméthyl}-amide de l'acide 1,2-diméthyl-1*H*- indole-3-carboxylique ;
{(1R*,2S*,5S*)-3-[5-(3-chloro-phényl)-2-méthyl-thiazole-4-carbonyl]-3-aza-bicyclo[3.1.0]hex-2-ylméthyl}-amide de l'acide 1*H*-indole-3-carboxylique ;
{(1R*,2S*,5S*)-3-[5-(3-chloro-phényl)-2-méthyl-thiazole-4-carbonyl]-3-aza-bicyclo[3.1.0]hex-2-ylméthyl}-amide de l'acide 1*H*-indazole-3-carboxylique ;
{(1R*,2S*,5S*)-3-[5-(3-chloro-phényl)-2-méthyl-thiazole-4-carbonyl]-3-aza-bicyclo[3.1.0]hex-2-ylméthyl}-amide de l'acide imidazo[1,2-a]pyridine-3-carboxylique ;
{(1R*,2S*,5S*)-3-[5-(3-chloro-phényl)-2-méthyl-thiazole-4-carbonyl]-3-aza-bicyclo[3.1.0]hex-2-ylméthyl}-amide de l'acide 2,5-diméthyl-2*H*-pyrazole-3-carboxylique ;
{(1R*,2S*,5S*)-3-[5-(3-chloro-phényl)-2-méthyl-thiazole-4-carbonyl]-3-aza-bicyclo[3.1.0]hex-2-ylméthyl}-amide de l'acide 2-éthyl-5-méthyl-2*H*-pyrazole-3-carboxylique ;
{(1R*,2S*,5S*)-3-[5-(3-chloro-phényl)-2-méthyl-thiazole-4-carbonyl]-3-aza-bicyclo[3.1.0]hex-2-ylméthyl}-amide de l'acide 2,3-dihydro-thiéno[3,4-b][1,4]dioxine-5-carboxylique ;
3-bromo-*N-*{(1R*,2S*,5S*)-3-[5-(3-chloro-phényl)-2-méthyl-thiazole-4-carbonyl]-3-aza-bicyclo[3.1.0]hex-2-ylméthyl}-benzamide ;
*N*-{(1R*,2S*,5S*)-3-[5-(3-chloro-phényl)-2-méthyl-thiazole-4-carbonyl]-3-aza-bicyclo[3.1.0]hex-2-ylméthyl}-3-trifluorométhyl-benzamide ;
*N*-{(1R*,2S*,5S*)-3-[5-(3-chloro-phényl)-2-méthyl-thiazole-4-carbonyl]-3-aza-bicyclo[3.1.0]hex-2-ylméthyl}-3-méthoxy-benzamide ;
{(1R*,2S*,5S*)-3-[5-(3-fluoro-phényl)-2-méthyl-thiazole-4-carbonyl]-3-aza-bicyclo[3.1.0]hex-2-ylméthyl}-amide de l'acide 3-méthyl-imidazo[2,1-b]thiazole-5-carboxylique ;
{(1R*,2S*,5S*)-3-[5-(3-fluoro-phényl)-2-méthyl-thiazole-4-carbonyl]-3-aza-bicyclo[3.1.0]hex-2-ylméthyl}-amide de l'acide 2-méthyl-imidazo[2,1-b]thiazole-5-carboxylique ;
{(1R*,2S*,5S*)-3-[5-(3-fluoro-phényl)-2-méthyl-thiazole-4-carbonyl]-3-aza-bicyclo[3.1.0]hex-2-ylméthyl}-amide de l'acide imidazo[2,1-b]thiazole-5-carboxylique ;
{(1R*,2S*,5S*)-3-[5-(3-fluoro-phényl)-2-méthyl-thiazole-4-carbonyl]-3-aza-bicyclo[3.1.0]hex-2-ylméthyl}-amide de l'acide 1-méthyl-1*H*-indole-3-carboxylique ;
{(1R*,2S*,5S*)-3-[5-(3-fluoro-phényl)-2-méthyl-thiazole-4-carbonyl]-3-aza-bicyclo[3.1.0]hex-2-ylméthyl}-amide de l'acide 3-méthyl-imidazo[2,1-b]thiazole-2-carboxylique ;
{(1R*,2S*,5S*)-3-[5-(3-fluoro-phényl)-2-méthyl-thiazole-4-carbonyl]-3-aza-bicyclo[3.1.0]hex-2-ylméthyl}-amide de l'acide 1-éthyl-3-méthyl-1*H*-pyrazole-4-carboxylique ;
{(1R*,2S*,5S*)-3-[5-(3-fluoro-phényl)-2-méthyl-thiazole-4-carbonyl]-3-aza-bicyclo[3.1.0]hex-2-ylméthyl}-amide de l'acide 5-*tert*-butyl-2-méthyl-2*H*-pyrazole-3-carboxylique ;
{(1R*,2S*,5S*)-3-[5-(3-fluoro-phényl)-2-méthyl-thiazole-4-carbonyl]-3-aza-bicyclo[3.1.0]hex-2-ylméthyl}-amide de l'acide isoquinoline-1-carboxylique ;
{(1R*,2S*,5S*)-3-[5-(3-fluoro-phényl)-2-méthyl-thiazole-4-carbonyl]-3-aza-bicyclo[3.1.0]hex-2-ylméthyl}-amide de l'acide 1*H*-indazole-3-carboxylique ;
{(1R*,2S*,5S*)-3-[5-(3-fluoro-phényl)-2-méthyl-thiazole-4-carbonyl]-3-aza-bicyclo[3.1.0]hex-2-ylméthyl}-amide de l'acide 4-méthoxy-quinoline-2-carboxylique ;
{(1R*,2S*,5S*)-3-[5-(3-fluoro-phényl)-2-méthyl-thiazole-4-carbonyl]-3-aza-bicyclo[3.1.0]hex-2-ylméthyl}-amide de l'acide quinoline-2-carboxylique ;
{(1R*,2S*,5S*)-3-[5-(3-fluoro-phényl)-2-méthyl-thiazole-4-carbonyl]-3-aza-bicyclo[3.1.0]hex-2-ylméthyl}-amide de l'acide 1*H*-indole-3-carboxylique ;
{(1R*,2S*,5S*)-3-[5-(3-fluoro-phényl)-2-méthyl-thiazole-4-carbonyl]-3-aza-bicyclo[3.1.0]hex-2-ylméthyl}-amide de l'acide 6-fluoro-4*H*-benzo[1,3]dioxine-8-carboxylique ;
{(1R*,2S*,5S*)-3-[5-(3-fluoro-phényl)-2-méthyl-thiazole-4-carbonyl]-3-aza-bicyclo[3.1.0]hex-2-ylméthyl}-amide de l'acide benzo[d]isoxazole-3-carboxylique ;
{(1R*,2S*,5S*)-3-[5-(3-fluoro-phényl)-2-méthyl-thiazole-4-carbonyl]-3-aza-bicyclo[3.1.0]hex-2-ylméthyl}-amide de l'acide benzo[1,3]dioxole-4-carboxylique ;
{(1R*,2S*,5S*)-3-[5-(3-fluoro-phényl)-2-méthyl-thiazole-4-carbonyl]-3-aza-bicyclo[3.1.0]hex-2-ylméthyl}-amide de l'acide 2-éthyl-5-méthyl-2*H*-pyrazole-3-carboxylique ;
{(1R*,2S*,5S*)-3-[5-(3-fluoro-phényl)-2-méthyl-thiazole-4-carbonyl]-3-aza-bicyclo[3.1.0]hex-2-ylméthyl}-amide de l'acide imidazo[1,2-a]pyridine-3-carboxylique ;
{(1R*,2S*,5S*)-3-[5-(3-fluoro-phényl)-2-méthyl-thiazole-4-carbonyl]-3-aza-bicyclo[3.1.0]hex-2-ylméthyl}-amide de l'acide 1-méthyl-5-trifluorométhyl-1*H* pyrazole-4-carboxylique ;
{(1R*,2S*,5S*)-3-[5-(3-fluoro-phényl)-2-méthyl-thiazole-4-carbonyl]-3-aza-bicyclo[3.1.0]hex-2-ylméthyl}-amide de l'acide 1,5-diméthyl-1*H-*pyrazole-3-carboxylique ;
{(1R*,2S*,5S*)-3-[5-(3-fluoro-phényl)-2-méthyl-thiazole-4-carbonyl]-3-aza-bicyclo[3.1.0]hex-2-ylméthyl}-amide de l'acide 2,5-diméthyl-2*H*-pyrazole-3-carboxylique ;
{(1R*,2S*,5S*)-3-[5-(3-fluoro-phényl)-2-méthyl-thiazole-4-carbonyl]-3-aza-bicyclo[3.1.0]hex-2-ylméthyl}-amide de l'acide 2,5-diméthyl-oxazole-4-carboxylique ;
{(1R*,2S*,5S*)-3-[5-(3-fluoro-phényl)-2-méthyl-thiazole-4-carbonyl]-3-aza-bicyclo[3.1.0]hex-2-ylméthyl}-amide de l'acide 2,3-dihydro-benzo[1,4]dioxine-5-carboxylique ;
{(1R*,2S*,5S*)-3-[5-(3-fluoro-phényl)-2-méthyl-thiazole-4-carbonyl]-3-aza-bicyclo[3.1.0]hex-2-ylméthyl}-amide de l'acide 2,3-dihydro-benzofuran-4-carboxylique ;
{(1R*,2S*,5S*)-3-[5-(3-fluoro-phényl)-2-méthyl-thiazole-4-carbonyl]-3-aza-bicyclo[3.1.0]hex-2-ylméthyl}-amide de l'acide 1,3-diméthyl-1*H*-pyrazole-4-carboxylique ;
{(1R*,2S*,5S*)-3-[5-(3-fluoro-phényl)-2-méthyl-thiazole-4-carbonyl]-3-aza-bicyclo[3.1.0]hex-2-ylméthyl}-amide de l'acide 7-fluoro-1*H*-indole-2-carboxylique ;
{(1R*,2S*,5S*)-3-[5-(3-fluoro-phényl)-2-méthyl-thiazole-4-carbonyl]-3-aza-bicyclo[3.1.0]hex-2-ylméthyl}-amide de l'acide 2-trifluorométhyl-1*H-*benzoimidazole-5-carboxylique ;
3-bromo-*N*-{(1R*,2S*,5S*)-3-[5-(3-fluoro-phényl)-2-méthyl-thiazole-4-carbonyl]-3-aza-bicyclo[3.1.0]hex-2-ylméthyl}-benzamide ;
*N*-{(1R*,2S*,5S*)-3-[5-(3-fluoro-phényl)-2-méthyl-thiazole-4-carbonyl]-3-aza-bicyclo[3.1.0]hex-2-ylméthyl}-3-trifluorométhyl-benzamide ;
[(1R*,2S*,5S*)-3-(2-amino-5-*m*-tolyl-thiazole-4-carbonyl)-3-aza-bicyclo [3.1.0]hex-2-ylméthyl]-amide de l'acide benzo[d]isoxazole-3-carboxylique ;
[(1R*,2S*,5S*)-3-(2-amino-5-*m*-tolyl-thiazole-4-carbonyl)-3-aza-bicyclo [3.1.0]hex-2-ylméthyl]-amide de l'acide 2,3-dihydro-benzo[1,4]dioxine-5-carboxylique ;
[(1R*,2S*,5S*)-3-(2-amino-5-*m*-tolyl-thiazole-4-carbonyl)-3-aza-bicyclo[3.1.0]hex-2-ylméthyl]-amide de l'acide 6-fluoro-4*H*-benzo[1,3]dioxine-8-carboxylique ;
[(1R*,2S*,5S*)-3-(2-amino-5-*m*-tolyl-thiazole-4-carbonyl)-3-aza-bicyclo[3.1.0]hex-2-ylméthyl]-amide de l'acide 2,3-dihydro-benzofuran-4-carboxylique ;
[(1R*,2S*,5S*)-3-(2-amino-5-*m*-tolyl-thiazole-4-carbonyl)-3-aza-bicyclo[3.1.0]hex-2-ylméthyl]-amide de l'acide 2,2-diméthyl-2,3-dihydro-benzofuran-7-carboxylique ;
[(1R*,2S*,5S*)-3-(2-amino-5-*m*-tolyl-thiazole-4-carbonyl)-3-aza-bicyclo[3.1.0]hex-2-ylméthyl]-amide de l'acide isoquinoline-1-carboxylique ;
[(1R*,2S*,5S*)-3-(2-amino-5-*m*-tolyl-thiazole-4-carbonyl)-3-aza-bicyclo[3.1.0]hex-2-ylméthyl]-amide de l'acide quinoline-8-carboxylique ;
[(1R*,2S*,5S*)-3-(2-amino-5-*m*-tolyl-thiazole-4-carbonyl)-3-aza-bicyclo[3.1.0]hex-2-ylméthyl]-amide de l'acide imidazo[2,1-b]thiazole-5-carboxylique ;
[(1R*,2S*,5S*)-3-(2-amino-5-*m*-tolyl-thiazole-4-carbonyl)-3-aza-bicyclo[3.1.0]hex-2-ylméthyl]-amide de l'acide 3-méthyl-imidazo[2,1-b]thiazole-2-carboxylique ;
[(1R*,2S*,5S*)-3-(2-amino-5-*m*-tolyl-thiazole-4-carbonyl)-3-aza-bicyclo[3.1.0]hex-2-ylméthyl]-amide de l'acide 1-méthyl-1*H*-indole-3-carboxylique ;
[(1R*,2S*,5S*)-3-(2-amino-5-*m*-tolyl-thiazole-4-carbonyl)-3-aza-bicyclo[3.1.0]hex-2-ylméthyl]-amide de l'acide 1*H*-indole-3-carboxylique ;
[(1R*,2S*,5S*)-3-(2-amino-5-*m*-tolyl-thiazole-4-carbonyl)-3-aza-bicyclo [3.1.0]hex-2-ylméthyl]-amide de l'acide 1*H*-indazole-3-carboxylique;
[(1R*,2S*,5S*)-3-(2-amino-5-*m*-tolyl-thiazole-4-carbonyl)-3-aza-bicyclo[3.1.0]hex-2-ylméthyl]-amide de l'acide imidazo[1,2-a]pyridine-3-carboxylique ; [(1R*,2S*,5S*)-3-(2-amino-5-*m*-tolyl-thiazole-4-carbonyl)-3-aza-bicyclo[3.1.0]hex-2-ylméthyl]-amide de l'acide 2-éthyl-5-méthyl-2*H*-pyrazole-3-carboxylique ;
[(1R*,2S*,5S*)-3-(2-amino-5-*m*-tolyl-thiazole-4-carbonyl)-3-aza-bicyclo[3.1.0]hex-2-ylméthyl]-amide de l'acide 1-éthyl-3-méthyl-1*H*-pyrazole-4-carboxylique ;
[(1R*,2S*,5S*)-3-(2-amino-5-*m*-tolyl-thiazole-4-carbonyl)-3-aza-bicyclo[3.1.0]hex-2-ylméthyl]-amide de l'acide 2,3-dihydro-thiéno[3,4-b][1,4]dioxine-5-carboxylique ; *N*-[(1R*,2S*,5S*)-3-(2-amino-5-*m*-tolyl-thiazole-4-carbonyl)-3-aza-bicyclo[3.1.0]hex-2-ylméthyl]-3-bromo-benzamide ;
*N*-[(1R*,2S*,5S*)-3-(2-amino-5-*m*-tolyl-thiazole-4-carbonyl)-3-aza-bicyclo[3.1.0]hex-2-ylméthyl]-3-méthoxy-benzamide ;
{(1R*,2S*,5S*)-3-[2-amino-5-(3-chloro-phényl)-thiazole-4-carbonyl]-3-aza-bicyclo[3.1.0]hex-2-ylméthyl}-amide de l'acide benzo[d]isoxazole-3-carboxylique ;
{(1R*,2S*,5S*)-3-[2-amino-5-(3-chloro-phényl)-thiazole-4-carbonyl]-3-aza-bicyclo[3.1.0]hex-2-ylméthyl}-amide de l'acide 2,3-dihydro-benzo[1,4]dioxine-5-carboxylique ;
{(1R*,2S*,5S*)-3-[2-amino-5-(3-chloro-phényl)-thiazole-4-carbonyl]-3-aza-bicyclo[3.1.0]hex-2-ylméthyl}-amide de l'acide 6-fluoro-4*H*-benzo[1,3]dioxine-8-carboxylique ;
{(1R*,2S*,5S*)-3-[2-amino-5-(3-chloro-phényl)-thiazole-4-carbonyl]-3-aza-bicyclo[3.1.0]hex-2-ylméthyl}-amide de l'acide 2,3-dihydro-benzofuran-4-carboxylique ;
{(1R*,2S*,5S*)-3-[2-amino-5-(3-chloro-phényl)-thiazole-4-carbonyl]-3-aza-bicyclo[3.1.0]hex-2-ylméthyl}-amide de l'acide isoquinoline-1-carboxylique ;
{(1Rµ,2S*,5S*)-3-[2-amino-5-(3-chloro-phényl)-thiazole-4-carbonyl]-3-aza-bicyclo[3.1.0]hex-2-ylméthyl}-amide de l'acide quinoline-8-carboxylique ;
{(1R*,2S*,5S*)-3-[2-amino-5-(3-chloro-phényl)-thiazole-4-carbonyl]-3-aza-bicyclo[3.1.0]hex-2-ylméthyl}-amide de l'acide imidazo[2,1-b]thiazole-5-carboxylique ;
{(1R*,2S*,5S*)-3-[2-amino-5-(3-chloro-phényl)-thiazole-4-carbonyl]-3-aza-bicyclo[3.1.0]hex-2-ylméthyl}-amide de l'acide 1-méthyl-1*H*-indole-3-carboxylique ;
{(1R*,2S*,5S*)-3-[2-amino-5-(3-chloro-phényl)-thiazole-4-carbonyl]-3-aza-bicyclo[3.1.0]hex-2-ylméthyl}-amide de l'acide 1*H*-indole-3-carboxylique ;
{(1R*,2S*,5S*)-3-[2-amino-5-(3-chloro-phényl)-thiazole-4-carbonyl]-3-aza-bicyclo[3.1.0]hex-2-ylméthyl}-amide de l'acide 1*H*-indazole-3-carboxylique ;
{(1R*,2S*,5S*)-3-[2-amino-5-(3-chloro-phényl)-thiazole-4-carbonyl]-3-aza-bicyclo[3.1.0]hex-2-ylméthyl}-amide de l'acide 2,5-diméthyl-2*H*-pyrazole-3-carboxylique;
{(1R*,2S*,5S*)-3-[2-amino-5-(3-chloro-phényl)-thiazole-4-carbonyl]-3-aza-bicyclo[3.1.0]hex-2-ylméthyl}-amide de l'acide 2-éthyl-5-méthyl-2*H*-pyrazole-3-carboxylique ;
{(1R*,2S*,5S*)-3-[2-amino-5-(3-chloro-phényl)-thiazole-4-carbonyl]-3-aza-bicyclo[3.1.0]hex-2-ylméthyl}-amide de l'acide 2,3-dihydro-thiéno[3,4-b][1,4]dioxine-5-carboxylique ;
*N*-{(1R*,2S*,5S*)-3-[2-amino-5-(3-chloro-phényl)-thiazole-4-carbonyl]-3-aza-bicyclo[3.1.0]hex-2-ylméthyl}-3-bromo-benzamide ;
*N*-{(1R*,2S*,5S*)-3-[2-amino-5-(3-chloro-phényl)-thiazole-4-carbonyl]-3-aza-bicyclo[3.1.0]hex-2-ylméthyl}-3-méthoxy-benzamide ;
[(1R*,2S*,5S*)-3-(3'-fluoro-5-méthyl-biphényl-2-carbonyl)-3-aza-bicyclo[3.1.0]hex-2-ylméthyl]-amide de l'acide 6-méthyl-imidazo[2,1-b]thiazole-5-carboxylique ;
[(1R*,2S*,5S*)-3-(3'-fluoro-5-méthyl-biphényl-2-carbonyl)-3-aza-bicyclo [3.1.0]hex-2-ylméthyl]-amide de l'acide imidazo[1,2-a]pyridine-3-carboxylique ;
[(1R*,2S*,5S*)-3-(3'-fluoro-5-méthyl-biphényl-2-carbonyl)-3-aza-bicyclo[3.1.0]hex-2-ylméthyl]-amide de l'acide 2,3-dihydro-benzofuran-4-carboxylique ;
[(1R*,2S*,5S*)-3-(3'-fluoro-5-méthyl-biphényl-2-carbonyl)-3-aza-bicyclo[3.1.0]hex-2-ylméthyl]-amide de l'acide 2,3-dihydro-benzofuran-7-carboxylique ;
{(1R*,2S*,5S*)-3-[5-(3-cyano-phényl)-2-méthyl-thiazole-4-carbonyl]-3-aza-bicyclo[3.1.0]hex-2-ylméthyl}-amide de l'acide 6-méthyl-imidazo[2,1-b]thiazole-5-carboxylique ;
{(1R*,2S*,5S*)-3-[5-(3-cyano-phényl)-2-méthyl-thiazole-4-carbonyl]-3-aza-bicyclo[3.1.0]hex-2-ylméthyl}-amide de l'acide benzofuran-4-carboxylique ;
{(1R*,2S*,5S*)-3-[5-(3-cyano-phényl)-2-méthyl-thiazole-4-carbonyl]-3-aza-bicyclo[3.1.0]hex-2-ylméthyl}-amide de l'acide 2,3-dihydro-benzo[1,4]dioxine-5-carboxylique ;
[(1R*,2S*,5S*)-3-(5-méthyl-biphényl-2-carbonyl)-3-aza-bicyclo[3.1.0]hex-2-ylméthyl]-amide de l'acide 2,3-dihydro-benzo[1,4]dioxine-5-carboxylique ;
[(1R*,2S*,5S*)-3-(2'-fluoro-5-méthyl-biphényl-2-carbonyl)-3-aza-bicyclo[3.1.0]hex-2-ylméthyl]-amide de l'acide 2,3-dihydro-benzo[1,4]dioxine-5-carboxylique ;
[(1R*,2S*,5S*)-3-(5,3'-diméthyl-biphényl-2-carbonyl)-3-aza-bicyclo[3.1.0]hex-2-ylméthyl]-amide de l'acide 2,3-dihydro-benzo[1,4]dioxine-5-carboxylique ;
[(1R*,2S*,5S*)-3-(3'-chloro-5-méthyl-biphényl-2-carbonyl)-3-aza-bicyclo[3.1.0]hex-2-ylméthyl]-amide de l'acide 2,3-dihydro-benzo[1,4]dioxine-5-carboxylique ;
[(1R*,2S*,5S*)-3-(3'-méthoxy-5-méthyl-biphényl-2-carbonyl)-3-aza-bicyclo[3.1.0]hex-2-ylméthyl]-amide de l'acide 2,3-dihydro-benzo[1,4]dioxine-5-carboxylique ;
[(1R*,2S*,5S*)-3-(5-méthyl-3'-trifluorométhyl-biphényl-2-carbonyl)-3-aza-bicyclo[3.1.0]hex-2-ylméthyl]-amide de l'acide 2,3-dihydro-benzo[1,4]dioxine-5-carboxylique ;
[(1R*,2S**,5S*)-3-(3'-fluoro-5-méthyl-biphényl-2-carbonyl)-3-aza-bicyclo[3.1.0]hex-2-ylméthyl]-amide de l'acide 2,3-dihydro-benzo[1,4]dioxine-5-carboxylique ;
[(1R*,2S*,5S*)-3-(5,4'-diméthyl-biphényl-2-carbonyl)-3-aza-bicyclo[3.1.0]hex-2-ylméthyl]-amide de l'acide 2,3-dihydro-benzo[1,4]dioxine-5-carboxylique ;
[(1R*,2S*,5S*)-3-(4'-fluoro-5,3'-diméthyl-biphényl-2-carbonyl)-3-aza-bicyclo[3.1.0]hex-2-ylméthyl]-amide de l'acide 2,3-dihydro-benzo[1,4]dioxine-5-carboxylique ;
{(1R*,2S*,5S*)-3-[4-méthyl-2-(4-méthyl-thiophén-2-yl)-benzoyl]-3-aza-bicyclo[3.1.0]hex-2-ylméthyl}-amide de l'acide 2,3-dihydro-benzo[1,4]dioxine-5-carboxylique ;
[(1R*,2S*,5S*)-3-(4'-fluoro-4,3'-diméthyl-biphényl-2-carbonyl)-3-aza-bicyclo[3.1.0]hex-2-ylméthyl]-amide de l'acide 2,3-dihydro-benzo[1,4]dioxine-5-carboxylique ;
[(1R*,2S*,5S*)-3-(6-méthyl-biphényl-2-carbonyl)-3-aza-bicyclo[3.1.0]hex-2-ylméthyl]-amide de l'acide 2,3-dihydro-benzo[1,4]dioxine-5-carboxylique ;
[(1R*,2S*,5S*)-3-(3'-fluoro-6-méthyl-biphényl-2-carbonyl)-3-aza-bicyclo[3.1.0]hex-2-ylméthyl]-amide de l'acide 2,3-dihydro-benzo[1,4]dioxine-5-carboxylique ;
[(1R*,2S*,5S*)-3-(2-cyclopropyléthynyl-4-méthyl-benzoyl)-3-aza-bicyclo[3.1.0]hex-2-ylméthyl]-amide de l'acide 2,3-dihydro-benzo[1,4]dioxine-5-carboxylique ;
[(1R,2S,5S)-3-(2-méthyl-5-*m*-tolyl-thiazole-4-carbonyl)-3-aza-bicyclo[3.1.0]hex-2-ylméthyl]-amide de l'acide benzofuran-4-carboxylique ;
{(1R,2S,5S)-3-[5-(3-fluoro-phényl)-2-méthyl-thiazole-4-carbonyl]-3-aza-bicyclo[3.1.0]hex-2-ylméthyl}-amide de l'acide benzofuran-4-carboxylique ;
[(1R,2S,5S)-3-(2-méthyl-5-*m*-tolyl-thiazole-4-carbonyl)-3-aza-bicyclo [3.1.0]hex-2-ylméthyl]-amide de l'acide 2,3-dihydro-benzo[1,4]dioxine-5-carboxylique ;
[(1R*,2S*,5S*)-3-(2-bromo-5-*m*-tolyl-thiazole-4-carbonyl)-3-aza-bicyclo[3.1.0]hex-2-ylméthyl]-amide de l'acide 6-méthyl-imidazo[2,1-b]thiazole-5-carboxylique ;
[(1R*,2S*,5S*)-3-(5-*m*-tolyl-2-triméthylsilanyléthynyl-thiazole-4-carbonyl)-3-aza-bicyclo[3.1.0]hex-2-ylméthyl]-amide de l'acide 6-méthyl-imidazo[2,1-b]thiazole-5-carboxylique ;
{(1R*,2S*,5S*)-3-[2-(3-hydroxy-prop-1-ynyl)-5-*m*-tolyl-thiazole-4-carbonyl]-3-aza-bicyclo[3.1.0]hex-2-ylméthyl}-amide de l'acide 6-méthyl-imidazo[2,1-b]thiazole-5-carboxylique ;
[(1R*,2S*,5S*)-3-(2-éthyl-5-*m*-tolyl-thiazole-4-carbonyl)-3-aza-bicyclo[3.1.0]hex-2-ylméthyl]-amide de l'acide 6-méthyl-imidazo[2,1-b]thiazole-5-carboxylique ;
{(1R*,2S*,5S*)-3-[2-(3-hydroxy-propyl)-5-*m*-tolyl-thiazole-4-carbonyl]-3-aza-bicyclo[3.1.0]hex-2-ylméthyl}-amide de l'acide 6-méthyl-imidazo[2,1-b]thiazole-5-carboxylique ;
[(1R*,2S*,5S*)-3-(5-*m*-tolyl-thiazole-4-carbonyl)-3-aza-bicyclo[3.1.0]hex-2-ylméthyl]-amide de l'acide 6-méthyl-imidazo[2,1-b]thiazole-5-carboxylique ; et
[(1R*,2S*,5S*)-3-(2-méthoxy-5-*m*-tolyl-thiazole-4-carbonyl)-3-aza-bicyclo[3.1.0]hex-2-ylméthyl]-amide de l'acide 6-méthyl-imidazo[2,1-b]thiazole-5-carboxylique ;
ou sel pharmaceutiquement acceptable d'un tel composé.

20. Composé selon l'une quelconque des revendications 1 à 19 en vue d'une utilisation comme médicament.

21. Utilisation d'un composé selon l'une quelconque des revendications 1 à 19 dans la préparation d'un médicament indiqué dans la prévention ou le traitement de maladies sélectionnées dans le groupe consistant en les suivantes : dérèglements de l'humeurs et troubles dysthymiques, psychotiques et anxieux ; diabète et troubles de l'appétit, du goût, du comportement alimentaire ou des apports de liquides ; maladies hypothalamiques ; perturbations des rythmes biologiques et circadiens ; tous les types de troubles du sommeil ; perturbations du sommeil associées à des maladies telles que des affections neurologiques, y compris la douleur neuropathique et le syndrome des jambes sans repos ; insomnies liées à des troubles psychiatriques ; apnée du sommeil ;
narcolepsie ; insomnies idiopathiques ; parasomnies ; syndromes liés à un stress ; adénome prostatique ; tous les types d'usages ou d'abus de substances psychoactives ; toutes les formes de démences et de dysfonctions cognitives dans la population saine et chez des patients atteints de troubles psychiatriques et neurologiques ; et autres maladies liées à des dysfonctionnements généraux du système des orexines.

22. Utilisation selon la revendication 21, où lesdits troubles du sommeil comprennent tous les types d'insomnies, la narcolepsie et d'autres affections associées à une torpeur excessive, les dystonies nocturnes, le syndrome des jambes sans repos, les apnées du sommeil, le syndrome du décalage horaire, la fatigue et les divers autres malaises attribués au travail en équipes, le syndrome de retard ou d'avance de phase du sommeil ou les insomnies liées à des troubles psychiatriques.

23. Utilisation selon la revendication 21, où lesdites dysfonctions cognitives comprennent tous les types de déficits d'attention, d'apprentissage et de fonctionnement de la mémoire qui se développent de manière transitoire ou chronique dans la population normale, saine, jeune, adulte ou vieillissante et qui sont également observés de manière transitoire ou chronique en association avec des maladies psychiatriques, neurologiques, cardio-vasculaires et immunitaires.

24. Utilisation selon la revendication 21, où lesdits troubles du comportement alimentaire comprennent un dysfonctionnement métabolique, un dérèglement du contrôle de l'appétit, l'obésité compulsive, la boulimie avec vomissements ou l'anorexie mentale.

25. Utilisation selon la revendication 21, où lesdits types d'usages ou d'abus de substances psychoactives comprennent toutes les formes d'addictions psychologiques ou physiques et les composants de tolérance et dépendance qui leur sont associés.
